(19) Europäisches Patentamt
European Patent Office
Office européen des brevets

(11) EP 2 153 847 A1

(12) EUROPEAN PATENT APPLICATION
published in accordance with Art. 153(4) EPC

(43) Date of publication:
**17.02.2010 Bulletin 2010/07**

(21) Application number: **08765479.4**

(22) Date of filing: **11.06.2008**

(51) Int Cl.:
***A61K 39/395*** *(2006.01)* ***A61K 31/7088*** *(2006.01)*
***A61K 38/00*** *(2006.01)* ***A61K 48/00*** *(2006.01)*
***A61P 19/00*** *(2006.01)* ***A61P 19/02*** *(2006.01)*
***A61P 19/10*** *(2006.01)* ***A61P 25/02*** *(2006.01)*
***A61P 29/00*** *(2006.01)* ***A61P 35/00*** *(2006.01)*
***A61P 43/00*** *(2006.01)* ***C12Q 1/68*** *(2006.01)*
***G01N 33/15*** *(2006.01)* ***G01N 33/50*** *(2006.01)*
***C07K 16/28*** *(2006.01)* ***C12Q 1/02*** *(2006.01)*

(86) International application number:
**PCT/JP2008/060700**

(87) International publication number:
**WO 2008/153072 (18.12.2008 Gazette 2008/51)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **12.06.2007 JP 2007155792**

(71) Applicants:
- **Riken**
**Wakou-shi, Saitama 351-0198 (JP)**
- **Takeda Pharmaceutical Company Limited**
**Osaka 541-0045 (JP)**

(72) Inventors:
- **IKEGAWA, Shiro**
**Yokohama-shi**
**Kanagawa 230-0045 (JP)**
- **MOTOTANI, Hideyuki**
**Osaka-shi**
**Osaka 532-8686 (JP)**

(74) Representative: **Keller, Günter et al**
**Lederer & Keller**
**Patentanwälte**
**Prinzregentenstrasse 16**
**80538 München (DE)**

# (54) GENE SENSITIVE TO BONE/JOINT DISEASE AND USE THEREOF

(57) The present invention provides a suppressing agent for inflammatory cytokine expression in joints and/or joint destruction (e.g., articular cartilage destruction) and/or pain in a bone/joint disease, comprising a substance that suppresses the expression or activity of a protein comprising the same or substantially the same amino acid sequence as the amino acid sequence shown by SEQ ID NO:2, a screening method for a substance that regulates inflammatory cytokine expression in joints and/or joint destruction (e.g., articular cartilage destruction) and/or pain in a bone/joint disease, comprising using the protein or a partial peptide thereof, and a diagnostic method for genetic susceptibility to a bone/joint disease, comprising detecting a polymorph in the base shown by base number 24007 in the partial base sequence of the human EDG2 gene shown by SEQ ID NO:3.

EP 2 153 847 A1

SEQUENCE LISTING

<110> RIKEN
Takeda Pharmaceutical Company Limited

<120> Bone and Joint Disease-Susceptibility Genes and Use Thereof

<130> 091257

<150> JP 2007-155792
<151> 2007-06-12

<160> 41

<170> PatentIn version 3.3

<210> 1
<211> 1092
<212> DNA
<213> Homo sapiens

<220>
<221> CDS
<222> (1)..(1092)

<400> 1
atg gct gcc atc tct act tcc atc cct gta att tca cag ccc cag ttc 48
Met Ala Ala Ile Ser Thr Ser Ile Pro Val Ile Ser Gln Pro Gln Phe
1 5 10 15

aca gcc atg aat gaa cca cag tgc ttc tac aac gag tcc att gcc ttc 96
Thr Ala Met Asn Glu Pro Gln Cys Phe Tyr Asn Glu Ser Ile Ala Phe
20 25 30

ttt tat aac cga agt gga aag cat ctt gcc aca gaa tgg aac aca gtc 144
Phe Tyr Asn Arg Ser Gly Lys His Leu Ala Thr Glu Trp Asn Thr Val
35 40 45

agc aag ctg gtg atg gga ctt gga atc act gtt tgt atc ttc atc atg 192
Ser Lys Leu Val Met Gly Leu Gly Ile Thr Val Cys Ile Phe Ile Met
50 55 60

ttg gcc aac cta ttg gtc atg gtg gca atc tat gtc aac cgc cgc ttc 240
Leu Ala Asn Leu Leu Val Met Val Ala Ile Tyr Val Asn Arg Arg Phe
65 70 75 80

cat ttt cct att tat tac cta atg gct aat ctg gct gct gca gac ttc 288
His Phe Pro Ile Tyr Tyr Leu Met Ala Asn Leu Ala Ala Ala Asp Phe
85 90 95

ttt gct ggg ttg gcc tac ttc tat ctc atg ttc aac aca gga ccc aat 336
Phe Ala Gly Leu Ala Tyr Phe Tyr Leu Met Phe Asn Thr Gly Pro Asn
100 105 110

act cgg aga ctg act gtt agc aca tgg ctc ctt cgt cag ggc ctc att 384
Thr Arg Arg Leu Thr Val Ser Thr Trp Leu Leu Arg Gln Gly Leu Ile
115 120 125

gac acc agc ctg acg gca tct gtg gcc aac tta ctg gct att gca atc 432
Asp Thr Ser Leu Thr Ala Ser Val Ala Asn Leu Leu Ala Ile Ala Ile
130 135 140

gag agg cac att acg gtt ttc cgc atg cag ctc cac aca cgg atg agc 480
Glu Arg His Ile Thr Val Phe Arg Met Gln Leu His Thr Arg Met Ser
145 150 155 160

aac cgg cgg gta gtg gtg gtc att gtg gtc atc tgg act atg gcc atc 528
Asn Arg Arg Val Val Val Val Ile Val Val Ile Trp Thr Met Ala Ile
165 170 175

gtt atg ggt gct ata ccc agt gtg ggc tgg aac tgt atc tgt gat att 576
Val Met Gly Ala Ile Pro Ser Val Gly Trp Asn Cys Ile Cys Asp Ile
180 185 190

```
gaa aat tgt tcc aac atg gca ccc ctc tac agt gac tct tac tta gtc      624
Glu Asn Cys Ser Asn Met Ala Pro Leu Tyr Ser Asp Ser Tyr Leu Val
        195                 200                 205

ttc tgg gcc att ttc aac ttg gtg acc ttt gtg gta atg gtg gtt ctc      672
Phe Trp Ala Ile Phe Asn Leu Val Thr Phe Val Val Met Val Val Leu
    210                 215                 220

tat gct cac atc ttt ggc tat gtt cgc cag agg act atg aga atg tct      720
Tyr Ala His Ile Phe Gly Tyr Val Arg Gln Arg Thr Met Arg Met Ser
225                 230                 235                 240

cgg cat agt tct gga ccc cgg cgg aat cgg gat acc atg atg agt ctt      768
Arg His Ser Ser Gly Pro Arg Arg Asn Arg Asp Thr Met Met Ser Leu
                245                 250                 255

ctg aag act gtg gtc att gtg ctt ggg gcc ttt atc atc tgc tgg act      816
Leu Lys Thr Val Val Ile Val Leu Gly Ala Phe Ile Ile Cys Trp Thr
            260                 265                 270

cct gga ttg gtt ttg tta ctt cta gac gtg tgc tgt cca cag tgc gac      864
Pro Gly Leu Val Leu Leu Leu Leu Asp Val Cys Cys Pro Gln Cys Asp
        275                 280                 285

gtg ctg gcc tat gag aaa ttc ttc ctt ctc ctt gct gaa ttc aac tct      912
Val Leu Ala Tyr Glu Lys Phe Phe Leu Leu Leu Ala Glu Phe Asn Ser
    290                 295                 300

gcc atg aac ccc atc att tac tcc tac cgc gac aaa gaa atg agc gcc      960
Ala Met Asn Pro Ile Ile Tyr Ser Tyr Arg Asp Lys Glu Met Ser Ala
305                 310                 315                 320

acc ttt agg cag atc ctc tgc tgc cag cgc agt gag aac ccc acc ggc     1008
Thr Phe Arg Gln Ile Leu Cys Cys Gln Arg Ser Glu Asn Pro Thr Gly
                325                 330                 335

ccc aca gaa ggc tca gac cgc tcg gct tcc tcc ctc aac cac acc atc     1056
Pro Thr Glu Gly Ser Asp Arg Ser Ala Ser Ser Leu Asn His Thr Ile
            340                 345                 350

ttg gct gga gtt cac agc aat gac cac tct gtg gtt                     1092
Leu Ala Gly Val His Ser Asn Asp His Ser Val Val
        355                 360
```

<210> 2
<211> 364
<212> PRT
<213> Homo sapiens

<400> 2

```
Met Ala Ala Ile Ser Thr Ser Ile Pro Val Ile Ser Gln Pro Gln Phe
1               5                   10                  15

Thr Ala Met Asn Glu Pro Gln Cys Phe Tyr Asn Glu Ser Ile Ala Phe
            20                  25                  30

Phe Tyr Asn Arg Ser Gly Lys His Leu Ala Thr Glu Trp Asn Thr Val
        35                  40                  45

Ser Lys Leu Val Met Gly Leu Gly Ile Thr Val Cys Ile Phe Ile Met
    50                  55                  60

Leu Ala Asn Leu Leu Val Met Val Ala Ile Tyr Val Asn Arg Arg Phe
65                  70                  75                  80

His Phe Pro Ile Tyr Tyr Leu Met Ala Asn Leu Ala Ala Ala Asp Phe
```

85 90 95

Phe Ala Gly Leu Ala Tyr Phe Tyr Leu Met Phe Asn Thr Gly Pro Asn
100 105 110

Thr Arg Arg Leu Thr Val Ser Thr Trp Leu Leu Arg Gln Gly Leu Ile
115 120 125

Asp Thr Ser Leu Thr Ala Ser Val Ala Asn Leu Leu Ala Ile Ala Ile
130 135 140

Glu Arg His Ile Thr Val Phe Arg Met Gln Leu His Thr Arg Met Ser
145 150 155 160

Asn Arg Arg Val Val Val Val Ile Val Val Ile Trp Thr Met Ala Ile
165 170 175

Val Met Gly Ala Ile Pro Ser Val Gly Trp Asn Cys Ile Cys Asp Ile
180 185 190

Glu Asn Cys Ser Asn Met Ala Pro Leu Tyr Ser Asp Ser Tyr Leu Val
195 200 205

Phe Trp Ala Ile Phe Asn Leu Val Thr Phe Val Val Met Val Val Leu
210 215 220

Tyr Ala His Ile Phe Gly Tyr Val Arg Gln Arg Thr Met Arg Met Ser
225 230 235 240

Arg His Ser Ser Gly Pro Arg Arg Asn Arg Asp Thr Met Met Ser Leu
245 250 255

Leu Lys Thr Val Val Ile Val Leu Gly Ala Phe Ile Ile Cys Trp Thr
260 265 270

Pro Gly Leu Val Leu Leu Leu Leu Asp Val Cys Cys Pro Gln Cys Asp
275 280 285

Val Leu Ala Tyr Glu Lys Phe Phe Leu Leu Leu Ala Glu Phe Asn Ser
290 295 300

Ala Met Asn Pro Ile Ile Tyr Ser Tyr Arg Asp Lys Glu Met Ser Ala
305 310 315 320

Thr Phe Arg Gln Ile Leu Cys Cys Gln Arg Ser Glu Asn Pro Thr Gly
325 330 335

Pro Thr Glu Gly Ser Asp Arg Ser Ala Ser Ser Leu Asn His Thr Ile
340 345 350

Leu Ala Gly Val His Ser Asn Asp His Ser Val Val
355 360

<210> 3
<211> 35879
<212> DNA

<213> Homo sapiens

<400> 3

```
atgaaaacat gagtcttcga aaagacaacc tattgtctgc attctggagg gaactctact      60
tagactatca agatcttgga ataacctata cacatctcat cactgcagcc tctttgtttc     120
aacagcattc acgtgcactg gtctgccagt ctcctcagtg tccttatgtc ctccacggga     180
aggcatgtgc taatgtcagg tgcccctgta tggctcacac aaatgtggga agccatctat     240
ccagccactc tctcggccag tgctgtgtta cagctctcat acgttgttgg acatcacatt     300
gctcaagtgt agttgggggt tcccctagtc ccaagaaagc actccatgag ctccagggtt     360
ctcagtttcc ctgacacctt ttcttttctg ggtgttttcc tcttctctaa gaggaggaaa     420
gtgaggacac tggtgtctga tttcatcctt tcctttctct caaaactctg ctagaaaaag     480
aaaggatgga ctttcactct ttcctatgtg gcaggggctt tgttgagtgc tgtctacttt     540
atacttgtaa tgaaactcta tgatcaaact agtgattatg taagtgagaa ctccgataat     600
ttaggaaatc cccttcctgc cagtcaaagc catttgacag tcagaagctg aacattgact     660
cctttctttc tcttgtgctc ctgctgttaa cagtcctaat catccctgac tatcgcccaa     720
attagatgga tcctcaaaaa aggaaaacca cgttaaaata gttcaaaaaa taattttcat     780
attgcatatt ttccccagtt tataattttt aaaacacatt ccttgttttc atttataggc     840
attttaaatt gttgtttaat ttaaatctat cagtcttttt ctttaggctt tcttccagtg     900
gtgtcatgcc aagccatccc caaatcaaaa gattttttta aaaaatcaat catatagttt     960
tctaatgcct ttgtatgtaa tgtaggtatt tatttcatga atgaccactg cagtaccccg    1020
tcttagtaaa taatacttcc tttcttcctg ttgttcaggg caaaactttg gcatcgtctt    1080
tactctgtct tcctcttatg ttaacatatt caatccttca gcaaaccctc ttagctttgc    1140
ctttgaaata tattcagaat ccaaccatct cttgtctggt caattgcggt agcctcctaa    1200
atggtctcct tttccctgcc ccctggtagt ctcttctcaa catggcaggc agattgttct    1260
tttaatacat gttatttttc tgcctaaaac ctaccagatt accatctcat ttagaggaaa    1320
atccaaagtc cttcccacgg gctgcgaggc catctgtggt ctctaccaca acacacacac    1380
caactcccac cacatccaac cttctctgac ttcctctccc agccttcttt cccttactcc    1440
tttctcttca gctatgacct tgacatcatt gctgttcatt gaataaggga agcaaactgc    1500
catctcagga ctattgcact tgccttccct tctacctgga atgtgcactc ctgtcccttc    1560
ctgacctctt tatctgcttt cctcaggtct ctcttcaaag gccatcatta caaaagcttg    1620
tttctgatca ctttatctaa aataagcact cacaacactt attgctacct gacatattgc    1680
ttattagtaa attttctgtc tcctcccatt aaaacagaag cttgagggag agctgtgttt    1740
tgtatactgt catatcccca aggtctaaaa tgggactcta ctacctttat taagatgtct    1800
ttttaaaaat ataccacatt ttggtatgat ttatgaataa aattacaagt cacttttaaa    1860
cacctaaaat taattttatg tacttatttt taaatttcat aataatggag tctactctat    1920
aatgttgcct ttaaaacatt ttaatctata tttaacgtca gattggtttg cactttgttt    1980
tactgtatta aaatctcctt gtatttctaa taatttttgc tttagataat ttaaggcaat    2040
attgcttgaa gctcaaaggt tcactcctat acctttttga taaattatgt tgtgtattcc    2100
tatgaactgt ccaggtttac ctcatttaat tttttccttg aatattactt aatctgatct    2160
```

taataatgcc acccatgagt ctttaaatta gcattggctt aatactttcc catcctgttt 2220

attctcaaac ttatgttttc ttttcattct aggcatgctt cttaaaaaca gcagactttt 2280

aaaattcact caacctttaa aaaaatgttc aaacttgttt ttattgctac catccatgtg 2340

tccttcaatt atttagaaag aaatctcaga catatcattt tacatttaaa tatttcagtt 2400

aatatctcaa aaagatttct tttaaaacat aactataata gtgttatcac acctaaaaat 2460

taataatagt ttctaatatc atgaagtact caattacaat tcgaatattc caattgtctg 2520

tctttttct tttacttttt ttgaggtagg gtcttgctct gttgcccagg ctggaatgca 2580

atgtcatgat ctgagttcac tgcaacctgc gtctcctgag ctcaagccaa cctcccactt 2640

cagtctcctg agtagctggg actacaggtg catgccacca tgcccagcta atttttgtat 2700

tttttataga gatggggttt catcatgttt cccagggtag tctcaaattc ctgagctcaa 2760

gcagtccacc agcctcggcc tcccaaagtg ctaagattac aggtgggagc catcatgtct 2820

ggctcatttt tcaaaatata atttatttgt atcataatcc aactaatgtc taagcattga 2880

aattggccaa tatgtccctt aaattctgtt taatctaaag gtccacctct atctctgatt 2940

ttgtttttct tactccaccc tttaaattcc tcacctaaaa ctgtcccaga gtgagttgtc 3000

aagagagagc ccttggactt aacaatgaat ggtgcatttc actggtagcc tctcatttat 3060

tttacatttt tcaggtttca tcaacatctt ggaccagagt caggtggatt agatacaaac 3120

atcatctttc cctgctatgt ctgctatgtc catggcttag tagtgatttc acaaggtagt 3180

atgtataatc agtgagaagc atgtgtagtc atttacatga actttctgaa aactcttacc 3240

tttctacttt ctattattat atactgaggt tgggggaaaa agtgttgtta atagctagga 3300

catattatgc catctcttat tacagcttgt aagtctgccc ccacttttta gcatctctct 3360

ttagaaagtg cacacttgga agctctttat tttcagctgt caaccttaat tacccacttt 3420

tgaaaaatga gaaaacttat actcagcacc atatcagttg ctgtaaagca gttgatctcc 3480

catatctttg ctttatatta ataacaatag ctaacatttg ttaagtattt tatgtcagac 3540

cttcagaatt ttgcataaat tacctcattt aatcctcatg gcaattcagt aacaggggca 3600

ctactagctc aatataatag gtgagaaaat tgacacctag aaaggttaac taaagcagta 3660

tgaggctaaa tagctaaaca ggagtggagc tatgatttga actcaagtct tctaactaaa 3720

tgttcatgat gccatcacaa tgctgccatg gtcatactct gttctagaag acgaacagtg 3780

tatacccgtg gctcaaactc attaatttga tttccagact tctgctactt aattggtttt 3840

gcttttcttt gttggcagtc atgtttttaa atctattaga attcattctt cttttccaat 3900

tactactttc taatacattc taatggaagc atgtttttta ttgtatttat gcattatcca 3960

atagatttta ttggaaatgc cacttcaaaa tttttagagg ccttatttac ggttcttcat 4020

taccattgtt tccactgagg ccattttttt ctgatcttaa aaaaatttcc ttttctcttc 4080

tagtgactga ttgttttctt tcatctggtg atttttcact gaccacttac ttatgaatga 4140

ggattagaat gatcagcacc agtgggcttc cctaacaatc tggttggatt gtgtttaata 4200

ttctttggtg gggcccttcc ctcctttccc ttcttccctt ctctgccttc ttctcccctc 4260

ctttcttccc ttctcttgcc ttcccttcat gggcttccct aacaatctgg taggattgtg 4320

tttaatattc tttggtgggg cctttccctc ctttctttcc ttcccttccc tcccttcttc 4380

tcccctcttt cttccctctc ttgccttctc ttcgtgggct tccttaacaa tctggttgga 4440
ctgtgtttaa tattctttgg tggggccctt ccctccattt cttccttccc ttcccttcct 4500
tcctctcccg ccctttcttc ccttctcttc ccttcccttc atgggcttcc ttaacaatct 4560
ggttggatta tgtttaatac tctttggtgg gactcttccc ttctttcatc cccttttat 4620
gagaaaggag agaaggctgt ggtgaaagat tcagaaaatg ggagtattag aataggtgtt 4680
aagctgctgt aaaaaaagaa cctcaaaata ccgggactta aataatatac cgatttattt 4740
ctctgtcaag taacagtcta gtagtgagta gtccaggttg gtggggcagc tctcctatct 4800
tcagttagtc atttgaggac attgtttctt ccatcttgtt gcttcttcaa tccctatggt 4860
gctattttaa ctggctgaaa ttagatcaag ctagaaggag aaagtacagt gatgaagggc 4920
aagcaatttc ctttcatgaa aatgatacag aagctgctgc agacatctct tccactcaca 4980
ttctttcaca tacctcttcc atttccattg atgagaacta atcacatgac cataactggt 5040
tgcaaagtag gctgggaagt ggaggctaaa actgaatatc catatgccta gctaaaattc 5100
cactactagg gaagaggaga agaaaagatt tctggtgacc actagcagtc tgccacacca 5160
agctgatagg taacagatct ctttaaaagt atgtaaagcc aggtcataat gtttctctaa 5220
gagcccaaag tcatctatgt ggctcacaag ccaaaaggtg agtctctgaa gtctggtgca 5280
ggcgcacaaa tggaatctct tttttaccac cataaaactt aattggtttt taaattgttg 5340
aaataatttt cttttccctt catccccaaa gattctgtgc aaaagtgcag aatacttttt 5400
ctgatgttct aaaaggtagc aatctaggct atttatttaa ataccctaaa tgaagaaagt 5460
ctattcaggg atatcctcaa atcccaactc ttgttctctc cagagccttc ttcaagtgct 5520
ccattgtttc ccttaacaaa ggaagatgga gaaattctcc ccaaaggggc cagggactct 5580
tggttctctt ccttctgttg gaactccttg gatagcaagg gaaaatttta aactctgaaa 5640
taaaaatggg aatgtattgg cttatgcaat gtaaaagttc acagaacaga caagagatac 5700
accttgatcc agggtttcaa aataggtcat caggatatgt ctcctaatgt tacttttctg 5760
agtgttggct tacccctttg gcaagttctc attcatggcg gtctccaaca tttcaagttt 5820
ttgtttcaca gtgccaattc tatcttattt tatcattatt ttttgagaca gggtctcact 5880
ctgttgccca ggctagaaaa ccgtggcatg atcatggctc actgcagctt caacctccca 5940
ggctcaagtg atcttcccac ttcagcctcc tgagtagctg ggactacagg cacatgccac 6000
cacacctggc tattttttt ttttttttt tgcctcatcc tcccaaagtg ctgggattac 6060
aggcatgagc cactgcacct gaactcaaca gccctaattc tagcaagaaa agagaatata 6120
ccactttctc atcaggtcaa gtgaaagtac ccaagatcat taacattggc tctgactgga 6180
tcactggtac atcttagaat ttttcaattc catgatcaca ttgaatgttc tgaatgaccc 6240
gtaccacatc atccacatca tagacaccca taaagagcaa aaggttaaca tcacacaaag 6300
tagtagaaag gaaaatcaag gttctgttag cagaagggag cttggatggt aggcaacaaa 6360
ataacaacaa atgtccacca ctcatccatt tgcctatgga gttaacgaat ctggctaatc 6420
ttaggatgac tcttaaatac ttgcctttga ataaggatgt tctctagagc cctttaaact 6480
actgagtttc cctgagtcgt cttcttgcag tgtcttattc caagtgtttg tggttatcat 6540
gtccccatgt tctttcactt tcaggtcagg catatgtctt tcccctagga cctactgatc 6600

ctgagtaata tcacaataat gaccatgttg taaaactcct tgtagtgttg attttatagg 6660
tcacttccat tttctgtaac ttcatgccac acttcgtttc tcctgcagat ggatattttt 6720
ttaaatttca tgtttcactt taagaaaatg ggggtacaca aagattcata gcaaggtagt 6780
ttgtaaagaa ttttaggggt aagaaacctt tatttaatga acagaataat ggatctctcc 6840
aagaattttt tttttttttg agacggagtc tcactctgtc gcccaggctg gaatgcagtg 6900
gcgcgatctc ggctcactgc aagctctgcc tcctgggttc atgccattct cctgcctcag 6960
cctcccgagt acctgggact acaggcacct gccaccacgc ctggctaatt ttttgtattt 7020
ttagtagaga tggggtttca ccgtgttagc caggatggtc tcgatctcct gacctcgtga 7080
tctgccctcc ttggcctccc aaagtgctgg gattacaggc gtgagtcacc acgcccagct 7140
ggctctccaa gattctttaa agactggacc tattgatagg tggttgtata ccgtggggtt 7200
ttctctaagg cttggaatag acaagccatt tgttacacta atgccaaggc tctgttcata 7260
ttgatgagga ataagtgggg agcagagaat tttgttttac cattgttctt ttgaataaga 7320
tagattagtt gggacttcca caatgtgctg tgtggcgagg tggctaagaa cacagactgt 7380
atcaacatcc tagttctgcc acttactgcc tacttgatct taggtaagtt acttgacttc 7440
agtattcctt attcttcaat aaaatggaaa taataacagt attcatgtca cagagttgtg 7500
atgctcatag gagttaatgt gtataaaatg tttagagtag tgtaagaacc tgaaagtaat 7560
ataagagcca ggtaaggaca tactaagaac tttgttagtg attattatat tatttttcat 7620
gagaattgct acataaatat tccatgaaca aagtgaaaga agtggctggg tgaagtgtaa 7680
tctcaatctc atgcgtgtaa tctcaacact ttgggaggct gaggcaggag gcttgcttga 7740
agccaggaat ttgcggccag gagttcgaga ccactctaga caacatagag agaatctgtt 7800
tctacaaaac aataacaata aaagaaaaga aaagaaataa aaagtttaaa gtgagagaaa 7860
tatgcagtag taagttcctt gaagaaacaa ccaaagcata aaacccagat tttcttttta 7920
tgaattctgc ctaaattatc cacttctgag taagaaggtg gaagtagcat actaacggaa 7980
aaacaggcta atgagcctac cctagtttca cattcaagag gtggaataat ttatgggaaa 8040
gctttcaaag cttccaaata ctgcaatcaa atcactatat ggagaaagct acaaaatcaa 8100
attccaaaca atgaagacat cagtttctta atggaacagt tccaaccatc tggcattcgt 8160
atgttgtttg taggggtggt tattgtacag atataatgac ccagttccac ctctaggcaa 8220
gaagctttca tgaaggaagt ttacgctggc cacttgtggc tggttaaaat gaccaaaaga 8280
gttttctttc cttttttttt ttttttttta aactgtgcta ttgttaatat ttcaaatttg 8340
gaccttttcc cagcaaaaca caaaaagaag aagcttcaaa tacattcata gagtgcaaga 8400
aagtgagcag aaggaggttt caatgtttca gaaagaaatt ttttcttcaa ggcaattttt 8460
tttaaaagaa gtgccttatt acataggctg gatccagtat taatgataat cagttatttt 8520
tattaaacag gtttaaattt tggatacttt ttttaaacta caccttaaaa acggactttg 8580
gataaaactg gaacacaaaa agcaaagaca ttttgttttc ttttggatgc actgccaatt 8640
ttgttagcat agtatctttt ccatttacta tggaacttga cctacagtct aaggttcaat 8700
tgttcattta aaattaatta attccacaaa tatttactgt ataactagaa agtgctagat 8760
actgaggtag gtatatttaa tggagtgcaa aaaagtggtt ttaaagaaat tggaacaggc 8820

aggttgtgaa aagggtaggt tggtacaaaa tgttaacagc atgagagatt tttttaaaaa 8880
agataaccag ataagatgaa aaagaaaatg gaagacaaaa gggagacatg caagaaaaat 8940
taaaatgcat tctaaataaa atctatattg gagtaattga agagaaaaat gttctggata 9000
atagcaatgt ggattttaca gtttataagc tctcctagga tgcagtgggg aaaggcaaag 9060
agataaaaat gacaagtgac tgactgaatg gagaacagag atctcaatat atggataagg 9120
ctgttcttga aggagactga aatgaacaaa agccacaatt aaatatatgg tagaggaaaa 9180
ctttcctgaa cctattttat gaaggtttat atacttattt ataatcctat tacacaaaga 9240
cctatttgtg tagatttgaa gggtttctta gttccaacat agctttttaa gagaaaaaaa 9300
catttaaaga tgtgctgaga ctaaaatata gatcattgct ggagtgcatt tgggctgcta 9360
tagtaaaata cactaaaatg ggtagctttt aaacaaaaga aatttattta aaacagttct 9420
ggaggttggg aagttcaaga tgaaggcacc agcagatttg gtgtctgatg agggcccatt 9480
tcctggttca cagatggtac cttcttgctg tgtcttcaca tgtgaaaggg gtcagggcat 9540
ctctttgagg cctcttttcc aaaggcacta atcccattcc taagcgcaga gacctcatga 9600
cttaattatc tttcaaaggc tataccttct gatatcatca ccctggtgat tagaattcaa 9660
catataaatt tgtgggtgac acaaatgttc agaccatagc aaccacccag caggccgtgt 9720
tgaaaaatgt gaagattagt ccaagcaact gagttgtgta ctaagttgag agcatttaaa 9780
aagtagtatt aagggctggg tgcggcagct catgtctgta attccagtgc tttgtgaggc 9840
ccaggaggaa gaatcgcttc aaggctggag tttaagacca gcttcagcaa tgtagtgaga 9900
ccccatctct acaaaaaatt aaaaagtaaa tttagccagg tgtggtgaca cgtgcctgta 9960
gtcctagcta ctcatgaggc tgagatggaa ggattgcttg agtctagttc gaggttacag 10020
taagctatga ttgtgccact gcactccagc ttgggtaaca gggtaagact ctgtcccagt 10080
aaataaataa taaagataat tattaaaaag gaagaaaaaa ataaactgag cgttcagcac 10140
cagaaaggaa agaggataac aacataaacc caaggaagtc agtagttaaa cccaaggatt 10200
tgactgtaag caaaataatt aatgagatac acaacagtaa aatgaacagc attgataaat 10260
acatcctgga tctgattttg caaattattt ttttaaaaag tatctacagt actagggtaa 10320
acaaatgggt aaaatacagt aagcaaatac cattaacaat ttcagttcag gcaatttgaa 10380
atctcaacaa aaagattatt acataaaaat tatttaaaga taaatagaat agtagctatg 10440
gaaaagactt gttgtagggt gagttctcag gaagtagatg ggggagattt aggagtgcaa 10500
acggttcact ggggagtaac atctgtgaaa ggaaaaggtt gagaagcagg atagggcagg 10560
ggatgccaac agatcatgat gcagacccta caaagtcttt gctatcccga cgtggatctt 10620
gggaaaaagg tggcccatta gagaagctac ttattaggcc cttgctcagt cattgggcat 10680
ggtacaccat gagaagagca taatctcagc tatcacctct ttgctcattc attggctgaa 10740
aggatgcccc gagaggaata tgaacccact tccaaagcta cacaggaccc tgaaggcgct 10800
aaataccatg ggctattagc taactacatt ctttgcagct ggtcagtgtg ttttcttgaa 10860
agggatctga atggcacatt tccatgtctt caatagtcca ccccttgctc cgtgtggagc 10920
tatttcccca aacttgttga ggtagcagca cctccaagtg tctgatgggc ctctttgcct 10980
gcaggaaaac ttagaagagg aagactagca ggatgaatgt caggcttcat cacaatagtt 11040

ggtctcagga ccaaaaatag tactcaccct cttattccct tattatacag gctaattctc 11100
taaaccctca gtgaacacct ctgccagtct tgctaatgta cctcatagtg tgtcccaaac 11160
cctcattcct cacagatctg tgtttttgta accgtactct tctttagttg tgatgtttgt 11220
ccattcatag ccatagttgg acaaggaagt accaagaggc accaagtggg tcctgtgagt 11280
ttcataaatc ttccttcctg cccccattta attacagcag ccctatctcc tcctgttaat 11340
tatcagggtg gattattcct gaaaggatgg tgattgcact tcttgtctgc tgatttctag 11400
gcgtaaggac cacagttttc ccagacaaca accatggctt gtagttcaat ggaaactttg 11460
caagagtgca gaacctctaa ccctgaagag cttaaagttg tgtgaagggg ggcacaaagt 11520
gccacaggga gtcactgaca gtgatgataa gtggggtcac ttctgcttcc atcttcttgg 11580
tccatggacc catgtatttt tcgtgttagt ggcaaagcac tgcataaaca tccctgatcc 11640
aatgcatata ctgcatcctg aataacagtt ccgtattctt gcaaggcact gtcccaagtt 11700
ggtgccttag ctgcgtcttc attgaaacat tccatacttt agtaggaaac ttctggatgg 11760
tgcagtatgt gatatgccca tatgcacctt cttcactgta aactaaatgc cctggttgaa 11820
tgttgcatgg tattccatgc ctatgaatca ggcattccat aagccctgga tagccatact 11880
ggctgaggcc ccaagggcag gaaaggaaat gacatataca gactagatgt ctattcctaa 11940
ggaaatgaac tactcatccg tccagaaaga agaggcccaa cctaacaatc ttgctaccaa 12000
gttgctagtt ggtctcatca aggaatccag gggctgaact ggtctctgtt ttagcagttt 12060
gaacgctcag aaataacact agctagatca gctttggtaa gtggaaattc catgctcttg 12120
ggtccgtgca gcctccctct ttgccatggt ggccacttca ttcatttgtt cattgtgtta 12180
gcactggtag cgctgctaac aaaggctgac tcagtgttcc tgcttgagaa gataagcagc 12240
aattctacct ctttcctctc ctttgcctgt tggaagaatt atggaagcgg gggccaggct 12300
cccgcctgta atcccagcac tgtaggagac tgaggcaggt ggatcacttg aggccaggag 12360
ttcgaggcta gcctggccaa catggtgaaa acctgtctct actaaaaaca caaaaattag 12420
ccagacatgg tggcacacac ctatagtccc agctactcag gaggctgagg caggagaatc 12480
acttgaagct gggaggcgga ggttgtagtg aacctagatt gtgctactgc actccagcct 12540
agccaacaaa gcgagacttc gtcctaaaaa aaaaaaaaaa aaaattacag acagggagga 12600
tcgtgttcat tcaggttcat cttgggattg attatggtac tcaatttaat acacttgcca 12660
aaacttgtcc ctaatggtca gatccatgct ctctcaatct aaatttctac aagtcagtgc 12720
agatctctct ctgggaagaa gtctagtaat ttctattgtt aatctttcag aaacctaaag 12780
tctcagaaaa aggttttaaa gataactttt catactcctt cctttctaga tattttctta 12840
gaaattggaa ataaattatc ctcatcagca aatcctagta acgttcaagt gctatcactt 12900
tctttcttct gatcatattg catcttccca aggggtttaa agtgtgctct gttactgttt 12960
caaactcatt acagtcaatt tgtattctgc aaattagcag tgattttgca aggtcacctc 13020
ttgtgtgcat agtaatcctc tccaggttgt aactgtctta taccaaagtg gttcttactc 13080
taggatttgt aatctttcca tggtgcagac atgaggtgcc caagggttcc tggcaatcat 13140
ggatgaggac tccacacttc gtgggtatta taggctgaaa catttccctc tctaaattcc 13200
tattttgaaa ttctaaaccc cagtacctta gaatgtgtct gtgtttggag ataagtcctt 13260

aaaacagtaa ttaaggtaaa tgaggtcata cggatgggtc ctaattcaat atgactcata 13320
tccttataaa aagaggagat taggacatag atatacacag atcaagggaa gatgaaggga 13380
agatacgagg agaagacaag ccaaggagaa tggtctgaga agaaacaacc ctgccaacac 13440
caggaacttg gactgccaga ctccagaact ttgagcaaat aaatttctgt tgcttaagcc 13500
agccagtctg caatatttca ttatactagc cttatcaaac taatacagtg ggtttattcc 13560
ttttttattt atttaaatta ttttaaaaaa tagagacgaa gctggtcttg aactcccggg 13620
ctcaagcaat cctcctgcct cggcctccca aagtgccagg attataggtg tgagccactg 13680
aacctggcca gtacagtaga tttgaatcac catcaataaa cttagatata caagagtatt 13740
tttctttatt tcagattaaa ttcctgctct ggtaccctta ttcttcagag ttagtgggaa 13800
aacaggagct cagctctttt ggagttttct ttcattttta gggaacagtt acacatacct 13860
acccaggtca gtcagcattc cagagttttc tgtagttgca acatgttgga gaaggtccag 13920
ctagttttgg tccttagagg ttaactttgc tacttcattt gcccaagtcc attgccacta 13980
tgccattcat gggacatgag catctgtgac taagctattc tagggcacat ctacctaaga 14040
gttcctatcc cttgaagtct tttattctcc agggtctttc tgtctcccca gcttcactgc 14100
tgggaagcag ggcagatgag ctgtctgctc tccaggatac acagacctct gtttatcact 14160
ctttcacttg tagaatatct gtttttagtc tggggaacta aggtctattc atgccacttt 14220
ttaaatccct ttctcctaat ctttctgtaa agatttttgg ctcttctgtt ctctgtcctg 14280
tcatgtactt tcctgagcaa taagcacaga gcatattact attcgacaat gagagaggga 14340
aaagtggaaa tacagggaga agacaatatg gaataagaat ctcaaaatag agtcctgtca 14400
attaattttt ctatcccact acagacagaa aaactataca cactgattat caaagtatat 14460
tttatggtta catggaatta ggatactatc tatgagaaac cagaagcttt atgcatagga 14520
acaaactact aacttgtttc ttataatttg tcttaggaca tgtttatcgt gtgtcttggt 14580
tttacctagg agtacaatca ctttctgctg ttaggtgtta tagtgataat atgtggttaa 14640
tcacaacagt agccccccaaa ggatctaatg gctgaacatt attccataat agtttgtttt 14700
agaaatatta ttgtatggat agaccccaat aatttcattt accttttttt tttttttttt 14760
ttgagacata gtcttgctct gtcacccagg ctggagtgca gtggcgtgat cttggcccac 14820
tgcaacctcc acctcccggg ctccagcgat gctgcctcag cctcctgagt agctgggact 14880
gcaggtggtg ccaccacacc cagctaattt tttgtatttc tagtagagac atcattttgc 14940
tatgttggcc aggctggtct cgagcgtctg gcctaaagtg atctgctgcc ttggcctccc 15000
gaagtgctgg gattacaggc atgagccatc acaacctgcc tcatttgcat ttaaaatata 15060
ttattgaggt gtaatttatg aacgttaaat tctaccaatc ttaaatgttc agtgcaatgt 15120
cctgcatctt gctttgtgtg gtggttacat atacccatgt tagtggaata catataccca 15180
tgcaatcacc acacaaagca agatatacag gatatttccc tgactgcagt ttccttgtgt 15240
gccttttcag tcaatcccca cccccaaggc aatcaatgct ctatttcttt catcatagat 15300
tagttttttt ctattctaga gctttataaa catgaaatca tgtagtatgt actgttttgt 15360
gtcatttttt gctcagcgtg ttttgagatt cacccatatt gtgtatatca gtagtccatt 15420
cctgatagtg ctaactggtc ttccatttgt gtctttaaaa aaaaaaatca ctggaatgta 15480

ctctgtttct gtatctggca gagtatatgt tggcctctga gaaatgcagg ggtttggcag 15540
agccattcag ttaaatataa tcagtgccta atccagcagt ttctgcaaag gtgtcacttc 15600
tttaacttgt ccacttatag tgagtactca ctctaggggc agtacatttg agtctctcct 15660
tttctctgat ccatccatga tctaggctac agggtgtcat tgaacccctg agattacgtt 15720
cagaaggtgg gtgaatatat gcattagtta agctaaaatt ctcacaattt aatggatcaa 15780
ggaaatttct tttttcatac aagaatccat agtgggtatc catgttagca gagaggtgct 15840
actctacatg gatgcttagg tgtcaggact atggctgctt attatcttgg cttccaaagt 15900
tggtgtgggc atcaccatct tagccagaag ggactcacag cacagaggag cacccatggg 15960
agaattttac aggtagtctc agaactggca gaagtcactt atgctcacat tcgactggag 16020
agaactgtta catgtcctca actatctcca aaagaagctg atgtccaaga gggcaagaat 16080
ggatattgtc actagaggcc tctcctacaa tatgtatatt ctacatttat ctacagcttt 16140
gattaggttc tcaaagggcc gggtctatac ccagaaatgt ttcagagcct gctgtaaggg 16200
catgtgggca ctgctgcttc caccttgccc tagaaggtat actgggccat caaggcaaga 16260
cacaaaaatg acataaaata cacataatgt aggaaaatcc tgctgttgca caggatgaca 16320
taatactgtt catattcctt ggaatactca ttgcagaact tgaggtcgac atcctatgag 16380
ctactagatc tcacaaaaat ccagcatatt agaacaagcc atattttatg tgaaagaaag 16440
gagcaatttt ttgtggtatt tttgtgtctt ttgtaagaga ttcttctcta ccctgaagtg 16500
atgaagatat tccaaaaatc tggaagtttc actttgcaaa tttaggtttt taattcatta 16560
agaacttttt gtttttaaaa aaacttattt aatactcata tataacaaag taacttttta 16620
attaattaat tgattaattt ttaaaaatta tactttaagt tctgggatac atgtacagaa 16680
tgtgcaggtt tgttacatag gtatacatgt gccatgatgc taagataatt ggcactttat 16740
gtagaggaaa aaatatcaga tccctacatc acactatttt ttaaaaggtt cttacagagg 16800
aaaaaatatc agatccctac atcacactac tttttaaaag gttcttatag aggaaaaaat 16860
atcagatccc tatgtcacac tatttttttaa aggttcttaa agaggaagaa atatcggatc 16920
cctatgtcaa aacctatgtt taaaaggtta acaacctttt aaaaaatagc gtgatgtaga 16980
gaactgattt ttttcctgta tataaagtgc caattatctc agcatcattt attaagcaac 17040
aacacatgtt ttttttttag cttttatttt aagttcaggg gtacatgtgc aggtttgtta 17100
cataggtaaa gttgtgtcat gggggtttgt tgtacagact atttcatcac ccaggtatta 17160
agcccagtac ctattagtta tttttcctga tcctctccct cctcccaccc tccacactcc 17220
aataggcccc agtgtgtttt gttcccctct atgtgtccat gtgttgtcat catttagctc 17280
tcacttataa gtgagaatat gcggtgtttg gttttctatt cctgcattaa tttgctaagg 17340
ataatggcct ctagctccat ccatgtccct gcaaaggaca agatctcatt ccttgttatg 17400
gctgcatagt attccatggc gtatatgtac cacattttct ttatccacat tttctttagc 17460
ccatctgtca ctgatgggca tttaggttga ctgcatgtct ttgctattgt gaatagtgct 17520
tcaatgaaca tatgtgtgca tgtgtcctta taatggaatg atttctgtat tcctttgggt 17580
atattcccag taatgggatt gctgggttga atggcatttc tgtctttggg tctttgagga 17640
cttgccaccc tgtcttccag aatggttgaa ctaatttaca ctcccaccaa cagtgtatac 17700

actgtttttt tttttcctcc acaacctcac cagcatctgt tattctttga atttttaata 17760
ataaccattc tgaccggtgt aagatggtat ggtacctcat tgtggttttg atttgcattt 17820
ttccaatgat cagagatgtt gagctttttt ttcatatgat tgttggctgc atgtatgtct 17880
tcttttgaaa aatgtctgtg ttctttgccc acttttcaat ggggttgctt gtttttttct 17940
tgtaaatttg tttaagttcc ttatagaggc tggatattac acctttgtca gatgcaaagt 18000
ttgcaaaaat gttctctcat tcggtaggtt gtttactgtg ttgatagttt cttttgctct 18060
gcagaagcgc tttagtttga ttagatccca tttgccaatt tttgcatttg gtgtcttcat 18120
catgaaatct ttacctgtgc ctatgtcctg agtgatattg tctaggcagt cttcaagggg 18180
tttttatagt ttggggtttt acatttaagt ctttaatcca tattgagtta attttggtat 18240
atagtgtaag gaaagggtcc agtttcaatc ttctgcatat ggctagccgg ttatcccagc 18300
accatttatt gaaaagggaa tcctttcccc attgcttgtt tttgtcaggt tagtcaaaga 18360
tcagatagtt gtaggtgtgc agtcttattt ctcagttctt tattctgttc cattggtcta 18420
tgtgtctgtt tttgtaccag tatcatgctg ttttggttac cgtagccctg ttgtatagtt 18480
ggaagttggg tagggtggcc ttcagctttg ttgttttaag caaatcatct ttgatgtatg 18540
aatttacatg gacttggaca tttacatgta gttgtttctg aattctcttt cgctacgtta 18600
tcagccactg caccaatacc acattattat tatttttttt aatttgagaa agaagcctta 18660
gattatcttg atagctggat ggaaaatttt ccctccttaa gcttctccag aattctcttg 18720
attattcttg gcttattagg cttacccttg aattttagaa tcaagttgtc aggggctgtg 18780
aaaaacccta ttgcaatttt gagtagattg cattacatat attggaggtt ttgcatccac 18840
aaacacggtg catattcagg cctttttatg tattttaata aagttttata attttccata 18900
taggcactgt actttcctgt tagtttccat taaagttata ataaagactt gttgctaata 18960
tgggccaggc acagtggctc atgcctgtaa tcccagcact ctgtgaggcc aaggtgggca 19020
gatcactgga gcccaggagt tcaaaaccag cctggccaaa atggtaaaac cccgtctcta 19080
ctaaacatac aaaattagct gggcgtggtg gtgcatgcct gtaatcccag ctactcagga 19140
ggctgaggca ggagaatgac ttgaacctgg aaggcagaag ttgcagtgag ccgagatagt 19200
gccactgcac tccagtctgg gcaacatagc tagactccat ctcaaaaaaa aataaaaaaa 19260
ttaaaaaaat ttgttgctaa tattgagatg gtatttgttc ccttgacctt gaccccccttc 19320
atggacagga atggagtggc tcatttcact cagcctgcag tccatggatg gctaagtgtt 19380
aacagctcag tgaagggtca gggtgacagc ctcctgcacc tgcccttttt gaccctgagt 19440
tcttgtttgg tgtccaggga gaatcaggtc aaatgaactg tttaaaagat gaatgcagaa 19500
gactttattg agcagtggaa gtggctcttg gtgaaagggg agctggaaag gggatggtgt 19560
gggaaaaagg tgatctttcc ctgaagccca gctgtctctt gctgggcacc cttctgaaac 19620
tgcaccctct gaagttagcc acatttatcc atagcctctg atgcttagtt gcctctctgc 19680
ttgctgcttg gctgcttgta tccccgccag tcagctgctt gtgcttctct tttccttttt 19740
tttttctgcc agctggtctg gtttttatgg gcacaggata gggggcaggg tgggccaaaa 19800
ggcaattatt tgggacgaaa aatggggtca gctgttttca cttagggcca aggttccagg 19860
cttgagggtg aggtttagcc agaagcccag cccttcagga tcggctgttt tcacttaggt 19920

taggggttct aggcttgagg gtggggttta gccaggagct cagccattct gtatcaatat 19980
gtgtgagagg cctttcaaaa attatttttt ctaagggggt gtggcttttt tgcatattga 20040
tctcatattc aacaatgttg ctaagctctc tttattagat actttattgg atttgcttgg 20100
atttcttaag cagacaacta tataacctat aaatcatatt tttcttgttt tctaactttc 20160
ataccttcta atttgttttt cttgtcttat tatattgaca agtggcttca gtacaatgtt 20220
gaatggagat ggcaaaagct atctttgttt tgttgtcaat tttgaaagaa aaatattgaa 20280
tgaattaaca ttaaaaataa tgcttctgga gttgggttat tggtagcaac tctatcatgt 20340
tcaggaaatt cctccctatt cctactttgc taagataaaa aaatgagttg tgaattactt 20400
tacattttca tatatataca tacgtatata tgtacataca tatatacgta tatattacac 20460
atatacatat atacatatat gtacacttac gtatatgtgt acatgtatat acatatatat 20520
atatgaaaat gtaaagtaat tcacaactca tttttttatc ttagcaaagt agtaataggg 20580
aggaatttcc tgaacatgaa acatgtacgt atatatatat acacctatat acgtatatat 20640
gtacatatat atacgtatat aggtgtatat atatatatgt gtgtgtatat atatatatat 20700
atatatattt tttttttttt tttcccctgg agtctcacac aggctggagt gcaggggtgc 20760
gatcttggct cactgcaacc tccacctccc aggttcaagt gattcttttg cctcagcctc 20820
ctgagtagct gggactacag gcgtgcgcca ccacgcctgg ctaatttttg tatttttagt 20880
agagatgggg tttcactatg ttggtcaggc tggtcttgaa ctcctgacct cgtgatctgc 20940
ccacctcagc ctcccaaagt gctgggatta caggcgtgag ccactgcatc ccgccacatt 21000
ttctaatatt aagccatctt tacatttatg gtatcaaccc aatatgttta ttatgcatat 21060
tgtgaaaaca cattgaaatt ggtttaataa tactatatgt agaatttatc atttttttatt 21120
ccttattaga ttggtctagt attttctttt ctatggcttt gtcaggtttt ggtatcaaaa 21180
ttttattagc ttataatgga ctggggtgct ttttttcttt ttctatgctt tgcaaccatt 21240
tgaatagagg ttatttgtcc atgaagattt ggtaagtctt accaaccaaa actttgaact 21300
tttttgcata tttatttaga aataagaatt taaattattt aatggtttta aatacatttg 21360
tattattttt gaattaatgc aattttacaa tttattaaaa agtatttcag ctctgtaatt 21420
ttgctgtttt tcaagtttta aaaatactga gctttgtatt atttaaaaat tttgcacata 21480
aagtaaaatt ttcttgatgt gtttatcata catttaaatg tactttggcc aggtgccgtg 21540
gctcatgcct gtaatcccag catttgggga ggccgaggtg ggcagatcac ctgagatcgg 21600
gagttcgaga ccagcctgac caacatggag aaaccccgtt tctactaaaa atacaaaatt 21660
agctgggcat ggtagcgcac gcctgtaatg ccagctactc gggaggctga ggcaggagaa 21720
tcacttgaac ccaggaggtg gaggttgtgg tgagccaaga ttgtgccatt gcagcctgga 21780
caacaagagt gaaactccgt cttaaataaa tgtacttcaa ttaaaatcat acaaaatgta 21840
actgtactgt tttgctaaat tacattacag agagtttgca tgagtttaca cctatttttc 21900
aagtatcatt atttaaaaga aatctttgtc aacaaaggaa aatgtaaaat catgtattat 21960
tttaattttc atcttttatt gaattaaaaa ttaatttatt gaagaaagta tttcagccaa 22020
gtttttttaat gttcatagta ttcttaggat ttaaaaaatt ctactgaaac tgtaattata 22080
atctccatat tcatagcctt cttttctctt ttttcattgt caattttacc agaaatttat 22140

ctatcttgct attgttctat agaactaggt ttttgttttg ttgaccettt ctatgatttc 22200
tttgtatcca gatccatttt tagctttata gttgtatata taacacacat atatttttt 22260
ccttttcctt gtctttcttc actctgtttt agatctaagc cttttttatt ttggatgagg 22320
gagactacag agaaaggatc tgctagcctt gacctacttc tgatgttacc cagagtattc 22380
atgcagatgt atgtgataag aaaaaggtga acacagagtt actctgtttg gggctttcat 22440
tgcagcctag tccttgcagg aggctgctgc atgcaaaccc aaattctctc cattcttgga 22500
atctccttat gccacctgca ctctgaagag tgttattatt attacttttt actaaattgg 22560
gctgccccac ccacctttgc cgctggactt ttaaaataga aaggtttcct aaggagaatg 22620
cttttttttc ccccctagcc aaggggaaaa acactggaga ggcacaaata ccccatttca 22680
aggaggaaga aaactatgcc tggaggcatc atctgcaaaa ctttccggaa aaggctgagt 22740
tctctcaggg tattgtttca ccaaaatact gtctttgaat cagtctctac cttaatagac 22800
tggagggaaa gtttccaaca gatcgttttg ctgatagttt tgattactct cgaacagaaa 22860
atttcaaaag ctttttgcca gagggaagcg aacagaggga ggaagagaga actcttccat 22920
gagtgtgcca tgactgggga acctttgaa caaataatag tgccaaaggc agtgacagcc 22980
aatttttcca cccgaaatgc ttctcaaatt atagtccttg gatcactagc ttatatcaat 23040
agtctgacaa agtctatggt gtccttccag atattaatgt aaaacctgaa cttgaaatat 23100
ccatcacaat gtttcatttt gctgaacttt ttctgagaca cagtcttgtt ctgttgccca 23160
ggctggagtg cagtggtgca atcttggctc actgcaacct ctctctccca agttcaagag 23220
attcttatgc ctcagcctcc caacagctgg gactacaggc atgcaccacc atgccggcta 23280
atttttatat ttttagtaga gacagggttt tagtagagac agacatgttg gccaggctgg 23340
tctcaaactc ttggcctcaa gcgatctgcc caccttggcc tcccaaggtg ctgggattac 23400
aggtgtgagc caccatgcct ggcttcattt tgctgaacat acatatatat ttaaaaattt 23460
taaaactttt taagagacag ggcctggctg ttgcccaggc tggagtgcag tggcacaatg 23520
gcagctcact gcagcttcga actcctgggc tcaagcgctc ctctcgcctc agcctcccaa 23580
aatgctggga ttacaagcat gagccactgt ggctaagaat attttttgtc atgtttgaca 23640
gcgccagctg tctttgggtc tttacctctg ctgaagtcat ttgaagaaac aaatctaaag 23700
aaatactgct tggcagaggg gaggagagaa aggttaggac agaaagcatc aggatgactc 23760
tccaagaaag ggaatgtctg aatgacaaga ggactacaga caggagaaaa cattcaagaa 23820
aatttatcaa ggtattcttt gttcctgttg aaacctcatc atcaatgtta gtgtcttcag 23880
aagcccaccc aaatatatta ttttactttg ataaaagaca acttggaacc atataaaaga 23940
ctgcactggt accattattc cattttacag atgagaaaag tagctcattt ggaacataat 24000
gagctaggtt ttatggatta atctagatgc tgaactgaat tctgtattgt atttcatttt 24060
ctctgtcctt ttctactgga tgtacatacc tacatttaat tttagagtta tcgatctaaa 24120
gctggctgca gaagctgtct catttagaca ggtccacgct tgtcaggaag acaggtctgt 24180
ccaggcagac ctttcagttt gttctatatt tcatttcagg ccacagcaga ccaaataaag 24240
aggaaatgaa gtgttttata gcaaataact tactgttgaa tacacatgtc ttgtacggac 24300
aggcctccca atttgatgtt agaaggggcc acagagtagt taagatagtc aaaacagaag 24360

caactgtatt ccttaagaag gtaagagaca aaatcaacat attaaaaata ttcaagttga 24420
gtggtaggta agaaactcct tttactttac ctttgattta tggggaaagg ctaaacatgg 24480
aaataattaa ttttaaaatg tctttatatt agatatagaa gtattggtgt taagaaatct 24540
gatagtaagt acagtgtata ggaagaaaga gacgggaaaa ttagctaata agtgattttt 24600
tgacttgagt agagctatgg ttaaaaatat atggaaacct ctttcgagta tatttttttc 24660
ttaccaatat atacttttta tgatactgag attttatagt gaacatttgc ctattcatcc 24720
caatataata tttcttatag tttggtatca accoctatga ttatttgtga gataagggag 24780
tggtttgtat tcatgggtat acatatctat acacatttct gcatgccgta cacaaacata 24840
agtagcattg tgcatgaaca tattagcact gtgcatgtac taaaatttca attttttcac 24900
atagtaggtt ataaaataaa tcatatattc gtaggaaagg tatagaaagc aaagttattt 24960
aaagtggatt tttaaaaaag atttgaatga tttttttaa aaaaaagatt agaaccattg 25020
tttttaaaag gaaacagctt tagatagtaa attatttaca gccaggactc agccctgagc 25080
atcctgtgaa aaccacacga gcttggtcaa ggttctaagc agtcaccggt gggagatggt 25140
catggtcccg tcacagcata actcacttga ggcttgaaaa gtttccgtcc agctacggtt 25200
tgcagtgacc tttctcttct gcaaagccca cggttacctg gtggccacca ccaacatagc 25260
cccgcctcgc tgtcctgcgt ttctctcaat cagatcagtc ctgccgagtc cccatgcctt 25320
actgctggtg tgctgtgtcc tctgtgccat ttcaaggcga gggctcatga aacttttact 25380
cacacgctgc tatttttgat atgcggtggc cttagctgtg gagcgagagg ccgataattg 25440
tgatcactcc ggaaatcctg ggaagccaga agtgggagat gggtgttgtg aggtggcgtg 25500
gctagggaga ccccatcgag tgggtgtgtt ataagacctg ggcgaatccc tgtggctgcc 25560
actctcctga gaatgttccc taggccttag tcccgcgccg ctcccaccca cacctccagg 25620
tgtgcagtcc ccgcccttaa ttactctcac taaaattgat agtttacact tgcaaagcta 25680
cactgggaaa gcggaagaga aatttataat cgtggatatg gagaactagg ggagcagaca 25740
cacttgcttt cgtttacaga tccagtgaag tgaaaaatca gaactagaaa cgtatgcacc 25800
ttcctagcag caaagccgct tctgcgttct tcgcagcctc cagtgcaggg cggcgctggg 25860
agaaactttg cgccttctgg aaagtttaga aagtgagcca cgaaagagag gccacatttc 25920
cggggttttg cgggccccgc gatgttttcc agagcttttc gagtgggaag aggagagcga 25980
caacgtgaaa atgccccgtg ccggggcgtc caccggagtc ctgccagctg tccggcgctg 26040
gggtaagcgc aggaggggcg ggggtgggac ccgagctggc ggccacgggt ctcccgctgc 26100
gggtgtgtcg actcgggggc ggggcggggg aggctgctga gataatgaat gggaggctaa 26160
ggccaccccc cagccccggc cctgccacca ccgtgggctg tcgagccaat gaatggagga 26220
gggggcgcag aggtcagggg cgctgggggc gccacaccag gtaaggggtc cagcttggga 26280
gcggggaggg cggactctgg gggttcgggt gttgctgact tgtgctacgt ggaacaagca 26340
gaaaagaagg aaggcggagg aagagaggag gctggcgtcg gcgctgctcc tctggccctc 26400
cctctgcgcc ccctcctcct gccagcgcgc caaagccggg cagtaggggc cgactggcgg 26460
ctgacgctcc ctgagtggcg actccgtctc cagccccgct gcggagcgcg ggccggatct 26520
ggggcggcca gggcccggag ccgcggagcc ctccccgccg cccggccgag cacgggaccc 26580

cggcggggtg ggcgcagggg gcggccccgc tctgggcgac tgccgagggg cgggcggagg 26640
gccgggctcg gctggcggtg gggcgggggc cgccgggact gggcgcgcgg cctgaagcca 26700
gcccgggggc ggcaggagag ggacgcgcgg cggcagcgag cgcaggtaag ggggccggcg 26760
cggcgtgtgg ggacggcgcc ccctgggcgc gaagccagag gccgcggcga ctgctcggcc 26820
cgccacacgg cgcgctgggc tcacactgtc ccgccgcgga cgggctttgt ggttgggggc 26880
gcgcgtgcga gtgccagtga gagtgtgggt gcgcgctgtg ggccgcggcg cgggtgggtg 26940
gccgtgcgtt cttgcgagcc ggcctgcagg aggcgaggct cccctggcct cccgcaccca 27000
gcggcggacc gagcccctgg agggaagttg ccgcagccgc ccgggccgcc ggccctcctg 27060
tcccgcgcca ggtcagtgcg ccccggggcc cgcccagtga cacgtaggtg gcctccggtt 27120
acctgggtcg gggtgggtgc gcgggagggc ttgcgagggg ccctcgaaat tctcccaaaa 27180
cctcgtcccg ctggacttgc acctccgagg ggtccctgcg cccctggggc tccgcacctc 27240
tcgcttccca ctgacgactc ggttgtcccc caccaccggg ctgaggactt ttaactaagt 27300
ttctgtccag ccgcctaaca aacgtgctta acaagtaaca ggaggttgta aggcaaggga 27360
gggacctggc tttgaaagtt gatggttttg aaatcgacgg ctcctttgac ttgcgtagga 27420
ctcctttgtg gtgaatggat ttaaagttta ttcttgtttt tctttgctgc catttggtct 27480
ttgcaatgtt aagagtgaag gcattcagga cttgctgctg cctgctgtct ggggcctgag 27540
gttgtggctc tccgcttctg ctctcctctc ttcatttttg ttgaaatccg tggatttttt 27600
cataaatgtg ggtgctggct tgtttcacta tggacgcgtc gtttcctatg gggttagata 27660
gatattggta aacataaata ttgaaaatgt gatgatcaca tattgatgat cacagcgaaa 27720
tcctctattc cttcaggctc taacagtttg tttttcacgt atttgcataa gtgtgtttgc 27780
tcagctaagc ataattggta agccagaggt ctgtgattca gagtctgcag ttctggttaa 27840
ggggagactg tacacttgga aaatgcgaag ttttttaaga agttattccc ttgccggata 27900
gaagagtggg tcccaaggaa aaagggtgta ttttagaggt atggttattt gaaccaaact 27960
aaacatcatt aaaatacaca gggaggtaga atgtcaccta gttctttctg cccttgattt 28020
ccatatccca gtaggagcga gtataattgc cccaaatgca aaattatgaa taataatcag 28080
cattgtaagt atattaaggt tgcatttttt tcttgatact tgtctgattt gaggataaaa 28140
acaccaagtg gcaagagaag gaggtagctt gaggcaggaa ggcacgggcg aggagcgagg 28200
agggaaggct gggttgtcat catccgagcc cgcaaggtac accacgtccc ctagcggcgc 28260
gacctcagcc agtcgggtca gctcagatcc tggtctccgt ctccccatcc ggaaaatgga 28320
aggggttggt gatcgttgct gttccttcca gcttcccaaa tagcgagaaa ccggtgggag 28380
gttatcttat tgtgttagag cagtaggaaa aggtaggagg gggaagtgag aggtccagag 28440
ggccaggaag atggaagaaa acaagcaaca gaggatgctt ggaggagaaa aattcttgag 28500
ctgggagctc atttcctctt tttttttttt ttttcctttc ctcaagggac tcaataatac 28560
ttcccccacc tcagatcaat ctcttgttac ctttattatt taaagcttgt gagtttgctt 28620
agctaatgca ctagggtgag gtcaaggtga tccttgcagt ctgacttcta ctaggcatca 28680
cttgactcat ttgcatcatt gtctctcaag ggaacagctc ctgcccaggt ctgtgggcac 28740
tcagcatgga tttcagtctc cctgtgagtg atgggaaaga actaacagag gtaagaatgt 28800

aagtggcagc cctttcgaaa cttctatttt tgttcaaacc taatattttc caaaaagtga 28860
tttgattttt ttgttgtttc aaattatacc gtaggctgca aggttttact gaatctattc 28920
cattagtgac ctactggaac gttcaaagaa taaaaatctc acttgctcag tgtttttgat 28980
acacagtgac attcagtctg aagtaagtga tatttagggc caagaatcat ttcaaatgct 29040
tagtatggaa agttgcaatc tgggcagaat acactgtatc attttcactg ggaagctcca 29100
agtattcagc agataacaga actttcagaa tttagtttga ggtcaagatt ttaatgtctg 29160
tgtttgatgt gtggcctgtc ttccttttct ttgatgtttt ggtatcaata tgcctacacc 29220
cttgaggaac attatttatt gtaaaagctg aactgtgatg taataaaaac ttaaacataa 29280
gctcttggtt taaagtccaa tagtcttctc tggccttaag tcagcatcat actactgttt 29340
ggtttcttat ttttacatat catgttaccc tcttatttaa atatcccggg ccaggtgtgg 29400
tggcttatgc ctgtaatctc aacactttgg gaggctgagg caggacgatc gcttgaagcc 29460
aggagtttga gaccaacctg ggcaacatag tgagactctg tctctataaa aaaaatagaa 29520
acattagctg ggtatggtgg catgtgcgtg tagtctcagc tacttgggag gcggaggagg 29580
gaggatggct tcagcccagg attcaaggct gcagtgagct gtgattgtgc cattgcacta 29640
cagcctgggt gacagagaga ctctgtctct taaaaaaaaa aatcttaggg aaaactatta 29700
tggatgtctt agaaagttga gagaaaagga ggtaattcta tttcagcaaa caattattga 29760
attccttcca tgtggtagat gcaaggatgg tgaaaaggaa gctccagaaa gaactgcaac 29820
acagggggga aagatgcagt gagcctcacg gttcataccg aatatgtata tgcagtgttg 29880
tgtaactggg tggtagctgt agagtccaag aactttggca tgccagggaa tggagaatta 29940
cgttttgatt caggggacga atttataaat gtggcttgaa gaataacata tcttttaaaa 30000
gatgaatggt gggggaatga agacaaagtg agagtgtggg gtgatgaagg ctggaaagtt 30060
acgaagggac aaattgtggg ggacctctga tgttatttta aggagttatg ctacatgggg 30120
atagggaggc aaggaggaag tattacataa cctcaagatg tggaaagaca ctgagtaaca 30180
catattgggg cagagtgagg ggtgcgatga tggatggagg actgggcttt tattccatta 30240
caatccgtgt aacttatggc tgctacgttt taagacacta ttgcctgttt actcaaatta 30300
tataaaggtt gtagaataaa ctaataagta gtttcttcct ccctactctc cgcaaattga 30360
tagtgcttta tacttttgag aaatttaatt tagtaaaaat taatgatgct attgtgttat 30420
agctggacct tgtggagcct tttgaacatg tggggttgaa gtcattctgc aggcacagag 30480
ctgtccaaga aaacattttt tttcccctct cttttttgtt tagggaaggg cttgctggtt 30540
aatgctaatt taaacatgac tcttctggca gctggcattc ttgaccctgt ttatgttata 30600
catggtattt aaccacagtg attgggtatt tgcagcacag aagaaaaaga attattatta 30660
gtttgaaacc ggcattaatg cctctgtaaa tgatagggca aggcagtaga tggaaggaga 30720
gagggaagcc aagtagcaca ctcggtactg cagtgagaga tgacataacc atgagaattc 30780
tttaagttta acttccagta gaagtaactt gctttctata tattttaaat ccctagagct 30840
aaagcattta actcattatc ttcactctgt gggatccatt tgggagaggt attcaggagc 30900
tttataggtt cacacttgct ccccagtacc tctgtgtcac aggaggatat acactggttt 30960
tcagttgcat gtcagaggtg gaactgactt ggatgtcttt gaattgctgt tgaatctgga 31020

gatgctaggg tacccaggag acagacagga aaaagaagag gctgggcacg gcggctcatg 31080
cctgtaatcc cagcactttg ggaggctgag gcgggtggat cacctgaggt caagagtttg 31140
agaccagcct ggctaacatg gtgaaaccct gtctctacta aaaatacaaa aaattagcca 31200
ggtgtgatgc tgggcgcctg taatcccagc tactcaggag gctgaggcag aagaatcact 31260
tgaacccagg agggagaggt tgcagtgagc caagattgtg ccattgcact ccagcctggg 31320
cagacagagt gagactccat ctcaaaaaac ttaagaaaaa gaaaaagaaa aagaaggtgg 31380
ctgaatttct ttcaattacc ttgtgaattc aatttaatga atgatcttcc cagcagtttg 31440
ttttatcttc tgcaagggaa cttatgtttg gcatgtttaa taaattaagt taattaagtt 31500
ggagaagccc aaggttagta tactttattt taggatacat ttttggtagg agaggaggag 31560
gggtggcatg gtggtggtga tgattttatt taaactcttt ggcatttttt agcaggttag 31620
tatgatttca aagtagtgtt gttgttgtat tatcaacttg tggggctggg ccaaagaata 31680
ttgtcttaat acttgcatgt ccctgtcatc taatcagtgc tatagaaact ttaaccctga 31740
agtccatgta aaattgtaat tatttttttc cagaaatgaa agagaactat tttactacat 31800
tcatggattt aggttataat ttattttatt tttgtgatac tgttttaaaa aagtgatata 31860
atgacagggc agacccttaa ctttagtcca gtgctaaaac aaacgtgcaa taagcctgca 31920
tgaggcaggg caagctgtgt ttgttgtatt atgaaaataa aggaaaatgt tttcaaaacc 31980
agcatttttc tctaaaagaa aaaatttgga ctaataatac ctggccatgg gtgggatttc 32040
cagctgttgg ttgaaggaaa ttttgttcat tatggccatt atgtgtgcta tgtctcttag 32100
agtttaggat ttgctatgct gagattgcta ctgtgaacca ttcctgttat agcaagtttc 32160
cctatattca ttaacttatg tgtctaaaca atgctctaga ttagacttta tatcgatctg 32220
ttctactaaa ttttcttccc ctatgcctag gtggttctct ttgaccaacc cttaactgcc 32280
ctgattctga aattctgctc taattgaagg atattcctgg gtctttggag ggagaaatgg 32340
ttcaggggca gaggaaactt tttttccccc catctcagga gcacttaact gactgcctgc 32400
tatataccag gctttgtgct agttttacta ggggtgagtg tgaataaggc aggattccca 32460
ccctccagga ggagcttaca cacgattaac agataattac aattcagtgt aacaagtgtt 32520
atgagagaga agactttgcg ggagcaccaa gcgggggcac ttgaccctca gcggtgaggt 32580
ggtggaggtc agggaatgct cctggaaggt taggatctgg aaggatcctg ggagataaac 32640
caagcctaga agaggtaaaa aagggatcaa gaggctttct atctccagtt tcgctactcc 32700
tgggtaggtc aaagtgcctc tctttttaca tctggaaaat aagaataaga actgatatct 32760
ggttggtaat tctagttttc atttactatg tgttccttaa tcctttatat tactctttct 32820
tccatattca gtaggcattt gtacccaccc tgtactggga agatagcact gcaggaaaca 32880
agatgtggac agacccccttc tctcatggag cttgccctct atcatatgac attagtaaga 32940
gaagttcttt tagaatgcct tcaaactcag gcatatgtat cttgttattc ttccatttta 33000
ggaacatttt ctccaaggta gtctcaatac tttgaggtga gttggccttt ccctttttaag 33060
gaaggagacc acagagcccg gtaatctcct tttcatttta tatgcttgaa atattttgct 33120
aggatttatc tgttttaaag tctagagata gacatagctg tgttttatct gtagccctgc 33180
cttgcttcca tctcattttc cctattctta cttttaaccc aacttgtgtt tgagattctt 33240

tgctcagtat atagtttgct ttctagaata attcaaacct agtgcctgta gaaagtcaga 33300
cttatttttt atttatttga gatagggtct tggagtgcag tggcataatc ctggctcacg 33360
gcagctttga cctcctgggc tcaagctatt ctcccactac agccttccaa gtagctggga 33420
ccgcaggtgt gtgccgctat gcctggctat tttttttctt tttctttctt tcttcttctt 33480
tctttttctt ttcttttttt ttttcttttt ttttttttaa agaaatgggc tctcactaca 33540
ttacccaggc tgttctcaaa ctcctgggct caagggatcc tctcaccttg gcctcccaaa 33600
gtgttgggat gacagctgtg agccaccgca tctggctaat acttattttg aagtcaccct 33660
gtcatggtgc tttgcttcac taatgtattt cacttatgat cttgaagtca ctcagggata 33720
caaacacaat aaaatgcatc ccaaccagga gagggtagct gcattcattt acaaaatttt 33780
tttttgcaat gattcaaaca ctatagatgt gtctaaagta aatgtaaaaa tctctctctg 33840
ccactttaac cttacccatc tgatatcagt ttgttccata gccttccata gttgtccttt 33900
tacttataca aacctacaaa aattaatatt tatgtatatt tatgtgtgtg tgggataagt 33960
gtggatatgt ttatacatac tcatatgtat gtttgtgtgt gtatctgtct gtgcctgcct 34020
ccattttctt ttgctttaga gaggctatac ttgaggctgc catcaagagt gagaagtttg 34080
aagctggaag agcctgcatg ggcccttctt gaactggtgc agcatgtgca gcatgacatc 34140
actcaagagt tcttgtcaga gtgataatga atgtctggct attgtaaacg ggaataagaa 34200
aactatttcc agctgtgtga caaccaagac gacaaaaagc attgcagaga atattattgc 34260
cacaaggacc ctgcttcatc tgggtctcag acgacgggag gaggggcatt ttggagcacg 34320
tgtttggcat ctgtgaacct tttgttaggt agaaaacaag gcctgaatga aaggcctttc 34380
aaccacttct ggagcagaga agataggtag agttactcat tataggcagg tttcattgta 34440
ggagtattca gtgaggaccc ccgccttgga agtctgtaat cagcagatga taaggatggt 34500
gtgttcttac taagagaata acacaactga aacagaattg ccttttgtta aggggatgct 34560
ttgccttctt ggactacgat tgtggggaga aggattattg tcaactaagt gaggcattca 34620
ttctgtaccc actatttatt gtagttccac agagaactgc ttgctttact ttctgactag 34680
gcacagaaaa gtaaaggttc aaaggctagg gcaagatgac tgacttttcc agatttagca 34740
caatctgttc tctggtcact ttgagacgct gtcagtttag tttcatgcag ctgatatctt 34800
ggagaacttc tgtgtcctta cgtttggctg aggacactaa attttttttt tttttttttt 34860
ttttttgagg cagagcctca ctttattgcc caggctggag tgcaatggca cgatcttggc 34920
tcactgcctc ctgggttcaa gcaattctct tgcctcagcc ttccaagtag ctgggaccac 34980
aggcgtgcac caccatgcct ggctaatttt ttttgtattt tttggtagag atggggtttc 35040
actgtgttgg ccaggctggt cttgaactcc tggcctcaag tgaaccaccc gcctcggcct 35100
ctcaaagtgc tgggtttaca ggcccaagcc accgtgcctg gcctgaggac actaaaatta 35160
aaaaaaaatt aagaaagtac ggtccccgct cttgagtagc tcatgaacat ggagcgatat 35220
ggactcaaat gattaagatc aggtaggtag tgatgaatac ggtaaaccaa gtttcaaaca 35280
aagagctgtc tgcatatctg aggatggaga gggtaagtca gccttagagg agggaaatga 35340
cttgcagcag gatgccagcc tcaatggtct gtggagctca ttccatgcag aagagtaatg 35400
aggaagaccc agtgagtgac aggcttgggg aggagtgcct gaaattgacg gtttgtggca 35460

ggaagtggtg ggaccaatct cgaagcttgt ggaggtagaa gggtactgga gactactgtg 35520
gacaaaaaag ggcatgttga tgccattacc agacaggaca cgagagcatg atgattgttg 35580
cagtgaggct tgttagtttt agacatgtct aaaaccatgc aggcagagat tttcagctgg 35640
tggctggcaa tttgagtttg aagtttagct gagaagtcag ttggcagtat tcaggcataa 35700
ttgctaaatg tagaagtaaa tgccagtaaa atgtgtgtga taagctggag agcactttta 35760
gagtgaaaag attgatatat tttaacaagg acaaggctta tttcaattct ttaggttatt 35820
tttctttagc agattaaagt agttttatcg gttatcaagc atttgttgag cgtttacta 35879

<210> 4
<211> 20
<212> DNA
<213> Artificial

<220>
<223> Primer

<400> 4
tgtctcggca tagttctgga 20

<210> 5
<211> 20
<212> DNA
<213> Artificial

<220>
<223> Primer

<400> 5
catttctttg tcgcggtagg 20

<210> 6
<211> 20
<212> DNA
<213> Artificial

<220>
<223> Primer

<400> 6
ttgtcttcct gctcatggtg 20

<210> 7
<211> 20
<212> DNA
<213> Artificial

<220>
<223> Primer

<400> 7
tgcaggactc acagcctaaa 20

<210> 8
<211> 20
<212> DNA
<213> Artificial

<220>
<223> Primer

<400> 8
tgactgtctt aggggcgttt 20

<210> 9
<211> 20
<212> DNA
<213> Artificial

<220>
<223> Primer

<400> 9
ttctcctgag agaagcagca 20

<210> 10
<211> 20
<212> DNA
<213> Artificial

<220>
<223> Primer

<400> 10
ttcctggcca ttgtctatcc 20

<210> 11
<211> 23
<212> DNA
<213> Artificial

<220>
<223> Primer

<400> 11
ggaatgataa acccaacaac ttc 23

<210> 12
<211> 20
<212> DNA
<213> Artificial

<220>
<223> Primer

<400> 12
cctgccccaa tccctttatt 20

<210> 13
<211> 20
<212> DNA
<213> Artificial

<220>
<223> Primer

<400> 13
ccctaagccc ccaattctct 20

<210> 14
<211> 20
<212> DNA
<213> Artificial

<220>
<223> Primer

<400> 14
tccagggaca ggatatggag 20

<210> 15
<211> 21
<212> DNA
<213> Artificial

<220>
<223> Primer

<400> 15
tgaagacaaa tcgcttttcc a 21

<210> 16
<211> 20
<212> DNA
<213> Artificial

<220>
<223> Primer

<400> 16
agtgaggaac aagccagagc 20

<210> 17
<211> 20
<212> DNA
<213> Artificial

<220>
<223> Primer

<400> 17
gaggtgccca tgctacattt 20

<210> 18
<211> 20
<212> DNA
<213> Artificial

<220>
<223> Primer

<400> 18
aatcgggaca ccatgatgag 20

<210> 19
<211> 20
<212> DNA
<213> Artificial

<220>
<223> Primer

<400> 19
cagaggatct gcctgaaggt 20

<210> 20
<211> 20
<212> DNA
<213> Artificial

<220>
<223> Primer

<400> 20
atggtcaccc tcagatcagc 20

<210> 21
<211> 20

<212> DNA
<213> Artificial

<220>
<223> Primer

<400> 21
ttgaccgctg aagagaacct 20

<210> 22
<211> 20
<212> DNA
<213> Artificial

<220>
<223> Primer

<400> 22
tggcacgtat gagctgaaag 20

<210> 23
<211> 20
<212> DNA
<213> Artificial

<220>
<223> Primer

<400> 23
ccagctgcag ggtaggttta 20

<210> 24
<211> 20
<212> DNA
<213> Artificial

<220>
<223> Primer

<400> 24
tggtgctgaa gaacatccaa 20

<210> 25
<211> 19
<212> DNA
<213> Artificial

<220>
<223> Primer

<400> 25
aaattccgca agcaaggac 19

<210> 26
<211> 20
<212> DNA
<213> Artificial

<220>
<223> Primer

<400> 26
tcccaggaat tggaaacaag 20

<210> 27
<211> 20
<212> DNA
<213> Artificial

<220>
<223> Primer

<400> 27
acatgactgg gctcacaaag 20

<210> 28
<211> 20
<212> DNA
<213> Artificial

<220>
<223> Primer

<400> 28
tgggtttccc ttcaaccata 20

<210> 29
<211> 20
<212> DNA
<213> Artificial

<220>
<223> Primer

<400> 29
ttgggtcaaa ctccgactgt 20

<210> 30
<211> 20
<212> DNA
<213> Artificial

<220>
<223> Primer

<400> 30
attttgaaga cacgggcaag 20

<210> 31
<211> 20
<212> DNA
<213> Artificial

<220>
<223> Primer

<400> 31
gggaatttgt tggcatgact 20

<210> 32
<211> 20
<212> DNA
<213> Artificial

<220>
<223> Primer

<400> 32
atcactgcca cccagaagac 20

<210> 33
<211> 20
<212> DNA
<213> Artificial

<220>

<223> Primer

<400> 33
atgaggtcca ccaccctgtt 20

<210> 34
<211> 36
<212> DNA
<213> Artificial

<220>
<223> Probe

<400> 34
ctagctttgg aacataatga gctaggtttt atggat 36

<210> 35
<211> 36
<212> DNA
<213> Artificial

<220>
<223> Probe

<400> 35
ctagatccat aaaacctagc tcattatgtt ccaaag 36

<210> 36
<211> 36
<212> DNA
<213> Artificial

<220>
<223> Probe

<400> 36
ctagctttgg aacataatga gctaagtttt atggat 36

<210> 37
<211> 36
<212> DNA
<213> Artificial

<220>
<223> Probe

<400> 37
ctagatccat aaaacttagc tcattatgtt ccaaag 36

<210> 38
<211> 25
<212> DNA
<213> Artificial

<220>
<223> Probe

<400> 38
ctagcgcttg atgagtcagc cggaa 25

<210> 39
<211> 25
<212> DNA
<213> Artificial

<220>
<223> Probe

<400> 39
ctagttccgg ctgactcatc aagcg 25

<210> 40
<211> 21
<212> DNA/RNA
<213> Artificial

<220>
<223> siRNA

<400> 40
gaaaugagcg ccaccuuuat t 21

<210> 41
<211> 21
<212> DNA/RNA
<213> Artificial

<220>
<223> siRNA

<400> 41
uaaagguggc gcucauuuct t 21

SEQUENCE LISTING

<110> RIKEN
Takeda Pharmaceutical Company Limited

<120> Bone and Joint Disease-Susceptibility Genes and Use Thereof

<130> 091257

<150> JP 2007-155792
<151> 2007-06-12

<160> 41

<170> PatentIn version 3.3

<210> 1
<211> 1092
<212> DNA
<213> Homo sapiens

<220>
<221> CDS
<222> (1)..(1092)

<400> 1
atg gct gcc atc tct act tcc atc cct gta att tca cag ccc cag ttc 48
Met Ala Ala Ile Ser Thr Ser Ile Pro Val Ile Ser Gln Pro Gln Phe
1 5 10 15

aca gcc atg aat gaa cca cag tgc ttc tac aac gag tcc att gcc ttc 96
Thr Ala Met Asn Glu Pro Gln Cys Phe Tyr Asn Glu Ser Ile Ala Phe
20 25 30

ttt tat aac cga agt gga aag cat ctt gcc aca gaa tgg aac aca gtc 144
Phe Tyr Asn Arg Ser Gly Lys His Leu Ala Thr Glu Trp Asn Thr Val
35 40 45

agc aag ctg gtg atg gga ctt gga atc act gtt tgt atc ttc atc atg 192
Ser Lys Leu Val Met Gly Leu Gly Ile Thr Val Cys Ile Phe Ile Met
50 55 60

ttg gcc aac cta ttg gtc atg gtg gca atc tat gtc aac cgc cgc ttc 240
Leu Ala Asn Leu Leu Val Met Val Ala Ile Tyr Val Asn Arg Arg Phe
65 70 75 80

cat ttt cct att tat tac cta atg gct aat ctg gct gct gca gac ttc 288
His Phe Pro Ile Tyr Tyr Leu Met Ala Asn Leu Ala Ala Ala Asp Phe
85 90 95

ttt gct ggg ttg gcc tac ttc tat ctc atg ttc aac aca gga ccc aat 336
Phe Ala Gly Leu Ala Tyr Phe Tyr Leu Met Phe Asn Thr Gly Pro Asn
100 105 110

act cgg aga ctg act gtt agc aca tgg ctc ctt cgt cag ggc ctc att 384
Thr Arg Arg Leu Thr Val Ser Thr Trp Leu Leu Arg Gln Gly Leu Ile
115 120 125

gac acc agc ctg acg gca tct gtg gcc aac tta ctg gct att gca atc 432
Asp Thr Ser Leu Thr Ala Ser Val Ala Asn Leu Leu Ala Ile Ala Ile

```
    130                 135                 140

gag agg cac att acg gtt ttc cgc atg cag ctc cac aca cgg atg agc      480
Glu Arg His Ile Thr Val Phe Arg Met Gln Leu His Thr Arg Met Ser
145                 150                 155                 160

aac cgg cgg gta gtg gtg gtc att gtg gtc atc tgg act atg gcc atc      528
Asn Arg Arg Val Val Val Val Ile Val Val Ile Trp Thr Met Ala Ile
                165                 170                 175

gtt atg ggt gct ata ccc agt gtg ggc tgg aac tgt atc tgt gat att      576
Val Met Gly Ala Ile Pro Ser Val Gly Trp Asn Cys Ile Cys Asp Ile
            180                 185                 190

gaa aat tgt tcc aac atg gca ccc ctc tac agt gac tct tac tta gtc      624
Glu Asn Cys Ser Asn Met Ala Pro Leu Tyr Ser Asp Ser Tyr Leu Val
        195                 200                 205

ttc tgg gcc att ttc aac ttg gtg acc ttt gtg gta atg gtg gtt ctc      672
Phe Trp Ala Ile Phe Asn Leu Val Thr Phe Val Val Met Val Val Leu
    210                 215                 220

tat gct cac atc ttt ggc tat gtt cgc cag agg act atg aga atg tct      720
Tyr Ala His Ile Phe Gly Tyr Val Arg Gln Arg Thr Met Arg Met Ser
225                 230                 235                 240

cgg cat agt tct gga ccc cgg cgg aat cgg gat acc atg atg agt ctt      768
Arg His Ser Ser Gly Pro Arg Arg Asn Arg Asp Thr Met Met Ser Leu
                245                 250                 255

ctg aag act gtg gtc att gtg ctt ggg gcc ttt atc atc tgc tgg act      816
Leu Lys Thr Val Val Ile Val Leu Gly Ala Phe Ile Ile Cys Trp Thr
            260                 265                 270

cct gga ttg gtt ttg tta ctt cta gac gtg tgc tgt cca cag tgc gac      864
Pro Gly Leu Val Leu Leu Leu Leu Asp Val Cys Cys Pro Gln Cys Asp
        275                 280                 285

gtg ctg gcc tat gag aaa ttc ttc ctt ctc ctt gct gaa ttc aac tct      912
Val Leu Ala Tyr Glu Lys Phe Phe Leu Leu Leu Ala Glu Phe Asn Ser
    290                 295                 300

gcc atg aac ccc atc att tac tcc tac cgc gac aaa gaa atg agc gcc      960
Ala Met Asn Pro Ile Ile Tyr Ser Tyr Arg Asp Lys Glu Met Ser Ala
305                 310                 315                 320

acc ttt agg cag atc ctc tgc tgc cag cgc agt gag aac ccc acc ggc     1008
Thr Phe Arg Gln Ile Leu Cys Cys Gln Arg Ser Glu Asn Pro Thr Gly
                325                 330                 335

ccc aca gaa ggc tca gac cgc tcg gct tcc tcc ctc aac cac acc atc     1056
Pro Thr Glu Gly Ser Asp Arg Ser Ala Ser Ser Leu Asn His Thr Ile
            340                 345                 350

ttg gct gga gtt cac agc aat gac cac tct gtg gtt                     1092
Leu Ala Gly Val His Ser Asn Asp His Ser Val Val
        355                 360


<210>  2
<211>  364
```

<212>  PRT
<213>  Homo sapiens

<400>  2

```
Met Ala Ala Ile Ser Thr Ser Ile Pro Val Ile Ser Gln Pro Gln Phe
1               5                   10                  15

Thr Ala Met Asn Glu Pro Gln Cys Phe Tyr Asn Glu Ser Ile Ala Phe
            20                  25                  30

Phe Tyr Asn Arg Ser Gly Lys His Leu Ala Thr Glu Trp Asn Thr Val
        35                  40                  45

Ser Lys Leu Val Met Gly Leu Gly Ile Thr Val Cys Ile Phe Ile Met
    50                  55                  60

Leu Ala Asn Leu Leu Val Met Val Ala Ile Tyr Val Asn Arg Arg Phe
65                  70                  75                  80

His Phe Pro Ile Tyr Tyr Leu Met Ala Asn Leu Ala Ala Ala Asp Phe
                85                  90                  95

Phe Ala Gly Leu Ala Tyr Phe Tyr Leu Met Phe Asn Thr Gly Pro Asn
            100                 105                 110

Thr Arg Arg Leu Thr Val Ser Thr Trp Leu Leu Arg Gln Gly Leu Ile
        115                 120                 125

Asp Thr Ser Leu Thr Ala Ser Val Ala Asn Leu Leu Ala Ile Ala Ile
    130                 135                 140

Glu Arg His Ile Thr Val Phe Arg Met Gln Leu His Thr Arg Met Ser
145                 150                 155                 160

Asn Arg Arg Val Val Val Val Ile Val Val Ile Trp Thr Met Ala Ile
                165                 170                 175

Val Met Gly Ala Ile Pro Ser Val Gly Trp Asn Cys Ile Cys Asp Ile
            180                 185                 190

Glu Asn Cys Ser Asn Met Ala Pro Leu Tyr Ser Asp Ser Tyr Leu Val
        195                 200                 205

Phe Trp Ala Ile Phe Asn Leu Val Thr Phe Val Val Met Val Val Leu
    210                 215                 220
```

```
Tyr Ala His Ile Phe Gly Tyr Val Arg Gln Arg Thr Met Arg Met Ser
225                 230                 235                 240

Arg His Ser Ser Gly Pro Arg Arg Asn Arg Asp Thr Met Met Ser Leu
                245                 250                 255

Leu Lys Thr Val Val Ile Val Leu Gly Ala Phe Ile Ile Cys Trp Thr
            260                 265                 270

Pro Gly Leu Val Leu Leu Leu Leu Asp Val Cys Cys Pro Gln Cys Asp
        275                 280                 285

Val Leu Ala Tyr Glu Lys Phe Phe Leu Leu Leu Ala Glu Phe Asn Ser
    290                 295                 300

Ala Met Asn Pro Ile Ile Tyr Ser Tyr Arg Asp Lys Glu Met Ser Ala
305                 310                 315                 320

Thr Phe Arg Gln Ile Leu Cys Cys Gln Arg Ser Glu Asn Pro Thr Gly
                325                 330                 335

Pro Thr Glu Gly Ser Asp Arg Ser Ala Ser Ser Leu Asn His Thr Ile
            340                 345                 350

Leu Ala Gly Val His Ser Asn Asp His Ser Val Val
        355                 360


<210>  3
<211>  35879
<212>  DNA
<213>  Homo sapiens

<400>  3
atgaaaacat gagtcttcga aaagacaacc tattgtctgc attctggagg gaactctact     60

tagactatca agatcttgga ataacctata cacatctcat cactgcagcc tctttgtttc    120

aacagcattc acgtgcactg gtctgccagt ctcctcagtg tccttatgtc ctccacggga    180

aggcatgtgc taatgtcagg tgcccctgta tggctcacac aaatgtggga agccatctat    240

ccagccactc tctcggccag tgctgtgtta cagctctcat acgttgttgg acatcacatt    300

gctcaagtgt agttgggggt tcccctagtc ccaagaaagc actccatgag ctccagggtt    360

ctcagtttcc ctgacacctt ttcttttctg ggtgttttcc tcttctctaa gaggaggaaa    420

gtgaggacac tggtgtctga tttcatcctt tcctttctct caaaactctg ctagaaaaag    480

aaaggatgga ctttcactct ttcctatgtg gcaggggctt tgttgagtgc tgtctacttt    540

atacttgtaa tgaaactcta tgatcaaact agtgattatg taagtgagaa ctccgataat    600
```

```
ttaggaaatc cccttcctgc cagtcaaagc catttgacag tcagaagctg aacattgact    660
cctttctttc tcttgtgctc ctgctgttaa cagtcctaat catccctgac tatcgcccaa    720
attagatgga tcctcaaaaa aggaaaacca cgttaaaata gttcaaaaaa taattttcat    780
attgcatatt ttccccagtt tataattttt aaaacacatt ccttgttttc atttataggc    840
attttaaatt gttgtttaat ttaaatctat cagtcttttt ctttaggctt tcttccagtg    900
gtgtcatgcc aagccatccc caaatcaaaa gattttttta aaaaatcaat catatagttt    960
tctaatgcct ttgtatgtaa tgtaggtatt tatttcatga atgaccactg cagtaccccg   1020
tcttagtaaa taatacttcc tttcttcctg ttgttcaggg caaaactttg gcatcgtctt   1080
tactctgtct tcctcttatg ttaacatatt caatccttca gcaaaccctc ttagctttgc   1140
ctttgaaata tattcagaat ccaaccatct cttgtctggt caattgcggt agcctcctaa   1200
atggtctcct tttccctgcc ccctggtagt ctcttctcaa catggcaggc agattgttct   1260
tttaatacat gttatttttc tgcctaaaac ctaccagatt accatctcat ttagaggaaa   1320
atccaaagtc cttcccacgg gctgcgaggc catctgtggt ctctaccaca acacacacac   1380
caactcccac cacatccaac cttctctgac ttcctctccc agccttcttt cccttactcc   1440
tttctcttca gctatgacct tgacatcatt gctgttcatt gaataaggga agcaaactgc   1500
catctcagga ctattgcact tgccttccct tctacctgga atgtgcactc ctgtcccttc   1560
ctgacctctt tatctgcttt cctcaggtct ctcttcaaag gccatcatta caaaagcttg   1620
tttctgatca ctttatctaa aataagcact cacaacactt attgctacct gacatattgc   1680
ttattagtaa attttctgtc tcctcccatt aaaacagaag cttgagggag agctgtgttt   1740
tgtatactgt catatcccca aggtctaaaa tgggactcta ctacctttat taagatgtct   1800
ttttaaaaat ataccacatt ttggtatgat ttatgaataa aattacaagt cacttttaaa   1860
cacctaaaat taattttatg tacttatttt taaatttcat aataatggag tctactctat   1920
aatgttgcct ttaaaacatt ttaatctata tttaacgtca gattggtttg cactttgttt   1980
tactgtatta aaatctcctt gtatttctaa taatttttgc tttagataat ttaaggcaat   2040
attgcttgaa gctcaaaggt tcactcctat accttttttga taaattatgt tgtgtattcc   2100
tatgaactgt ccaggtttac ctcatttaat tttttccttg aatattactt aatctgatct   2160
taataatgcc acccatgagt ctttaaatta gcattggctt aatactttcc catcctgttt   2220
attctcaaac ttatgttttc ttttcattct aggcatgctt cttaaaaaca gcagactttt   2280
aaaattcact caacctttaa aaaaatgttc aaacttgttt ttattgctac catccatgtg   2340
tccttcaatt atttagaaag aaatctcaga catatcattt tacatttaaa tatttcagtt   2400
```

aatatctcaa aaagatttct tttaaaacat aactataata gtgttatcac acctaaaaat 2460
taataatagt ttctaatatc atgaagtact caattacaat tcgaatattc caattgtctg 2520
tctttttct tttacttttt ttgaggtagg gtcttgctct gttgcccagg ctggaatgca 2580
atgtcatgat ctgagttcac tgcaacctgc gtctcctgag ctcaagccaa cctcccactt 2640
cagtctcctg agtagctggg actacaggtg catgccacca tgcccagcta atttttgtat 2700
tttttataga gatggggttt catcatgttt cccagggtag tctcaaattc ctgagctcaa 2760
gcagtccacc agcctcggcc tcccaaagtg ctaagattac aggtgggagc catcatgtct 2820
ggctcatttt tcaaaatata atttatttgt atcataatcc aactaatgtc taagcattga 2880
aattggccaa tatgtccctt aaattctgtt taatctaaag gtccacctct atctctgatt 2940
ttgtttttct tactccaccc tttaaattcc tcacctaaaa ctgtcccaga gtgagttgtc 3000
aagagagagc ccttggactt aacaatgaat ggtgcatttc actggtagcc tctcatttat 3060
tttacatttt tcaggtttca tcaacatctt ggaccagagt caggtggatt agatacaaac 3120
atcatctttc cctgctatgt ctgctatgtc catggcttag tagtgatttc acaaggtagt 3180
atgtataatc agtgagaagc atgtgtagtc atttacatga actttctgaa aactcttacc 3240
tttctacttt ctattattat atactgaggt tgggggaaaa agtgttgtta atagctagga 3300
catattatgc catctcttat tacagcttgt aagtctgccc ccacttttta gcatctctct 3360
ttagaaagtg cacacttgga agctctttat tttcagctgt caaccttaat tacccacttt 3420
tgaaaaatga gaaaacttat actcagcacc atatcagttg ctgtaaagca gttgatctcc 3480
catatctttg ctttatatta ataacaatag ctaacatttg ttaagtattt tatgtcagac 3540
cttcagaatt ttgcataaat tacctcattt aatcctcatg gcaattcagt aacaggggca 3600
ctactagctc aatataatag gtgagaaaat tgacacctag aaaggttaac taaagcagta 3660
tgaggctaaa tagctaaaca ggagtggagc tatgatttga actcaagtct tctaactaaa 3720
tgttcatgat gccatcacaa tgctgccatg gtcatactct gttctagaag acgaacagtg 3780
tatacccgtg gctcaaactc attaatttga tttccagact tctgctactt aattggtttt 3840
gcttttcttt gttggcagtc atgtttttaa atctattaga attcattctt cttttccaat 3900
tactactttc taatacattc taatggaagc atgtttttta ttgtatttat gcattatcca 3960
atagatttta ttggaaatgc cacttcaaaa tttttagagg ccttatttac ggttcttcat 4020
taccattgtt tccactgagg ccattttttt ctgatcttaa aaaaatttcc ttttctcttc 4080
tagtgactga ttgttttctt tcatctggtg atttttcact gaccacttac ttatgaatga 4140
ggattagaat gatcagcacc agtgggcttc cctaacaatc tggttggatt gtgtttaata 4200
ttctttggtg gggcccttcc ctcctttccc ttcttccctt ctctgccttc ttctcccctc 4260

```
ctttcttccc ttctcttgcc ttcccttcat gggcttccct aacaatctgg taggattgtg   4320
tttaatattc tttggtgggg cctttccctc ctttctttcc ttcccttccc tcccttcttc   4380
tcccctcttt cttccctctc ttgccttctc ttcgtgggct tccttaacaa tctggttgga   4440
ctgtgtttaa tattctttgg tggggccctt ccctccattt cttccttccc ttcccttcct   4500
tcctctcccg ccctttcttc ccttctcttc ccttcccttc atgggcttcc ttaacaatct   4560
ggttggatta tgtttaatac tctttggtgg gactcttccc ttctttcatc cccttttat   4620
gagaaaggag agaaggctgt ggtgaaagat tcagaaaatg ggagtattag aataggtgtt   4680
aagctgctgt aaaaaaagaa cctcaaaata ccgggactta aataatatac cgatttattt   4740
ctctgtcaag taacagtcta gtagtgagta gtccaggttg gtggggcagc tctcctatct   4800
tcagttagtc atttgaggac attgtttctt ccatcttgtt gcttcttcaa tccctatggt   4860
gctattttaa ctggctgaaa ttagatcaag ctagaaggag aaagtacagt gatgaagggc   4920
aagcaatttc ctttcatgaa aatgatacag aagctgctgc agacatctct tccactcaca   4980
ttctttcaca tacctcttcc atttccattg atgagaacta atcacatgac cataactggt   5040
tgcaaagtag gctgggaagt ggaggctaaa actgaatatc catatgccta gctaaaattc   5100
cactactagg gaagaggaga agaaaagatt tctggtgacc actagcagtc tgccacacca   5160
agctgatagg taacagatct ctttaaaagt atgtaaagcc aggtcataat gtttctctaa   5220
gagcccaaag tcatctatgt ggctcacaag ccaaaaggtg agtctctgaa gtctggtgca   5280
ggcgcacaaa tggaatctct tttttaccac cataaaactt aattggtttt taaattgttg   5340
aaataatttt cttttccctt catccccaaa gattctgtgc aaaagtgcag aatacttttt   5400
ctgatgttct aaaaggtagc aatctaggct atttatttaa ataccctaaa tgaagaaagt   5460
ctattcaggg atatcctcaa atcccaactc ttgttctctc cagagccttc ttcaagtgct   5520
ccattgtttc ccttaacaaa ggaagatgga gaaattctcc ccaaaggggc cagggactct   5580
tggttctctt ccttctgttg gaactccttg gatagcaagg gaaaatttta aactctgaaa   5640
taaaaatggg aatgtattgg cttatgcaat gtaaaagttc acagaacaga caagagatac   5700
accttgatcc agggtttcaa aataggtcat caggatatgt ctcctaatgt tacttttctg   5760
agtgttggct taccccttttg gcaagttctc attcatggcg gtctccaaca tttcaagttt   5820
ttgtttcaca gtgccaattc tatcttattt tatcattatt ttttgagaca gggtctcact   5880
ctgttgccca ggctagaaaa ccgtggcatg atcatggctc actgcagctt caacctccca   5940
ggctcaagtg atcttcccac ttcagcctcc tgagtagctg ggactacagg cacatgccac   6000
cacacctggc tattttttt ttttttttt tgcctcatcc tcccaaagtg ctgggattac   6060
```

aggcatgagc cactgcacct gaactcaaca gccctaattc tagcaagaaa agagaatata 6120
ccactttctc atcaggtcaa gtgaaagtac ccaagatcat taacattggc tctgactgga 6180
tcactggtac atcttagaat ttttcaattc catgatcaca ttgaatgttc tgaatgaccc 6240
gtaccacatc atccacatca tagacaccca taaagagcaa aaggttaaca tcacacaaag 6300
tagtagaaag gaaaatcaag gttctgttag cagaagggag cttggatggt aggcaacaaa 6360
ataacaacaa atgtccacca ctcatccatt tgcctatgga gttaacgaat ctggctaatc 6420
ttaggatgac tcttaaatac ttgcctttga ataaggatgt tctctagagc cctttaaact 6480
actgagtttc cctgagtcgt cttcttgcag tgtcttattc caagtgtttg tggttatcat 6540
gtccccatgt tctttcactt tcaggtcagg catatgtctt tcccctagga cctactgatc 6600
ctgagtaata tcacaataat gaccatgttg taaaactcct tgtagtgttg attttatagg 6660
tcacttccat tttctgtaac ttcatgccac acttcgtttc tcctgcagat ggatattttt 6720
ttaaatttca tgtttcactt taagaaaatg ggggtacaca aagattcata gcaaggtagt 6780
ttgtaaagaa ttttaggggt aagaaacctt tatttaatga acagaataat ggatctctcc 6840
aagaattttt tttttttttg agacggagtc tcactctgtc gcccaggctg gaatgcagtg 6900
gcgcgatctc ggctcactgc aagctctgcc tcctgggttc atgccattct cctgcctcag 6960
cctcccgagt acctgggact acaggcacct gccaccacgc ctggctaatt ttttgtattt 7020
ttagtagaga tggggtttca ccgtgttagc caggatggtc tcgatctcct gacctcgtga 7080
tctgccctcc ttggcctccc aaagtgctgg gattacaggc gtgagtcacc acgcccagct 7140
ggctctccaa gattctttaa agactggacc tattgatagg tggttgtata ccgtggggtt 7200
ttctctaagg cttggaatag acaagccatt tgttacacta atgccaaggc tctgttcata 7260
ttgatgagga ataagtgggg agcagagaat tttgttttac cattgttctt ttgaataaga 7320
tagattagtt gggacttcca caatgtgctg tgtggcgagg tggctaagaa cacagactgt 7380
atcaacatcc tagttctgcc acttactgcc tacttgatct taggtaagtt acttgacttc 7440
agtattcctt attcttcaat aaaatggaaa taataacagt attcatgtca cagagttgtg 7500
atgctcatag gagttaatgt gtataaaatg tttagagtag tgtaagaacc tgaaagtaat 7560
ataagagcca ggtaaggaca tactaagaac tttgttagtg attattatat tatttttcat 7620
gagaattgct acataaatat tccatgaaca aagtgaaaga agtggctggg tgaagtgtaa 7680
tctcaatctc atgcgtgtaa tctcaacact ttgggaggct gaggcaggag gcttgcttga 7740
agccaggaat ttgcggccag gagttcgaga ccactctaga caacatagag agaatctgtt 7800
tctacaaaac aataacaata aaagaaaaga aaagaaataa aaagtttaaa gtgagagaaa 7860
tatgcagtag taagttcctt gaagaaacaa ccaaagcata aaacccagat tttcttttta 7920

tgaattctgc ctaaattatc cacttctgag taagaaggtg gaagtagcat actaacggaa 7980
aaacaggcta atgagcctac cctagtttca cattcaagag gtggaataat ttatgggaaa 8040
gctttcaaag cttccaaata ctgcaatcaa atcactatat ggagaaagct acaaaatcaa 8100
attccaaaca atgaagacat cagtttctta atggaacagt tccaaccatc tggcattcgt 8160
atgttgtttg taggggtggt tattgtacag atataatgac ccagttccac ctctaggcaa 8220
gaagctttca tgaaggaagt ttacgctggc cacttgtggc tggttaaaat gaccaaaaga 8280
gttttctttc cttttttttt ttttttttta aactgtgcta ttgttaatat ttcaaatttg 8340
gaccttttcc cagcaaaaca caaaaagaag aagcttcaaa tacattcata gagtgcaaga 8400
aagtgagcag aaggaggttt caatgtttca gaaagaaatt ttttcttcaa ggcaattttt 8460
tttaaaagaa gtgccttatt acataggctg gatccagtat taatgataat cagttatttt 8520
tattaaacag gtttaaattt tggatacttt ttttaaacta caccttaaaa acggactttg 8580
gataaaactg gaacacaaaa agcaaagaca ttttgttttc ttttggatgc actgccaatt 8640
ttgttagcat agtatctttt ccatttacta tggaacttga cctacagtct aaggttcaat 8700
tgttcattta aaattaatta attccacaaa tatttactgt ataactagaa agtgctagat 8760
actgaggtag gtatatttaa tggagtgcaa aaaagtggtt ttaaagaaat tggaacaggc 8820
aggttgtgaa aagggtaggt tggtacaaaa tgttaacagc atgagagatt tttttaaaaa 8880
agataaccag ataagatgaa aaagaaaatg gaagacaaaa gggagacatg caagaaaaat 8940
taaaatgcat tctaaataaa atctatattg gagtaattga agagaaaaat gttctggata 9000
atagcaatgt ggattttaca gtttataagc tctcctagga tgcagtgggg aaaggcaaag 9060
agataaaaat gacaagtgac tgactgaatg gagaacagag atctcaatat atggataagg 9120
ctgttcttga aggagactga aatgaacaaa agccacaatt aaatatatgg tagaggaaaa 9180
ctttcctgaa cctattttat gaaggtttat atacttattt ataatcctat tacacaaaga 9240
cctatttgtg tagatttgaa gggtttctta gttccaacat agctttttaa gagaaaaaaa 9300
catttaaaga tgtgctgaga ctaaaatata gatcattgct ggagtgcatt tgggctgcta 9360
tagtaaaata cactaaaatg ggtagctttt aaacaaaaga aatttattta aaacagttct 9420
ggaggttggg aagttcaaga tgaaggcacc agcagatttg gtgtctgatg agggcccatt 9480
tcctggttca cagatggtac cttcttgctg tgtcttcaca tgtgaaaggg gtcagggcat 9540
ctctttgagg cctcttttcc aaaggcacta atcccattcc taagcgcaga gacctcatga 9600
cttaattatc tttcaaaggc tataccttct gatatcatca ccctggtgat tagaattcaa 9660
catataaatt tgtgggtgac acaaatgttc agaccatagc aaccacccag caggccgtgt 9720

```
tgaaaaatgt gaagattagt ccaagcaact gagttgtgta ctaagttgag agcatttaaa    9780
aagtagtatt aagggctggg tgcggcagct catgtctgta attccagtgc tttgtgaggc    9840
ccaggaggaa gaatcgcttc aaggctggag tttaagacca gcttcagcaa tgtagtgaga    9900
ccccatctct acaaaaaatt aaaaagtaaa tttagccagg tgtggtgaca cgtgcctgta    9960
gtcctagcta ctcatgaggc tgagatggaa ggattgcttg agtctagttc gaggttacag   10020
taagctatga ttgtgccact gcactccagc ttgggtaaca gggtaagact ctgtcccagt   10080
aaataaataa taaagataat tattaaaaag gaagaaaaaa ataaactgag cgttcagcac   10140
cagaaaggaa agaggataac aacataaacc caaggaagtc agtagttaaa cccaaggatt   10200
tgactgtaag caaaataatt aatgagatac acaacagtaa aatgaacagc attgataaat   10260
acatcctgga tctgattttg caaattattt ttttaaaaag tatctacagt actagggtaa   10320
acaaatgggt aaaatacagt aagcaaatac cattaacaat ttcagttcag gcaatttgaa   10380
atctcaacaa aaagattatt acataaaaat tatttaaaga taaatagaat agtagctatg   10440
gaaaagactt gttgtagggt gagttctcag gaagtagatg ggggagattt aggagtgcaa   10500
acggttcact ggggagtaac atctgtgaaa ggaaaaggtt gagaagcagg atagggcagg   10560
ggatgccaac agatcatgat gcagacccta caaagtcttt gctatcccga cgtggatctt   10620
gggaaaaagg tggcccatta gagaagctac ttattaggcc cttgctcagt cattgggcat   10680
ggtacaccat gagaagagca taatctcagc tatcacctct ttgctcattc attggctgaa   10740
aggatgcccc gagaggaata tgaacccact tccaaagcta cacaggaccc tgaaggcgct   10800
aaataccatg ggctattagc taactacatt ctttgcagct ggtcagtgtg ttttcttgaa   10860
agggatctga atggcacatt tccatgtctt caatagtcca ccccttgctc cgtgtggagc   10920
tatttcccca aacttgttga ggtagcagca cctccaagtg tctgatgggc ctctttgcct   10980
gcaggaaaac ttagaagagg aagactagca ggatgaatgt caggcttcat cacaatagtt   11040
ggtctcagga ccaaaaatag tactcaccct cttattccct tattatacag gctaattctc   11100
taaaccctca gtgaacacct ctgccagtct tgctaatgta cctcatagtg tgtcccaaac   11160
cctcattcct cacagatctg tgtttttgta accgtactct tctttagttg tgatgtttgt   11220
ccattcatag ccatagttgg acaaggaagt accaagaggc accaagtggg tcctgtgagt   11280
ttcataaatc ttccttcctg cccccattta attacagcag ccctatctcc tcctgttaat   11340
tatcagggtg gattattcct gaaaggatgg tgattgcact tcttgtctgc tgatttctag   11400
gcgtaaggac cacagttttc ccagacaaca accatggctt gtagttcaat ggaaactttg   11460
caagagtgca gaacctctaa ccctgaagag cttaaagttg tgtgaagggg ggcacaaagt   11520
gccacaggga gtcactgaca gtgatgataa gtggggtcac ttctgcttcc atcttcttgg   11580
```

tccatggacc catgtatttt tcgtgttagt ggcaaagcac tgcataaaca tccctgatcc 11640
aatgcatata ctgcatcctg aataacagtt ccgtattctt gcaaggcact gtcccaagtt 11700
ggtgccttag ctgcgtcttc attgaaacat tccatacttt agtaggaaac ttctggatgg 11760
tgcagtatgt gatatgccca tatgcacctt cttcactgta aactaaatgc cctggttgaa 11820
tgttgcatgg tattccatgc ctatgaatca ggcattccat aagccctgga tagccatact 11880
ggctgaggcc ccaagggcag gaaaggaaat gacatataca gactagatgt ctattcctaa 11940
ggaaatgaac tactcatccg tccagaaaga agaggcccaa cctaacaatc ttgctaccaa 12000
gttgctagtt ggtctcatca aggaatccag gggctgaact ggtctctgtt ttagcagttt 12060
gaacgctcag aaataacact agctagatca gctttggtaa gtggaaattc catgctcttg 12120
ggtccgtgca gcctccctct ttgccatggt ggccacttca ttcatttgtt cattgtgtta 12180
gcactggtag cgctgctaac aaaggctgac tcagtgttcc tgcttgagaa gataagcagc 12240
aattctacct ctttcctctc ctttgcctgt tggaagaatt atggaagcgg gggccaggct 12300
cccgcctgta atcccagcac tgtaggagac tgaggcaggt ggatcacttg aggccaggag 12360
ttcgaggcta gcctggccaa catggtgaaa acctgtctct actaaaaaca caaaaattag 12420
ccagacatgg tggcacacac ctatagtccc agctactcag gaggctgagg caggagaatc 12480
acttgaagct gggaggcgga ggttgtagtg aacctagatt gtgctactgc actccagcct 12540
agccaacaaa gcgagacttc gtcctaaaaa aaaaaaaaaa aaaattacag acagggagga 12600
tcgtgttcat tcaggttcat cttgggattg attatggtac tcaatttaat acacttgcca 12660
aaacttgtcc ctaatggtca gatccatgct ctctcaatct aaatttctac aagtcagtgc 12720
agatctctct ctgggaagaa gtctagtaat ttctattgtt aatctttcag aaacctaaag 12780
tctcagaaaa aggttttaaa gataactttt catactcctt cctttctaga tattttctta 12840
gaaattggaa ataaattatc ctcatcagca aatcctagta acgttcaagt gctatcactt 12900
tctttcttct gatcatattg catcttccca aggggtttaa agtgtgctct gttactgttt 12960
caaactcatt acagtcaatt tgtattctgc aaattagcag tgattttgca aggtcacctc 13020
ttgtgtgcat agtaatcctc tccaggttgt aactgtctta taccaaagtg gttcttactc 13080
taggatttgt aatctttcca tggtgcagac atgaggtgcc caagggttcc tggcaatcat 13140
ggatgaggac tccacacttc gtgggtatta taggctgaaa catttccctc tctaaattcc 13200
tattttgaaa ttctaaaccc cagtacctta gaatgtgtct gtgtttggag ataagtcctt 13260
aaaacagtaa ttaaggtaaa tgaggtcata cggatgggtc ctaattcaat atgactcata 13320
tccttataaa aagaggagat taggacatag atatacacag atcaaggaa gatgaaggga 13380

agatacgagg agaagacaag ccaaggagaa tggtctgaga agaaacaacc ctgccaacac 13440
caggaacttg gactgccaga ctccagaact ttgagcaaat aaatttctgt tgcttaagcc 13500
agccagtctg caatatttca ttatactagc cttatcaaac taatacagtg ggtttattcc 13560
ttttttattt atttaaatta ttttaaaaaa tagagacgaa gctggtcttg aactcccggg 13620
ctcaagcaat cctcctgcct cggcctccca aagtgccagg attataggtg tgagccactg 13680
aacctggcca gtacagtaga tttgaatcac catcaataaa cttagatata caagagtatt 13740
tttctttatt tcagattaaa ttcctgctct ggtaccctta ttcttcagag ttagtgggaa 13800
aacaggagct cagctctttt ggagttttct ttcattttta gggaacagtt acacatacct 13860
acccaggtca gtcagcattc cagagttttc tgtagttgca acatgttgga gaaggtccag 13920
ctagttttgg tccttagagg ttaactttgc tacttcattt gcccaagtcc attgccacta 13980
tgccattcat gggacatgag catctgtgac taagctattc tagggcacat ctacctaaga 14040
gttcctatcc cttgaagtct tttattctcc agggtctttc tgtctcccca gcttcactgc 14100
tgggaagcag ggcagatgag ctgtctgctc tccaggatac acagacctct gtttatcact 14160
ctttcacttg tagaatatct gtttttagtc tggggaacta aggtctattc atgccacttt 14220
ttaaatccct ttctcctaat ctttctgtaa agatttttgg ctcttctgtt ctctgtcctg 14280
tcatgtactt tcctgagcaa taagcacaga gcatattact attcgacaat gagagaggga 14340
aaagtggaaa tacagggaga agacaatatg gaataagaat ctcaaaatag agtcctgtca 14400
attaattttt ctatcccact acagacagaa aaactataca cactgattat caaagtatat 14460
tttatggtta catggaatta ggatactatc tatgagaaac cagaagcttt atgcatagga 14520
acaaactact aacttgtttc ttataatttg tcttaggaca tgtttatcgt gtgtcttggt 14580
tttacctagg agtacaatca ctttctgctg ttaggtgtta tagtgataat atgtggttaa 14640
tcacaacagt agcccccaaa ggatctaatg gctgaacatt attccataat agtttgtttt 14700
agaaatatta ttgtatggat agaccccaat aatttcattt accttttttt tttttttttt 14760
ttgagacata gtcttgctct gtcacccagg ctggagtgca gtggcgtgat cttggcccac 14820
tgcaacctcc acctcccggg ctccagcgat gctgcctcag cctcctgagt agctgggact 14880
gcaggtggtg ccaccacacc cagctaattt tttgtatttc tagtagagac atcattttgc 14940
tatgttggcc aggctggtct cgagcgtctg gcctaaagtg atctgctgcc ttggcctccc 15000
gaagtgctgg gattacaggc atgagccatc acaacctgcc tcatttgcat ttaaaatata 15060
ttattgaggt gtaatttatg aacgttaaat tctaccaatc ttaaatgttc agtgcaatgt 15120
cctgcatctt gctttgtgtg gtggttacat atacccatgt tagtggaata catataccca 15180
tgcaatcacc acacaaagca agatatacag gatatttccc tgactgcagt ttccttgtgt 15240

gccttttcag tcaatcccca cccccaaggc aatcaatgct ctatttcttt catcatagat 15300
tagttttttt ctattctaga gctttataaa catgaaatca tgtagtatgt actgttttgt 15360
gtcatttttt gctcagcgtg ttttgagatt cacccatatt gtgtatatca gtagtccatt 15420
cctgatagtg ctaactggtc ttccatttgt gtctttaaaa aaaaaaatca ctggaatgta 15480
ctctgtttct gtatctggca gagtatatgt tggcctctga gaaatgcagg ggtttggcag 15540
agccattcag ttaaatataa tcagtgccta atccagcagt ttctgcaaag gtgtcacttc 15600
tttaacttgt ccacttatag tgagtactca ctctaggggc agtacatttg agtctctcct 15660
tttctctgat ccatccatga tctaggctac agggtgtcat tgaacccctg agattacgtt 15720
cagaaggtgg gtgaatatat gcattagtta agctaaaatt ctcacaattt aatggatcaa 15780
ggaaatttct tttttcatac aagaatccat agtgggtatc catgttagca gagaggtgct 15840
actctacatg gatgcttagg tgtcaggact atggctgctt attatcttgg cttccaaagt 15900
tggtgtgggc atcaccatct tagccagaag ggactcacag cacagaggag cacccatggg 15960
agaattttac aggtagtctc agaactggca gaagtcactt atgctcacat tcgactggag 16020
agaactgtta catgtcctca actatctcca aaagaagctg atgtccaaga gggcaagaat 16080
ggatattgtc actagaggcc tctcctacaa tatgtatatt ctacatttat ctacagcttt 16140
gattaggttc tcaaagggcc gggtctatac ccagaaatgt ttcagagcct gctgtaaggg 16200
catgtgggca ctgctgcttc caccttgccc tagaaggtat actgggccat caaggcaaga 16260
cacaaaaatg acataaaata cacataatgt aggaaaatcc tgctgttgca caggatgaca 16320
taatactgtt catattcctt ggaatactca ttgcagaact tgaggtcgac atcctatgag 16380
ctactagatc tcacaaaaat ccagcatatt agaacaagcc atattttatg tgaaagaaag 16440
gagcaatttt ttgtggtatt tttgtgtctt ttgtaagaga ttcttctcta ccctgaagtg 16500
atgaagatat tccaaaaatc tggaagtttc actttgcaaa tttaggtttt taattcatta 16560
agaacttttt gtttttaaaa aaacttattt aatactcata tataacaaag taacttttta 16620
attaattaat tgattaattt ttaaaaatta tactttaagt tctgggatac atgtacagaa 16680
tgtgcaggtt tgttacatag gtatacatgt gccatgatgc taagataatt ggcactttat 16740
gtagaggaaa aaatatcaga tccctacatc acactatttt ttaaaaggtt cttacagagg 16800
aaaaaatatc agatccctac atcacactac tttttaaaag gttcttatag aggaaaaaat 16860
atcagatccc tatgtcacac tattttttaa aggttcttaa agaggaagaa atatcggatc 16920
cctatgtcaa aacctatgtt taaaaggtta acaacctttt aaaaaatagc gtgatgtaga 16980
gaactgattt ttttcctgta tataaagtgc caattatctc agcatcattt attaagcaac 17040

```
aacacatgtt ttttttttag cttttatttt aagttcaggg gtacatgtgc aggtttgtta  17100
cataggtaaa gttgtgtcat gggggtttgt tgtacagact atttcatcac ccaggtatta  17160
agcccagtac ctattagtta tttttcctga tcctctccct cctcccaccc tccacactcc  17220
aataggcccc agtgtgtttt gttcccctct atgtgtccat gtgttgtcat catttagctc  17280
tcacttataa gtgagaatat gcggtgtttg gttttctatt cctgcattaa tttgctaagg  17340
ataatggcct ctagctccat ccatgtccct gcaaaggaca agatctcatt ccttgttatg  17400
gctgcatagt attccatggc gtatatgtac cacattttct ttatccacat tttctttagc  17460
ccatctgtca ctgatgggca tttaggttga ctgcatgtct ttgctattgt gaatagtgct  17520
tcaatgaaca tatgtgtgca tgtgtcctta taatggaatg atttctgtat tcctttgggt  17580
atattcccag taatgggatt gctgggttga atggcatttc tgtctttggg tctttgagga  17640
cttgccaccc tgtcttccag aatggttgaa ctaatttaca ctcccaccaa cagtgtatac  17700
actgtttttt tttttcctcc acaacctcac cagcatctgt tattctttga atttttaata  17760
ataaccattc tgaccggtgt aagatggtat ggtacctcat tgtggttttg atttgcattt  17820
ttccaatgat cagagatgtt gagctttttt ttcatatgat tgttggctgc atgtatgtct  17880
tcttttgaaa aatgtctgtg ttctttgccc acttttcaat ggggttgctt gtttttttct  17940
tgtaaatttg tttaagttcc ttatagaggc tggatattac acctttgtca gatgcaaagt  18000
ttgcaaaaat gttctctcat tcggtaggtt gtttactgtg ttgatagttt cttttgctct  18060
gcagaagcgc tttagtttga ttagatccca tttgccaatt tttgcatttg gtgtcttcat  18120
catgaaatct ttacctgtgc ctatgtcctg agtgatattg tctaggcagt cttcaagggg  18180
tttttatagt ttggggtttt acatttaagt ctttaatcca tattgagtta attttggtat  18240
atagtgtaag gaaagggtcc agtttcaatc ttctgcatat ggctagccgg ttatcccagc  18300
accatttatt gaaaagggaa tcctttcccc attgcttgtt tttgtcaggt tagtcaaaga  18360
tcagatagtt gtaggtgtgc agtcttattt ctcagttctt tattctgttc cattggtcta  18420
tgtgtctgtt tttgtaccag tatcatgctg ttttggttac cgtagccctg ttgtatagtt  18480
ggaagttggg tagggtggcc ttcagctttg ttgttttaag caaatcatct ttgatgtatg  18540
aatttacatg gacttggaca tttacatgta gttgtttctg aattctcttt cgctacgtta  18600
tcagccactg caccaatacc acattattat tatttttttt aatttgagaa agaagcctta  18660
gattatcttg atagctggat ggaaaatttt ccctccttaa gcttctccag aattctcttg  18720
attattcttg gcttattagg cttacccttg aattttagaa tcaagttgtc aggggctgtg  18780
aaaaacccta ttgcaatttt gagtagattg cattacatat attggaggtt ttgcatccac  18840
aaacacggtg catattcagg ccttttttatg tattttaata aagttttata attttccata  18900
```

```
taggcactgt actttcctgt tagtttccat taaagttata ataaagactt gttgctaata  18960
tgggccaggc acagtggctc atgcctgtaa tcccagcact ctgtgaggcc aaggtgggca  19020
gatcactgga gcccaggagt tcaaaaccag cctggccaaa atggtaaaac cccgtctcta  19080
ctaaacatac aaaattagct gggcgtggtg gtgcatgcct gtaatcccag ctactcagga  19140
ggctgaggca ggagaatgac ttgaacctgg aaggcagaag ttgcagtgag ccgagatagt  19200
gccactgcac tccagtctgg gcaacatagc tagactccat ctcaaaaaaa aataaaaaaa  19260
ttaaaaaaat ttgttgctaa tattgagatg gtatttgttc ccttgacctt gaccccccttc  19320
atggacagga atggagtggc tcatttcact cagcctgcag tccatggatg gctaagtgtt  19380
aacagctcag tgaagggtca gggtgacagc ctcctgcacc tgcccttttt gaccctgagt  19440
tcttgtttgg tgtccaggga gaatcaggtc aaatgaactg tttaaaagat gaatgcagaa  19500
gactttattg agcagtggaa gtggctcttg gtgaaagggg agctggaaag gggatggtgt  19560
gggaaaaagg tgatctttcc ctgaagccca gctgtctctt gctgggcacc cttctgaaac  19620
tgcaccctct gaagttagcc acatttatcc atagcctctg atgcttagtt gcctctctgc  19680
ttgctgcttg gctgcttgta tccccgccag tcagctgctt gtgcttctct tttccttttt  19740
tttttctgcc agctggtctg gtttttatgg gcacaggata gggggcaggg tgggccaaaa  19800
ggcaattatt tgggacgaaa aatggggtca gctgttttca cttagggcca aggttccagg  19860
cttgagggtg aggtttagcc agaagcccag cccttcagga tcggctgttt tcacttaggt  19920
taggggttct aggcttgagg gtggggttta gccaggagct cagccattct gtatcaatat  19980
gtgtgagagg cctttcaaaa attatttttt ctaagggggt gtggcttttt tgcatattga  20040
tctcatattc aacaatgttg ctaagctctc tttattagat actttattgg atttgcttgg  20100
atttcttaag cagacaacta tataacctat aaatcatatt tttcttgttt tctaactttc  20160
ataccttcta atttgttttt cttgtcttat tatattgaca agtggcttca gtacaatgtt  20220
gaatggagat ggcaaaagct atctttgttt tgttgtcaat tttgaaagaa aaatattgaa  20280
tgaattaaca ttaaaaataa tgcttctgga gttgggttat tggtagcaac tctatcatgt  20340
tcaggaaatt cctccctatt cctactttgc taagataaaa aaatgagttg tgaattactt  20400
tacattttca tatatataca tacgtatata tgtacataca tatatacgta tatattacac  20460
atatacatat atacatatat gtacacttac gtatatgtgt acatgtatat acatatatat  20520
atatgaaaat gtaaagtaat tcacaactca tttttttatc ttagcaaagt agtaataggg  20580
aggaatttcc tgaacatgaa acatgtacgt atatatatat acacctatat acgtatatat  20640
gtacatatat atacgtatat aggtgtatat atatatatgt gtgtgtatat atatatatat  20700
```

atatatattt tttttttttt tttcccctgg agtctcacac aggctggagt gcaggggtgc 20760
gatcttggct cactgcaacc tccacctccc aggttcaagt gattcttttg cctcagcctc 20820
ctgagtagct gggactacag gcgtgcgcca ccacgcctgg ctaatttttg tatttttagt 20880
agagatgggg tttcactatg ttggtcaggc tggtcttgaa ctcctgacct cgtgatctgc 20940
ccacctcagc ctcccaaagt gctgggatta caggcgtgag ccactgcatc ccgccacatt 21000
ttctaatatt aagccatctt tacatttatg gtatcaaccc aatatgttta ttatgcatat 21060
tgtgaaaaca cattgaaatt ggtttaataa tactatatgt agaatttatc atttttttatt 21120
ccttattaga ttggtctagt attttctttt ctatggcttt gtcaggtttt ggtatcaaaa 21180
ttttattagc ttataatgga ctggggtgct ttttttcttt ttctatgctt tgcaaccatt 21240
tgaatagagg ttatttgtcc atgaagattt ggtaagtctt accaaccaaa actttgaact 21300
tttttgcata tttatttaga aataagaatt taaattattt aatggtttta aatacatttg 21360
tattattttt gaattaatgc aattttacaa tttattaaaa agtatttcag ctctgtaatt 21420
ttgctgtttt tcaagtttta aaaatactga gctttgtatt atttaaaaat tttgcacata 21480
aagtaaaatt ttcttgatgt gtttatcata catttaaatg tactttggcc aggtgccgtg 21540
gctcatgcct gtaatcccag cattttggga ggccgaggtg ggcagatcac ctgagatcgg 21600
gagttcgaga ccagcctgac caacatggag aaaccccgtt tctactaaaa atacaaaatt 21660
agctgggcat ggtagcgcac gcctgtaatg ccagctactc gggaggctga ggcaggagaa 21720
tcacttgaac ccaggaggtg gaggttgtgg tgagccaaga ttgtgccatt gcagcctgga 21780
caacaagagt gaaactccgt cttaaataaa tgtacttcaa ttaaaatcat acaaaatgta 21840
actgtactgt tttgctaaat tacattacag agagtttgca tgagtttaca cctatttttc 21900
aagtatcatt atttaaaaga aatctttgtc aacaaaggaa aatgtaaaat catgtattat 21960
tttaattttc atcttttatt gaattaaaaa ttaatttatt gaagaaagta tttcagccaa 22020
gtttttttaat gttcatagta ttcttaggat ttaaaaaatt ctactgaaac tgtaattata 22080
atctccatat tcatagcctt cttttctctt ttttcattgt caattttacc agaaatttat 22140
ctatcttgct attgttctat agaactaggt ttttgttttg ttgacccttt ctatgatttc 22200
tttgtatcca gatccatttt tagctttata gttgtatata taacacacat atattttttt 22260
ccttttcctt gtctttcttc actctgtttt agatctaagc cttttttatt ttggatgagg 22320
gagactacag agaaaggatc tgctagcctt gacctacttc tgatgttacc cagagtattc 22380
atgcagatgt atgtgataag aaaaaggtga acacagagtt actctgtttg gggctttcat 22440
tgcagcctag tccttgcagg aggctgctgc atgcaaaccc aaattctctc cattcttgga 22500
atctccttat gccacctgca ctctgaagag tgttattatt attacttttt actaaattgg 22560

gctgccccac ccacctttgc cgctggactt ttaaaataga aaggtttcct aaggagaatg 22620
ctttttttc ccccctagcc aaggggaaaa acactggaga ggcacaaata ccccatttca 22680
aggaggaaga aaactatgcc tggaggcatc atctgcaaaa ctttccggaa aaggctgagt 22740
tctctcaggg tattgtttca ccaaaatact gtctttgaat cagtctctac cttaatagac 22800
tggagggaaa gtttccaaca gatcgttttg ctgatagttt tgattactct cgaacagaaa 22860
atttcaaaag ctttttgcca gagggaagcg aacagaggga ggaagagaga actcttccat 22920
gagtgtgcca tgactgggga accttttgaa caaataatag tgccaaaggc agtgacagcc 22980
aatttttcca cccgaaatgc ttctcaaatt atagtccttg gatcactagc ttatatcaat 23040
agtctgacaa agtctatggt gtccttccag atattaatgt aaaacctgaa cttgaaatat 23100
ccatcacaat gtttcatttt gctgaacttt ttctgagaca cagtcttgtt ctgttgccca 23160
ggctggagtg cagtggtgca atcttggctc actgcaacct ctctctccca agttcaagag 23220
attcttatgc ctcagcctcc caacagctgg gactacaggc atgcaccacc atgccggcta 23280
atttttatat ttttagtaga gacagggttt tagtagagac agacatgttg gccaggctgg 23340
tctcaaactc ttggcctcaa gcgatctgcc caccttggcc tcccaaggtg ctgggattac 23400
aggtgtgagc caccatgcct ggcttcattt tgctgaacat acatatatat ttaaaaattt 23460
taaaactttt taagagacag ggcctggctg ttgcccaggc tggagtgcag tggcacaatg 23520
gcagctcact gcagcttcga actcctgggc tcaagcgctc ctctcgcctc agcctcccaa 23580
aatgctggga ttacaagcat gagccactgt ggctaagaat attttttgtc atgtttgaca 23640
gcgccagctg tctttgggtc tttacctctg ctgaagtcat ttgaagaaac aaatctaaag 23700
aaatactgct tggcagaggg gaggagagaa aggttaggac agaaagcatc aggatgactc 23760
tccaagaaag ggaatgtctg aatgacaaga ggactacaga caggagaaaa cattcaagaa 23820
aatttatcaa ggtattcttt gttcctgttg aaacctcatc atcaatgtta gtgtcttcag 23880
aagcccaccc aaatatatta ttttactttg ataaaagaca acttggaacc atataaaaga 23940
ctgcactggt accattattc cattttacag atgagaaaag tagctcattt ggaacataat 24000
gagctaggtt ttatggatta atctagatgc tgaactgaat tctgtattgt atttcatttt 24060
ctctgtcctt ttctactgga tgtacatacc tacatttaat tttagagtta tcgatctaaa 24120
gctggctgca gaagctgtct catttagaca ggtccacgct tgtcaggaag acaggtctgt 24180
ccaggcagac ctttcagttt gttctatatt tcatttcagg ccacagcaga ccaaataaag 24240
aggaaatgaa gtgttttata gcaaataact tactgttgaa tacacatgtc ttgtacggac 24300
aggcctccca atttgatgtt agaaggggcc acagagtagt taagatagtc aaaacagaag 24360

```
caactgtatt ccttaagaag gtaagagaca aaatcaacat attaaaaata ttcaagttga  24420
gtggtaggta agaaactcct tttactttac ctttgattta tggggaaagg ctaaacatgg  24480
aaataattaa ttttaaaatg tctttatatt agatatagaa gtattggtgt taagaaatct  24540
gatagtaagt acagtgtata ggaagaaaga gacgggaaaa ttagctaata agtgattttt  24600
tgacttgagt agagctatgg ttaaaaatat atggaaacct ctttcgagta tatttttttc  24660
ttaccaatat atacttttta tgatactgag attttatagt gaacatttgc ctattcatcc  24720
caatataata tttcttatag tttggtatca acccctatga ttatttgtga gataagggag  24780
tggtttgtat tcatgggtat acatatctat acacatttct gcatgccgta cacaaacata  24840
agtagcattg tgcatgaaca tattagcact gtgcatgtac taaaatttca attttttcac  24900
atagtaggtt ataaaataaa tcatatattc gtaggaaagg tatagaaagc aaagttattt  24960
aaagtggatt tttaaaaaag atttgaatga ttttttttaa aaaaaagatt agaaccattg  25020
tttttaaaag gaaacagctt tagatagtaa attatttaca gccaggactc agccctgagc  25080
atcctgtgaa aaccacacga gcttggtcaa ggttctaagc agtcaccggt gggagatggt  25140
catggtcccg tcacagcata actcacttga ggcttgaaaa gtttccgtcc agctacggtt  25200
tgcagtgacc tttctcttct gcaaagccca cggttacctg gtggccacca ccaacatagc  25260
cccgcctcgc tgtcctgcgt ttctctcaat cagatcagtc ctgccgagtc cccatgcctt  25320
actgctggtg tgctgtgtcc tctgtgccat ttcaaggcga gggctcatga aacttttact  25380
cacacgctgc tatttttgat atgcggtggc cttagctgtg gagcgagagg ccgataattg  25440
tgatcactcc ggaaatcctg ggaagccaga agtgggagat gggtgttgtg aggtggcgtg  25500
gctagggaga ccccatcgag tgggtgtgtt ataagacctg ggcgaatccc tgtggctgcc  25560
actctcctga gaatgttccc taggccttag tcccgcgccg ctcccaccca cacctccagg  25620
tgtgcagtcc ccgcccttaa ttactctcac taaaattgat agtttacact tgcaaagcta  25680
cactgggaaa gcggaagaga aatttataat cgtggatatg gagaactagg ggagcagaca  25740
cacttgcttt cgtttacaga tccagtgaag tgaaaaatca gaactagaaa cgtatgcacc  25800
ttcctagcag caaagccgct tctgcgttct tcgcagcctc cagtgcaggg cggcgctggg  25860
agaaactttg cgccttctgg aaagtttaga aagtgagcca cgaaagagag gccacatttc  25920
cggggttttg cgggccccgc gatgttttcc agagcttttc gagtgggaag aggagagcga  25980
caacgtgaaa atgccccgtg ccggggcgtc caccggagtc ctgccagctg tccggcgctg  26040
gggtaagcgc aggaggggcg ggggtgggac ccgagctggc ggccacgggt ctcccgctgc  26100
gggtgtgtcg actcgggggc ggggcggggg aggctgctga gataatgaat gggaggctaa  26160
ggccaccccc cagccccggc cctgccacca ccgtgggctg tcgagccaat gaatggagga  26220
```

gggggcgcag aggtcagggg cgctgggggc gccacaccag gtaaggggtc cagcttggga 26280
gcggggaggg cggactctgg gggttcgggt gttgctgact tgtgctacgt ggaacaagca 26340
gaaaagaagg aaggcggagg aagagaggag gctggcgtcg gcgctgctcc tctggccctc 26400
cctctgcgcc ccctcctcct gccagcgcgc caaagccggg cagtaggggc cgactggcgg 26460
ctgacgctcc ctgagtggcg actccgtctc cagccccgct gcggagcgcg ggccggatct 26520
ggggcggcca gggcccggag ccgcggagcc ctccccgccg cccggccgag cacgggaccc 26580
cggcggggtg ggcgcagggg gcggccccgc tctgggcgac tgccgagggg cgggcggagg 26640
gccgggctcg gctggcggtg gggcgggggc cgccgggact gggcgcgcgg cctgaagcca 26700
gcccgggggc ggcaggagag ggacgcgcgg cggcagcgag cgcaggtaag gggccggcg 26760
cggcgtgtgg ggacggcgcc ccctgggcgc gaagccagag gccgcggcga ctgctcggcc 26820
cgccacacgg cgcgctgggc tcacactgtc ccgccgcgga cgggctttgt ggttgggggc 26880
gcgcgtgcga gtgccagtga gagtgtgggt gcgcgctgtg ggccgcggcg cgggtgggtg 26940
gccgtgcgtt cttgcgagcc ggcctgcagg aggcgaggct cccctggcct cccgcaccca 27000
gcggcggacc gagcccctgg agggaagttg ccgcagccgc ccgggccgcc ggccctcctg 27060
tcccgcgcca ggtcagtgcg ccccggggcc cgcccagtga cacgtaggtg gcctccggtt 27120
acctgggtcg gggtgggtgc gcgggagggc ttgcgagggg ccctcgaaat tctcccaaaa 27180
cctcgtcccg ctggacttgc acctccgagg ggtccctgcg cccctggggc tccgcacctc 27240
tcgcttccca ctgacgactc ggttgtcccc caccaccggg ctgaggactt ttaactaagt 27300
ttctgtccag ccgcctaaca aacgtgctta acaagtaaca ggaggttgta aggcaaggga 27360
gggacctggc tttgaaagtt gatggttttg aaatcgacgg ctcctttgac ttgcgtagga 27420
ctcctttgtg gtgaatggat ttaaagttta ttcttgtttt tctttgctgc catttggtct 27480
ttgcaatgtt aagagtgaag gcattcagga cttgctgctg cctgctgtct ggggcctgag 27540
gttgtggctc tccgcttctg ctctcctctc ttcatttttg ttgaaatccg tggatttttt 27600
cataaatgtg ggtgctggct tgtttcacta tggacgcgtc gtttcctatg gggttagata 27660
gatattggta aacataaata ttgaaaatgt gatgatcaca tattgatgat cacagcgaaa 27720
tcctctattc cttcaggctc taacagtttg tttttcacgt atttgcataa gtgtgtttgc 27780
tcagctaagc ataattggta agccagaggt ctgtgattca gagtctgcag ttctggttaa 27840
ggggagactg tacacttgga aaatgcgaag ttttttaaga agttattccc ttgccggata 27900
gaagagtggg tcccaaggaa aaagggtgta ttttagaggt atggttattt gaaccaaact 27960
aaacatcatt aaaatacaca gggaggtaga atgtcaccta gttctttctg cccttgattt 28020

ccatatccca gtaggagcga gtataattgc cccaaatgca aaattatgaa taataatcag 28080
cattgtaagt atattaaggt tgcatttttt tcttgatact tgtctgattt gaggataaaa 28140
acaccaagtg gcaagagaag gaggtagctt gaggcaggaa ggcacgggcg aggagcgagg 28200
agggaaggct gggttgtcat catccgagcc cgcaaggtac accacgtccc ctagcggcgc 28260
gacctcagcc agtcgggtca gctcagatcc tggtctccgt ctccccatcc ggaaaatgga 28320
aggggttggt gatcgttgct gttccttcca gcttcccaaa tagcgagaaa ccggtgggag 28380
gttatcttat tgtgttagag cagtaggaaa aggtaggagg gggaagtgag aggtccagag 28440
ggccaggaag atggaagaaa acaagcaaca gaggatgctt ggaggagaaa aattcttgag 28500
ctgggagctc atttcctctt tttttttttt ttttcctttc ctcaagggac tcaataatac 28560
ttcccccacc tcagatcaat ctcttgttac ctttattatt taaagcttgt gagtttgctt 28620
agctaatgca ctagggtgag gtcaaggtga tccttgcagt ctgacttcta ctaggcatca 28680
cttgactcat ttgcatcatt gtctctcaag ggaacagctc ctgcccaggt ctgtgggcac 28740
tcagcatgga tttcagtctc cctgtgagtg atgggaaaga actaacagag gtaagaatgt 28800
aagtggcagc cctttcgaaa cttctatttt tgttcaaacc taatattttc caaaaagtga 28860
tttgattttt ttgttgtttc aaattatacc gtaggctgca aggttttact gaatctattc 28920
cattagtgac ctactggaac gttcaaagaa taaaaatctc acttgctcag tgtttttgat 28980
acacagtgac attcagtctg aagtaagtga tatttagggc caagaatcat ttcaaatgct 29040
tagtatggaa agttgcaatc tgggcagaat acactgtatc attttcactg ggaagctcca 29100
agtattcagc agataacaga actttcagaa tttagtttga ggtcaagatt ttaatgtctg 29160
tgtttgatgt gtggcctgtc ttccttttct ttgatgtttt ggtatcaata tgcctacacc 29220
cttgaggaac attatttatt gtaaaagctg aactgtgatg taataaaaac ttaaacataa 29280
gctcttggtt taaagtccaa tagtcttctc tggccttaag tcagcatcat actactgttt 29340
ggtttcttat ttttacatat catgttaccc tcttatttaa atatcccggg ccaggtgtgg 29400
tggcttatgc ctgtaatctc aacactttgg gaggctgagg caggacgatc gcttgaagcc 29460
aggagtttga gaccaacctg ggcaacatag tgagactctg tctctataaa aaaaatagaa 29520
acattagctg ggtatggtgg catgtgcgtg tagtctcagc tacttgggag gcggaggagg 29580
gaggatggct tcagcccagg attcaaggct gcagtgagct gtgattgtgc cattgcacta 29640
cagcctgggt gacagagaga ctctgtctct taaaaaaaaa aatcttaggg aaaactatta 29700
tggatgtctt agaaagttga gagaaaagga ggtaattcta tttcagcaaa caattattga 29760
attccttcca tgtggtagat gcaaggatgg tgaaaaggaa gctccagaaa gaactgcaac 29820
acagggggga aagatgcagt gagcctcacg gttcataccg aatatgtata tgcagtgttg 29880

tgtaactggg tggtagctgt agagtccaag aactttggca tgccagggaa tggagaatta 29940
cgttttgatt caggggacga atttataaat gtggcttgaa gaataacata tcttttaaaa 30000
gatgaatggt gggggaatga agacaaagtg agagtgtggg gtgatgaagg ctggaaagtt 30060
acgaagggac aaattgtggg ggacctctga tgttatttta aggagttatg ctacatgggg 30120
atagggaggc aaggaggaag tattacataa cctcaagatg tggaaagaca ctgagtaaca 30180
catattgggg cagagtgagg ggtgcgatga tggatggagg actgggcttt tattccatta 30240
caatccgtgt aacttatggc tgctacgttt taagacacta ttgcctgttt actcaaatta 30300
tataaaggtt gtagaataaa ctaataagta gtttcttcct ccctactctc cgcaaattga 30360
tagtgcttta tacttttgag aaatttaatt tagtaaaaat taatgatgct attgtgttat 30420
agctggacct tgtggagcct tttgaacatg tggggttgaa gtcattctgc aggcacagag 30480
ctgtccaaga aaacattttt tttcccctct ctttttttgtt tagggaaggg cttgctggtt 30540
aatgctaatt taaacatgac tcttctggca gctggcattc ttgaccctgt ttatgttata 30600
catggtattt aaccacagtg attgggtatt tgcagcacag aagaaaaaga attattatta 30660
gtttgaaacc ggcattaatg cctctgtaaa tgatagggca aggcagtaga tggaaggaga 30720
gagggaagcc aagtagcaca ctcggtactg cagtgagaga tgacataacc atgagaattc 30780
tttaagttta acttccagta gaagtaactt gctttctata tattttaaat ccctagagct 30840
aaagcattta actcattatc ttcactctgt gggatccatt tgggagaggt attcaggagc 30900
tttataggtt cacacttgct ccccagtacc tctgtgtcac aggaggatat acactggttt 30960
tcagttgcat gtcagaggtg gaactgactt ggatgtcttt gaattgctgt tgaatctgga 31020
gatgctaggg tacccaggag acagacagga aaaagaagag gctgggcacg gcggctcatg 31080
cctgtaatcc cagcactttg ggaggctgag gcgggtggat cacctgaggt caagagtttg 31140
agaccagcct ggctaacatg gtgaaaccct gtctctacta aaaatacaaa aaattagcca 31200
ggtgtgatgc tgggcgcctg taatcccagc tactcaggag gctgaggcag aagaatcact 31260
tgaacccagg agggagaggt tgcagtgagc caagattgtg ccattgcact ccagcctggg 31320
cagacagagt gagactccat ctcaaaaaac ttaagaaaaa gaaaaagaaa aagaaggtgg 31380
ctgaatttct ttcaattacc ttgtgaattc aatttaatga atgatcttcc cagcagtttg 31440
ttttatcttc tgcaagggaa cttatgtttg gcatgtttaa taaattaagt taattaagtt 31500
ggagaagccc aaggttagta tactttattt taggatacat ttttggtagg agaggaggag 31560
gggtggcatg gtggtggtga tgattttatt taaactcttt ggcatttttt agcaggttag 31620
tatgatttca aagtagtgtt gttgttgtat tatcaacttg tggggctggg ccaaagaata 31680

```
ttgtcttaat acttgcatgt ccctgtcatc taatcagtgc tatagaaact ttaaccctga  31740
agtccatgta aaattgtaat tattttttttc cagaaatgaa agagaactat tttactacat  31800
tcatggattt aggttataat ttattttatt tttgtgatac tgttttaaaa aagtgatata  31860
atgacagggc agacccttaa ctttagtcca gtgctaaaac aaacgtgcaa taagcctgca  31920
tgaggcaggg caagctgtgt ttgttgtatt atgaaaataa aggaaaatgt tttcaaaacc  31980
agcatttttc tctaaaagaa aaaattttga ctaataatac ctggccatgg gtgggatttc  32040
cagctgttgg ttgaaggaaa ttttgttcat tatggccatt atgtgtgcta tgtctcttag  32100
agtttaggat ttgctatgct gagattgcta ctgtgaacca ttcctgttat agcaagtttc  32160
cctatattca ttaacttatg tgtctaaaca atgctctaga ttagacttta tatcgatctg  32220
ttctactaaa ttttcttccc ctatgcctag gtggttctct ttgaccaacc cttaactgcc  32280
ctgattctga aattctgctc taattgaagg atattcctgg gtctttggag ggagaaatgg  32340
ttcaggggca gaggaaactt tttttccccc catctcagga gcacttaact gactgcctgc  32400
tatataccag gctttgtgct agttttacta ggggtgagtg tgaataaggc aggattccca  32460
ccctccagga ggagcttaca cacgattaac agataattac aattcagtgt aacaagtgtt  32520
atgagagaga agactttgcg ggagcaccaa gcgggggcac ttgaccctca gcggtgaggt  32580
ggtggaggtc agggaatgct cctggaaggt taggatctgg aaggatcctg ggagataaac  32640
caagcctaga agaggtaaaa aagggatcaa gaggctttct atctccagtt tcgctactcc  32700
tgggtaggtc aaagtgcctc tctttttaca tctggaaaat aagaataaga actgatatct  32760
ggttggtaat tctagttttc atttactatg tgttccttaa tcctttatat tactctttct  32820
tccatattca gtaggcattt gtacccaccc tgtactggga agatagcact gcaggaaaca  32880
agatgtggac agaccccttc tctcatggag cttgccctct atcatatgac attagtaaga  32940
gaagttcttt tagaatgcct tcaaactcag gcatatgtat cttgttattc ttccatttta  33000
ggaacatttt ctccaaggta gtctcaatac tttgaggtga gttggccttt cccttttaag  33060
gaaggagacc acagagcccg gtaatctcct tttcatttta tatgcttgaa atattttgct  33120
aggatttatc tgttttaaag tctagagata gacatagctg tgttttatct gtagccctgc  33180
cttgcttcca tctcattttc cctattctta cttttaaccc aacttgtgtt tgagattctt  33240
tgctcagtat atagtttgct ttctagaata attcaaacct agtgcctgta gaaagtcaga  33300
cttatttttt atttatttga gatagggtct tggagtgcag tggcataatc ctggctcacg  33360
gcagctttga cctcctgggc tcaagctatt ctcccactac agccttccaa gtagctggga  33420
ccgcaggtgt gtgccgctat gcctggctat ttttttcttt tttctttctt tcttcttctt  33480
tctttttctt ttcttttttt ttttcttttt tttttttaa agaaatgggc tctcactaca  33540
```

```
ttacccaggc tgttctcaaa ctcctgggct caagggatcc tctcaccttg gcctcccaaa  33600
gtgttgggat gacagctgtg agccaccgca tctggctaat acttattttg aagtcaccct  33660
gtcatggtgc tttgcttcac taatgtattt cacttatgat cttgaagtca ctcagggata  33720
caaacacaat aaaatgcatc ccaaccagga gagggtagct gcattcattt acaaaatttt  33780
tttttgcaat gattcaaaca ctatagatgt gtctaaagta aatgtaaaaa tctctctctg  33840
ccactttaac cttacccatc tgatatcagt ttgttccata gccttccata gttgtccttt  33900
tacttataca aacctacaaa aattaatatt tatgtatatt tatgtgtgtg tgggataagt  33960
gtggatatgt ttatacatac tcatatgtat gtttgtgtgt gtatctgtct gtgcctgcct  34020
ccattttctt ttgctttaga gaggctatac ttgaggctgc catcaagagt gagaagtttg  34080
aagctggaag agcctgcatg ggcccttctt gaactggtgc agcatgtgca gcatgacatc  34140
actcaagagt tcttgtcaga gtgataatga atgtctggct attgtaaacg ggaataagaa  34200
aactatttcc agctgtgtga caaccaagac gacaaaaagc attgcagaga atattattgc  34260
cacaaggacc ctgcttcatc tgggtctcag acgacgggag gaggggcatt ttggagcacg  34320
tgtttggcat ctgtgaacct tttgttaggt agaaaacaag gcctgaatga aaggcctttc  34380
aaccacttct ggagcagaga agataggtag agttactcat tataggcagg tttcattgta  34440
ggagtattca gtgaggaccc ccgccttgga agtctgtaat cagcagatga taaggatggt  34500
gtgttcttac taagagaata acacaactga aacagaattg ccttttgtta aggggatgct  34560
ttgccttctt ggactacgat tgtggggaga aggattattg tcaactaagt gaggcattca  34620
ttctgtaccc actatttatt gtagttccac agagaactgc ttgctttact ttctgactag  34680
gcacagaaaa gtaaaggttc aaaggctagg gcaagatgac tgacttttcc agatttagca  34740
caatctgttc tctggtcact ttgagacgct gtcagtttag tttcatgcag ctgatatctt  34800
ggagaacttc tgtgtcctta cgtttggctg aggacactaa attttttttt tttttttttt  34860
ttttttgagg cagagcctca ctttattgcc caggctggag tgcaatggca cgatcttggc  34920
tcactgcctc ctgggttcaa gcaattctct tgcctcagcc ttccaagtag ctgggaccac  34980
aggcgtgcac caccatgcct ggctaatttt ttttgtattt tttggtagag atggggtttc  35040
actgtgttgg ccaggctggt cttgaactcc tggcctcaag tgaaccaccc gcctcggcct  35100
ctcaaagtgc tgggtttaca ggcccaagcc accgtgcctg gcctgaggac actaaaatta  35160
aaaaaaaatt aagaaagtac ggtccccgct cttgagtagc tcatgaacat ggagcgatat  35220
ggactcaaat gattaagatc aggtaggtag tgatgaatac ggtaaaccaa gtttcaaaca  35280
aagagctgtc tgcatatctg aggatggaga gggtaagtca gccttagagg agggaaatga  35340
```

cttgcagcag gatgccagcc tcaatggtct gtggagctca ttccatgcag aagagtaatg 35400

aggaagaccc agtgagtgac aggcttgggg aggagtgcct gaaattgacg gtttgtggca 35460

ggaagtggtg ggaccaatct cgaagcttgt ggaggtagaa gggtactgga gactactgtg 35520

gacaaaaaag ggcatgttga tgccattacc agacaggaca cgagagcatg atgattgttg 35580

cagtgaggct tgttagtttt agacatgtct aaaaccatgc aggcagagat tttcagctgg 35640

tggctggcaa tttgagtttg aagtttagct gagaagtcag ttggcagtat tcaggcataa 35700

ttgctaaatg tagaagtaaa tgccagtaaa atgtgtgtga taagctggag agcactttta 35760

gagtgaaaag attgatatat tttaacaagg acaaggctta tttcaattct ttaggttatt 35820

tttctttagc agattaaagt agttttatcg gttatcaagc atttgttgag cgtttacta 35879

<210> 4
<211> 20
<212> DNA
<213> Artificial

<220>
<223> Primer

<400> 4
tgtctcggca tagttctgga 20

<210> 5
<211> 20
<212> DNA
<213> Artificial

<220>
<223> Primer

<400> 5
catttctttg tcgcggtagg 20

<210> 6
<211> 20
<212> DNA
<213> Artificial

<220>
<223> Primer

<400> 6
ttgtcttcct gctcatggtg 20

<210> 7
<211> 20
<212> DNA
<213> Artificial

<220>

<223> Primer

<400> 7
tgcaggactc acagcctaaa 20

<210> 8
<211> 20
<212> DNA
<213> Artificial

<220>
<223> Primer

<400> 8
tgactgtctt aggggcgttt 20

<210> 9
<211> 20
<212> DNA
<213> Artificial

<220>
<223> Primer

<400> 9
ttctcctgag agaagcagca 20

<210> 10
<211> 20
<212> DNA
<213> Artificial

<220>
<223> Primer

<400> 10
ttcctggcca ttgtctatcc 20

<210> 11
<211> 23
<212> DNA
<213> Artificial

<220>
<223> Primer

<400> 11
ggaatgataa acccaacaac ttc 23

<210> 12
<211> 20
<212> DNA
<213> Artificial

<220>
<223> Primer

<400> 12
cctgccccaa tccctttatt 20

<210> 13
<211> 20
<212> DNA
<213> Artificial

<220>
<223> Primer

<400> 13
ccctaagccc ccaattctct 20

<210> 14
<211> 20
<212> DNA
<213> Artificial

<220>
<223> Primer

<400> 14
tccagggaca ggatatggag 20

<210> 15
<211> 21
<212> DNA
<213> Artificial

<220>
<223> Primer

<400> 15
tgaagacaaa tcgcttttcc a 21

<210> 16
<211> 20
<212> DNA
<213> Artificial

<220>
<223> Primer

<400> 16
agtgaggaac aagccagagc 20

<210> 17
<211> 20
<212> DNA
<213> Artificial

<220>
<223> Primer

<400> 17
gaggtgccca tgctacattt 20

<210> 18
<211> 20
<212> DNA
<213> Artificial

<220>
<223> Primer

<400> 18
aatcgggaca ccatgatgag 20

<210> 19
<211> 20
<212> DNA
<213> Artificial

<220>
<223> Primer

<400> 19
cagaggatct gcctgaaggt 20

<210> 20
<211> 20
<212> DNA
<213> Artificial

<220>
<223> Primer

<400> 20
atggtcaccc tcagatcagc 20

<210> 21
<211> 20
<212> DNA
<213> Artificial

<220>
<223> Primer

<400> 21
ttgaccgctg aagagaacct 20

<210> 22
<211> 20
<212> DNA
<213> Artificial

<220>
<223> Primer

<400> 22

tggcacgtat gagctgaaag 20

<210> 23
<211> 20
<212> DNA
<213> Artificial

<220>
<223> Primer

<400> 23
ccagctgcag ggtaggttta 20

<210> 24
<211> 20
<212> DNA
<213> Artificial

<220>
<223> Primer

<400> 24
tggtgctgaa gaacatccaa 20

<210> 25
<211> 19
<212> DNA
<213> Artificial

<220>
<223> Primer

<400> 25
aaattccgca agcaaggac 19

<210> 26
<211> 20
<212> DNA
<213> Artificial

<220>
<223> Primer

<400> 26
tcccaggaat tggaaacaag 20

<210> 27
<211> 20
<212> DNA
<213> Artificial

<220>
<223> Primer

<400> 27
acatgactgg gctcacaaag 20

<210> 28
<211> 20
<212> DNA
<213> Artificial

<220>
<223> Primer

<400> 28
tgggtttccc ttcaaccata 20

<210> 29
<211> 20
<212> DNA
<213> Artificial

<220>
<223> Primer

<400> 29
ttgggtcaaa ctccgactgt 20

<210> 30
<211> 20
<212> DNA
<213> Artificial

<220>
<223> Primer

<400> 30
attttgaaga cacgggcaag 20

<210> 31
<211> 20
<212> DNA
<213> Artificial

<220>
<223> Primer

<400> 31
gggaatttgt tggcatgact 20

<210> 32
<211> 20
<212> DNA
<213> Artificial

<220>
<223> Primer

<400> 32
atcactgcca cccagaagac 20

<210> 33
<211> 20
<212> DNA
<213> Artificial

<220>
<223> Primer

<400> 33
atgaggtcca ccaccctgtt 20

<210> 34
<211> 36
<212> DNA
<213> Artificial

<220>
<223> Probe

<400> 34
ctagctttgg aacataatga gctaggtttt atggat 36

<210> 35
<211> 36
<212> DNA
<213> Artificial

<220>
<223> Probe

<400> 35
ctagatccat aaaacctagc tcattatgtt ccaaag 36

<210> 36
<211> 36
<212> DNA
<213> Artificial

<220>
<223> Probe

<400> 36
ctagctttgg aacataatga gctaagtttt atggat 36

<210> 37
<211> 36
<212> DNA
<213> Artificial

<220>
<223> Probe

<400> 37
ctagatccat aaaacttagc tcattatgtt ccaaag 36

<210> 38
<211> 25
<212> DNA
<213> Artificial

<220>
<223> Probe

<400> 38
ctagcgcttg atgagtcagc cggaa 25

<210> 39
<211> 25
<212> DNA
<213> Artificial

<220>
<223> Probe

<400> 39
ctagttccgg ctgactcatc aagcg 25

<210> 40
<211> 21
<212> DNA/RNA
<213> Artificial

<220>
<223> siRNA

<400> 40
gaaaugagcg ccaccuuuat t 21

<210> 41
<211> 21
<212> DNA/RNA
<213> Artificial

<220>
<223> siRNA

<400> 41
uaaagguggc gcucauuuct t 21

## Description

Technical Field

The present invention relates to identification of genes related to bone and joint diseases such as osteoarthritis and of polymorphisms in the gene that correlate with the diseases, prophylaxis or treatment of bone and joint diseases based thereon, diagnosis of genetic susceptibility to the diseases, and the like.

(Background of the Invention)

Although lifestyle-related diseases of bone and joints have direct effects on life in only a few cases, they represent the major cause of deterioration of the QOL of the elderly because they interfere with the activities of daily living (ADL) due to pain, gait disturbance and the like. Also, because these diseases are characterized by rapid rises in incidence with aging and by a chronic course, they impose a great burden on national medical economics, posing an important problem to be overcome in aging society as a whole. The World Health Organization (WHO) positioned the first 10 years of the 21 century as "The Bone and Joint Decade (BJD)" and begun efforts to conquer bone and joint diseases.

Osteoarthritis (hereinafter to be sometimes referred to as "OA"), a bone and joint disease accompanied by chronic arthritis, is characterized by cartilage destruction and proliferative changes in bone and cartilage due to regressive degeneration of cartilage. The number of patients suffering from this disease is as many as 5 million to 7 million in Japan alone; it is reported that not less than 80% of the people aged 60 years or more exhibit symptoms of osteoarthritis in their knees, elbows, hip joints, and spine, and this disease is a representative common disease. At present, no fundamental therapeutic approach for OA is available, with symptomatic therapies for pain relief, such as administration of non-steroidal anti-inflammatory analgesics, hyaluronic acid, or steroid agents, forming the mainstream. For advanced cases, surgeries such as arthroscopic surgery, osteotomy, or prosthetic replacement are indicated; however, because of the limited durability of joint prosthesis, it is thought to be desirable to avoid this surgery until about 55 years of age. Accordingly, there is a demand for the development of a therapeutic method for suppressing retrograde changes of joints.

OA is a multifactor disease involved by environmental factors and genetic factors. Elucidation of a genetic factor leads to the presentation of a drug discovery target. The present inventors have found to date that polymorphs in the CALM1 gene, which is one of the genes that encode calmodulin, and the gene for asporin, which is one of the extracellular substrate proteins that have been reported to be highly expressed in OA cartilage, correlate with OA, and have reported that abnormalities in these genes are associated with the onset and/or progression of OA (Patent Document 1 and Non-patent Documents 1 and 2).

About half the existing pharmaceuticals exhibit their actions via receptors on cell membrane, and G protein coupled receptors (GPCRs), in particular, account for a major percentage; therefore, GPCRs are the most promising drug discovery target. However, no GPCR associated with the onset or progression of OA has been reported.

Lysophosphatidic acid (LPA) is a bioactive lipid that controls a wide variety of biological processes; as receptors thereof, 5 kinds of GPCRs ($LPA_1$-$LPA_5$) have been reported to date. Of them, $LPA_1$ is also called EDG2 or VZG-1 (Non-patent Documents 3 and 4) (hereinafter, denoted as "EDG2" herein). Because LPA has algesic action (Non-patent Documents 5 and 6), and also because it is suggested that early intervention at the LPA receptor level may be effective in suppressing the progression or mitigating the severity of neurogenic pain (NP) (Non-patent Document 7), EDG2 antagonists are expected to serve as candidate therapeutics for neurogenic pain (NP) (Non-patent Document 8). However, there has been no report of the relationship between EDG2 and bone/joint diseases, including OA.


Patent Document 1: Pamphlet for International Patent Publication NO. 2006/014013

Non-patent Document 1: Mototani et al., "Human Molecular Genetics (Hum. Mol. Genet.)", (UK), 2005, Vol.14, p. 1009-1017

Non-patent Document 2: Kizawa et al., "Nature Genetics (Nat Genet.)", (US), 2005, Vol.37 (No.2), p. 138-44

Non-patent Document 3: Hecht, J.H. et al., "The Journal of Cell Biology (J. Cell Biol.)", (US), 1996, Vol.135 (No.4), p. 1071-1083

Non-patent Document 4: An, S. et al., "Biochemical and Biophysical Research Communications (Biochem. Biophys. Res. Commun.)", (US), 1997, Vol.231, p. 619-622

Non-patent Document 5: Renback, K. et al., "Molecular Brain Research (Mol. Brain Res.)", (Netherlands), 2000, Vol.75, p. 350-354

Non-patent Document 6: Inoue, M. et al., "Nature Medicine" (Nat. Med.)", (US), 2004, Vol.10 (No.7), p. 712-718

Non-patent Document 7: Renback, K. et al., "Neuroscience Letters (NeuroSci. Lett.)", (Ireland), 1999, Vol.270, p. 59-61

Non-patent Document 8: Gardell, S.E. et al., "Trends in Molecular Medicine (Trends Mol. Med.)", (UK), 2006, Vol.

12 (No.2), p. 65-75

Disclosure of the Invention

Problems to Be Solved by the Invention

It is an object of the present invention to identify genes involved in the onset and/or progression of bone/joint diseases, including OA, particularly GPCR genes, and elucidate the functions thereof, to thereby provide novel and radical means for the prevention/treatment of the diseases. It is another object of the present invention to provide a convenient and highly reliable diagnostic method for genetic susceptibility to bone and joint diseases.

Means of Solving the Problems

The present inventors, to accomplish the above-described objects, focused on the GPCR gene family, which is promising as a drug discovery target, to find candidate genes, and performed correlation analyses between a knee OA (KOA) group and a control group using already reported polymorphs (marker SNPs). As a result, the inventors found that polymorphs on the EDG2 gene exhibit a high correlation in two independent populations (general population and cohort population). Furthermore, the inventors identified a plurality of SNPs correlating with KOA by linkage disequilibrium (LD) mapping. One of them (i-EDG2-09) was present in the promoter region of the EDG2 gene; as a result of gel shift analysis, the existence of intranuclear factors that bind to disease-susceptible alleles more firmly was revealed. As a result of reporter assay, it was found that in disease-susceptible alleles, EDG2 transcription activity is elevated.
As a result of expression analysis in major organs and tissues, EDG2 was found to be highly expressed in the synovial membrane and spinal cord. Hence, to analyze the role of EDG2 in synovial cells, OA synovial cells were stimulated with LPA; the expression of inflammatory cytokines rose transiently, and 1 to 2 hours after the peak was reached, the expression of MMP-1, 3, and 13 rose. Meanwhile, since EDG2 is also highly expressed in the spinal cord, and LPA reportedly has algesic action, a correlation analysis was performed on a population with complaint of pain as a subjective symptom in a cohort population; a significant correlation was observed in i-EDG2-09.
These results show that polymorphs of the promoter region of the EDG2 gene alter the amount of EDG2 expressed, and are involved in the onset/progression of KOA, and strongly suggest that EDG2 antagonists suppress the induction of inflammatory cytokines to suppress cartilage destruction and lessen joint pain that accompanies KOA.
The present inventors conducted further investigations based on these findings, and have developed the present invention.
Accordingly, the present invention provides:

[1] a suppressing agent for inflammatory cytokine expression in joints and/or joint destruction (e.g., articular cartilage destruction) and/or pain in a bone/joint disease, comprising a substance that suppresses the expression or activity of a protein comprising the same or substantially the same amino acid sequence as the amino acid sequence shown by SEQ ID NO:2;
[2] the agent described in [1] above, wherein the aforementioned substance is any one of (a) to (d) below:

(a) a nucleic acid comprising a base sequence complementary to a base sequence that encodes a protein comprising the same or substantially the same amino acid sequence as the amino acid sequence shown by SEQ ID NO:2 or a portion thereof;
(b) a neutralizing antibody against a protein comprising the same or substantially the same amino acid sequence as the amino acid sequence shown by SEQ ID NO:2;
(c) an antagonist against a protein comprising the same or substantially the same amino acid sequence as the amino acid sequence shown by SEQ ID NO:2; or
(d) a dominant negative form of a protein comprising the same or substantially the same amino acid sequence as the amino acid sequence shown by SEQ ID NO:2;

[3] the agent described in [1] above, wherein the agent is to be used for the prevention/treatment of a bone/joint disease;
[4] the agent described in [3] above, wherein the bone/joint disease is selected from the group consisting of osteoarthritis, osteoporosis, rheumatoid arthritis, arthritis, synovitis, metabolic arthritis, sport-related joint disorder, congenital bone system disease, osteochondroma, osteoma and chondroma;
[5] a diagnostic reagent for a bone/joint disease, comprising either (a) or (b) below:

(a) an antibody against a protein comprising the same or substantially the same amino acid sequence as the amino acid sequence shown by SEQ ID NO:2; or
(b) a nucleic acid comprising a base sequence that encodes a protein comprising the same or substantially the same amino acid sequence as the amino acid sequence shown by SEQ ID NO:2 or a portion thereof;

[6] the diagnostic reagent described in [5] above, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma;
[7] a screening method for a substance that regulates inflammatory cytokine expression in joints and/or joint destruction (e.g., articular cartilage destruction) and/or pain in a bone/joint disease, comprising using a protein comprising the same or substantially the same amino acid sequence as the amino acid sequence shown by SEQ ID NO:2 or a partial peptide thereof;
[8] the method described in [7] above, wherein lysophosphatidic acid is further used;
[9] the method described in [7] above, wherein the protein comprising the same or substantially the same amino acid sequence as the amino acid sequence shown by SEQ ID NO:2 or partial peptide thereof is provided in the form of a cell that produces the same;
[10] the method described in [9] above, further comprising using an antibody against a protein comprising the same or substantially the same amino acid sequence as the amino acid sequence shown by SEQ ID NO:2, or a nucleic acid comprising a base sequence that encodes the protein or a portion thereof;
[11] the method described in [7] above, wherein the method is to be used for screening for a prophylactic/therapeutic substance for a bone/joint disease;
[12] the method described in [11] above, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma;
[13] a nucleic acid being a partial sequence of the base sequence shown by SEQ ID NO:3 comprising the bases shown by base numbers 8463 to 8464 in the base sequence (but thymine is inserted between the two bases), and comprising a continuous base sequence of about 15 bases or more;
[14] a diagnostic method for genetic susceptibility to a bone/joint disease, comprising detecting a polymorph in one or more bases or base sequences selected from the group consisting of the bases or base sequences shown by base numbers 8463 to 8464, 24007, 24999 and 34573 in the partial base sequence of the human EDG2 gene shown by SEQ ID NO:3;
[15] the method described in [14] above, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma;
[16] a suppressing agent for inflammatory cytokine expression in joints and/or joint destruction (e.g., articular cartilage destruction) and/or pain in a bone/joint disease, comprising a nucleic acid comprising a base sequence consisting of the base shown by base number 24007 in the base sequence shown by SEQ ID NO:3 (but the base is adenine) and a base or base sequence adjacent thereto;
[17] the agent described in [16] above, wherein the agent is to be used for the prevention/treatment of a bone/joint disease;
[18] the agent described in [17] above, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma;
[19] a screening method for a substance that regulates inflammatory cytokine expression in joints and/or joint destruction (e.g., articular cartilage destruction) and/or pain in a bone/joint disease, wherein the substance is to be administered to a human having an allele wherein the base shown by base number 24007 is thymine in the partial base sequence of the human EDG2 gene shown by SEQ ID NO:3, comprising using a nucleic acid comprising a base sequence consisting of the base shown by base number 24007 in the base sequence shown by SEQ ID NO: 3 (but the base is adenine) and a base or base sequence adjacent thereto, and a transcription regulatory factor that binds selectively to the base sequence;
[20] the method described in [19] above, wherein the method is to be used for screening for a prophylactic/therapeutic substance for a bone/joint disease;
[21] the method described in [20] above, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma;
[22] a suppressing agent for inflammatory cytokine expression in joints and/or joint destruction (e.g., articular cartilage destruction) and/or pain in a bone/joint disease, comprising a substance that suppresses the expression or activity of a protein comprising the same or substantially the same amino acid sequence as the amino acid sequence shown by SEQ ID NO:2, wherein the agent is administered to a human having one or more alleles selected from the group consisting of an allele with thymine inserted between the bases shown by base numbers 8463 and 8464, an allele wherein the base shown by base number 24007 is adenine, an allele wherein the base shown by base number 24999 is thymine, and an allele wherein the base shown by base number 34573 is thymine (but two or more alleles may be present on the same haplotype), in the partial base sequence of the human EDG2 gene shown by SEQ ID NO:3;

[23] a suppressing agent for inflammatory cytokine expression in joints and/or joint destruction (e.g., articular cartilage destruction) and/or pain in a bone/joint disease, comprising a substance that suppresses the production or activity of lysophosphatidic acid;

[24] the agent described in [22] above, wherein the aforementioned substance is a lysophosphatidic acid production enzyme inhibitory substance;

[25] the agent described in [23] above, wherein the agent is to be used for the prevention/treatment of a bone/joint disease;

[26] the agent described in [25] above, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma;

[27] a method of evaluating the pharmacological effect of a suppressing agent for inflammatory cytokine expression in joints and/or joint destruction (e.g., articular cartilage destruction) and/or pain, comprising examining the expression of a protein comprising the same or substantially the same amino acid sequence as the amino acid sequence shown by SEQ ID NO:2 or the gene that encodes the same, in a bone/joint disease in a subject receiving the suppressing agent; and the like.

Effect of the Invention

Because EDG2 induces inflammatory cytokines in synovial cells and raises the expression of cartilage substrate decomposing enzyme, increased expression of the EDG2 gene leads to cartilage destruction. Therefore, by inhibiting the expression and/or activity of EDG2 or LPA, which is a ligand thereof, inflammatory cytokine induction and/or cartilage destruction can be suppressed. Because the LPA-EDG2 signal is also involved in pain, by inhibiting the expression and/or activity of EDG2 or LPA, pain in a bone/joint disease can be suppressed. Furthermore, because polymorphs in the EDG2 gene correlate with bone/joint diseases, the polymorphs can be utilized for convenient determination of genetic susceptibility to bone/joint diseases.

Brief Description of the Drawings

Fig. 1 is a drawing showing the genomic structure of the rs3739708 LD region and the vicinity thereof. a) The rs3739708 LD region is shaded. b) Polymorphs in the rs3739708 LD region are shown. c) Pairwise D' and $\Delta$, including the polymorphs in the rs3739708 LD region, are shown.

Fig. 2 is a drawing showing the amounts of LPA receptor genes expressed in major human organs.

Fig. 3 is a drawing showing the amounts of LPA receptor genes expressed in human OA knee cartilage and OA synovial tissue.

Fig. 4 is a drawing showing the responsivity of E11 cells to PMA. a) Results of an evaluation of AP-1 activation with PMA stimulation by luciferase assay are shown. b) It is shown that the expression of the EDG2 gene rises over time with PMA stimulation.

Fig. 5 is a drawing showing a comparison of the transcription activities of the individual alleles in i-EDG2-09 by luciferase assay.

Fig. 6 is a drawing showing results of gel shift assay. a) It is shown that when E11 cells are stimulated with PMA, an intranuclear factor bindable to the region around i-EDG2-09 migrates into the nucleus over time. b) It is shown that the binding affinity of the intranuclear factor for i-EDG2-09 differs across the alleles. It is also shown that this intranuclear factor is AP-1.

Fig. 7 is a drawing showing an inflammatory cytokine gene induction reaction by LPA in human articular cartilage cells. a) Data on the TNFA gene, and b) data on the IL1B gene are shown.

Fig. 8 is a drawing showing inflammatory cytokine gene and MMP gene induction reactions by LPA in human OA synovial cells. a) - c) Changes in inflammatory cytokine genes over time are shown. d) - f) Changes in MMP genes over time are shown. g) - i) The LPA dose dependencies of inflammatory cytokine genes are shown.

Fig. 9 is a drawing showing inflammatory cytokine gene and MMP gene induction reactions by LPA in rabbit synovial cell line. a) and b) Changes in inflammatory cytokine genes over time are shown. c) - e) Changes in MMP genes over time are shown. f) and g) The LPA dose dependencies of inflammatory cytokine genes are shown.

Fig. 10 is a drawing showing that the frequency of the i-EDG2-09 disease-susceptible genotype correlates with the severity of knee OA in a Japanese general population.

[Fig. 11] A drawing showing the involvement of EDG2 in LPA-induced inflammatory cytokine raising reaction in human OA synovial cells. a) EDG2 expression suppression by an siRNA against EDG2, b) IL6 expression suppression by an siRNA against EDG2 and c) IL6 expression suppression by an LPA receptor inhibitory drug are shown.

(Detailed Description of the Invention)

The protein comprising the same or substantially the same amino acid sequence as the amino acid sequence shown by SEQ ID NO:2 used in the present invention (hereinafter, sometimes generically simply referred to as "EDG2") may be a natural EDG2 protein derived from a cell [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell or interstitial cell, or corresponding precursor cell or stem cell thereof, and the like] of a human or other warm-blooded animal (for example, monkey, bovine, horse, swine, sheep, goat, rabbit, mouse, rat, guinea pig, hamster, chicken, and the like), or any tissue or organ where such cells are present [for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum), spinal cord, hypophysis, stomach, pancreas, kidney, liver, gonad, thyroid, gall-bladder, bone marrow, adrenal gland, skin, muscle (e.g., skeletal muscle), lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), and the like], preferably synovial membrane, whole brain, spinal cord or the like, and, as described below, may be a protein synthesized chemically or biochemically using a cell-free protein synthesis system. Alternatively, the protein may be a recombinant protein produced from a transformant incorporating a nucleic acid comprising a base sequence that encodes the above-described amino acid sequence.

Substantially the same amino acid sequence as the amino acid sequence shown by SEQ ID NO:2 refers to an amino acid sequence having a homology of about 50% or more, preferably about 60% or more, more preferably about 70% or more, still more preferably about 80% or more, particularly preferably about 90% or more, most preferably about 95% or more, to the amino acid sequence shown by SEQ ID NO:2. Here, "a homology" means a ratio (%) of identical amino acid residues and similar amino acid residues to all overlapping amino acid residues in the optimal alignment (preferably, the algorithm considers introduction of gaps on one or both sides of the sequence for the best alignment) where two amino acid sequences are aligned using a mathematical algorithm known in the technical field. "Similar amino acid" means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such similar amino acids is expected not to change the phenotype of proteins (i.e., conservative amino acid substitution). Specific examples of the conservative amino acid substitution are known in the technical field and are described in various documents (see, for example, Bowie et al., Science, 247:1306-1310 (1990)).

Amino acid sequence homology in the present description can be calculated using the homology calculation algorithm NCBI BLAST (National Center for Biotechnology Information Basic Local Alignment Search Tool) under the following conditions (expectancy=10; gaps allowed; matrix=BLOSUM62; filtering=OFF). As examples of other algorithms for determination of amino acid sequence homology, the algorithm described in Karlin et al., Proc. Natl. Acad. Sci. USA, 90:5873-5877 (1993) [the algorithm is incorporated in the NBLAST and XBLAST programs (version 2.0) (Altschul et al., Nucleic Acids Res., 25:3389-3402 (1997))], the algorithm described in Needleman et al., J. Mol. Biol., 48:444-453 (1970) [the algorithm is incorporated in the GAP program in the GCG software package], the algorithm described in Myers and Miller, CABIOS, 4:11-17 (1988) [the algorithm is incorporated in the ALIGN program (version 2.0), which is part of the CGC sequence alignment software package], the algorithm described in Pearson et al., Proc. Natl. Acad. Sci. USA, 85: 2444-2448 (1988) [the algorithm is incorporated in the FASTA program in the GCG software package] and the like can be mentioned, which can likewise be used preferably.

A protein comprising the same or substantially the same amino acid sequence as the amino acid sequence shown by SEQ ID NO:2 refers to a protein comprising the above-described "substantially the same amino acid sequence as the amino acid sequence shown by SEQ ID NO:2", and possessing substantially the same quality of activity as a protein comprising the amino acid sequence shown by SEQ ID NO:2. As examples of substantially the same quality of activity, LPA binding activity, LPA signal transduction activities [for example, GTP binding activity, adenylate cyclase (AC) inhibitory activity, phospholipase C (PLC) β activity enhancement activity, inflammatory cytokine (e.g., TNFα, IL-1b, IL-6 and the like) induction activity, subsequent cartilage substrate decomposing enzyme (e.g., MMP-1, MMP-3, MMP-13 and the like) induction activity, algesic action and the like] and the like can be mentioned. "Substantially the same quality" means that the activities are qualitatively (e.g., physiologically or pharmacologically) equivalent to each other. Therefore, it is preferable that the above-described quantitative factors such as the extent of activity be equivalent to each other, but the quantitative factors may be different (for example, about 0.01 to about 100 times, preferably about 0.1 to about 10 times, more preferably about 0.5 to about 2 times).

A measurement of EDG2 activity can be performed in accordance with a method known per se. For example, LPA binding activity can be measured using $^{3}$H-labeled LPA, and GTP binding activity can be measured using a $^{35}$S-labeled GTP analogue (e.g., GTPγS and the like) with RI activity as an index. A measurement of AC activity can be performed by measuring the cAMP content using an anti-cAMP antibody, or with the expression of a reporter gene under the control of cAMP responding element (CRE) as an index. A measurement of PLCβ activity can be performed by measuring the $Ca^{2+}$ content in the cell using a fluorescent probe (e.g., fura-2, indo-1, fluor-3, Calcium-Green I and the like) or a Ca-sensitive luminescent protein (e.g., aequorin and the like), or by measuring the resulting inositol-1,4,5-triphosphate using $^{3}$H-labeled phosphatidylinositol-4,5-diphosphbric acid, or with the expression of a reporter gene under the control of TPA responding element (TRE) as an index. Algesic action can be evaluated by a publicly known pain test such as von Frey test.

The EDG2 used in the present invention also includes, for example, a protein comprising (1) an amino acid sequence resulting from deletion of 1 or 2 or more [preferably, 1 to about 30, more preferably 1 to about 10, still more preferably 1 to several (e.g., 5, 4, 3, 2)] amino acids in the amino acid sequence shown by SEQ ID NO:2, (2) an amino acid sequence resulting from addition of 1 or 2 or more [preferably, 1 to about 30, more preferably 1 to about 10, still more preferably 1 to several (e.g., 5, 4, 3, 2)] amino acids to the amino acid sequence shown by SEQ ID NO:2, (3) an amino acid sequence resulting from insertion of 1 or 2 or more [preferably, 1 to about 30, more preferably 1 to about 10, still more preferably 1 to several (e.g., 5, 4, 3, 2)] amino acids into the amino acid sequence shown by SEQ ID NO: 2, (4) an amino acid sequence resulting from substitution of 1 or 2 or more [preferably, 1 to about 30, more preferably 1 to about 10, still more preferably 1 to several (e.g., 5, 4, 3, 2)] amino acids in the amino acid sequence shown by SEQ ID NO:2 with other amino acids, or (5) an amino acid sequence being a combination thereof, and possessing substantially the same quality of activity as a protein comprising the amino acid sequence shown by SEQ ID NO:2.
When an amino acid sequence has been inserted, deleted or substituted as described above, the position of the insertion, deletion or substitution is not particularly limited, as far as the activity of the protein is not interfered with.

Meanwhile, a protein comprising substantially the same amino acid sequence as the amino acid sequence shown by SEQ ID NO:2, but not possessing substantially the same quality of activity as a protein consisting of the amino acid sequence shown by SEQ ID NO:2, for example, a protein incorporating a mutation (for example, deletion, insertion, substitution, modification and the like) in a region involved in the activation of a conjugated G protein α subunit (e.g., $G_{i/o}$) (G protein activating domain; GAD) (for example, Arg residue on the N-terminal side of intracellular third loop and/or RRNR sequence on the C-terminal side and the like) and the like (i.e., dominant negative mutant; DNM) is capable of blocking EDG2-mediated signal transduction from LPA by binding to LPA, so that such a protein can be useful for the purpose of suppressing the signal transduction action.

For the proteins identified by amino acid sequences herein, the left end indicates the N-terminal (amino terminal) and the right end indicates the C-terminal (carboxyl terminal), according to the common practice of peptide designation. In the EDG2 used in the present invention, including proteins comprising the amino acid sequence shown by SEQ ID NO:2, the C-terminal may be any of a carboxyl group (-COOH), a carboxylate ($-COO^-$), an amide ($-CONH_2$) and an ester (-COOR).
Here, as R in the ester, a $C_{1-6}$ alkyl group, for example, methyl, ethyl, n-propyl, isopropyl and n-butyl, a $C_{3-8}$ cycloalkyl group, for example, cyclopentyl and cyclohexyl, a $C_{6-12}$ aryl group, for example, phenyl and α-naphthyl, a phenyl-$C_{1-2}$ alkyl group, for example, benzyl and phenethyl, a $C_{7-14}$ aralkyl group, for example, an α-naphthyl-$C_{1-2}$ alkyl group, for example, α-naphthylmethyl, a pivaloyloxymethyl group; and the like can be used.

When the protein used in the present invention has a carboxyl group (or carboxylate) at a position other than the C-terminal, a protein wherein the carboxyl group is amidated or esterified is also included in the protein used in the present invention. In this case, as the ester, the above-described ester at the C terminal, and the like, for example, are used.
Furthermore, the protein used in the present invention also includes a protein wherein the amino group of the N-terminal amino acid residue (e.g., methionine residue) is protected by a protecting group (for example, $C_{1-6}$ acyl groups such as $C_{1-6}$ alkanoyls such as formyl group and acetyl group, and the like); a protein wherein the glutamine residue produced upon cleavage at the N terminal in vivo has been converted to pyroglutamic acid, a protein wherein a substituent (for example, -OH, -SH, amino group, imidazole group, indol group, guanidino group and the like) on a side chain of an amino acid in the molecule is protected by an appropriate protecting group (for example, $C_{1-6}$ acyl groups such as $C_{1-6}$ alkanoyl groups such as formyl group and acetyl group, and the like), a conjugated peptide such as what is called a glycopeptide having a sugar chain bound thereto (for example, the Asn residue shown by amino acid numbers 27 and 35 in the amino acid sequence shown by SEQ ID NO:2 is possibly an N-binding glycosylation site), and the like.

As specific examples of the protein used in the present invention, human EDG2 consisting of the amino acid sequence shown by SEQ ID NO:2 (GenBank accession No., NP_001392, NP_476500 and the like), or an ortholog thereof in another mammal and the like can be mentioned. For example, mouse EDG2 is registered with the NCBI database as GenBank accession No., NP_034466, and exhibits an amino acid identity of about 96% to human EDG2. Rat EDG2 is registered with the NCBI database as GenBank accession No. NP_446388, and exhibits an amino acid identity of about 97% to human EDG2.

The partial peptide of EDG2 used in the present invention may be any peptide comprising the same or substantially the same amino acid sequence as a partial amino acid sequence of the amino acid sequence shown by SEQ ID NO:2, as far as the aforementioned substantially the same quality of activity as EDG2 used in the present invention is possessed. Here, "substantially the same quality of activity" is defined as described above. A measurement of "substantially the same quality of activity" can be performed in the same manner as the above. Herein, the partial peptide is hereinafter referred to as "the active peptide of the present invention".

Specifically, as the partial peptide, a peptide consisting of at least 50 or more, preferably 70 or more, more preferably 100 or more, amino acid sequences of the amino acid sequences that constitute EDG2 used in the present invention, and the like are used. The partial peptide of EDG2 used in the present invention may have (1) 1 or 2 or more [preferably, 1 to about 30, more preferably 1 to about 10, still more preferably 1 to several (e.g., 5, 4, 3, 2)] amino acids deleted in the amino acid sequence thereof, or (2) 1 or 2 or more [preferably, 1 to about 30, more preferably 1 to about 10, still more preferably 1 to several (e.g., 5, 4, 3, 2)] amino acids added to the amino acid sequence thereof, or (3) 1 or 2 or more [preferably, 1 to about 30, more preferably 1 to about 10, still more preferably 1 to several (e.g., 5, 4, 3, 2)] amino acids inserted into the amino acid sequence thereof, or (4) 1 or 2 or more [preferably 1 to about 10, more preferably 1 to several (e.g., 5, 4, 3, 2)] amino acids in the amino acid sequence thereof substituted by other amino acids, or (5) a combination thereof.

Meanwhile, peptides comprising a partial amino acid sequence of EDG2, but not possessing substantially the same quality of activity as EDG2, for example, ones having a partial amino acid sequence of the amino acid sequence shown by SEQ ID NO:2 that contains a region involved in the binding to LPA (ligand-binding domain; LBD) but does not contain a G protein activation domain (GAD), and the like are not included in "the active peptide of the present invention". However, such peptides, like the above-described dominant negative mutant (DNM) of EDG2, are capable of blocking the EDG2-mediated signal transduction from LPA by binding to LPA, so that they can be useful for the purpose of suppressing the signal transduction action. Herein, such a partial peptide is hereinafter referred to as "the inhibitory peptide of the present invention".

In the partial peptide of EDG2 used in the present invention (encompasses both the active peptide of the present invention and the inhibitory peptide of the present invention; hereinafter, in this case, sometimes referred to as "the partial peptide of the present invention"), the C-terminal may be any of a carboxyl group (-COOH), a carboxylate ($-COO^-$), an amide ($-CONH_2$) and an ester (-COOR). Here, as R in the ester, the same as those mentioned for EDG2 above can be mentioned. When the partial peptide has a carboxyl group (or a carboxylate) at a position other than the C terminal, a partial peptide wherein the carboxyl group is amidated or esterified is also included in the partial peptide of EDG2 used in the present invention. In this case, as the ester, the above-described ester at the C terminal, and the like, for example, are used. Furthermore, the partial peptide also includes a protein wherein the amino group of the N terminal amino acid residue (e.g., methionine residue) is protected by a protecting group, a protein wherein Gln, which is produced upon cleavage at the N terminal in vivo, has been converted to pyroglutamic acid, a protein wherein a substituent on a side chain of an amino acid in the molecule is protected by an appropriate protecting group, a conjugated peptide such as what is called a glycopeptide having a sugar chain bound thereto, and the like, as with the case of EDG2.

As the salt of EDG2 or a partial peptide thereof used in the present invention, physiologically acceptable salts with acid (e.g., inorganic acid, organic acid) or base (e.g., alkali metal salts) can be mentioned, and physiologically acceptable acid addition salts are preferred. Useful salts include, for example, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

EDG2 or a salt thereof used in the present invention can be prepared from a cell or tissue of the aforementioned human or a warm-blooded animal by a method known per se of protein purification. Specifically, EDG2 used in the present invention can be purified or isolated by homogenizing tissue or cells of the animals, and extracting by an acid, and applying the extract to a combination of chromatographies such as reversed-phase chromatography, ion exchange chromatography, and the like.

EDG2 or its partial peptide or a salt thereof used in the present invention (hereinafter also comprehensively referred to as "EDG2s") can also be produced according to a publicly known method of peptide synthesis.
The method of peptide synthesis may be any of, for example, a solid phase synthesis process and a liquid phase synthesis process. A desired protein can be produced by condensing a partial peptide or amino acid capable of constituting the protein of the present invention with the remaining portion, and removing any protecting group the resultant product may have. Here, the condensation and the protecting group removal are conducted in accordance with methods known per se, for example, the methods indicated in (1) and (2) below:

(1) M. Bodanszky and M.A. Ondetti: Peptide Synthesis, Interscience Publishers, New York (1966)
(2) Schroeder and Luebke: The Peptide, Academic Press, New York (1965).

For the synthesis of EDG2s, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resin, an amino acid having an appropriately protected $\alpha$-amino group and side chain functional group is condensed on the resin in accordance with the sequence of the desired protein or the like according to one of various methods of condensation known per se. At the end of the reaction, the protein or a partial peptide thereof is cleaved from the resin and at the same time various protecting groups are removed, and a reaction to form an intramolecular disulfide bond is carried out in a highly diluted solution to obtain the desired protein or a partial peptide thereof or an amide thereof.

For the above-described condensation of protected amino acids, various activation reagents which can be used for protein synthesis can be used, and a carbodiimide is preferably used. As the carbodiimide, DCC, N, N'-diisopropylcarbodiimide, N-ethyl-N'-(3-dimethylaminopropyl)carbodiimide and the like are used. For the activation using these carbodiimides, the protected amino acid, along with a racemation-suppressing additive (for example, HOBt, HOOBt), may be added directly to the resin, or the protected amino acid may be activated in advance as a symmetric acid anhydride or HOBt ester or HOOBt ester and then added to the resin.

Solvents used for the activation of protected amino acids and condensation thereof with a resin can be appropriately selected from among solvents that are known to be usable for protein condensation reactions. As examples of useful solvents, acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; ethers such as pyridine dioxane and tetrahydrofuran; nitriles such as acetonitrile and propionitrile; esters such as methyl acetate and ethyl acetate; suitable mixtures thereof; and the like can be mentioned. Reaction temperature is appropriately selected from the range that is known to be usable for protein binding reactions, and is normally selected from the range of about -20˚C to about 50˚C. An activated amino acid derivative is normally used from 1.5 to 4 times in excess. When a test using the ninhydrin reaction reveals that the condensation is insufficient, sufficient condensation can be conducted by repeating the condensation reaction without elimination of protecting groups. If the condensation is insufficient even though the reaction is repeated, unreacted amino acids may be acetylated using acetic anhydride or acetylimidazole to prevent the subsequent reaction from being influenced.

A protecting method and a protecting group for a functional group that should not be involved in the reaction of raw materials, a method of removing the protecting group, a method of activating a functional group involved in the reaction, and the like can be appropriately selected from among publicly known groups or publicly known means.
As examples of the protecting group for an amino group of the starting material, Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc, and the like can be used.

A carboxyl group can be protected, for example, by alkyl esterification (for example, linear, branched or cyclic alkyl esterification with methyl, ethyl, propyl, butyl, t-butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, t-butoxycarbonyl hydrazidation, trityl hydrazidation, and the like.

The hydroxyl group of serine can be protected by, for example, esterification or etherification. As examples of a group suitable for this esterification, lower ($C_{1-6}$) alkanoyl groups such as an acetyl group, aroyl groups such as a benzoyl group, and groups derived from carbonic acid such as a benzyloxycarbonyl group and an ethoxycarbonyl group, and the like are used. As examples of a group suitable for etherification, a benzyl group, a tetrahydropyranyl group, a t-butyl group, and the like can be mentioned.

As examples of the protecting group for the phenolic hydroxyl group of tyrosine, Bzl, $Cl_2$-Bzl, 2-nitrobenzyl, Br-Z, t-butyl, and the like can be used.

As examples of the protecting group for the imidazole of histidine, Tos, 4-methoxy-2,3,6-trimethylbenzenesulfonyl, DNP, benzyloxymethyl, Bum, Boc, Trt, Fmoc, and the like are used.

As examples of the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like are used. The elimination reaction by the above-described acid treatment is generally carried out at a temperature of about -20˚C to about 40˚C; the acid treatment is efficiently conducted by adding a cation scavenger, for example, anisole, phenol, thioanisole, m-cresol, p-cresol, dimethylsulfide, 1,4-butanedithiol and 1,2-ethanedithiol. Also, a 2,4-dinitrophenyl group used as a protecting group of the imidazole of histidine is removed by thiophenol treatment; a formyl group used as a protecting group of the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As examples of those obtained by activation of the carboxyl group in the starting material, a corresponding acid anhydride, an azide, an activated ester [an ester with an alcohol (for example, pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like are used. As examples of those obtained by activation of the amino group in the starting material, a corresponding phosphoric amide is used.

In another method of preparing an amide of a protein or a partial peptide thereof, for example, the α-carboxyl group of the carboxy terminal amino acid is first amidated and hence protected, and a peptide (protein) chain is elongated to a desired chain length toward the amino group side, thereafter a protein or a partial peptide thereof having the protecting group for the N terminal α-amino group of the peptide chain only removed and a protein or a partial peptide thereof having the protecting group for the C terminal carboxyl group only removed are prepared, and these proteins or peptides are condensed in a mixed solvent described above. For details about the condensation reaction, the same as above applies. After the protected protein or peptide obtained by the condensation is purified, all protecting groups can be removed by the above-described method to yield a desired crude protein or peptide. By purifying this crude protein or peptide using various publicly known means of purification, and freeze-drying the main fraction, a desired amide of the protein or peptide can be prepared.

In order to obtain esters of the protein or peptide, a desired ester of the protein or peptide can be prepared by, for example, condensing the α-carboxyl group of the carboxy terminal amino acid with a desired alcohol to yield an amino acid ester, and then treating the ester in the same manner as with an amide of the protein or peptide.

The partial peptide of EDG2 or salt thereof used in the present invention can also be produced by cleaving the EDG2 obtained by any of the methods described above or below or a salt thereof with an appropriate peptidase.

EDG2s of the present invention thus obtained can be purified or isolated by a known method of purification. Here, as examples of the method of purification, solvent extraction, distillation, column chromatography, liquid chromatography, recrystallization, combinations thereof and the like can be mentioned.

When thus obtained protein or partial peptide is in a free form, the free form can be converted into a suitable salt form by a known method or an analogue thereto, and on the other hand, when the protein is obtained in the form of a salt, it can be converted into the free form or in the form of a different salt by a known method or an analogue thereto.

EDG2s of the present invention can also be produced by cultivating a transformant harboring expression vectors comprising nucleic acid that encodes EDG2 or a partial peptide thereof to produce EDG2s, and separating and purifying EDG2s from the culture obtained.

The nucleic acid that encodes EDG2 or a partial peptide thereof may be any one, as long as it comprises a base sequence that encodes the amino acid sequence of the aforementioned EDG2 used in the present invention or a partial amino acid sequence thereof. Although the nucleic acid may be a DNA, an RNA, or a DNA/RNA chimera, it is preferably a DNA. Additionally, the nucleic acid may be double-stranded or single-stranded. In the case of a double-stranded nucleic acid, it may be a double-stranded DNA, a double-stranded RNA, or a DNA:RNA hybrid.

As the DNA that encodes EDG2 or a partial peptide thereof, genomic DNA, a cDNA derived from a cell [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell fibroblast, fibrocyte, myocyte, adipocyte, immune cell (for example, macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell or interstitial cell, or corresponding precursor cell, stem cell or cancer cell thereof, and the like] of a human or other warm-blooded animal (for example, monkey, bovine, horse, swine, sheep, goat, rabbit, mouse, rat, guinea pig, hamster, chicken, and the like), or any tissue or organ where such cells are present [for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum), spinal cord, hypophysis, stomach, pancreas, kidney, liver, gonad, thyroid, gall-bladder, bone marrow, adrenal gland, skin, muscle (e.g., skeletal muscle), lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), and the like], preferably synovial membrane, whole brain, spinal cord and the like, synthetic DNA and the like can be mentioned. The genomic DNA and cDNA that encode EDG2 or a partial peptide thereof can also be directly amplified by polymerase chain reaction (hereinafter abbreviated as "PCR method") and reverse transcriptase-PCR (hereinafter abbreviated as "RT-PCR method") using a genomic DNA fraction and a total RNA or mRNA fraction prepared from one of the above-described cells or tissues as the respective templates. Alternatively, the genomic DNA and cDNA that encode EDG2 or a partial peptide thereof can also be cloned from a genomic DNA library and cDNA library prepared by inserting a fragment of a genomic DNA and total RNA or mRNA, respectively, prepared from one of the above-described cells or tissues into an appropriate vector, by the colony or plaque hybridization method or the PCR method and the like. The vector used for the library may be any of a bacteriophage, a plasmid, a cosmid, a phagemid and the like.

As examples of the DNA that encodes EDG2, DNA comprising the base sequence shown by SEQ ID NO:1, DNA that comprises a base sequence hybridizing to the base sequence shown by SEQ ID NO:1 under highly stringent conditions, and that encodes a protein or peptide having substantially the same quality of activity as the aforementioned protein comprising the amino acid sequence shown by SEQ ID NO:2, and the like can be mentioned.

As examples of the DNA capable of hybridizing to the base sequence shown by SEQ ID NO:1 under highly stringent conditions, DNA that comprises a base sequence showing a homology of about 60% or more, preferably about 70% or more, more preferably about 80% or more, particularly preferably about 90% or more, to the base sequence shown by SEQ ID NO:1, and the like are used.

Base sequence homology in the present description can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3). As preferable examples of other algorithms for determining base sequence homology, the above-described amino acid sequence homology calculation algorithm can also be mentioned.

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions.

As examples of the highly stringent conditions, conditions of a sodium salt concentration of about 19 to about 40 mM, preferably about 19 to about 20 mM, and a temperature of about 50˚C to about 70˚C, preferably about 60˚C to about 65˚C (in particular, the case of a sodium salt concentration of about 19 mM and a temperature of about 65˚C is preferred), or a hybridization reaction in 6×SSC (sodium chloride/sodium citrate) at 45˚C followed by washing in 0.2×SSC/0.1% SDS at 65˚C once or more and the like can be mentioned. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate.

The DNA that encodes EDG2 is preferably a human EDG2 cDNA comprising the base sequence shown by SEQ ID NO:1 (GenBank accession No., NM_001401) or an allele mutant thereof or an ortholog thereof in another warm-blooded animal (for example, mouse, rat, guinea pig, hamster, rabbit, sheep, goat, swine, bovine, horse, bird, cat, dog, monkey, chimpanzee and the like) and the like. For example, mouse EDG2 is registered with the NCBI database as GenBank accession No., NM_010336. Rat EDG2 is registered with the NCBI database as GenBank accession No., NM_053936.

The DNA that encodes the partial peptide of EDG2 may be any one comprising the base sequence that encodes the same or substantially the same amino acid sequence as a portion of the amino acid sequence shown by SEQ ID NO:2. The DNA may be any of genomic DNA, cDNA derived from the above-described cell or tissue, and synthetic DNA. Specifically, as examples of the DNA that encodes the partial peptide,

(1) DNA that comprises a partial base sequence of DNA comprising the base sequence shown by SEQ ID NO:1,
(2) DNA that comprises a base sequence hybridizing to DNA comprising the base sequence shown by SEQ ID NO:1 under highly stringent conditions, and that encodes a peptide having substantially the same quality of activity (e.g., activity to promote chondrocyte differentiation and the like) as that of a protein comprising the amino acid sequence encoded by the DNA, and the like are used.

As examples of the DNA capable of hybridizing to the DNA comprising the base sequence shown by SEQ ID NO:1 under highly stringent conditions, a DNA comprising a base sequence showing a homology of about 60% or more, preferably about 70% or more, more preferably about 80% or more, and particularly preferably about 90% or more, to the corresponding part in the base sequence, and the like are used.

The DNA that encodes EDG2 or a partial peptide thereof can be cloned by amplifying it by the PCR method using a synthetic DNA primer comprising a portion of the base sequence that encodes the protein or peptide, or by hybridizing DNA incorporated in an appropriate expression vector to a labeled DNA fragment or synthetic DNA that encodes a portion or the entire region of EDG2. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known per se, such as the ODA-LA PCR method, the Gapped duplex method, the Kunkel method and the like, or a method based thereon, using a publicly known kit, for example, Mutan™-super Express Km (Takara Shuzo Co., Ltd.), Mutan™-K (Takara Shuzo Co., Ltd.) and the like. The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

By transforming a host with an expression vector comprising the above-described DNA that encodes EDG2 or a partial peptide thereof, and culturing the transformant obtained, the protein or peptide can be produced.

A expression vector comprising DNA that encodes EDG2 or a partial peptide thereof can be produced by, for example, cutting out a desired DNA fragment from the DNA that encodes EDG2, and joining the DNA fragment downstream of a promoter in an appropriate expression vector.

Useful expression vectors include plasmids derived from *Escherichia coli* (e.g., pBR322, pBR325, pUC12, pUC13); plasmids derived from *Bacillus subtilis* (e.g., pUB110, pTP5, pC194); plasmids derived from yeast (e.g., pSH19, pSH15); bacteriophages such as $\lambda$ phage; animal viruses such as retrovirus, vaccinia virus and baculovirus; pAl-11, pXT1, pRc/CMV, pRc/RSV, pcDNAI/Neo, and the like.

The promoter may be any promoter, as long as it is appropriate for the host used to express the gene.

For example, when the host is an animal cell, the SR$\alpha$ promoter, the SV40 promoter, the LTR promoter, the CMV (cytomegalovirus) promoter, the HSV-TK promoter and the like are used. Of these, the CMV promoter, the SR$\alpha$ promoter and the like are preferred.

When the host is a bacterium of the genus *Escherichia,* the trp promoter, the lac promoter, the recA promoter, the $\lambda P_L$ promoter, the lpp promoter, the T7 promoter and the like are preferred.

When the host is a bacterium of the genus *Bacillus,* the SPO1 promoter, the SPO2 promoter, the penP promoter and the like are preferred.

When the host is yeast, the PHO5 promoter, the PGK promoter, the GAP promoter, the ADH promoter and the like are preferred.

When the host is an insect cell, the polyhedrin prompter, the P10 promoter and the like are preferred.

Useful expression vectors include, in addition to the above, those optionally harboring an enhancer, a splicing signal, a polyA addition signal, a selection marker, an SV40 replication origin (hereinafter also abbreviated as SV40ori) and the like. As examples of the selection marker, the dihydrofolate reductase (hereinafter also abbreviated as dhfr) gene [methotrexate (MTX) resistance], the ampicillin resistance gene (hereinafter also abbreviated as *$Amp^r$),* the neomycin resistance gene (hereinafter also abbreviated as *$Neo^r$,* G418 resistance) and the like can be mentioned. In particular, when a Chinese hamster cell lacking the dhfr gene is used in combination with the dhfr gene as the selection marker, a target gene can also be selected using a thymidine-free medium.

In addition, as required, a base sequence encoding a signal sequence (signal codon) that matches the host may be added to the 5' terminal side of the DNA encoding EDG2 or a partial peptide thereof. Useful signal sequences include a PhoA signal sequence, an OmpA signal sequence and the like when the host is a bacterium of the genus *Escherichia;* an $\alpha$-amylase signal sequence, a subtilisin signal sequence and the like when the host is a bacterium of the genus *Bacillus;* an MF$\alpha$ signal sequence, an SUC2 signal sequence and the like when the host is yeast; and an insulin signal sequence, an $\alpha$-interferon signal sequence, an antibody molecule signal sequence and the like when the host is an animal cell.

Useful hosts include, for example, a bacterium of the genus *Escherichia*, a bacterium of the genus *Bacillus,* yeast, an insect cell, an insect, an animal cell and the like.

Useful bacteria of the genus *Escherichia* include, for example, *Escherichia coli* K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)) and the like.

Useful bacteria of the genus *Bacillus* include, for example, *Bacillus subtilis* MI114 (Gene, Vol. 24, 255 (1983)), 207-21 (Journal of Biochemistry, Vol. 95, 87 (1984)) and the like.

Useful yeasts include, for example, *Saccharomyces cerevisiae* AH22, AH22R$^-$, NA87-11A, DKD-5D and 20B-12, *Schizosaccharomyces pombe* NCYC1913 and NCYC2036, *Pichia pastoris* KM71, and the like.

Useful insect cells include, for example, *Spodoptera frugiperda* cell (Sf cell), MG1 cell derived from the mid-intestine of *Trichoplusia ni,* High Five™ cell derived from an egg of *Trichoplusia ni,* cell derived from Mamestra brassicae, cell derived from *Estigmena acrea,* and the like can be mentioned when the virus is AcNPV. When the virus is BmNPV, useful insect cells include *Bombyx mori* N cell (BmN cell) and the like. Useful Sf cells include, for example, Sf9 cell (ATCC CRL1711), Sf21 cell (both in Vaughn, J. L. et al., In Vivo, 13, 213-217 (1977) and the like.

Useful insects include, for example, a larva of *Bombyx mori* (Maeda et al., Nature, Vol. 315, 592 (1985)) and the like.

Useful animal cells include, for example, monkey cell COS-7, Vero, Chinese hamster cell CHO (hereafter abbreviated as CHO cell), Chinese hamster cell lacking the dhfr gene CHO (hereafter abbreviated as CHO(dhfr$^-$) cell), mouse L cell, mouse AtT-20, mouse myeloma cell, rat GH3, human FL cell and the like.

Transformation can be carried out according to the kind of host in accordance with a publicly known method. A bacterium of the genus *Escherichia* can be transformed, for example, in accordance with a method described in Proc. Natl. Acad. Sci. U.S.A., Vol. 69, 2110 (1972), Gene, Vol. 17, 107 (1982) and the like.

A bacterium of the genus *Bacillus* can be transformed, for example, according to a method described in Molecular and General Genetics, Vol. 168, 111 (1979) and the like.

Yeast can be transformed, for example, in accordance with a method described in Methods in Enzymology, Vol. 194, 182-187 (1991), Proc. Natl. Acad. Sci. USA, Vol. 75, 1929 (1978) and the like.

An insect cell and an insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably contains a carbon source, a nitrogen source, an inorganic substance and the like necessary for the growth of the transformant. Here, as examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, potato extract and the like can be mentioned; as examples of the inorganic substance, calcium chloride, sodium dihydrogen phosphate, magnesium chloride and the like can be mentioned. In addition, the medium may be supplemented with yeast extract, vitamins, growth promoting factor and the like. Preferably, the pH of the medium is about 5 to about 8.

As an example of the medium used to cultivate a transformant whose host is a bacterium of the genus *Escherichia,* a M9 medium supplemented with glucose and a casamino acid (Miller, Journal of Experiments in Molecular Genetics, 431-433, Cold Spring Harbor Laboratory, New York, 1972) can be mentioned. As required, in order to increase promoter efficiency, a chemical agent such as 3β-indolylacrylic acid may be added to the medium.

Cultivation of a transformant whose host is a bacterium of the genus *Escherichia* is normally carried out at about 15˚C to about 43˚C for about 3 to about 24 hours. As necessary, the culture may be aerated or agitated.

Cultivation of a transformant whose host is a bacterium of the genus *Bacillus* is normally carried out at about 30˚C to about 40˚C for about 6 to about 24 hours. As necessary, the culture may be aerated or agitated.

As examples of the medium for cultivating a transformant whose host is a yeast, Burkholder's minimum medium [Bostian, K.L. et al., Proc. Natl. Acad. Sci. USA, vol. 77, 4505 (1980)] and SD medium supplemented with 0.5% casamino acid [Bitter, G.A. et al., Proc. Natl. Acad. Sci. USA, vol. 81, 5330 (1984)] can be mentioned. The medium's pH is preferably about 5 to 8. Cultivation is normally carried out at about 20˚C to about 35˚C for about 24 to about 72 hours. As necessary, the culture may be aerated or agitated.

Useful medium for cultivating a transformant whose host is an insect cell or an insect include, for example, Grace's insect medium [Grace, T.C.C., Nature, 195, 788 (1962)] supplemented with additives such as inactivated 10% bovine serum as appropriate. The medium's pH is preferably about 6.2 to 6.4. Cultivation is normally carried out at about 27˚C for about 3 to 5 days. As necessary, the culture may be aerated or agitated.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396 (1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally carried out at about 30˚C to 40˚C for about 15 to 60 hours. As necessary, the culture may be aerated or agitated.

As described above, EDG2s can be produced in a cell of the transformant or outside the cell.

EDG2s can be separated and purified from the culture obtained by cultivating the aforementioned transformant according to a method known per se.

For example, when EDG2s is extracted from a cultured bacterum, a method is used as appropriate wherein bacteria or cells are collected by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100™.

Isolation and purification of EDG2s contained in the thus-obtained soluble fraction can be conducted according to a method know per se. Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained EDG2 or a partial peptide thereof is a free form, it can be converted to a salt by a method known per se or a method based thereon; when the protein or peptide is obtained as a salt, it can be converted to a free form or another salt by a method known per se or a method based thereon.

Note that EDG2s produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained EDG2s can be confirmed by enzyme immunoassay, Western blotting and the like using a specific antibody.

Furthermore, EDG2 or a partial peptide thereof can also be synthesized by in vitro translation using a cell-free protein translation system comprising a rabbit reticulocyte lysate, wheat germ lysate, *Escherichia coli* lysate and the like, with RNA corresponding to the above-described DNA that encodes the calmodulin or a partial peptide thereof as the template. Alternatively, EDG2 or a partial peptide thereof can be synthesized using a cell-free transcription/translation system containing RNA polymerase, with the DNA that encodes EDG2 or a partial peptide thereof as the template. The cell-free protein (transcription/) translation system used may be a commercial product, and may be prepared in accordance with a method known per se; specifically, an *Escherichia coli* extract can be prepared in accordance with the method described in Pratt J.M. et al., Transcription and Translation, Hames B.D. and Higgins S.J. eds., IRL Press, Oxford 179-209 (1984) and the like. Commercially available cell lysates include those derived from *Escherichia coli* such as the *E. coli* S30 extract system (manufactured by Promega) and the RTS 500 Rapid Translation System (manufactured by Roche), those derived from rabbit reticulocytes such as the Rabbit Reticulocyte Lysate System (manufactured by Promega), and those derived from wheat germ such as PROTEIOS™ (manufactured by TOYOBO). Of these, one using a wheat germ lysate is suitable. For preparing a wheat germ lysate, a method described in, for example, Johnston F.B. et al., Nature, 179:160-161 (1957) or Erickson A.H. et al., Meth. Enzymol., 96:38-50 (1996), can be used.

As a system or apparatus for protein synthesis, the batch method (Pratt, J.M. et al. (1984), ibidem), a continuous cell-free protein synthesis system wherein amino acids, energy sources and the like are continuously supplied to the reaction system [Spirin A.S. et al., Science, 242:1162-1164 (1988)], the dialysis method (Kigawa et al., 21st general assembly of the Molecular Biology Society of Japan, WID6) or the overlay method (instruction manual of the PROTEIOS™ Wheat germ cell-free protein synthesis core kit: manufactured by TOYOBO) and the like can be mentioned. Furthermore, a method wherein template RNA, amino acids, energy sources and the like are supplied to the synthetic reaction system whenever necessary, and synthesized products and decomposed products are discharged whenever necessary (JP-A-2000-333673) and the like can be used.

The nucleic acids having "the base sequence encoding the protein comprising an amino acid sequence which is the same or substantially the same as the amino acid sequence represented by SEQ ID NO 2, or a part thereof", or "the base sequence which is complementary to the base sequence or a part thereof" are meant to include not only above-described nucleic acids encoding EDG2 or its partial peptide, but also a base sequence having mismatched codon-frame. The nucleic acid may be a DNA, an RNA, or a DNA/RNA chimera. It is preferably a DNA. Additionally, the nucleic acid may be double-stranded or single-stranded. In the case of a double-stranded nucleic acid, it may be a double-stranded DNA, a double-stranded RNA, or a DNA:RNA hybrid.

The nucleic acid comprising a base sequence complementary to a subject region of the objective nucleic acid, i.e., the nucleic acid capable of hybridizing with the objective nucleic acid can be said to be "antisense" against the objective nucleic acid. On the other hand, the nucleic acid comprising a base sequence having homology to a subject region of the objective nucleic acid can be said to be "sense" against the objective nucleic acid. As used herein, "having homology" or "(being) complementary" means having homology or complementarity of about 70% or more, preferably about 80% or more, more preferably about 90% or more, most preferably about 95% or more between the base sequences.

Nucleic acid comprising a base sequence which is complementary to the base sequence encoding EDG2 or a part thereof (hereinafter, also referred to as the "antisense EDG2") can be designed and synthesized on the basis of base sequence information of cloned or sequenced nucleic acid encoding EDG2. Such nucleic acid can inhibit replication or expression of the gene encoding the protein of the present invention. That is, the antisense EDG2 can hybridize to RNA transcripted from the genes encoding EDG2 and inhibit mRNA synthesis (processing) or function (translation into protein).

The length of the subject region of the antisense EDG2 is not particularly limited as long as the antisense nucleic acid inhibits translation of EDG2 protein of as results of hybridization of the antisense nucleic acid, and may be whole sequence or partial sequence of mRNA encoding the protein, for example, about 15 bases or so in the case of a short one and full-length in the case of a long one, of mRNA or initial transcription product. Considering ease of synthesis and antigenicity, an oligonucleotide comprising about 15 to about 30 bases is preferred but not limited thereto. Specifically, for example, the 5' end hairpin loop; 5' end 6-base-pair repeats, 5' end untranslated region, translation initiation codon, protein coding region, translation initiation codon, 3' end untranslated region, 3' end palindrome region, and 3' end hairpin loop of nucleic acid encoding EDG2, may be selected as subject regions, though any other region maybe selected as a target in the genes encoding EDG2. For example, the subject region is also preferably intron part of the gene.

Further, the antisense EDG2 may inhibit RNA transcription by forming triple strand (triplex) by binding to the genes encoding EDG2 which is double stranded DNA as well as inhibits translation into protein by hybridizing with mRNA or initial transcription product encoding EDG2.

Examples of the antisense nucleic acid include deoxypolynucleotides containing 2-deoxy-D-ribose, ribonucleotides containing D-ribose, any other type of nucleotides which are N-glycosides of a purine or pyrimidine base, or other polymers containing non-nucleotide backbones (e.g., commercially available nucleic acid polymers specific for protein nucleic acids and synthetic sequence) or other polymers containing particular linkages (provided that the polymers contain nucleotides having such an alignment that allows base pairing or base bonding, as found in DNA or RNA), etc. It may be double-stranded DNA; single-stranded DNA, double-stranded RNA, single-stranded RNA or a DNA:RNA hybrid, and may further include unmodified polynucleotides (or unmodified oligonucleotides), those with known modifications, for example, those with labels known in the art, those with caps, those which are methylated, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g.; alpha anomeric nucleic acids, etc.), etc. As used herein, the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides or modified nucleotides also include modifications on the sugar moiety, wherein, for example, one or more hydroxyl groups may optionally be substituted with a halogen atom(s), an aliphatic group(s), etc., or may be converted into the functional groups such as ethers, amines, or the like.

Preferably, the antisense nucleic acid is optionally modified RNA or DNA. Specific examples of the modified nucleic acid (RNA, DNA) are, but not limited to, sulfur and thiophosphate derivatives of nucleic acids and those resistant to degradation of polynucleoside amides or oligonucleoside amides. The antisense EDG2 can be designed preferably based on the following plan, that is by increasing the intracellular stability of the antisense nucleic acid, increasing the cell permeability of the antisense nucleic acid, increasing the affinity of the nucleic acid to the targeted sense strand to a higher level, or minimizing the toxicity, if any, of the antisense nucleic acid. Many of such modifications are known in the art, as disclosed in J. Kawakami, et al., Pharm. Tech. Japan, Vol. 8, 247, 1992; Vol. 8, 395, 1992; S. T. Crooke, et al. ed., Antisense Research and Applications, CRC Press, (1993); etc.

The antisense nucleic acids may contain sugars, bases or bonds, which are changed or modified. The antisense nucleic acids may also be provided in a specialized form such as liposomes, microspheres, or may be applied to gene therapy, or may be provided in combination with attached moieties. Such attached moieties include polycations such as polylysine that act as charge neutralizers of the phosphate group backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other groups may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol, etc.

Ribozymes which can cleave specifically mRNA or initial transcription product encoding EDG2 inside the code region (comprising intron moiety in the case of initial transcription product) can be also included in the antisense EDG2. The "ribozyme" means RNA having enzyme activity cleaving nucleic acid. However, it has been shown recently that oligo DNA having base sequence of the enzyme activity site also has nucleic acid cleavage activity similarly. Thus, in the present specification, ribozyme is meant to include DNA as long as it has sequence-specific nucleic acid cleavage activity. As most highly used ribozyme, there is self-splicing RNA found in infectious RNA such as viroid and virusoid. Hammerhead type and hairpin type, etc. are known. The hammerhead type exhibits enzyme activity at about 40 bases or so, and can specifically cleave only target mRNA by rendering several bases (about 10 bases or so in total) at the both ends which are adjacent to hammerhead structure moiety, to a sequence complementary to mRNA of the desired cleavage site. This type of ribozyme takes RNA only as a substrate, and thus has an advantage of not attacking genome DNA. When mRNA encoding EDG2 has double strand structure by itself, the target sequence can be made to be single stranded by using hybrid ribozyme ligated to RNA motif derived from virus nucleic acid which can bind specifically to RNA helicase [Proc. Natl. Acad. Sci. USA, 98(10): 5572-5577 (2001)]. Further, when ribozyme is used in the form of an expression vector comprising DNA encoding the same, it can be also made to be a hybrid ribozyme further ligated to the sequence obtained by modifying tRNA to promote transfer of the transcription product to cytoplasm [Nucleic Acids Res., 29(13): 2780-2788 (2001)].

Double stranded oligo RNA (siRNA) which comprise a base sequence complementary to a partial sequence (comprising intron part in the case of initial transcription product) in the code region of mRNA or initial transcription product encoding EDG2 can be also included in the antisense EDG2. It has been known that so-called RNA interference (RNAi), which is a phenomenon that if short double stranded RNA is introduced into cells, mRNA complementary to its RNA is degraded, occur in the nematodes, insect, plant, etc. Recently, it has been found that this phenomenon also occurs in mammal cells [Nature, 411(6836): 494-498 (2001)], which is drawing attention as an alternative technique to ribozymes.

The antisense oligonucleotide and ribozyme of the present invention can be prepared by determining a subject region of mRNA or initial transcription product on the basis of sequence information of cDNA or genome DNA encoding EDG2, and synthesizing its complementary sequence using commercially available DNA/RNA automatic synthesizer (Applied Biosystems, Beckman, etc.). siRNA having RNAi activity can be prepared by synthesizing sense strand and antisense strand with a DNA/RNA automatic synthesizer, respectively, denaturing them in a suitable annealing buffer, at about 90 to about 95°C for about 1 minute or so, and annealing them at about 30 to about 70°C for about 1 to about 8 hours. It can be also prepared as longer double stranded polynucleotide by synthesizing complementary oligonucleotide chains to overlap alternately, annealing them, and ligating them with ligase. Alternatively, the siRNA can be designed to be synthesized as an RNA (shRNA) by joining a sense strand and an antisense strand via a linker of an appropriate length (for example, about 3 to about 10 bases), and to be processed by an enzyme dicer (dicer) in the animal cell into which it is introduced, and the like. Furthermore, an expression vector wherein the DNAs that encode a sense strand and an antisense strand are placed under the control of respective Pol III system promoters such as U6 and H1, or an expression vector wherein the DNA that encodes an RNA strand wherein the above-described sense strand and antisense strand have been joined via a linker is placed under the control of a Pol III system promoter, may be prepared and allowed to be expressed in an animal cell to form of an siRNA.

The inhibitory activity of gene expression of the antisense EDG2 can be examined using a transformant comprising nucleic acid encoding EDG2, a gene expression system for gene encoding EDG2 in vivo and in vitro, or a translation system of EDG2 in vivo and in vitro.

The present invention also provides an antibody to EDG2 or its partial peptide or a salt thereof (hereinafter, also abbreviated as "anti- EDG2 antibody"). The antibodies may be any of polyclonal antibodies and monoclonal antibodies as long as they have specific affinity to EDG2 or its partial peptide or a salt thereof. The antibodies may be manufactured by known methods for manufacturing antibodies or antisera, using EDG2 or its partial peptide or a salt thereof as antigens.

[Preparation of monoclonal antibody]

(a) Preparation of monoclonal antibody-producing cells

EDG2 or its partial peptide or a salt thereof (EDG2s) is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually performed once in every 2 to 6 weeks and approximately 2 to 10 times in total. Examples of the applicable mammals are monkeys, rabbits, dogs, guinea pigs, mice, rats, sheep, goats and the like, with mice and rats being preferred.

For example, from mammals, e.g., mice, immunized with an antigen, one wherein the antibody titer is noted is selected, then the spleen or lymph node is collected after 2 to 5 days from the final immunization and antibody-producing cells contained therein are fused with myeloma cells from same or different race of animal to give monoclonal antibody-producing hybridomas. Measurement of the antibody titer in antisera may be made, for example, by reacting labeled EDG2, which will be described later, with the antiserum followed by assaying the binding activity of the labeling agent bound to the antibody. The fusion may be operated, for example, by the known Koehler and Milstein method [Nature, vol. 256, 495 (1975)]. Examples of the fusion accelerator are polyethylene glycol (PEG), Sendai virus, etc., of which PEG is preferably employed.

Examples of the myeloma cells are mammalian myelomas such as NS-1, P3U1, SP2/0, AP-1 etc. In particular, P3U1 is preferably employed. A preferred ratio of the count of the antibody-producing cells used (spleen cells) to the count of myeloma cells is within a range of approximately 1: 1 to 20: 1. When PEG (preferably, PEG 1000 to PEG 6000) is added in a concentration of approximately 10 to 80% or so followed by incubating at 20˚C to 40˚C, preferably at 30˚C to 37˚C for 1 to 10 minutes, an efficient cell fusion can be performed.

A monoclonal antibody-producing hybridoma can be screen for by a method which comprises adding the culture supernatant of a hybridoma to a solid phase (e.g., microplate) adsorbed with an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase; a method which comprises adding the culture supernatant of a hybridoma to a solid phase adsorbed with an anti-immunoglobulin antibody or Protein A, adding EDG2 labeled with a radioactive substance or an enzyme and detecting the monoclonal antibody bound to the solid phase; etc.

The monoclonal antibody can be selected by known methods or by analogues of these methods. In general, the selection of the monoclonal antibody can be effected in a medium for animal cells supplemented with HAT (hypoxanthine, aminopterin and thymidine). Any medium for the selection for the monoclonal antibody and growth can be employed as far as the hybridoma can grow therein. For example, RPMI 1640 medium containing 1% to 20%, preferably 10% to 20% fetal calf serum, GIT medium (Wako Pure Chemical Industries, Ltd.) containing 1% to 10% fetal calf serum, a serum free medium for culture of a hybridoma (SFM-101, Nissui Seiyaku Co., Ltd.), etc. may be used for the selection and growth medium. The cultivation is performed generally at 20˚C to 40˚C, preferably at about 37˚C, for 5 days to 3 weeks, preferably 1 to 2 weeks. The cultivation may be performed normally in 5% $CO_2$. The antibody titer of the culture supernatant of hybridomas can be determined as in the assay for the antibody titer in the antisera described above.

Separation and purification of the obtained monoclonal antibody can be performed by a method known per se, for example methods applied to separation and purification of immunoglobulins [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to give the antibody].

When the antibody of the present invention is utilized to inhibit the activity of EDG2, the antibody must possess EDG2 activity inhibitory activity (i.e., neutralizing activity), so that it is necessary to examine the degree of the neutralizing activity of the monoclonal antibody obtained. The neutralizing activity can be measured by performing the screening method of the present invention described below in the presence and absence of the antibody, and comparing the cell-stimulating activities mediated by EDG2, and the like.

In a preferred embodiment, because the antibody of the present invention is used as a pharmaceutical for a human recipient, the antibody (preferably monoclonal antibody) of the present invention is an antibody having a reduced risk of exhibiting antigenicity when administered to humans, specifically a fully human antibody, a humanized antibody, a mouse-human chimera antibody or the like, and particularly preferably a fully human antibody. A humanized antibody and a chimera antibody can be prepared by gene engineering according to the method described below. Although a fully human antibody can also be produced from a human-human (or mouse) hybridoma, it is desirable, for supplying a large amount of antibody stably and at low cost, that the antibody be produced using a human antibody-producing animal (e.g., mouse) or the phage display method.

(i) Preparation of chimeric antibody

As used herein, "a chimeric antibody" means an antibody wherein the sequences of the variable regions of the H chain and L chain ($V_H$ and $V_L$) thereof are derived from a mammalian species, and wherein the sequences of the constant regions ($C_H$ and $C_L$) are derived from another mammalian species. The sequences of the variable regions are preferably derived from, for example, an animal species permitting easy preparation of a hybridoma, such as mouse, and the sequences of the constant regions are preferably derived from the recipient mammalian species.

As examples of the method of preparing a chimeric antibody, the method described in US Patent No. 6,331,415 or a partially modified method thereof and the like can be mentioned. Specifically, first, mRNA or total RNA is prepared from a monoclonal antibody-producing hybridoma (for example, mouse-mouse hybridoma) obtained as described above, according to a conventional method, to synthesize cDNA. DNAs that encode $V_H$ and $V_L$ are amplified and purified by PCR according to a conventional method with the cDNA as the template, using appropriate primers (for example, oligo DNAs comprising the base sequences that encode the N-terminal sequences of $V_H$ and $V_L$, respectively, as the sense primers, and oligo DNAs that hybridize to the base sequences that encode the N-terminal sequences of $C_H$ and $C_L$, respectively, as the antisense primer (see, for example, Bio/Technology, 9: 88-89, 1991)). In the same manner, DNAs that encode $C_H$ and $C_L$ are amplified and purified from an RNA prepared from lymphocytes and the like of another mammal (e.g., human) by RT-PCR. $V_H$ and $C_H$, and $V_L$ and $C_L$, are ligated together, respectively, using a conventional method, and the chimeric H chain DNA and chimeric L chain DNA obtained are inserted into respective appropriate expression vectors (for example, vectors comprising promoters that have transcription activity in CHO cells, COS cells, mouse myeloma cells and the like (e.g., CMV promoter, SV40 promoter and the like)). The DNAs that encode the two chains may be inserted into separate vectors, and may be inserted into a single vector in tandem. Host cells are transformed with the chimeric H chain and chimeric L chain expression vectors obtained. As the host cells, animal cells, for example, Chinese hamster ovary (CHO) cells, monkey-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter recovering the culture supernatant and purifying it in the same manner as the above. Alternatively, it is also possible to obtain a chimeric monoclonal antibody easily and in large amounts from milk or eggs of transgenic animals which are produced by a conventional method using germ line cells of an animal such as bovine, goat, or chicken as the host cells, for which a transgenic technique has been established and a know-how of mass propagation as a domestic animal (domestic fowl) has been compiled. Furthermore, it is also possible to obtain a large amount of the chimeric monoclonal antibody from seeds, leaves and the like obtained from a transgenic plant prepared by microinjection or electroporation for protoplast, the particle gun method, the Ti vector method and the like for intact cells, using as the host cell a cell of a plant for which transgenic technology has been established, and which is cultured in large amounts as a major crop, such as corn, rice, wheat, soy or tobacco.
When the chimeric monoclonal antibody obtained is digested with papain, Fab is obtained; when the same is digested with pepsin, $F(ab')_2$ is obtained.

It is also possible to reformat into scFv by ligating DNAs that encode mouse $V_H$ and $V_L$ via a suitable linker, for example, a DNA that encodes a peptide consisting of 1 to 40 amino acids, preferably 3 to 30 amino acids, more preferably 5 to 20 amino acids (e.g., [Ser-(Gly)m]n or [(Gly)m-Ser]n (m is an integer from 0 to 10, n is an integer from 1 to 5) and the like); furthermore, it is possible to reformat into a minibody monomer by ligating a DNA that encodes $C_{H3}$ via a suitable linker, or reformat into a scFv-Fc by ligating a DNA that encodes full-length $C_H$ via a suitable linker. The DNA encoding such an antibody molecule modified (coupled) by gene engineering can be expressed in a microorganism such as *Escherichia coli* or yeast under the control of a suitable promoter, to produce the antibody molecule in large amounts.

When DNAs encoding mouse $V_H$ and $V_L$ are inserted downstream of one promoter in tandem and introduced into *Escherichia coli,* a dimer called Fv is formed by monocistronic gene expression. When an appropriate amino acid in the framework regions (FRs) of $V_H$ and $V_L$ is substituted with Cys using molecule modeling, a dimer called dsFv is formed via the intermolecular disulfide bond between the two chains.

(ii) Humanized antibody

As used herein, "a humanized antibody" means an antibody wherein the sequences of all regions present in the variable region, other than the complementarity determining region (CDR), (i.e., FRs in the constant region and variable region) are derived from a human, and wherein only the sequence of CDR is derived from another mammalian species. The other mammalian species is preferably an animal species that permits easy preparation of a hybridoma, for example, mouse.
As examples of the method of preparing a humanized antibody, the methods described in US Patent Nos. 5,225,539, 5,585,089, 5,693,761 and 5,693,762 or partially modified methods therefrom and the like can be mentioned. Specifically, DNAs that encode $V_H$ and $V_L$ derived from a non-human mammalian species (e.g., mouse) are isolated in the same manner as the above for a chimeric antibody, after which sequencing is performed by a conventional method using an automated DNA sequencer (e.g., manufactured by Applied Biosystems Co. and the like), and the base sequences obtained or amino acid sequences deduced therefrom are analyzed using a publicly known antibody sequence database [for example, Kabat database (see Kabat et al., "Sequences of Proteins of Immunological Interest", edited by the US Department of Health and Human Services, Public Health Service, NIH, 5th edition, 1991) and the like] to determine the CDRs and FRs of the two chains. A base sequence wherein the CDR encoding region of base sequences that encode the L chain and H chain of a human antibody having FR sequences similar to the determined FR sequences [e.g., human κ type L chain subgroup I and human H chain subgroup II or III (see Kabat et al., 1991 (supra))] is substituted with a determined base sequence that encodes the CDR of another animal species, is designed, and the base sequence is divided into fragments of about 20 to 40 bases, and a sequence complementary to the base sequence is further divided into fragments of about 20 to 40 bases so that they alternatively overlap with the aforementioned fragments. It is possible to construct DNAs that encode $V_H$ and $V_L$ having a human-derived FR and a CDR derived from another mammalian species by synthesizing individual fragments using a DNA synthesizer, and hybridizing and ligating them in accordance with conventional methods. In order to transfer a CDR derived from another mammalian species to human-derived $V_H$ and $V_L$ more quickly and more efficiently, it is preferable to use PCR-based site directed mutagenesis. As examples of such a method, the sequential CDR grafting method described in JP-A-5-227970 and the like can be mentioned. Such methods include, for example, the sequential CDR transplantation described in JP-A-HEI-5-227970. By joining the $V_H$- and $V_L$-encoding DNAs thus obtained to human derived $C_H$- and $C_L$-encoding DNAs, respectively, in the same manner as the above for a chimeric antibody, and introducing them into an appropriate host cell, a cell or transgenic animal or plant that produces a humanized antibody can be obtained.

A humanized antibody, like a chimeric antibody, can be modified to scFv, scFv-Fc, minibody, dsFv, Fv and the like using gene engineering techniques, and can be produced in microorganisms such as Escherichia coli and yeast by using an appropriate promoter.

The technology for producing a humanized antibody can also be applied to, for example, the production of a monoclonal antibody that can preferably be administered to another animal species for which no hybridoma production technology has been established. For example, animals widely propagated as domestic animals (domestic fowls) such as bovine, swine, sheep, goat, and chicken, and pet animals such as dogs and cats, and the like can be mentioned as the subject animal species.

(iii) Preparation of fully human antibody using human antibody-producing animal

Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. First, some of the human monoclonal antibodies generated by using a human antibody-producing mouse (see Immunol. Today, 17: 391-397, 1996) obtained by crossing a mouse transfected with minigenes of the human Ig H chain and L chain using an ordinary transgenic (Tg) technique with a mouse wherein the endogenous mouse Ig gene has been inactivated using an ordinary KO technique, are already in clinical stage, and to date production of anti-human Ig human antibody (HAHA) has not been reported.

Later, Abgenix Inc. [trade name: XenoMouse (see Nat. Genet., 15: 146-156, 1997; US Patent No. 5,939,598 and the like)] and Medarex Inc. [trade name: Hu-Mab Mouse (see Nat. Biotechnol., 14: 845-851, 1996; US Patent No. 5,545,806 and the like)] established Tg mice transfected with even a larger human Ig gene using a yeast artificial chromosome (YAC) vector, thus enabling the production of human antibodies of richer repertoire. However, because the human Ig gene, for example, in the case of the H chain, exhibits its diversity as the VDJ exon, which is a variable combination of about 80 kinds of V fragments, about 30 kinds of D fragments and 6 kinds of J fragments, encodes the antigen binding site, the full length thereof is as large as about 1.5 Mb (14th chromosome) for the H chain, about 2 Mb (2nd chromosome) for the κL chain, and about 1 Mb (22nd chromosome) for the λL chain. To reproduce a diverse antibody repertoire like in humans in another animal species, it is desirable to introduce the full length of each Ig gene; however, a DNA that is insertable into a conventional transfection vector (plasmid, cosmid, BAC, YAC and the like) is normally several kb to several hundred kb in length, and it has been difficult to introduce the full length of Ig genes by the conventional technique for establishing a transgenic animal, which comprises inserting a cloned DNA into a fertilized egg.

Tomizuka et al. (Nat. Genet., 16: 133-143, 1997) prepared a mouse having the full-length human Ig gene by introducing a natural fragment of a human chromosome harboring the Ig gene (hCF) into a mouse [transchromosomic (TC) mouse]. That is, first, a human-mouse hybrid cell having human chromosomes in which the 14th chromosome comprising the H chain gene and the 2nd chromosome comprising the κL chain gene, both labeled with, for example, a drug-resistance marker and the like, is treated with a spindle formation inhibitor (e.g., colcemid) for about 48 hours to prepare a microcell wherein one to several chromosomes or fragments thereof are enveloped in nuclear membrane, and the chromosomes are introduced into a mouse ES cell by the micronuclear fusion method. A hybrid ES cell retaining the chromosomes having the human Ig gene or fragments thereof is selected using a medium containing a drug, and the cell is microinjected into a mouse embryo in the same manner as with the preparation of an ordinary KO mouse. A germ line chimera is selected among the chimeric mice obtained, with coat color as the index, and the like, to establish a TC mouse strain carrying the human 14th chromosome fragment (TC(hCF14)) and a TC mouse strain carrying the human 2nd chromosome fragment (TC(hCF2)). After preparing mice wherein the endogenous H chain gene and κL chain gene are knocked out (KO (IgH) and KO (Igκ)) by a conventional method, it is possible to establish a mouse strain having all the four kinds of gene modifications (double TC/KO) by repeating the crossing of these four strains.

Provided that the same method as that for producing an ordinary mouse monoclonal antibody is applied to a double TC/KO mouse prepared as described above, it is possible to prepare an antigen-specific human monoclonal antibody-producing hybridoma. However, there is the drawback of a lower efficiency of hybridoma acquirement than that with the ordinary mouse, because hCF2 containing the κL chain gene is unstable in the mouse cells.

Meanwhile, because the aforementioned Hu-Mab mouse has a structure wherein the variable region cluster is doubled although it has about 50% of the κL chain gene, it exhibits a κ chain diversity equivalent to that with full length (on the other hand, HuMab mouse exhibits a low H chain diversity and inadequate response to antigen because it carries only about 10% of the H chain gene), and the κ chain is stably retained in the mouse cells because it is inserted in mouse chromosome via a YAC vector (Igκ-YAC). Making use of this advantage, it is possible to obtain an efficiency for hybridoma acquirement and antigen affinity of antibody that are equivalent to those with the ordinary mouse, by crossing a TC(hCF14) mouse with a Hu-Mab mouse to prepare a mouse that stably retains both hCF14 and Igκ-YAC (trade name: KM mouse).

Furthermore, it is also possible to prepare a human antibody-producing animal in which the λL chain gene is further transfected to reconstruct the diverse human antibody repertoire more completely. Such an animal can also be obtained by producing a TC mouse in which the human 22nd chromosome or a fragment thereof harboring the λL chain gene is introduced in the same manner as the above (TC(hCF22)), and crossing the mouse with the above-described double TC/KO mouse or KM mouse, or can also be obtained by, for example, constructing a human artificial chromosome (HAC) comprising both the H chain locus and the λL chain locus, and introducing it into a mouse cell (Nat. Biotechnol., 18: 1086-1090, 2000).

When the antibody of the present invention is used as a pharmaceutical, the antibody of the present invention is desirably a monoclonal antibody, but it may be a polyclonal antibody. When the antibody of the present invention is a polyclonal antibody, it is not necessary to utilize a hybridoma; therefore, provided that a human antibody-producing animal is produced in the same manner as the above using an animal species for which no technique for preparing a hybridoma has been established but a transgenic technique has been established, preferably an ungulate such as bovine, it is also possible to produce a human antibody in larger amounts at low costs (see, for example, Nat. Biotechnol., 20: 889-894, 2002). The human polyclonal antibody obtained can be purified by collecting blood, ascites fluid, milk, egg and the like, preferably milk or egg, of the human antibody-producing animal, in combination with the same purification techniques as the above.

(iv) Preparation of fully human antibody using phage display human antibody library

Another approach to produce a fully human antibody is a method using phage display. This method sometimes encounters cases in which a mutation due to PCR is introduced into a site other than CDRs; for this reason, a few reports of cases of HAHA production in clinical stage are available; on the other hand, however, the method has advantages such as no risk of cross-species viral infection derived from the host animal and the indefinite specificity of the antibody (antibodies against forbidden clone, sugar chain and the like can also be easily prepared).

Examples of the method of preparing a phage display human antibody library include, but are not limited to, the methods described below.

Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, besides 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) an Escherichia coli having a phagemid vector harboring the gene that encodes the fusion protein is infected with a helper phage having the wild-type coat protein gene to produce phage particles that express the fusion protein and the wild-type coat protein simultaneously, and the like can be mentioned; however, a phage display vector of the type 2) or 3) is used for the preparation of an antibody library, because in the case of 1), the capability of infection is lost when a large foreign protein is fused.

As specific vectors, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein a DNA encoding the κL chain constant region allocated to downstream of the g3p signal peptide, and a DNA encoding $C_{H3}$, His-tag, c-myc tag, and the amber stop codon (TAG) followed by the g3p coding sequence, allocated to downstream of the g3p signal peptide, are arranged under the control of one lactose promoter. When this is introduced into an *Escherichia coli* having an amber mutation, Fab is presented onto the g3p coat protein, but when it is expressed in the HB2151 strain and the like, which do not have an amber mutation, a soluble Fab antibody is produced. As the scFv-expressing phagemid vector, for example, pHEN1 (J. Mol. Biol., 222:581-597, 1991) and the like are used.

Meanwhile as examples of the helper phage, M13-KO7, VCSM13 and the like can be mentioned.

As another phage display vector, a vector designed to comprise sequences comprising a cysteine-encoding codon linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay™ technology of Morphosys Co.), and the like can be mentioned.

As the kind of human antibody library, a naive/non-immunized library, a synthetic library, an immunized library and the like can be mentioned.

The naive/non-immunized library is a library obtained by acquiring the $V_H$ and $V_L$ genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived-mRNA in which a class switch due to antigen sensitization has not occurred, to avoid V gene biases such as clinical history, is particularly called a naive library. Representatively, the library of CAT Co. (see J. Mol. Biol., 222: 581-597, 1991; Nat. Biotechnol., 14: 309-314, 1996), the library of MRC Co. (see Annu. Rev. Immunol., 12: 433-455, 1994), the library of Dyax Co. (see J. Biol. Chem., 1999 (supra); Proc. Natl. Acad. Sci. USA,14: 7969-7974, 2000) and the like can be mentioned.

The synthetic library is obtained by selecting a functional particular antibody gene in human B cells, and replacing of a portion of a V gene fragment, for example, an antigen-binding region such as CDR3, with a DNA encoding a random amino acid sequence of appropriate length, to construct a library. The synthetic library is recognized as being excellent in antibody expression efficiency and stability because the library can be constructed with the combination of the $V_H$ and $V_L$ genes, which produce functional scFv and Fab, since the beginning. Representatively, the HuCAL library of Morphosys Co. (see J.Mol. Biol., 296: 57-86, 2000), the library of BioInvent Co. (see Nat. Biotechnol., 18: 852, 2000), the library of Crucell Co. (see Proc. Natl. Acad. Sci. USA, 92: 3938, 1995; J. Immunol. Methods, 272: 219-233, 2003) and the like can be mentioned.

An immunized library is a library obtained by preparing an mRNA from lymphocytes collected from a human such as a patient with cancer, autoimmune disease, infectious disease and the like or a recipient of vaccination, having an elevated blood antibody titer against the target antigen, or from human lymphocytes and the like which are artificially immunized with the target antigen by the above-described *in vitro* immunization method, in the same manner as with the above-described naive/non-immunized library, and amplifying the $V_H$ and $V_L$ genes by RT-PCR, to construct a library. It is possible to obtain the desired antibody even from a library of relatively small size because the desired antibody gene is contained in the library already at the beginning.

The wider the diversity of the library is, the better; actually, however, an appropriate library size is about $10^8$ to $10^{11}$ clones, taking into consideration of the number of phages handlable in the following panning operation ($10^{11}$ to $10^{13}$ phages) and the number of phages necessary to isolate and amplify clones in ordinary panning (100 to 1,000 phages/clone); it is possible to screen for an antibody normally having a Kd value on the order of $10^{-9}$ with a library of about $10^8$ clones.

The process for selecting an antibody against the target antigen by the phage display method is referred to as panning. Specifically, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to *Escherichia coli* to proliferate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin-(strept) avidin system and the like are preferably used. When the endogenous ligand that is a target antigen is a small molecule such as a peptide, it is necessary to pay special attention to prevent masking of the portion used as the antigen determinant by conjugated with the carrier. For washing the unbound phage, a blocking solution such as BSA solution (once or twice), a PBS containing a surfactant such as Tween (3 to 5 times) and the like can be used. There is also a report mentioning that the use of citrate buffer (pH 5) and the like is preferable for the washing. For elution of the specific phage, an acid (e.g., 0.1 M hydrochloric acid and the like) is normally used; cleavage with a specific protease (for example, a gene sequence that encodes the trypsin cleavage site can be introduced into the linkage site between the antibody gene and the coat protein gene. In this case, *E. coli* infection and proliferation are possible even if all the coat protein is expressed in the form of a fusion protein because the wild-type coat protein is presented on the surface of the eluted phage), competitive elution with a soluble antigen, or elution by reduction of S-S bond (for example, in the aforementioned CysDisplay™, the antigen-specific phage can be recovered by dissociating the antibody and the coat protein by using a suitable reducing agent after performing panning) is also possible. When elution has been performed with an acid, the eluate is neutralized with Tris and the like, and the eluted phage is then infected to *Escherichia coli,* which is cultured; after which the phage is recovered by a conventional method.

After the phage presenting the antigen-specific antibody is concentrated by panning, the phage is infected to *Escherichia coli* and the cells are seeded onto a plate to perform cloning. The phage is again collected, and the antigen binding activity is confirmed by an antibody titer assay (e.g., ELISA, RIA, FIA and the like) or a measurement utilizing FACS or SPR.

Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an Escherichia coli that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as the above. Provided that the His-tag or c-myc tag has been introduced in advance, the antibody can easily be purified by using IMAC, an anti-c-myc antibody column and the like. When cleavage with a specific protease is utilized in panning, the antibody molecule is separated from the phage surface by an action with the protease, so that the desired antibody can be purified by performing the same purification operation as above mentioned.

The technology for producing a fully human antibody using a human antibody-producing animal and a phage display human antibody library can also be applied to the production of a monoclonal antibody of another animal species. For example, animals widely propagated as domestic animals (domestic fowls) such as bovine, swine, sheep, goat, and chicken, and pet animals such as dogs and cats, and the like can be mentioned as the subject animal species. In non-human animals, the utilization of an immunized library is more effective because there are fewer ethical problems concerning artificial immunization with the target antigen.

[Preparation of polyclonal antibody]

The polyclonal antibody to EDG2 or its partial peptide or a salt thereof can be manufactured by methods known per se. For example, an immunogen (an antigen such as EDG2s) or a complex of the immunogen and a carrier protein is prepared, and a warm-blooded animal is immunized with the antigen in a manner similar to the method described above for the manufacture of monoclonal antibodies. The product containing the anti-EDG2 antibody is collected from the immunized animal followed by separation and purification of the antibody.

In the complex of an immunogen and a carrier protein used to immunize a warm-blooded animal, the type of carrier protein and the mixing ratio of the carrier to hapten may be of any type in any ratio, as long as the antibody is efficiently produced to the hapten immunized by crosslinking to the carrier. For example, bovine serum albumin, bovine thyroglobulins, keyhole limpet hemocyanin, etc. is coupled to hapten with the weight ratio of approximately 0.1 to 20, preferably about 1 to about 5, per one hapten.

A variety of condensing agents (e.g., glutaraldehyde, carbodiimide, maleimide activated ester, activated ester reagents containing thiol group or dithiopyridyl group, etc.) can be used for the coupling of a carrier to hapten. The condensation product is administered to a warm-blooded animal either solely or together with carriers or diluents to the site in which the antibody may be prepared by the administration. In order to potentiate the antibody productivity upon the administration, complete Freund's adjuvant or incomplete Freund's adjuvant may be administered.

The administration is usually made once approximately in approximately every 2 to 6 weeks and about 3 to about 10 times in total.

The polyclonal antibody can be collected from the blood, ascites, breast milk etc. of the blood of mammal immunized by the method described above, or from the blood, egg etc. when the immunized animal is a bird.

The polyclonal antibody titer in antiserum can be assayed by the same procedure as that for the determination of antiserum antibody titer described above. The separation and purification of the polyclonal antibody can be performed, following the method for the separation and purification of immunoglobulins performed as applied to the separation and purification of monoclonal antibodies described hereinabove.

When a partial peptide of EDG2 or a salt thereof is used as the antigen, its position on EDG2 is not subject to limitation; for example, a polypeptide or oligopeptide comprising a partial amino acid sequence of a region well conserved among various warm-blooded animals or a salt thereof can be used.

The (i) EDG2s, (ii) nucleic acid that encodes EDG2 or a partial peptide thereof (preferably, DNA), (iii) anti-EDG2 antibody, (iv) antisense EDG2, (v) EDG2 DNM or the inhibitory peptide of the present invention, and (vi) EDG2 antagonist, described above have the following applications, for example.

As shown in Examples below, the EDG2 gene is highly expressed in joint tissue, particularly in synovial membrane, and the expression thereof rises in OA synovial membrane. In OA synovial membrane, the expression of inflammatory cytokines rises transiently upon LPA stimulation, and subsequently the expression of cartilage substrate decomposing enzymes rises, but the rises in the expression thereof are suppressed by the addition of an siRNA against EDG2. These facts show that a substance capable of suppressing the expression or activity of EDG2 suppresses inflammatory cytokine expression in joints and/or joint destruction (e.g., articular cartilage destruction), and is effective in the prevention/treatment of bone/joint diseases, particularly diseases associated with cartilage substrate degeneration/production abnormalities or abnormalities of differentiation from cartilage progenitor cells to chondrocytes. Here, "a disease associated with" refers to a disease that is caused by the condition or that results in the condition.

Furthermore, the EDG2 gene is also highly expressed in the spinal cord, and is a dominant LPA receptor. The OA susceptibility polymorph i-EDG2-09 in the EDG2 gene described below exhibits an increased transcription activity of EDG2 promoter in a susceptible allele, and the polymorph correlates significantly with symptomatic OA with a complaint of knee pain. These facts show that a substance capable of suppressing the expression or activity of EDG2 not only suppresses joint destruction (e.g., articular cartilage destruction), but also acts to mitigate pain, which is the chief complaint of OA patients, and is effective in the prevention/treatment of bone/joint diseases with pain.

[1] Suppressing agent for inflammatory cytokine expression in joints / joint destruction (e.g., articular cartilage destruction) / pain in bone/joint disease

As stated above, EDG2 is thought to increase the expression of cartilage substrate decomposing enzyme and promote articular cartilage destruction by eliciting a rise in the expression of inflammatory cytokines in synovial cells, and to induce pain in bone/joint diseases by transmitting signals from LPA; therefore, if there is an abnormality in EDG2 or a nucleic acid (e.g., gene, mRNA and the like) that encodes the same in a living organism (emergence of highly active mutant), or if the amount expressed has been abnormally increased, or if an increased expression of an inflammatory cytokine in joints occurs due to a certain other factor, and cartilage substrate decomposition is abnormally accentuated, or if a painful symptom is manifested in a bone/joint disease, a) by (i) administering antisense EDG2 to the patient to introduce (and expressing) the same into a target cell, or (ii) introducing antisense EDG2 into an isolated target cell and expressing the same, and thereafter transplanting the cell to the patient, b) by administering an anti-EDG2 antibody to the patient to inactivate (neutralize) EDG2, c) by administering an EDG2 antagonist to the patient to block the action of EDG2, d) by administering EDG2 DNM or the inhibitory peptide of the present invention to the patient to block the action of EDG2, or e) by introducing/expressing a nucleic acid that encodes EDG2 DNM or the inhibitory peptide of the present invention into the patient in vivo or ex vivo in the same manner as with the above-described antisense EDG2, and the like, it is possible to reduce the amount and/or activity of EDG2 in the patient's body, suppress inflammatory cytokine expression and/or joint destruction (e.g., articular cartilage destruction), and mitigate pain, so as to prevent/treat diseases underlain by cartilage substrate abnormalities and the like, and bone/joint diseases with pain and the like.
Therefore, a) antisense EDG2, b) anti-EDG2 antibody, c) EDG2 antagonist, d) EDG2 DNM or the inhibitory peptide of the present invention, or e) a nucleic acid that encodes EDG2 DNM or the inhibitory peptide of the present invention can be used as a prophylactic/therapeutic agent for the above-described diseases, for example, diseases such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, and chondroma, preferably osteoarthritis [e.g., hip OA (HOA), knee OA (KOA)], more preferably KOA.

Here, the term EDG2 antagonist is used with a meaning that encompasses both substances that antagonistically bind to the LBD of EDG2, but have almost no or totally no influence on the equilibrium between the active form and inactive form of EDG2 (neutral antagonists) and substances that bind to an optionally chosen site of EDG2 to shift the equilibrium between the active form and inactive form of EDG2 toward the more inactive side (inverse agonists). As EDG2 antagonists, for example, publicly known antagonists such as the isoxazol derivatives disclosed in EP 1258484 A1 (preferably, Ki16425 (see Mol. Pharmacol., 2003, 64(4): 994-1005) and the like), dioctylglycerol pyrophosphate, dioctylphosphatidic acid (Mol. Pharmacol., 2001, 60(4): 776-784), VPC32183, VPC32179, and VPC12249, which are commercially available from Avanti Polar Lipids, Inc., as well as substances selected by the screening method of the present invention described below, can also be included.

When an anti-EDG2 antibody, EDG2 antagonist, or EDG2 DNM or the inhibitory peptide of the present invention is used as the above-described prophylactic/therapeutic agent, the same can be prepared as a pharmaceutical formulation according to a routine means. Meanwhile, when a nucleic acid that encodes antisense EDG2, EDG2 DNM or the inhibitory peptide of the present invention is used as the above-described prophylactic/therapeutic agent, the nucleic acid, alone or after being inserted into an appropriate vector such as retrovirus vector, adenovirus vector, or adeno-associated virus vector, can be prepared as a pharmaceutical formulation according to a routine means. The nucleic acid can be administered as it is, or along with an auxiliary for promoting its ingestion, using a gene gun or a catheter such as a hydrogel catheter.

For example, antisense EDG2, anti-EDG2 antibody, EDG2 antagonist, EDG2 or the inhibitory peptide of the present invention or a nucleic acid that encodes EDG2 DNM or the inhibitory peptide of the present invention (hereinafter, sometimes generically referred to as "EDG2 suppressant") can be used orally as tablets, capsules, elixirs, microcapsules and the like, coated with sugar as required, or can be used parenterally in the form of an injection such as a sterile solution or suspension in water or another pharmaceutically acceptable liquid. For example, by mixing an EDG2 suppressant with a publicly known physiologically acceptable carrier, a sweetener, a filler, a vehicle, an antiseptic, a stabilizer, a binder and the like, in a unit dosage form required for generally accepted preparation design, such a preparation can be produced. The active ingredient contents in these preparations are intended to ensure that an appropriate dose in the specified range is obtained.

Examples of additives that can be mixed in tablets, capsules and the like include binders such as gelatin, cornstarch, tragacanth and gum arabic, fillers such as crystalline cellulose, swelling agents such as cornstarch, gelatin, alginic acid and the like, lubricants such as magnesium stearate, sweeteners such as sucrose, lactose or saccharin, flavoring agents such as peppermint, acamono oil or cherry, and the like can be used. When the formulation unit form is a capsule, a liquid carrier such as a grease (can be further contained in addition to the above-described type of material. A sterile composition for injection can be formulated according to an ordinary procedure for making a pharmaceutical preparation, such as dissolving or suspending an active substance in a vehicle like water for injection, or a naturally occurring vegetable oil such as sesame oil or coconut oil. The aqueous solution for injectable preparations is exemplified by saline, isotonic solutions containing glucose and another auxiliary (for example, D-sorbitol, D-mannitol, sodium chloride and the like) and the like, and may be used in combination with an appropriate solubilizer, for example, an alcohol (e.g., ethanol), a polyalcohol (e.g., propylene glycol, polyethylene glycol), a non-ionic surfactant (e.g., Polysorbate 80™, HCO-50) and the like. The oily liquid is exemplified by sesame oil, soybean oil and the like, and may be used in combination with a solubilizer such as benzyl benzoate or benzyl alcohol.

Also, the above-described prophylactic or therapeutic agent may be combined with, for example, a buffer (for example, phosphate buffer solution, sodium acetate buffer solution), an analgesic (for example, benzalkonium chloride, procaine hydrochloride and the like), a stabilizer (for example, human serum albumin, polyethylene glycol and the like), a preservative (for example, benzyl alcohol, phenol and the like), an antioxidant and the like. The injectable preparation prepared is usually filled in an appropriate ampoule.

The preparations thus obtained are safe and less toxic, can be administered to human or other warm-blooded animals (e.g., rats, mice, hamsters, rabbits, sheep, goats, swine, bovine, horses, cats, dogs, monkey, chimpanzee, birds, etc.).

The dose of the EDG2 suppressant varies depending on the recipient, target organ, symptoms, method of administration and the like; in oral administration, the dose is normally about 0.1 to 100 mg, preferably about 1.0 to 50 mg, and more preferably about 1.0 to 20 mg per day for, for example, an OA patient (assumed to weigh 60 kg). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an OA patient (as 60 kg body weight). In the case that the subject to be administered to is other than human, the corresponding dose as converted per 60 kg body weight can be administered.

[2] Screening for prophylactic/therapeutic substance for bone/joint disease

As stated above, a substance capable of suppressing the activity of EDG2 suppresses inflammatory cytokine expression in joints and joint destruction (e.g., articular cartilage destruction) and mitigates pain, so that the substance is effective in the prevention/treatment of diseases underlain by cartilage substrate abnormalities and the like, and bone/joint diseases with pain. Accordingly, the present invention provides a screening method for a prophylactic/therapeutic substance for a bone/joint disease by measuring a change in the activity thereof using EDG2s. Specifically, by using EDG2s, or by constructing an expression system for recombinant EDG2, and using an affinity assay system using the expression system, a substance that reduces the bindability of EDG2 and ligand can be efficiently screened for.
Such compounds include (a) compounds that block or suppress cell-stimulating activities via EDG2 (for example, GTP-GDP exchange reaction promotion in conjugated G$\alpha$, intracellular cAMP production inhibition, inositol triphosphate (IP3) production promotion, intracellular $Ca^{2+}$ uptake promotion, inflammatory cytokine expression induction, cartilage substrate decomposing enzyme expression induction, algesic action and the like) (antagonists), (b) compounds that reduce the binding force of EDG2 and ligand, and the like.

Accordingly, the present invention provides a screening method for a compound that alters the bindability of EDG2 and LPA or a salt thereof, comprising comparing (i) a case wherein EDG2 and LPA are brought into contact with each other and (ii) a case wherein EDG2, LPA and a test compound are brought into contact with each other.
In the above-described screening method, the amount of LPA bound to EDG2, cell-stimulating activities and the like in cases (i) and (ii) are measured and compared.

More specifically, the present invention provides:

(1) a screening method for a compound that alters the bindability of EDG2 and LPA or a salt thereof, comprising measuring and comparing the amounts of labeled LPA bound to EDG2s in a case wherein the labeled LPA is brought into contact with EDG2s and a case wherein the labeled LPA and a test compound are brought into contact with EDG2s,

(2) a screening method for a compound that alters the bindability of EDG2 and LPA or a salt thereof, comprising measuring and comparing the amounts of labeled LPA bound to a EDG2s-producing cell or a membrane fraction thereof in a case wherein the labeled LPA is brought into contact with the cell or a membrane fraction thereof and a case wherein the labeled LPA and a test compound are brought into contact with the cell or a membrane fraction thereof,

(3) a screening method for a compound that alters the bindability of EDG2 and LPA or a salt thereof, comprising measuring and comparing the amounts of labeled LPA bound to EDG2s in a case wherein the labeled LPA is brought into contact with EDG2 or EDG2s expressed on the cell membrane by culturing a transformant containing a DNA that encodes the active peptide of the present invention and a case wherein the labeled LPA and a test compound are brought into contact with the EDG2 or EDG2s expressed on the cell membrane by culturing a transformant containing a DNA that encodes the active peptide of the present invention,

(4) a screening method for a compound that alters the bindability of EDG2 and LPA or a salt thereof, comprising measuring and comparing cell-stimulating activities via EDG2s (for example, GTP-GDP exchange reaction promotion in conjugated Gα, intracellular cAMP production inhibition, IP3 production promotion, intracellular $Ca^{2+}$ uptake promotion, inflammatory cytokine expression induction, cartilage substrate decomposing enzyme expression induction, algesic action and the like) in a case wherein LPA is brought into contact with a cell that expresses EDG2s on the cell membrane and a case wherein the LPA and a test compound are brought into contact with the cell that expresses EDG2s on the cell membrane, and

(5) a screening method for a compound that alters the bindability of EDG2 and LPA or a salt thereof, comprising measuring and comparing cell-stimulating activities via EDG2s (for example, GTP-GDP exchange reaction promotion in conjugated Gα, intracellular cAMP production inhibition, IP3 production promotion, intracellular $Ca^{2+}$ uptake promotion, inflammatory cytokine expression induction, cartilage substrate decomposing enzyme expression induction, algesic action and the like) in a case wherein the LPA is brought into contact with EDG2 or EDG2s expressed on the cell membrane by culturing a transformant containing a DNA that encodes the active peptide of the present invention and a case wherein the LPA and a test compound are brought into contact with the EDG2 or EDG2s expressed on the cell membrane by culturing a transformant containing a DNA that encodes the active peptide of the present invention.

In the methods (1) to (5) above, an EDG2 agonist (for example, VPC31143 and VPC31144 commercially available from Avanti Polar Lipids, Inc. and the like) can also be used in place of LPA.

The screening method of the present invention is hereinafter described specifically.
First, although the EDG2 used in the screening method of the present invention may be any one comprising the above-described EDG2, a cell membrane fraction of an organ of an EDG2-producing mammal is preferred. However, because human-derived organs, in particular, are difficult to obtain, the EDG2 used in the screening is suitably a human-derived EDG2s expressed in a large amount using a recombinant and the like.

An EDG2s is produced using the method described above, which is preferably performed by expressing a DNA that encodes EDG2 or an active peptide of the present invention in mammalian cells or insect cells. A cDNA is used as the DNA fragment encoding the desired portion of the protein, but is not construed as limiting. For example, a gene fragment or a synthetic DNA may also be used. For introducing a DNA fragment that encodes EDG2 or the active peptide of the present invention into host animal (insect) cells and efficiently expressing the same, it is preferable to insert the DNA fragment downstream of an SV40-derived promoter, a retrovirus promoter, a metallothionein promoter, a human heat shock promoter, a cytomegalovirus promoter, an SRα promoter, the polyhedrin promoter of nuclear polyhedrosis virus (NPV), which is a baculovirus having insect hosts, or the like.
Therefore, the EDG2s used in the screening method of the present invention may be an EDG2s purified according to a method known per se, or may be used as cells producing the an EDG2s or a cell membrane fraction thereof.

In the above-described screening method, when using cells producing EDG2s, the cells may be fixed with glutaraldehyde, formalin and the like. This fixation can be performed according to a method known per se.
Cells producing EDG2s refer to host cells expressing EDG2s; as the host cells, yeasts, insect cells, animal cells and the like are used.
The aforementioned cell membrane fraction refers to a fraction rich in cell membrane obtained by a method known per se after cell disruption. As the method of cell disruption, cell squashing using a Potter-Elvehjem homogenizer, disruption using a Waring blender or Polytron (manufactured by Kinematica Inc.), disruption by ultrasonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is further centrifuged at a higher speed (15000 rpm to 30000 rpm) normally for 30 minutes to 2 hours, and the precipitate obtained is used as the membrane fraction. The membrane fraction is rich in the EDG2s expressed and cell-derived membrane components such as phospholipids and membrane proteins.

The amount of EDG2s in the cells producing the EDG2s and the membrane fraction thereof is preferably $10^3$

to $10^8$ molecules per cell, more preferably $10^5$ to $10^7$ molecules. Note that as the amount expressed increases, LPA-binding activity per unit of membrane fraction (specific activity) increases so that it is possible to construct a highly sensitive screening system, and to assay a large number of samples in the same lot.

In performing (1) to (3) above for screening for a compound that alters the bindability of EDG2 and LPA, for example, an appropriate EDG2s-containing membrane fraction and labeled LPA are required.
As the EDG2s-containing membrane fraction, a membrane fraction containing natural type EDG2, or a recombinant EDG2s-containing membrane fraction possessing an equivalent activity and the like are desirable. Here, an equivalent activity refers to an equivalent LPA binding activity, signal transduction action or the like.
As the labeled LPA, one labeled with, for example, [$^3$H], [$^{32}$P], [$^{125}$I], [$^{14}$C] or the like, according to a conventional method is used.
Specifically, in performing screening for a compound that alters the bindability of EDG2 and LPA, cells producing EDG2s or a membrane fraction thereof is first suspended in a buffer suitable for the screening to prepare a standard preparation of EDG2s. The buffer may be any buffer that does not interfere with the binding of EDG2s and LPA, such as a phosphate buffer or Tris-hydrochloride buffer having a pH value of 4 to 10 (desirably a pH value of 6 to 8). To reduce non-specific binding, a surfactant such as CHAPS, Tween-80™ (Kao-Atlas Co.), digitonin or deoxycholate may be added to the buffer. Furthermore, to suppress the degradation of EDG2s by proteases, a protease inhibitor such as PMSF, leupeptin, E-64 (manufactured by Peptide Research Laboratory Co.), or pepstatin may be added. A given amount (5,000 cpm to 500,000 cpm) of labeled LPA is added to 0.01 to 10 ml of a suspension of EDG2s, in the presence of a $10^{-4}$M to $10^{-10}$M test compound. To determine non-specific binding (NSB), a reaction tube containing the unlabeled ligand in large excess is also provided. The reaction is carried out at about 0 to about 50°C, desirably at about 4 to about 37°C, for about 20 minutes to about 24 hours, desirably for about 30 minutes to about 3 hours. After the reaction, the reaction mixture is filtered through glass fiber filter paper and the like, and washed with an appropriate amount of the same buffer; thereafter, the residual radioactivity on the glass fiber filter paper is counted using a liquid scintillation counter or a γ-counter. A test compound showing a specific binding (B-NSB) of, for example, not more than 50%, as the percent ratio to the count ($B_0$-NSB) calculated by subtracting non-specific binding (NSB) from the count in the absence of antagonizing substance ($B_0$), can be selected as a candidate substance capable of inhibiting the antagonism.

In performing the methods (4) to (5) above for screening for a compound that alters the bindability of EDG2 and LPA, for example, cell-stimulating activities via EDG2s (for example, GTP-GDP exchange reaction promotion in conjugated Gα, intracellular cAMP production inhibition, IP3 production promotion, intracellular $Ca^{2+}$ uptake promotion, inflammatory cytokine expression induction, cartilage substrate decomposing enzyme expression induction, algesic action and the like) can be measured using a publicly known method or a commercially available measuring kit.
Specifically, first, EDG2s-producing cells are cultured on a multiwell plate and the like. In performing screening, the medium is previously replaced with a fresh medium or an appropriate buffer that does not exhibit toxicity to the cells, and the cells are incubated for a given length of time with the addition of a test compound and the like, after which the cells are extracted or the supernatant is recovered, and the resulting product is quantified according to each method. If it is difficult to test the production of the substance serving as an index for cell-stimulating activities because of a decomposing enzyme contained in the cell, the assay may be performed with the addition of an inhibitor of the decomposing enzyme. An activity such as cAMP production suppression can be detected as a production suppressing action on cells having the basic productivity increased with forscolin or the like.
In performing screening by measuring a cell-stimulating activity, an appropriate cell wherein EDG2s is expressed on the membrane is required. As the cell wherein EDG2s is expressed, a cell line that produces natural type EDG2, a cell line expressing the aforementioned recombinant EDG2s and the like are desirable.
As examples of test compounds, peptides, proteins, non-peptide compounds, synthetic compounds, fermentation products, cell extracts, plant extracts, animal tissue extracts and the like can be used; these compounds may be novel compounds or publicly known compounds.

The screening kit for a compound that alters the bindability of EDG2 and LPA or a salt thereof comprises EDG2s, a cell that produces EDG2s or a membrane fraction thereof and the like.
As examples of the screening kit of the present invention, the following can be mentioned.

1. Screening reagents

(1) Assay buffer solution and wash buffer solution

Hanks' balanced salt solution (manufactured by Gibco Co.) supplemented with 0.05% bovine serum albumin (manufactured by Sigma Co.).
The solution is sterilized by filtration through a filter of 0.45 μm pore size and stored at 4°C, or may be prepared freshly just before use.

(2) Reference standards of EDG2s

CHO cells allowed to express an EDG2, passaged with a 12-well plate at $5\times10^5$ cells/well, cultured at 37°C, 5% $CO_2$, 95% air for 2 days.

(3) Labeled LPAs

LPAs (including analogues) labeled with a commercially available [$^3$H], [$^{32}$P], [$^{125}$I], [$^{14}$C] or the like
An aqueous solution of the LPA is stored at 4°C or -20°C and diluted to 1 μM with the assay buffer solution just before use.

(4) Standard solution of LPA

LPA (including analogues) is dissolved in a PBS containing 0.1% bovine serum albumin (manufactured by Sigma Co.) to obtain a concentration of 1 mM, and preserved at -20°C.

2. Assay method

(1) After EDG2s-expressing CHO cells cultured on a 12-well tissue culture plate are twice washed with 1 ml of the assay buffer solution, 490 μl of the assay buffer solution is added to each well.
(2) After 5 μl of $10^{-3}$ to $10^{-10}$M test compound solution is added, 5 μl of labeled LPA is added and the reaction is performed at room temperature for 1 hour. To determine non-specific binding, 5 μl of $10^{-3}$M standard LPA solution, instead of the test compound, is added in advance.
(3) The reaction liquid is removed and the wells are washed 3 times with 1 ml of the wash buffer solution. The labeled LPA bound to the cells (or plate) is dissolved in 0.2N NaOH-1% SDS, and mixed with 4 ml of liquid scintillator A (manufactured by Wako Pure Chemical Industries, Ltd.).
(4) Radioactivity is measured using a liquid scintillation counter (manufactured by Beckman Co.), and percent maximum binding (PMB) is calculated using the following equation [Equation 1].

[Equation 1]

$$PMB = [(B - NSB)/(B_0 - NSB)] \times 100$$

PMB: percent maximum binding
B: value obtained in the presence of test compound
NSB: non-specific binding
$B_0$: maximum binding

In another preferred mode of embodiment, cells that express EDG2s on the cell membrane further express conjugated Gα. A family of Gαs that conjugates with EDG2 each can be identified by determining whether or not the signal transduction action of EDG2 is activated when LPA, which is a ligand, is allowed to act. As conjugated Gαs of EDG2, $G_{i/o}$, $G_{q/11}$ and the like are known. Preferably, as cells that express EDG2s and a conjugated Gα thereof, transformant cells co-transfected with EDG2 or a DNA that encodes the active peptide of the present invention and a DNA that encodes the conjugated Gα can be mentioned.
When LPA binds to EDG2s, the Gα activation domain (GAD) of the receptor and the receptor binding region of the conjugated Gα interact with each other to produce a conformational change in Gα, and GDP dissociates from the GTP/GDP-binding region and binds with GTP immediately. The Gα-GTP acts on an effector to promote or suppress the activity thereof. Meanwhile, when an antagonist is bound, a conformational change in the receptor does not occur and the Gα activation domain is in the inactivated state, so that the activated form Gα-GTP level decreases, and the action on the effector is inhibited. Here, provided that a GTP analogue that does not undergo hydrolysis by the GTPase activity of Gα, for example, $^{35}$S-labeled GTPγS, in place of GTP, is added to the system in advance, by measuring/comparing the radioactivities bound to the membrane in the presence and absence of a test compound, the effect of the test compound on the GDP-GTP exchange reaction in Gα can be evaluated, and a substance possessing EDG2 agonist/antagonist activity can be screened for. That is, if the radioactivity decreases in the presence of the test compound, the test compound is judged to have antagonist activity against EDG2.

In another preferred mode of embodiment, screening method is provided which comprises comparing the activity of an effector that interacts with Gα in the presence and absence of a test compound in a co-expression system for EDG2s and conjugated Gα. Therefore, cells that express EDG2s and a conjugated Gα thereof also further express an effector that accepts signals from the Gα. Gαs elonging to the $G_{i/o}$ family have adenylate cyclase (AC) as the effector, and suppress the activity thereof. Gαs elonging to the $G_{q/11}$ family have phospholipase C (PLC) β as the effector, and promote the activity thereof.

In a screening system comprising AC as the effector, the action of Gα on the effector can be evaluated by directly measuring the AC activity. For the measurement of AC activity, any publicly known technique may be used; examples include, but are not limited to, a method wherein ATP is added to an AC-containing membrane fraction, and the resulting cAMP content is measured using an anti-cAMP antibody by a competitive immunoassay with cAMP labeled with an RI (for example, $^{125}I$), enzyme (for example, alkaline phosphatase, peroxidase and the like), fluorescent substance (for example, FITC, rhodamine and the like) and the like, a method wherein [$\alpha$-$^{32}P$] ATP is added to an AC-containing membrane fraction, and the resulting, and the resulting [$^{32}P$] cAMP is separated using an alumina column and the like, and thereafter the radioactivity thereof is measured, and the like. When the conjugated Gα is $G_{i/o}$, if the AC activity increases in the presence of the test compound, the test compound is judged to have antagonist activity against EDG2, and conversely, if the AC activity decreases, the test compound is judged to have agonist activity against EDG2.

When an intact eukaryotic cell is used as the screening system, the action of Gα on AC can also be evaluated by measuring the cAMP content in the cell, or by labeling the cell with [$^3H$] adenine, and measuring the radioactivity of the resulting [$^3H$] cAMP. Although the cAMP content in the cell can be measured by incubating the cell in the presence and absence of a test compound for an appropriate length of time, and subjecting the extract obtained by cell disruption to the above-described competitive immunoassay, any other publicly known method can be used.

In another mode, a method is available wherein the cAMP content is evaluated by measuring the amount expressed of a reporter gene under the control of cAMP responding element (CRE). That is, a eukaryotic cell incorporating a vector containing an expression cassette with a DNA that encodes a reporter protein (for example, luciferase, GFP, peroxidase, alkaline phosphatase and the like) linked downstream of a CRE-containing promoter is cultured in the presence and absence of a test compound for an appropriate length of time, and the expression of the reporter gene in the extract obtained by cell disruption is measured/compared using a publicly known technique, whereby the cAMP content in the cell is evaluated.

Therefore, when the conjugated Gα is $G_{i/o}$, if the intracellular the cAMP content (or the amount expressed of reporter gene under the control of CRE) increases in the presence of the test compound, the test compound is judged to have antagonist activity against EDG2; conversely, if the cAMP content (or the amount of reporter gene expressed) decreases, the test compound is judged to have agonist activity against EDG2.

Meanwhile, in a screening system comprising PLCβ as the effector (i.e., if the conjugated Gα polypeptide is $G_{q/11}$), the action of Gα on the effector can be evaluated by directly measuring the PLCβ activity. PLCβ activity can be evaluated by, for example, adding [$^3H$]-labeled phosphatidylinositol-4,5-diphosphate (PIP) to a PLCβ-containing membrane fraction, and measuring the amount of inositol phosphate (IP3) produced using a publicly known technique. PLCβ activity is measured and compared in the presence and absence of a test compound; if the PLCβ activity increases in the presence of the test compound, the test compound is judged to have agonist activity against EDG2, and conversely, if the PLCβ activity decreases, the test compound is judged to have antagonist activity against EDG2.

When an intact eukaryotic cell is used as the screening system, the action of Gα on PLCβ can also be evaluated by adding [$^3H$]-labeled inositol to the cell, and measuring the radioactivity of the resulting [$^3H$]-labeled IP3, or measuring the $Ca^{2+}$ content in the cell. The $Ca^{2+}$ content in the cell can be measured spectroscopically using a fluorescent probe (fura-2, indo-1, fluor-3, Calcium-Green I and the like), or can be measured using aequorin, a calcium-sensitive luminescent protein, and the like, after the cell is incubated in the presence and absence of a test compound for an appropriate length of time, but any other publicly known method can be used. As an apparatus suitable for spectroscopic measurements using a fluorescent probe, the FLIPR (Molecular Devices Co.) system can be mentioned.

As another mode, a method is available wherein the $Ca^{2+}$ content is evaluated by measuring the amount expressed of a reporter gene under the control of TPA (12-O-tetradecanoylphorbor-13-acetate) responding element (TRE), upregulated by $Ca^{2+}$. That is, a eukaryotic cell incorporating a vector containing an expression cassette with a DNA that encodes a reporter protein (for example, luciferase, GFP, peroxidase, alkaline phosphatase and the like) linked downstream of a TRE-containing promoter is cultured in the presence and absence of a test compound for an appropriate length of time, and the expression of the reporter gene in the extract obtained by cell disruption is measured/compared using a publicly known technique, whereby the $Ca^{2+}$ content in the cell is evaluated.

Therefore, if the intracellular $Ca^{2+}$ content (or the amount expressed of a reporter gene under the control of TRE) increases in the presence of the test compound, the test compound is judged to have agonist activity against EDG2, and conversely, if the intracellular $Ca^{2+}$ content (or the amount of reporter gene expressed) decreases, the test compound is judged to have antagonist activity against EDG2.

By expressing a chimeric Gα wherein the region that interacts with the AC of $G_{i/o}$ polypeptide (about 5 amino acids at the C-terminal) is substituted by the sequence of $G_{q/11}$, in a eukaryotic cell, irrespective of the conjugated Gα of EDG2, an EDG2 agonist/antagonist can be screened for using PLCβ as the effector.

In another preferred mode of embodiment, for example, as disclosed in WO 03/055507, EDG2 can be permanently activated by expressing a fusion protein wherein conjugated Gα is joined to the C-terminal side of EDG2 in a host eukaryotic cell such as a mammalian cell or an insect cell. In this case, without using a ligand such as LPA, agonist and inverse agonist can be screened for.

In another preferred mode of embodiment, yeast (for example, baker's yeast and the like) is used as the host cell. Yeast has STE2, which is a receptor of conjugation pheromone α factor, as the only GPCR. Upon receiving a ligand stimulation, STE2 activates GPA1, which is a conjugated Gα, and becomes dissociated from Gβ/Gγ. By the kinase cascade signal from the released Gβ/Gγ, the expression of FUS1 is induced, and conjugation occurs. Here, provided that STE2 is destroyed, and instead EDG2 is expressed in yeast, and conjugated Gα of EDG2 is co-expressed under the control of GPA1 promoter, upon LPA stimulation, EDG2, just like STE2, mediates intracellular signal transduction. Here, provided that EDG2 and conjugated Gα are co-expressed under the control of GPA1 promoter, and further fused with a gene that confers an auxotrophic mutation to FUS1, a reporter gene (for example, LacZ) and the like, signal activation can easily be detected. Details of this method are described in, for example, Yeast, 16: 11-22 (2000) and the like.

A compound obtained using the above-described screening method or screening kit or a salt thereof is a compound possessing an action to alter the bindability of EDG2 and LPA, specifically, (a) a compound possessing a cell-stimulating activity via LPA-EDG2 interaction (for example, GTP-GDP exchange reaction promotion in conjugated Gα, intracellular cAMP production inhibition, IP3 production promotion, intracellular $Ca^{2+}$ uptake promotion, inflammatory cytokine expression induction, cartilage substrate decomposing enzyme expression induction, algesic action and the like) (agonist), (b) a compound not having the cell-stimulating activity (antagonist), (c) a compound that enhances the binding force of EDG2 and LPA, or (d) a compound that reduces the binding force of EDG2 and LPA.

Because an antagonist against EDG2 is capable of suppressing the bioactivity of LPA on EDG2, is useful as a safe pharmaceutical of low toxicity that suppresses LPA activity. A compound that reduces the binding force of EDG2 and LPA is useful as a safe pharmaceutical of low toxicity for reducing the bioactivity of LPA on EDG2. Therefore, these compounds can be used as suppressants of the expression of inflammatory cytokines in joints, joint destruction that can be elicited thereby (e.g., articular cartilage destruction), or pain in a bone/joint disease, and are useful as prophylactic/therapeutic drugs for a bone/joint disease resulting in an cartilage substrate abnormality, a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, chondroma, or the like, preferably osteoarthritis [e.g., hip OA (HOA), knee OA (KOA)], more preferably KOA.

Meanwhile, an agonist against EDG2 possesses an action similar to the bioactivity of LPA on EDG2, and is therefore useful as a safe pharmaceutical of low toxicity that enhances LPA activity. A compound that enhances the binding force of EDG2 and LP is useful as a safe pharmaceutical of low toxicity for enhancing the bioactivity of LPA on EDG2. These compounds are useful as, for example, prophylactic/therapeutic drugs for schizophrenia (schizophrenia), obesity and the like.

A measurement of cell-stimulating activities in the above-described screening method, according to the purpose of screening, can also be performed by evaluating a more direct activity of EDG2.

For example, as stated above, EDG2, in response to LPA stimulation, induces in synovial cells, the expression of inflammatory cytokines (for example, TNFα, IL-1b, IL-6 and the like) and cartilage substrate decomposing enzyme (for example, collagenases such as MMP-1, MMP-3, and MMP-13, and the like). Therefore, by comparing the expression of any one of the above-described genes in a case wherein synovial cells with forcibly expressed EDG2 are cultured in the presence of LPA and a case wherein the same are cultured in the presence of both LPA and a test compound, a substance that suppresses the inflammatory cytokine expression induction and/or joint destruction (e.g., articular cartilage destruction) action of EDG2 can be screened for.

If the expression of the gene (genes) has decreased in the presence of the test compound, the test compound can be selected as a substance that suppresses the activity of EDG2.

As stated above, EDG2 is involved in pain in bone/joint diseases (for example, joint pain in OA, particularly knee pain in KOA). Therefore, for example, a pain test such as the von Frey test is performed on laboratory animals having EDG2 overexpressed therein (for example, EDG2 transgenic mouse, rat and the like), allocated to a test compound dosing group and a non-dosing group; if a significant analgesic effect is observed in the test compound dosing group, the compound can be selected as a substance that suppresses pain in bone/joint disease.

When a substance that suppresses EDG2 activity, obtained as described above (EDG2 activity suppressant) is used as the above-described prophylactic/therapeutic agent for bone/joint disease, the substance can be prepared as a pharmaceutical formulation in the same manner as the above for EDG2 antagonist and the like.

The preparations thus obtained are safe and less toxic, can be administered to human or other warm-blooded animals (e.g., rats, mice, hamsters, rabbits, sheep, goats, swine, bovine, horses, cats, dogs, monkey, chimpanzee, birds, etc.). The dose of the EDG2 activity suppressant varies depending on the recipient, target organ, symptoms, method of administration and the like; in oral administration, the dose is normally about 0.1 to 100 mg, preferably about 1.0 to 50 mg, and more preferably about 1.0 to 20 mg per day for, for example, an OA patient (assumed to weigh 60 kg). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an OA patient (as 60 kg body weight). In the case that the subject to be administered to is other than human, the corresponding dose as converted per human (60 kg body weight) can be administered.

As stated above, a substance that suppresses the expression of EDG2 also suppresses inflammatory cytokine expression in joints, joint destruction (e.g., articular cartilage destruction), and pain in bone/joint diseases, and is effective in the prevention/treatment of bone/joint diseases resulting in an cartilage substrate abnormality, or symptomatic bone/joint diseases with pain. Accordingly, the present invention provides a screening method for a substance that suppresses inflammatory cytokine expression in joints and/or joint destruction (e.g., articular cartilage destruction) and/or pain in a bone/joint disease, comprising comparing the expression of EDG2s in cells having the capability of producing EDG2s in the presence and absence of a test compound.

The amount of EDG2s expressed can also be measured at the transcription level by detecting the mRNA thereof using a nucleic acid capable of hybridizing to a nucleic acid that encodes EDG2 under high stringent conditions (that is, a nucleic acid comprising the aforementioned base sequence that encodes EDG2 or a portion thereof (hereinafter also referred to as "the sense EDG2") or nucleic acid comprising a base sequence complementary to a base sequence that encodes EDG2 or a portion thereof (the antisense EDG2)). Alternatively, the amount expressed can also be measured at the translation level by detecting a protein (peptide) using the aforementioned anti-EDG2 antibody.

Accordingly, more specifically, the present invention provides:

(a) a screening method for a substance that suppresses inflammatory cytokine expression in joints and/or joint destruction (e.g., articular cartilage destruction) and/or pain in a bone/joint disease, comprising culturing cells having the capability of producing EDG2s in the presence and absence of a test compound, and measuring and comparing the amount of EDG2s-encoding mRNA under the two conditions using sense or antisense EDG2, and

(b) a screening method for a substance that suppresses inflammatory cytokine expression in joints and/or joint destruction (e.g., articular cartilage destruction) and/or pain in a bone/joint disease, comprising culturing cells having the capability of producing EDG2s in the presence and absence of a test compound, and measuring and comparing EDG2s protein (peptide) content under the two conditions using anti-EDG2 antibody.

In the screening methods (a) and (b) above, as cells having the capability of producing EDG2s, the same as used in the above-described screening method for an EDG2 activity suppressant are preferably used.

For example, a measurement of the mRNA level or protein (peptide) level of EDG2s can specifically be performed as described below.

(i) A test substance is administered to a normal or disease model non-human warm-blooded animal (for example, mouse, rat, rabbit, sheep, swine, bovine, cat, dog, monkey, bird, and the like) a given time before (30 minutes to 24 hours before, preferably 30 minutes to 12 hours before, more preferably 1 hour to 6 hours before), or a given time after (30 minutes to 3 days after, preferably 1 hour to 2 days after, more preferably 1 hour to 24 hours after) a chemical or physical stimulation and the like, or at the same time as a chemical or physical stimulation; after a given time has passed from the administration of the test substance, articular fluid, articular cartilage and the like are collected. The mRNA of EDG2 expressed in the cells contained in the biological sample obtained can be quantified by, for example, extracting the mRNA from the cells and the like by an ordinary method, and using a technique such as RT-PCR and the like, or can also be quantified by Northern blot analysis known per se. On the other hand, EDG2 protein levels can be quantified using Western blot analysis or the various immunoassay methods described in detail below.

(ii) The measurement can be performed by preparing a transformant incorporating a nucleic acid that encodes EDG2 or a partial peptide thereof according to the above-described method, culturing the transformant according to a conventional method for a given time with a test substance added to the medium, and then quantifying and analyzing the level of the mRNA or protein (peptide) of EDG2s contained in the transformant.

As the test substance, peptides, proteins, non-peptide compounds, synthetic compounds, fermentation products and the like can be mentioned, and these substances may be novel substances or known substances.

As specific examples of the method of measuring the amount of EDG2s in the screening method (b) above,

(i) a method comprising quantifying EDG2s in a sample liquid by competitively reacting an EDG2 antibody with the sample liquid and labeled EDG2s, and detecting the labeled EDG2s bound to the antibody,
(ii) a method comprising quantifying EDG2s in a sample liquid by simultaneously or sequentially reacting the sample liquid with an anti-EDG2 antibody insolubilized on a carrier and another labeled anti-EDG2 antibody, and then measuring the amount (activity) of the labeling agent on the insolubilized carrier, and the like can be mentioned.

In the quantitation method (ii) above, it is desirable that the two kinds of antibodies be ones that recognize different portions of EDG2s. For example, if one antibody is an antibody that recognizes the N-terminal portion of EDG2s, an antibody that reacts with the C-terminal portion of EDG2s can be used as the other antibody.

As labeling agents used for the assay methods using labeled substances, there are employed, for example, radioisotopes, enzymes, fluorescent substances, luminescent substances, etc. As the radioisotopes, there are employed, for example, [$^{125}I$], [$^{131}I$], [$^{3}H$], [$^{14}C$], etc. As the enzymes described above, stable enzymes with a high specific activity are preferred; for example, beta -galactosidase, beta - glucosidase, alkaline phosphatase, peroxidase, malate dehydrogenase, etc. are used. Examples of the fluorescent substance used are fluorescamine, fluorescein isothiocyanate, etc. As the luminescent substances, there are employed, for example, luminol, luminol derivatives, luciferin, lucigenin, etc. Furthermore, the biotin-avidin system may also be used for binding of an antibody or antigen to the labeling agent.

As the sample liquid, a cell membrane-containing fraction obtained by a method known per se after cell disruption is used. As the method of cell disruption, cell squashing using a Potter-Elvehjem homogenizer, disruption using a Waring blender or Polytron (manufactured by Kinematica Inc.), disruption by ultrasonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is centrifuged at a higher speed (15000 rpm to 30000 rpm) normally for 30 minutes to 2 hours, and the precipitate obtained is used as the membrane fraction.
Also, as long as the labeling agent is detectable, intact cells may be used as the sample.

The methods for quantifying EDG2s using the anti-EDG2 antibody are not to be limited particularly. Any method can be used, so long as the amount of antibody, antigen, or antibody-antigen complex corresponding to the amount of antigen in a test fluid can be detected by chemical or physical means and can be calculated from a standard curve prepared from standard solutions containing known amounts of the antigen. For example, nephrometry, competitive method, immunometric method, and sandwich method are advantageously used, among which the sandwich method described below is particularly preferable in terms of sensitivity and specificity.

For immobilization of the antigen or antibody, physical adsorption may be used. Chemical binding methods conventionally used for insolubilization or immobilization of proteins, enzymes, etc. may be used as well. For the carriers, examples include insoluble polysaccharides such as agarose, dextran, cellulose, etc.; synthetic resin such as polystyrene, polyacrylamide, silicone, etc., or glass, etc.

In the sandwich method, the insolubilized anti-EDG2 antibody is reacted with a test fluid (primary reaction), then with a labeled form of another anti-EDG2 antibody (secondary reaction), and the activity of the labeling agent on the immobilizing carrier is assayed, whereby the amount of EDG2s in the test fluid can be quantified. The order of the primary and secondary reactions may be reversed, and the reactions may be performed simultaneously or with some time intervals. The labeling agent and the methods for insolubilization can be performed by modifications of those methods described above.
In the immunoassay by the sandwich method, the antibody used for immobilized antibody or labeled antibody is not necessarily from one species, but a mixture of two or more species of antibodies may be used to increase the measurement sensitivity.

The anti-EDG2 antibody can be used for the assay systems other than the sandwich method, for example, the competitive method, immunometric method, nephrometry, etc.
In the competitive method, EDG2s in a test fluid and labeled EDG2s are competitively reacted with an antibody, and the unreacted labeled antigen (F) and the labeled antigen bound to the antibody (B) are separated (B/F separation). The amount of the labeled antigen in B or F is measured, and the amount of EDG2s in the test fluid is quantified. This reaction method includes a liquid phase method using a soluble antibody as an antibody, polyethylene glycol and a secondary antibody to the soluble antibody (primary antibody) for B/F separation, etc. and an immobilized method either using an immobilized antibody as the primary antibody (direct method), or using a soluble antibody as the primary antibody and an immobilized antibody as the secondary antibody (indirect method).

In the immunometric method, EDG2s in a test fluid and immobilized EDG2s are competitively reacted with a definite amount of labeled antibody, the solid phase is separated from the liquid phase, or EDG2s in a test fluid is reacted with an excess amount of labeled antibody, the immobilized EDG2s is then added to bind the unreacted labeled antibody to the solid phase, and the solid phase is separated from the liquid phase. Then, the amount of the labeled antibody in either phase is measured to quantify an amount of the antigen in the test fluid.

In the nephrometry, an amount of insoluble precipitates produced after the antigen-antibody reaction in gel or solution are measured. Even when the amount of EDG2s in a test fluid is small and only a small amount of precipitates is obtained, laser nephrometry utilizing scattering of laser can be advantageously employed.

For applying these individual immunological assay methods to the quantification methods of the present invention, any particular conditions, and setting of procedures and the like are not required. The assay systems for EDG2s may be constructed by adding ordinary technical consideration in the art to conventional conditions and procedures in the respective methods. For the details of these general technical means, reference can be made to the reviews and texts. For example, Meth. Enzymol., Vol. 70: (Immunochemical Techniques (Part A)), ibidem Vol. 73 (Immunochemical Techniques (Part B)), ibidem Vol. 74 (Immunochemical Techniques (Part C)), ibidem Vol. 84 (Immunochemical Techniques (Part D: Selected Immunoassays)), ibidem Vol. 92 (Immunochemical Techniques (Part E: Monoclonal Antibodies and General Immunoassay Methods)), ibidem Vol. 121 (Immunochemical Techniques (Part I: Hybridoma Technology and Monoclonal Antibodies)) (all published by Academic Press) and the like can be referenced to.
As described above, by using an anti-EDG2 antibody, the amount of EDG2s produced in cells can be quantified with high sensitivity.

In the screening methods (a) and (b) above, a substance that has increased the amount of the EDG2s expressed (mRNA content or protein (peptide) content) can be selected as EDG2 expression promoting drug, and a substance that has reduced the amount expressed can be selected as an EDG2 expression suppressant. An EDG2 expression suppressant can be used as a suppressant for the expression of inflammatory cytokines in joints, joint destruction that can be elicited thereby (e.g., articular cartilage destruction), or pain in a bone/joint disease, and is useful as a prophylactic/therapeutic drug for a bone/joint disease resulting in an cartilage substrate abnormality, a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, chondroma or the like, preferably osteoarthritis [e.g., hip OA (HOA), knee OA (KOA)], more preferably KOA.
Meanwhile, an EDG2 expression promoting drug is useful as prophylactic/therapeutic drug for, for example, schizophrenia (schizophrenia), obesity and the like.

When an EDG2 expression suppressant is used as the above-described prophylactic/therapeutic agent, the same can be prepared as a pharmaceutical formulation in the same manner as with the aforementioned EDG2 antagonist and the like.
The preparations thus obtained are safe and less toxic, can be administered to human or other warm-blooded animals (e.g., rats, mice, hamsters, rabbits, sheep, goats, swine, bovine, horses, cats, dogs, monkey, chimpanzee, birds, etc.). The dose of the EDG2 expression suppressant varies depending on the recipient, target organ, symptoms, method of administration and the like; in oral administration, the dose is normally about 0.1 to 100 mg, preferably about 1.0 to 50 mg, and more preferably about 1.0 to 20 mg per day for, for example, an OA patient (assumed to weigh 60 kg). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an OA patient (as 60 kg body weight). In the case that the subject to be administered to is other than human, the corresponding dose as converted per 60 kg body weight can be administered.

The above-described screening method for an EDG2 expression suppressant can also be used to evaluate the pharmacological effect of an existing suppressing agent for inflammatory cytokine expression in joints, a suppressing agent for joint destruction (e.g., articular cartilage destruction),
a suppressing agent for pain in bone/joint disease and the like on the recipient. That is, from a subject receiving any one of the above-described drugs, a biological sample, for example, articular fluid, articular cartilage or the like is collected, and the EDG2 mRNA content or EDG2 protein content in the sample is measured using the above-described method. For example, if the amount of EDG2 expressed decreases significantly compared with the level before drug administration, the drug can be judged to have a prophylactic/therapeutic effect on the subject (drug recipient).

(4) Genetic diagnostic reagent

Because a nucleic acid comprising a base sequence that encodes EDG2 or a portion thereof (hereinafter also referred to as "the sense EDG2") or a nucleic acid comprising a base sequence complementary to the base sequence or a portion thereof (the antisense EDG2) is capable of detecting abnormalities in the EDG2-encoding DNA or mRNA (gene abnormalities) in a human or another warm-blooded animal (for example, rat, mouse, hamster, rabbit, sheep, goat, swine, bovine, horse, cat, dog, monkey, chimpanzee, bird and the like) when used as a probe and the like, it is useful as, for example, a genetic diagnostic agent for damage or mutation of the DNA, a splicing abnormality or decreased expression of mRNA, amplification of the DNA, increased expression of mRNA, and the like. The nucleic acid comprising a portion of the base sequence that encodes EDG2 is not subject to limitation, as long as it has a necessary length for a probe (for example, about 15 bases or more), and needs not encode a partial peptide of EDG2.

The above-described genetic diagnosis using the sense or antisense EDG2 can be performed by, for example, Northern hybridization known per se, quantitative RT-PCR, the PCR-SSCP method, allele-specific PCR, the PCR-SSOP method, the DGGE method, the RNase protection method, the PCR-RFLP method and the like.

As stated above, EDG2 raises the expression of inflammatory cytokines, induces joint destruction (e.g., articular cartilage destruction), or provokes pain in bone/joint disease. For this reason, it is thought that if the subject is suffering a bone/joint disease resulting in an cartilage substrate abnormality or a symptomatic bone/joint disease with pain, or is at an increased risk for suffering the same in the future, the expression of the EDG2 gene is elevated compared with normal state. Therefore, for example, if an increase in the expression of the EDG2 gene is detected as a result of Northern hybridization or quantitative RT-PCR for an RNA fraction extracted from cells of a subject warm-blooded animal, the subject can be judged as being suffering, or being likely to suffer in the future, a bone/joint disease resulting in an cartilage substrate abnormality, a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, chondroma or the like.

Because the aforementioned anti-EDG2 antibody is capable of measuring the amount of EDG2 or a salt thereof in humans or other warm-blooded animals (for example, rat, mouse, hamster, rabbit, sheep, goat, swine, bovine, horse, cat, dog, monkey, chimpanzee, bird and the like), it is useful as, for example, a genetic diagnostic agent for decreased expression or increased expression of the protein and the like.

The above-described genetic diagnosis using an anti-EDG2 antibody can be performed by performing immunoassay using as cells having the capability of producing EDG2s a biological sample (e.g., articular fluid, biopsy and the like) collected from a subject warm-blooded animal in a screening method for an EDG2 expression suppressant using the aforementioned anti-EDG2 antibody (screening method (b)).

If as a result of the immunoassay, an increase in the EDG2 or a salt thereof in the sample is detected, the subject can be judged as being suffering, or being likely to suffer in the future, a bone/joint disease resulting in an cartilage substrate abnormality, a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, chondroma and the like.

The present invention also provides a method of judging (diagnosing) genetic susceptibility to bone/joint diseases, including OA (hereinafter, sometimes simply referred to as "the diagnostic method of the present invention"). The diagnostic method of the present invention comprises detecting polymorphs in the subject's EDG2 gene.

Herein, "a (gene) polymorph" refers to a change (substitution, deletion, insertion, translocation, inversion and the like) in 1 or a plurality of bases on genomic DNA, the change being present in a population at a frequency of 1% or more; for example, one wherein one base has been substituted by another base (SNP), one wherein 1 to several tens of bases have been deleted or inserted (DIP), one wherein the copy number differs at a site where a sequence of 2 to several tens of bases as 1 unit is repeatedly present (one wherein the copy number is 2 to 4 bases is referred to as a microsatellite polymorph, one wherein the copy number is several to several tens of bases is referred to as a variable number of tandem repeat (VNTR)) and the like can be mentioned. The polymorph that can be utilized in the diagnostic method of the present invention may be of any type, as far as the following requirements are met.

The polymorph that can be utilized in the diagnostic method of the present invention is not particularly limited, as far as it is a polymorph present in the EDG2 gene, and the frequency of either one allele is higher in an optionally chosen population of patients with bone/joint disease significantly than in an optionally chosen population of patients without bone/joint disease; preferably, the polymorph is an SNP in the base shown by base number 24007 in the base sequence shown by SEQ ID NO:3 [corresponds to the 2820th (-2820) base upstream of the transcription initiation point ("A" shown by base number 26827 in the base sequence shown by SEQ ID NO:3). Hereinafter, the position of each polymorph in the EDG2 gene is indicated by a distance from the transcription initiation point.] [-2820 G>A (hereinafter, any polymorph as mentioned herein is denoted as ""position of polymorph" "base of major allele">"base of minor allele"". "-" means that no base exists, and "N/ins" means that the base N has been inserted.); being the 9th polymorph, from the 5' upstream side, of the 27 polymorphs found in the region consisting of the 5' flanking region, first exon and first intron of the EDG2 gene in Examples below, the polymorph is referred to as "i-EDG2-09". Other polymorphs are named in the same way.] or a polymorph that is in a linkage disequilibrium state of a linkage disequilibrium coefficient D' of 0.9 or more with respect to the SNP. Here, "linkage disequilibrium coefficient D'" can be obtained using the following equation, if each allele of the first SNP of the two SNPs is written as (A, a), each allele of the second SNP as (B, b), and the frequencies of four haplotypes (AB, Ab, aB, ab) are written as $P_{AB}$, $P_{Ab}$, $P_{aB}$, and $P_{ab}$:

$$D' = (P_{AB}P_{ab} - P_{Ab}P_{aB}) / \mathrm{Min}\ [(P_{AB}+P_{aB})(P_{aB}+P_{ab}),\ (P_{AB}+P_{Ab})(P_{Ab}+P_{ab})]$$

[wherein Min [$(P_{AB}+P_{aB})$ $(P_{aB}+P_{ab})$, $(P_{AB}+P_{Ab})$ $(P_{Ab}+P_{ab})$] means that the smaller of the values $(P_{AB}+P_{aB})$ $(P_{aB}+P_{ab})$ and $(P_{AB}+P_{Ab})$ $(P_{Ab}+P_{ab})$ is assumed.]

i-EDG2-09 is an SNP in the 2820th base upstream of the transcription initiation point of the EDG2 gene, registered with the NCBI SNP database (http://www.ncbi.nlm.nih.gov/SNP/) as RefSNP ID: rs10980705, wherein the base of the major allele is "G", and the base of the minor allele is "A". Of them, A allele is an OA-susceptible allele that is found at a high frequency in the OA patient group.

As polymorphs, other than i-EDG2-09, present in the region consisting of the 5' flanking region, first exon and first intron of the EDG2 gene, 17 publicly known polymorphs registered with the NCBI SNP database [RefSNP ID: rs3030215 (-23718 to -23719 ->ACAGGAG/ins; i-EDG2-02), rs4576485 (-14799 T>C; i-EDG2-05), rs10980706 (-3262 A>C; i-EDG2-08), rs9919025 (-2254 C>T; i-EDG2-10), rs4978978 (-2068 C>G; i-EDG2-11), rs10980704 (-1828 A>T; i-EDG2-12), rs10980702 (-1340 G>T; i-EDG2-13), rs10980701 (-1076 G>C; i-EDG2-14), rs12344515 (-866 A>G; i-EDG2-15), rs7858454 (-77 A>G; i-EDG2-17), rs12236414 (1039 G>T; i-EDG2-18), rs7041855 (1637 A>C; i-EDG2-19), rs1861157 (3389 C>T; i-EDG2-21), rs3739706 (7369 C>T; i-EDG2-23), rs3739707 (7660 T>G; i-EDG2-24), rs3739708 (7747 A>T; i-EDG2-25) and rs2287005 (9053 A>G; i-EDG2-27)], and 9 novel polymorphs found in the present invention [-26825 to -26826 - >A/ins (i-EDG2-01), -18363 to -18364 ->T/ins (i-EDG2-03), - 14946 G>A (i-EDG2-04), -13850 A>T (i-EDG2-06), -3289 C>T (i-EDG2-07), -425 T>C (i-EDG2-17), 1728 A>G (i-EDG2-20), 3624 G>A (i-EDG2-22) and 8510 A>G (i-EDG2-26)] can be mentioned. Of them, all polymorphs but i-EDG2-24 are mutually in the relationship of D'>0.9.

Of the above-described polymorphs, one wherein the frequency of either one allele is significantly higher in an optionally chosen population of patients with bone/joint disease than in an optionally chosen population of patients without bone/joint disease can be utilized in the diagnostic method of the present invention. Herein, hereinafter, such a polymorph is sometimes referred to as a bone/joint disease marker polymorph.

Provided that each of the population of patients with bone/joint disease and population of patients without bone/joint disease is a population consisting of a sufficient number of patients to give statistically reliable results, there is no particular limitation on the size thereof (number of samples), background factors of each sample (for example, birth place, age, sex, disease and the like) and the like. As examples of the bone/joint disease, osteoarthritis, osteoporosis, rheumatoid arthritis, arthritis, synovitis, metabolic arthritis, sport-related joint disorder, osteochondroma, osteoma, chondroma, congenital bone system disease and the like can be mentioned, and the disease is preferably osteoarthritis, osteoporosis, rheumatoid arthritis, arthritis, synovitis, metabolic arthritis, sport-related joint disorder, congenital bone system diseases resulting in an cartilage substrate abnormality or the like, more preferably osteoarthritis (OA), still more preferably hip OA (HOA) or knee OA (KOA), particularly KOA. Because there is an ethical need for informed consent of the sample donor, as the population of patients without bone/joint disease, usually, a patient group other than a group of patients with diseases other than bone/joint diseases at a certain medical institution, or a group of subjects diagnosed as not being suffering a bone/joint disease in a screening checkup in a certain area and the like are preferably used.

As polymorphs, out of the above-described 26 polymorphs other than i-EDG2-09, wherein the frequency of either one allele is significantly higher in a population of OA patients than in a population of non-OA patients in case/control correlation analysis in a Japanese general population, i.e., i-EDG2-03, i-EDG2-12 and i-EDG2-25, can be mentioned. For all these polymorphs, the minor alleles (T/ins allele for i-EDG2-03, T allele for i-EDG2-12, T allele for i-EDG2-25) are OA susceptible alleles. These polymorphs are mutually nearly completely linked.

Therefore, in a preferred mode of embodiment, the diagnostic method of the present invention comprises detecting one or more polymorphs out of i-EDG2-03, i-EDG2-09, i-EDG2-12 and i-EDG2-25. More preferably, the polymorph utilized in the diagnostic method of the present invention is i-EDG2-09 or i-EDG2-12, particularly preferably i-EDG2-09. The i-EDG2-09 polymorph influences the transcription activity of the EDG2 gene; in A allele (-2820A), the transcription activity rises under conditions that activate the transcriptional factor AP-1. Therefore, considering the above-described functions of EDG2 (i.e., induction of inflammatory cytokine expression in joints, joint destruction (e.g., articular cartilage destruction) elicitation, elicitation of pain in bone/joint diseases and the like), it is suggested that A allele may exhibit susceptibility not only to osteoarthritis, but also to a bone/joint disease resulting in an cartilage substrate abnormality and symptomatic bone/joint diseases with pain, for example, a disease such as osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma.

Because the i-EDG2-03, i-EDG2-12 and i-EDG2-25 polymorphs, particularly the i-EDG2-12 polymorph, are mutually nearly completely linked with i-EDG2-09, by detecting any one of these polymorphs, it is possible to determine whether or not a haplotype susceptible to the above-described bone/joint disease is retained with high accuracy.

In the diagnostic method of the present invention, polymorph detection can be achieved using any publicly known method of SNP detection. Examples of classical methods of detection include a method comprising performing hybridization with accurate control of stringency in accordance with, for example, the method of Wallace et al. (Proc. Natl. Acad. Sci. USA, 80, 278-282 (1983)), with a genomic DNA extracted from cells or the like of a subject as the sample, using as the probe a nucleic acid containing a continuous base sequence of about 15 to about 500 bases, which is a partial base sequence of the EDG2 gene [for example, the base sequence shown by SEQ ID NO:3 can be referred to. The base sequence corresponds to the base sequence shown by base numbers 112795 to 76917 in the base sequence (complementary strand sequence) registered as GenBank accession No. AC007157 (VERSION: AC007157.6, GI: 4646258, updated on April 27, 1999; herein sometimes simply abbreviated as "AC007157.6").], and which contains the base of one of the above-described polymorph sites (preferably, i-EDG2-03, i-EDG2-09, i-EDG2-12 or i-EDG2-25, more preferably i-EDG2-09 or i-EDG2-12, particularly preferably i-EDG2-09), and detecting only a sequence that is completely complementary to the probe, a method comprising performing hybridization with gradual reductions in reaction temperature from denaturation temperature using a pair of mixed probes which are the above-described nucleic acid and a nucleic acid resulting from substitution of the base(s) at the polymorph site by other base(s) in the above-described nucleic acid, with one of the nucleic acids labeled and the other unlabeled, to allow a sequence completely complementary to one probe to be hybridized in advance, to thereby prevent cross-reactions with the probe containing the mismatch. Here, As the labeling agent, for example, radioisotopes, enzymes, fluorescent substances, luminescent substances and the like are used. As the radioisotopes, for example, [$^{125}$I], [$^{131}$I], [$^{3}$H], [$^{14}$C] and the like are used. As the enzymes described above, stable enzymes with a high specific activity are preferred; for example, beta-galactosidase, beta-glucosidase, alkaline phosphatase, peroxidase, malate dehydrogenase and the like are used. As the fluorescent substance, for example, fluorescamine, fluorescein isothiocyanate and the like are used. As the luminescent substances, for example, luminol, luminol derivatives, luciferin, lucigenin and the like are used.

Preferably, polymorph detection can be performed by, for example, the various methods disclosed in WO 03/023063, for example, the RFLP method, the PCR-SSCP method, ASO hybridization, the direct sequencing method, the ARMS method, the denaturant density gradient gel electrophoresis method, the RNase A cleavage method, the chemical cleavage method, the DOL method, the TaqMan PCR method, the invader method, the MALDI-TOF/MS method, the TDI method, the molecular beacon method, the dynamic allele-specific hybridization method, the padlock probe method, the UCAN method, the nucleic acid hybridization method using a DNA chip or DNA microarray, the ECA method and the like (see WO 03/023063, 5th line on page 17 to 20th line on page 28). Hereinafter, as representative methods, the TaqMan PCR method and the invader method used in Examples below are described in more detail.

(1) TaqMan PCR method

The TaqMan PCR method is a method utilizing a fluorescently labeled allele-specific oligonucleotide (TaqMan probe) and a PCR with Taq DNA polymerase. As a TaqMan probe, an oligonucleotide consisting of a continuous base sequence of about 15 to about 30 bases being a partial base sequence of the EDG2 gene (see, for example, the base sequence shown by SEQ ID NO:3), and containing the base(s) at any of the above-described polymorph sites (preferably, i-EDG2-03, i-EDG2-09, i-EDG2-12 or i-EDG2-25, more preferably i-EDG2-09 or i-EDG2-12, particularly preferably i-EDG2-09), is used. For example, i-EDG2-03 is a polymorph in the base sequence shown by base numbers 8463 to 8464 in the base sequence shown by SEQ ID NO:3, i-EDG2-09 is a polymorph in the base shown by base number 24007 in the base sequence shown by SEQ ID NO:3, i-EDG2-12 is a polymorph in the base shown by base number 24999 in the base sequence shown by SEQ ID NO:3, and i-EDG2-25 is a polymorph in the base shown by base number 34573 in the base sequence shown by SEQ ID NO:3; therefore, as the probe for detection of each polymorph, an oligonucleotide consisting of a continuous partial base sequence of about 15 to about 30 bases, containing one of the numbered bases (or base sequence), is used.

The probe is labeled with a fluorescent dye such as FAM or VIC at the 5' end thereof, and with a quencher (quenching substance) such as TAMRA at the 3' end thereof; when the probe is in the form as it is, the quencher absorbs the fluorescence energy, so that fluorescence will not be detected. It is preferable that the probe be prepared for both alleles, and labeled with fluorescent dyes having mutually different fluorescence wavelengths (for example, FAM for one allele, VIC for the other) for the sake of one-time detection. To prevent a PCR extension reaction from occurring from the TaqMan probe, the 3' end has been phosphorylated. Provided that PCR is performed along with a primer designed to amplify a partial sequence of genomic DNA comprising the region that hybridizes with the TaqMan probe and Taq DNA polymerase, the TaqMan probe hybridizes with the template DNA, and simultaneously an extension reaction from the PCR primer occurs; however, as the extension reaction proceeds, the TaqMan probe hybridized is cleaved by the 5' nuclease activity of Taq DNA polymerase, whereby the fluorescent dye is released and becomes no longer influenced by the quencher, and fluorescence is detected. With the amplification of the template, fluorescence intensity increases exponentially.

For example, in the detection of the i-EDG2-09 polymorph, when the allele-specific oligonucleotide comprising the bases of the polymorph site (about 15 to about 30 bases long; A allele and G allele were labeled with FAM and VIC, respectively, at the 5' end, and both were labeled with TAMRA at the 3' end) was used as TaqMan probe, if the subject's genotype is AA or GG, strong fluorescence intensity of FAM or VIA, respectively, is observed, with almost no fluorescence of the other observed. Meanwhile, if the subject's genotype is AG, fluorescences from both FAM and VIC are detected.

(2) Invader method

In the invader method, unlike in the TaqMan PCR method, the allele-specific oligonucleotide (allele probe) itself is not labeled, but a sequence lacking complementarity to the template DNA (flap) is present on the 5' side of the base of the polymorph site, and a complementary sequence specific for the template is present on the 3' side. In the invader method, further, an oligonucleotide having a complementary sequence specific for the 3' side of the polymorph site of the template (invader probe; the polymorph site which is the 5' end of the base corresponding to the probe is optionally chosen) and a FRET (fluorescence resonance energy transfer) probe wherein a sequence that can assume a hairpin structure on the 5' side is present, and after formation of the hairpin structure, the sequence continuous from the base pairing with the base at the 5' end toward the 3' side is a sequence complementary to the flap of the allele probe, are used. The 5' end of the FRET probe is labeled with a fluorescent substance (for example, FAM, VIC and the like), and a quencher (for example, TAMRA and the like) is bound to the vicinity thereof; no fluorescence will be detected in the state as it is (hairpin structure).
When the genomic DNA which is the template is reacted with an allele probe and an invader probe, the invader probe's 3' end invades the polymorph site upon complementary binding of the three entities. When the single-stranded portion of the allele probe (i.e., flap portion on the 5' side of the polymorph site base) is cut out using an enzyme that recognizes the structure of this polymorph site (cleavase), the flap binds complementarily with the FRET probe, and the polymorph site of the flap enters the hairpin structure of the FRET probe. This structure is recognized and cleaved by the cleavase, whereby the terminally labeled fluorescent dye in the FRET probe is released and becomes no longer influenced by the quencher, and fluorescence is detected. Although allele probes wherein the base of the polymorph site does not match the template are not cleaved by the cleavase, even allele probes that are not cleaved are capable of hybridizing to the FRET probe, so that fluorescence is detected likewise. However, because reaction efficiency differs, fluorescence intensity is remarkably stronger in allele probes wherein the base of the polymorph site matches the template, than in allele probes wherein the base does not matches the template.
Usually, it is preferable that before being reacted with the three kinds of probes and cleavase, the template DNA be previously amplified by PCR using a primer capable of amplifying a region comprising the portion to which the allele probes and invader probe hybridize.
As a result of an examination of polymorphs as described above, if the subject is judged to retain an allele susceptible to a bone/joint disease, particularly if the subject is judged to be a homozygous with respect to the allele, the subject can be judged to have a high genetic susceptibility to the bone/joint disease. For example,

(1) if a T/ins allele is retained for i-EDG2-03,
(2) if A allele is retained for i-EDG2-09,
(3) if T allele is retained for i-EDG2-12, or
(4) if T allele is retained for i-EDG2-25,

the subject can be judged (diagnosed) as being likely to contract (being susceptible to) a bone/joint disease resulting in an cartilage substrate abnormality and symptomatic bone/joint diseases with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA.
When examining two or more polymorphs, if one or more results disagree with other results, the results for the i-EDG2-09 and/or the i-EDG2-12 polymorph prevail, and if the results disagree within these bases, the results for the i-EDG2-09 polymorph prevail.
As stated above, the i-EDG2-03polymorphis a novel polymorph that was for the first time found in the present invention, and is also a marker polymorph that can be utilized for the diagnosis of genetic susceptibility to bone/joint diseases. In more detail, the publicly known base sequence at the position of i-EDG2-03 is "TA" as shown by base numbers 8463 to 8464 in the base sequence shown by SEQ ID NO:3, but the present inventors identified a novel polymorph wherein "thymine (T)" is inserted between the two bases (DIP). Because the frequency of T/ins allele is significantly higher in a population of OA patients than in a population of non-OA patients, the allele is an allele susceptible to a bone/joint disease resulting in an cartilage substrate abnormality and symptomatic bone/joint diseases with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA.
Accordingly, the present invention also provides a nucleic acid being a partial base sequence of the EDG2 gene, and comprising a continuous base sequence of about 15 to about 500 bases (preferably about 15 to about 200 bases, more preferably about 15 to about 50 bases) containing the bases shown by base numbers 8463 to 8464 in the base sequence shown by SEQ ID NO:3 (but "T" is inserted between the two bases). A novel nucleic acid comprising such a polymorph site can preferably used in the above-described diagnostic method of the present invention to detect the i-EDG2-03 polymorph. Such a nucleic acid can be synthesized on the basis of the base sequence information shown by SEQ ID NO:3 using an automated DNA/RNA synthesizer.

The present invention also provides a kit to be used for the above-described the diagnostic method of the present invention. That is, the diagnostic kit of the present invention comprises one pair or more of nucleic acid probe and/or primer capable of detecting one or more polymorphs selected from the group consisting of a polymorph present in the EDG2 gene wherein the frequency of either one allele is higher in an optionally chosen population of patients with bone/joint disease than in an optionally chosen population of patients without bone/joint disease, preferably i-EDG2-09 or each polymorph present in the EDG2 gene in a linkage disequilibrium state (preferably, completely linked) wherein the linkage disequilibrium coefficient D' is 0.9 or more.

Specifically, the nucleic acid probe used in the diagnostic kit of the present invention is a nucleic acid that hybridizes with the genomic DNA in the above-described region containing the bases of the polymorph site to be detected in the diagnostic method of the present invention; as far as it is specific for the target site, and is capable of easily detecting polymorphism, the length thereof (the base length of the portion that hybridizes with genomic DNA) is not particularly limited; for example, the length is about 15 bases or more, preferably about 15 to about 500 bases, more preferably about 15 to about 200 bases, still more preferably about 15 to about 50 bases.
The probe may comprise an additional sequence suitable for polymorphism detection (a sequence not complementary to the genomic DNA). For example, the allele probe used in the above-described invader method has an additional sequence called a flap at the 5' end of the base of the polymorph site.
The probe may be previously labeled with an appropriate labeling agent, for example, a radioisotope (e.g., $^{125}I$, $^{131}I$, $^{3}H$, $^{14}C$ and the like), enzyme (e.g., β-galactosidase, β-glucosidase, alkaline phosphatase, peroxidase, malate dehydrogenase and the like), fluorescent substance (e.g., fluorescamine, fluorescein isothiocyanate and the like), luminescent substance (e.g., luminol, luminol derivative, luciferin, lucigenin and the like) and the like. Alternatively, in the vicinity of a fluorescent substance (e.g., FAM, VIC and the like), a quencher (quenching substance) that absorbs the fluorescence energy produced by the fluorescent substance may further be bound. In this mode of embodiment, upon the detection reaction, the fluorescent substance and the quencher become separated from each other and fluorescence is detected.

Preferably, the nucleic acid probe used in the diagnostic kit of the present invention is a nucleic acid comprising a continuous base sequence of about 15 to about 500 bases, preferably about 15 to about 200 bases, more preferably about 15 to about 50 bases, comprising the bases at a polymorph site selected from the group consisting of the polymorph site (i-EDG2-03) shown by base numbers 8463 to 8464 in the base sequence shown by SEQ ID NO:3, the polymorph site shown by base number 24007 (i-EDG2-09), the polymorph site shown by base number 24999 (i-EDG2-12) and the polymorph site shown by base number 34573 (i-EDG2-25), more preferably the i-EDG2-09 and/or i-EDG2-12 polymorph site, particularly preferably the i-EDG2-09 polymorph site. Here,

(1) the bases shown by base numbers 8463 to 8464 are TA or T(T/ins)A,
(2) the base shown by base number 24007 is G or A,
(3) the base shown by base number 24999 is A or T,
(4) the base shown by base number 34573 is A or T,

and according to the method of polymorph detection used, a nucleic acid having either one base at each polymorph site can be used, and 2 kinds of nucleic acids having bases corresponding to each allele can be used. For the invader probe used in the above-described invader method, the base of the polymorph site (i.e., the base at the 3' end) may be an optionally chosen base.

The nucleic acid primer used in the diagnostic kit of the present invention may be any one, as far as it is designed to be capable of specifically amplifying a region of the genomic DNA comprising the base(s) of the polymorph site to be detected in the above-described diagnostic method of the present invention. For example, a pair of nucleic acids which is a combination of a nucleic acid comprising about 15 to about 50 bases, preferably about 15 to about 30 bases, which is a partial base sequence of the base sequence shown by SEQ ID NO:3, which nucleic acid hybridizes with a portion of a complementary strand sequence on the 5' side from the base(s) of the polymorph site to be detected, and a nucleic acid comprising a base sequence of about 15 to about 50 bases, preferably about 15 to about 30 bases, which nucleic acid hybridizes with a portion of the sequence on the 3' side from the base(s) of the polymorph site, wherein the length of the fragment of the nucleic acid amplified thereby is about 50 to about 1,000 bases, preferably about 50 to about 500 bases, more preferably about 50 to about 200 bases, can be mentioned.
The primer may comprise an additional sequence suitable for polymorphism detection (a sequence not complementary to the genomic DNA), for example, a linker sequence.
The primer may be previously labeled with an appropriate labeling agent, for example, a radioisotope (e.g., $^{125}I$, $^{131}I$, $^{3}H$, $^{14}C$ and the like), enzyme (e.g., β-galactosidase, β-glucosidase, alkaline phosphatase, peroxidase, malate dehydrogenase and the like), fluorescent substance (e.g., fluorescamine, fluorescein isothiocyanate and the like), luminescent substance (e.g., luminol, luminol derivative, luciferin, lucigenin and the like) and the like.

Preferably, the nucleic acid primers used in the diagnostic kit of the present invention are a pair of nucleic acids capable of amplifying a continuous base sequence of about 50 to about 1,000 bases, preferably about 50 to about 500 bases, more preferably about 50 to about 200 bases, comprising the bases at a polymorph site selected from the group consisting of the polymorph site shown by base numbers 8463 to 8464 in the base sequence shown by SEQ ID NO:3 (i-EDG2-03), the polymorph site shown by base number 24007 (i-EDG2-09), the polymorph site shown by base number 24999 (i-EDG2-12) and the polymorph site shown by base number 34573 (i-EDG2-25), more preferably the i-EDG2-09 and/or i-EDG2-12 polymorph site, particularly preferably the i-EDG2-09 polymorph site.

The nucleic acid probe or primer used in the diagnostic kit of the present invention may be a DNA or an RNA, and may be single-stranded or double-stranded. In the case of a double strand, the same may be a double-stranded DNA, a double-stranded RNA, or a DNA/RNA hybrid. Therefore, when a nucleic acid having a certain base sequence is described herein, it should be understood that the term nucleic acid is used with a meaning that encompasses all of single-stranded nucleic acids having the base sequence, single-stranded nucleic acids having a sequence complementary to the base sequence, and double-stranded nucleic acids which are hybrids thereof unless otherwise stated.
The above-described nucleic acid probe or primer can be synthesized, for example, on the basis of information on the base sequence shown by SEQ ID NO:3 using an automated DNA/RNA synthesizer according to a conventional method.

The above-described nucleic acid probe and/or primer can be separately (or in a mixed form if possible) dissolved in water or an appropriate buffer solution (e.g., TE buffer and the like) to obtain an appropriate concentration (e.g., 1 to 50 μM at 2× to 20× concentration and the like), and preserved at about -20°C.
The diagnostic kit of the present invention may further contain other ingredients necessary for the implementation of the method as constituents thereof according to the method of polymorph detection. For example, provided that the kit is for detection of polymorphs by the TaqMan PCR method, the kit can further contain a 10×PCR reaction buffer solution, 10×$MgCl_2$ aqueous solution, 10×dNTPs aqueous solution, Taq DNA polymerase (5 U/μL) and the like.

As shown in Examples below, an intranuclear factor that binds more selectively to A allele (-2820A) is present in i-EDG2-09, suggesting that the factor may act as a transcriptional factor. Therefore, a nucleic acid comprising a base sequence consisting of the base shown by base number 24007 in the base sequence shown by SEQ ID NO:3 (but the base is "adenine (A)") and a base (or base sequence) adjacent thereto, by functioning as a decoy nucleic acid, is capable of inhibiting the binding of the intranuclear factor to the transcription regulatory region of the EDG2 gene to reduce the transcription activity of the EDG2 gene, and can be used as a suppressing agent for inflammatory cytokine expression in joints, joint destruction (e.g., articular cartilage destruction), and pain in a bone/joint disease, and the like, and can therefore be used as a prophylactic/therapeutic agent for a bone/joint disease resulting in an cartilage substrate abnormality and a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA.
Here, "neighboring bases" may exist on any of the 5' upstream and 3' downstream sides of -2820A, and may exist on both sides. The base sequence consisting of -2820A and neighboring bases thereof (or base sequence) is preferably a base sequence consisting of about 5 to about 30 bases, more specifically base sequence consisting of a full length of about 5 to about 30 bases, which consist of -2820A, 0 to 20 bases on the 5' upstream thereof, and 0 to 20 bases on the 3' downstream of 16T.

When the above-described decoy nucleic acid is used as the above-described prophylactic or therapeutic agent, it can be prepared as a pharmaceutical formulation in the same manner as the aforementioned case of anti-EDG2 antagonist and the like.
The preparations thus obtained are safe and less toxic, can be administered to human or other warm-blooded animals (e.g., rats, mice, hamsters, rabbits, sheep, goats, swine, bovine, horses, cats, dogs, monkey, chimpanzee, birds, etc.). The dose of the decoy nucleic acid varies depending on the subject to be administered, the subject organ, symptoms, route for administration, etc.; for example, in oral administration, the dose is normally about 0.1 to 100 mg, preferably about 1.0 to 50 mg, and more preferably about 1.0 to 20 mg per day for an OA patient (as 60 kg body weight). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an OA patient (as 60 kg body weight). In the case that subject to be administered is other than human, the corresponding dose as converted per 60 kg body weight can be administered.

As stated above, an intranuclear factor that binds more selectively to A allele (-2820A) is present in i-EDG2-09, and it is suggested that the factor may act as a transcriptional factor; therefore, a substance that inhibits the binding of the factor to -2820A and the region therearound is effective in the prevention/treatment of bone/joint diseases, bone/joint diseases resulting in an cartilage substrate abnormality, and symptomatic bone/joint diseases with pain, for example, diseases such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA.

Accordingly, the present invention also provides a screening method for a prophylactic/therapeutic substance for the above-described bone/joint disease, comprising using a nucleic acid comprising a base sequence consisting of the base shown by base number 24007 in the base sequence shown by SEQ ID NO:3 (but the base is "A") and a base (or base sequence) adjacent thereto and a transcriptional factor that selectively binds to the base sequence. For example,

1) a method comprising detecting the regulation of the binding of the nucleic acid and the transcriptional factor in the presence of a test compound, and
2) a method comprising comparing the expression, in animal cells comprising a gene under the control of a promoter comprising the nucleic acid, of the gene in the presence and absence of a test compound, and the like can be mentioned.

When the binding of the nucleic acid and the transcriptional factor is used as the index, the screening can be performed by, for example, incubating the nucleic acid, previously labeled (e.g., $^{32}P$, digoxigenin and the like), and the transcriptional factor (which can be provided in the form of, for example, a nuclear extract derived from human or other warm-blooded animal cells, or can also be used after affinity purification from the extract using the nucleic acid) in the presence of a test compound, subjecting the reaction mixture to non-denatured gel electrophoresis, and detecting an increase or decrease in the signal intensity of a band corresponding to the nucleic acid-transcriptional factor complex.

As examples of the test compound, peptides, proteins, non-peptide compounds, synthetic compounds, fermentation products, cell extracts, plant extracts, animal tissue extracts and the like can be mentioned.

When the expression of a gene under the control of a promoter comprising the nucleic acid is used as the index, any promoter capable of functioning in animal cells can be used; for example, the SRα promoter, the SV40 promoter, the LTR promoter, the CMV (cytomegalovirus) promoter, the HSV-tk promoter and the like are used. The nucleic acid can be inserted to an appropriate position in the promoter using a gene engineering technique known per se. Alternatively, a native promoter of the EDG2 gene comprising -2820A may be used.

Although the gene under the control of a promoter comprising the nucleic acid is not subject to limitation, as long as it permits an easy measurement of the expression level thereof, reporter genes such as luciferase, GFP, alkaline phosphatase, peroxidase, β-galactosidase and the like are preferably used. Also, the EDG2 gene comprising -2820A can also be used as is. In this case, a cell or tissue derived from an animal (preferably a human) that naturally carries the EDG2 gene can be used as "an animal cell containing a gene under the control of a promoter comprising the nucleic acid". When a reporter gene is used as the gene under the control of a promoter comprising the nucleic acid, a reporter gene ligated downstream of a promoter comprising the above-described nucleic acid using a gene engineering technique known per se can be inserted to an appropriate transfer vector, for example, a vector such as an *Escherichia* coli-derived plasmid (e.g., pBR322, pBR325, pUC12, pUC13), a *Bacillus* subtilis-derived plasmid (e.g., pUB110, pTP5, pC194), a yeast-derived plasmid (e.g., pSH19, pSH15), or a bacteriophage such as λ phage, and transferred to a host animal cell. The transfer vector may comprise another enhancer, polyA addition signal, selection marker, SV40 replication origin (hereinafter sometimes abbreviated as SV40ori) and the like as required. As examples of the selection marker, the dihydrofolate reductase gene [methotrexate (MTX) resistance], the ampicillin resistance gene, the neomycin resistance gene (G418 resistance) and the like can be mentioned.

The animal cell is not subject to limitation, as long as it is a cell capable of expressing the transcriptional factor; examples of useful animal cells include, but are not limited to, Huh-7 cells and the like. The animal cell can be transformed according to, for example, a method described in Saibo Kogaku (Cell Engineering), extra issue 8, Shin Saibo Kogaku Jikken Protocol (New Cell Engineering Experimental Protocol), 263-267 (1995), published by Shujunsha, and Virology, 52:456 (1973).

As examples of the test compound, peptides, proteins, non-peptide compounds, synthetic compounds, fermentation products, cell extracts, plant extracts, animal tissue extracts and the like can be mentioned.
Cells are cultured for a given time in an appropriate medium (e.g., minimal essential medium, Dulbecco's modified Eagle medium, Ham medium, F12 medium, RPMI1640 medium, William's E medium and the like) in the presence and absence of the test compound, after which the expressions of a gene under the control of a promoter comprising the nucleic acid are compared between those under the two conditions. The expression of the EDG2 gene can be detected and quantified by an immunoassay method such as ELISA using the above-described anti-EDG2 antibody, and the RT-PCR method.
Alternatively, it is also possible to screen for a prophylactic or therapeutic substance for bone and joint diseases by using animal cells that can be used in the above-described method, and comparing the intracellular localizations of the transcriptional factor, for example, the degree of transfer of the factor from cytoplasm to nucleus in the animal cells, in the presence and absence of the test compound. More specifically, for example, by immunostaining the cells with a fluorescein labeled antibody against the transcriptional factor, the transfer of the factor from cytoplasm to nucleus can be monitored. Alternatively, by using a transformant capable of expressing the transcriptional factor in the form of a fusion protein with a fluorescent protein such as GFP, it is also possible to directly monitor the transfer of the factor from cytoplasm to nucleus (see, for example, Biochem. Biophys. Res. Commun., 278: 659-664 (2000)).
In the above-described screening method, a test compound that has inhibited the binding of the nucleic acid and the transcriptional factor can be used as a suppressing agent for inflammatory cytokine expression in joints, joint destruction (e.g., articular cartilage destruction), pain in a bone/joint disease and the like, and can therefore be used as a prophylactic/therapeutic agent for a bone/joint disease resulting in an cartilage substrate abnormality and a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA.
When the above-described binding inhibitory compound is used as the above-described prophylactic/therapeutic agent, the same can be prepared as a pharmaceutical formulation in the same manner as the above for EDG2 antagonist and the like.
The preparations thus obtained are safe and less toxic, can be administered to human or other warm-blooded animals (e.g., rats, mice, hamsters, rabbits, sheep, goats, swine, bovine, horses, cats, dogs, monkey, chimpanzee, birds, etc.). The dose of the above-described binding inhibitory compound varies depending on the recipient, target organ, symptoms, method of administration and the like; in oral administration, the dose is normally about 0.1 to 100 mg, preferably about 1.0 to 50 mg, and more preferably about 1.0 to 20 mg per day for, for example, an osteoarthritis patient (assumed to weigh 60 kg). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an osteoarthritis patient (as 60 kg body weight). In the case that the subject to be administered to is other than human, the corresponding dose as converted per human (60 kg body weight) can be administered.
As stated above, in each of the i-EDG2-03, i-EDG2-09, i-EDG2-12 and i-EDG2-25 polymorphs, the minor alleles (T/ins allele for i-EDG2-03, A allele for i-EDG2-09, T allele for i-EDG2-12, T allele for i-EDG2-25) are alleles susceptible to bone/joint diseases, including OA. For i-EDG2-09, in A allele, compared with G allele, the transcription activity of the EDG2 gene rises under conditions that activate the transcriptional factor AP-1. Meanwhile, because EDG2 induces inflammatory cytokine expression in joints, it is strongly suggested that EDG2 may raise the expression of cartilage substrate decomposing enzymes, and elicit joint destruction (e.g., articular cartilage destruction), and may elicit pain in a bone/joint disease. Judging from the above, it can be thought that in animal individuals having the above-described susceptible allele, inflammatory cytokine expression in joints rises with the overexpression of EDG2, and the risk for causing joint destruction (e.g., articular cartilage destruction), or for manifesting a painful symptom in a bone/joint disease is higher than in individuals having a non-susceptible allele. Therefore, for individuals having the above-described susceptible allele, administration of a substance that suppresses the expression or activity of EDG2 (EDG2 suppressant) is effective in the prevention/treatment of a bone/joint disease, particularly a bone/joint disease resulting in an cartilage substrate abnormality and a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA.
Accordingly, the present invention also provides a suppressing agent for inflammatory cytokine expression in joints, joint destruction (e.g., articular cartilage destruction), and pain in a bone/joint disease, and a prophylactic/therapeutic agent for a bone/joint disease resulting in an cartilage substrate abnormality and a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA, comprising the aforementioned EDG2 suppressant, wherein the suppressing agent or prophylactic/therapeutic agent is administered to an animal individual (preferably, a human) having an allele susceptible to any one of the polymorphs i-EDG2-03, i-EDG2-09, i-EDG2-12 and i-EDG2-25, which is preferably homozygous with respect to the susceptible allele.
In this case as well, the EDG2 suppressant can be prepared as a pharmaceutical formulation by the same technique as the above, and the same dose/route for administration as the above can be used.

EDG2, in response to stimulation by LPA, which is a ligand thereof, induces the expression of inflammatory cytokines, to thereby induce the expression of cartilage substrate decomposing enzymes and elicit articular cartilage destruction, and elicit pain in a bone/joint disease. Therefore, also by blocking the simulation by LPA, an effect as with the aforementioned EDG2 suppressant can be obtained.
Accordingly, the present invention also provides a suppressing agent for inflammatory cytokine expression in joints, joint destruction (e.g., articular cartilage destruction), or pain in a bone/joint disease, and a prophylactic/therapeutic agent for joint destruction (e.g., articular cartilage destruction), a bone/joint disease resulting in an cartilage substrate abnormality and a symptomatic bone/joint disease with pain, for example, a disease such as osteoarthritis, osteoporosis, rheumatoid arthritis, arthritis, synovitis, metabolic arthritis, sport-related joint disorder, congenital bone system disease (e.g., achondroplasia, multiple apophysial dysplasia, vertebral apophysial dysplasia, metaphysial dysplasia, Stickler syndrome, pseudoachondroplasia, multiple exostosis, hemihypertrophy, Ollier's disease, Maffucci's syndrome and the like), osteochondroma, osteoma, or chondroma, preferably OA (for example, HOA or KOA), more preferably KOA, comprising a substance that suppresses the production or activity of LPA.

Examples of the substance that inhibits LPA production include, but are not limited to, substances that inhibit the expression or activity of LPA production enzyme and the like. LPA is produced from phosphatidylcholine by the action of phospholipase D (PLD) and secretory phospholipase $A_2$ ($sPLA_2$), or by the action of phospholipase $A_2$ ($PLA_2$), lecithin cholesterol acyltransferase (LCAT) and autotaxin/lisophospholipase D (ATX/lysoPLD), or from monoacyl glycerol (MAG) by the action of MAG kinase. Therefore, by inhibiting the expression or production of one or more of these enzymes, the production of LPA can be inhibited. As the substance that inhibits the expression of the enzymes, antisense nucleic acids, siRNAs and the like of each enzyme gene can be mentioned. These molecules can be acquired on the basis of nucleotide sequence information on each enzyme gene known per se by the same technique as the above for antisense EDG2. Meanwhile, substances that inhibit the activity of the enzymes, neutralizing antibodies against each enzyme protein, publicly known enzyme inhibitors and the like can be mentioned. As the neutralizing antibody, a commercially available one can be used as it is, and the antibody can also be acquired with each enzyme protein or a peptide fragment thereof as the antigen, by the same technique as the above for anti-EDG2 antibody. As examples of LPA production enzyme inhibitors, $PLA_2$ inhibitors such as chloropromadine, quinacrine, arachidonyl trifluoromethyl ketone, palmityl trifluoromethyl ketone and anti-annexin antibody, PLD inhibitors such as ethanol, 1-butanol, and 2,3-diphosphoglycerate, LCAT inhibitors such as DTNB and PCMPS, ATX inhibitors such as cyclic phosphatidic acid analogues and the like can be mentioned.

The substance that inhibits LPA activity is exemplified by, but is not limited to, the above-described EDG2 antagonists, as well as an antibody against LPA and the like. As the anti-LPA neutralizing antibody, a commercially available one can be used as it is, and the antibody can also be acquired by conjugating LPA as the hapten with an appropriate carrier protein (for example, KLH, albumin and the like), and treating this conjugate as the antigen by the same technique as the above for anti-EDG2 antibody.

An antisense nucleic acid and siRNA of LPA production enzyme gene can be prepared as a pharmaceutical formulation in the same manner as with antisense EDG2. An anti-LPA production enzyme neutralizing antibody or an anti-LPA neutralizing antibody can be prepared as a pharmaceutical formulation in the same manner as with anti-EDG2 antibody. Furthermore, an LPA production enzyme inhibitor can be prepared as a pharmaceutical formulation in the same manner as with EDG2 antagonist.

The dose of the above-described substance that inhibits the production or activity of LPA varies depending on the recipient, target organ, symptoms, method of administration and the like; in oral administration, the dose is normally about 0.1 to 100 mg, preferably about 1.0 to 50 mg, and more preferably about 1.0 to 20 mg per day for, for example, an OA patient (assumed to weigh 60 kg). In parenteral administration, a single dose varies depending on the subject to be administered, the subject organ, symptoms, route for administration, etc.; for example, in injection administration, the dose is normally about 0.01 to 30 mg, preferably about 0.1 to 20 mg, and more preferably about 0.1 to 10 mg per day for an OA patient (as 60 kg body weight). In the case that the subject to be administered to is other than human, the corresponding dose as converted per 60 kg body weight of human can be administered.

In the description and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
DNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediamine tetraacetic acid
SDS : sodium dodecyl sulfate
Gly : glycine
Ala : alanine
Val : valine
Leu : leucine
Ile : : isoleucine
Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gin : glutamine
pGlu : pyroglutamic acid
Sec : selenocysteine

Furthermore, substituent group, protecting group and reagents which are often used in the present specification are denoted as follows.

Me : methyl group
Et : ethyl group
Bu : butyl group
Ph : phenyl group
TC : thiazolidin-4(R)-carboxamide group
Tos : p-toluenesulfonyl
CHO : formal
Bzl : benzyl
Cl2Bzl: 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl

| | |
|---|---|
| DNP : | dinitrophenol |
| Trt : | trityl |
| Bum : | t-butoxymethyl |
| Fmoc : | N-9-fluorenyl methoxycarbonyl |
| Hobs : | 1-hydroxybenztriazole |
| HOOBt : | 3,4-dihydro-3-hydroxy-4-oxo-1,2,3-benztriazine |
| HONE : | 1- hydroxy-5-norbornene-2,3-dicarboximide |
| DCC : | N,N'-dicyclohexylcarbodiimide |

The sequence identification numbers 1 to 3 in the sequence listing herein show the following sequences.

[SEQ ID NO:1]
Shows the base sequence of the CDS of human EDG2 cDNA.
[SEQ ID NO:2]
Shows the amino acid sequence of the human EDG2 protein encoded by the EDG2 gene.
[SEQ ID NO:3]
Shows the base sequence of a region consisting of the 5' flanking region, first exon and first intron of the human EDG2 gene.

Examples

The present invention is hereinafter described more specifically by means of the following Examples, which, however, are for illustrative purposes only and do not limit the invention in any way.

Example 1 Selection of GPCRs expressed in cartilage by oligonucleotide microarray analysis

As a technique for identifying an osteoarthritis (OA) susceptibility gene using a correlation analysis, a candidate gene approach to G protein coupled receptors (GPCRs) expressed in articular cartilage was adopted. By targeting GPCRs as candidate genes for OA susceptibility genes, it is possible to identify a direct drug discovery target with a low molecular compound. It is also possible to identify an OA susceptibility signal transduction pathway, and a molecule involved in the signal can also be investigated as a drug discovery target. However, it is unrealistic to deal with all GPCRs as candidate genes. Hence, it was decided to perform correlation analyses for GPCRs expressed in articular cartilage, which is the major lesion site in OA.

GPCR expression analyses were performed using microarray sets configured with oligonucleotide probes for about 60,000 kinds of target sequences (GeneChip U95 and U133, Affymetrix). A series of operations for biotinylated cRNA preparation, array hybridization and the like were performed as directed in the Affymetrix GeneChip expression analysis manual. RNAs of OA articular cartilage and normal articular cartilage were purchased from Direct Clinical Access Co. with the approval of the in-house ethical committee of Takeda Pharmaceutical Co., Ltd. First, with 5-10 μg of total RNA as the template, using the T7-poly T primer and Superscript II (Invitrogen), a first strand cDNA was synthesized. Next, using E. coli DNA polymerase I (Invitrogen) and a ligase (Invitrogen), a second strand cDNA was synthesized. Using the double-stranded cDNA obtained, in vitro transcription (Ambion) was performed in the presence of biotinylated UTP and CTP (Enzo Diagnostics). Biotinylated cRNA was fragmented by being incubated in a buffer containing Tris (pH 8.1), 100 mM potassium acetate, and 30 mM magnesium acetate at 94˚C for 30 minutes, and array hybridization was performed. Washing and staining were performed using dedicated Fluidics Station (Affymetrix). Signals were detected using dedicated Confocal Scanner (Molecular Dynamics). By the above-described method, a microarray analysis was performed using biotinylated cRNA probes prepared from total RNAs extracted from knee OA patient-derived articular cartilages (n=5), hip OA patient-derived cartilages (n=5), normal knee articular cartilages (n=3) and normal hip articular cartilage (n=3); GPCRs receiving a judgment "presence" in any person were identified as "articular cartilage expression GPCRs". As a result, 64 genes were selected as cartilage expression GPCRs (Table 1).

Table 1

Table 1 GPCR genes shown to be present in articular cartilage by microarray analysis

| Official code | Name of gene |
|---|---|
| *BDKRB2* | bradykinin receptor B2 |
| *CX3CR1* | chemokine (C-X3-C) receptor 1 |
| *CMKLR1* | chemokine-like receptor 1 |

(continued)

| Official code | Name of gene |
|---|---|
| *FPR1* | formyl peptide receptor 1 |
| *FPRL2* | formyl peptide receptor-like 2 |
| *C3AR1* | complement component 3a receptor 1 |
| *AGTR1* | angiotensin II receptor, type 1 |
| *F2R* | coagulation factor II receptor |
| *GABBR1* | GABAB receptor, 1 |
| *ADRA2A* | alpha-2A adrenergic receptor |
| *DRD5* | dopamine receptor D5 |
| *HRH1* | histamime receptor H1 |
| *PTGFR* | prostaglandin F receptor |
| *TBXA2R* | thromboxane A2 receptor |
| *CYSLTR1* | cysteinyl leukotriene receptor 1 |
| *SSTR5* | somatostatin receptor 5 |
| *CALCRL* | calcitonin receptor-like |
| *P2Y5* | purinergic receptor P2Y5 |
| *P2RY4* | pyrimidinergic receptor P2Y4 |
| *EDG1* | endothelial differentiation, sphingolipid G-protein-coupled receptor, 1 |
| *EDG2* | endothelial differentiation, lysophosphatidic acid G-protein-coupled |
| *EDG4* | endothelial differentiation, lysophosphatidic acid G-protein-coupled |
| *CMKOR1* | chemokine orphan receptor |
| *GPR1* | G protein-coupled receptor 1 |
| *GPR64* | G protein-coupled receptor 64 |
| *CXCR4* | chemokine (C-X-C motif), receptor 4 (fusin) |
| *EDNRA* | endothelin receptor type A |
| *EDNRB* | endothelin receptor type B |
| *PLAUR* | plasminogen activator, urokinase receptor |
| *PTGER2* | prostaglandin E receptor 2 |
| *PTGER3* | prostaglandin E receptor 3 |
| *PTHR1* | parathyroid hormone receptor 1 |
| *ADORA1* | adenosine A1 receptor |
| *ADORA2A* | adenosine A2a receptor |
| *ADORA3* | adenosine A3 receptor |
| *NPY2R* | neuropeptide Y peptide YY Y2 receptor |
| *BDKRB1* | bradykinin receptor B1 |
| *CCR9* | CC chemokine receptor 9 |
| *OPRM1* | opioid receptor, mn 1 |
| *OXTR* | oxytocin receptor |
| *GRM4* | metabotropic glutamate receptor 4 |
| *GRM6* | metabotropic glutamate receptor 6 |
| *GRM7* | metabotropic glutamate receptor 7 |
| *GRM8* | metabotropic glutamate receptor 8 |
| *GPR51/ GABBR2* | GABA-B receptor R2 |
| *ADRA1A* | alpha-1A adrenergic receptor |
| *CHRM3* | m3 muscarinic acetylcholine receptor |
| *DRD2* | dopamine receptor D2 |
| *HRH4* | histamine H4 receptor |
| *PTGDR* | prostaglandin D2 receptor |
| *SSTR2* | somatostatin receptor 2 |
| *GHRHR* | growth hormone releasing hormone receptor |
| *GNRHR* | gonadotropin-releasing hormone receptor |

(continued)

| Official code | Name of gene |
|---|---|
| *CRHR2* | corticotropin releasing hormone receptor 2 |
| *CNR2* | cannabinoid receptor 2 |
| *P2RY1* | purinergic receptor P2Y, G-protein coupled, 1 |
| *P2RY10* | purinergic receptor P2Y, G-protein coupled, 10 |
| *GPR2* | G protein-coupled receptor 2/ CC chemokine receptor 10 |
| *GPR3* | G protein-coupled receptor 3 |
| *GPR6* | G protein-coupled receptor 6 |
| *GPR12* | G protein-coupled receptor 12 |
| *GPR65* | G protein-coupled receptor 65 |
| *GPR68* | G protein-coupled receptor 68/ Proton-sensing receptor |
| *GPR91* | G-protein coupled receptor 91 |

Example 2 Selection of genotyping probes

For performing a correlation analysis, it is necessary to determine the genotypes of single nucleotide polymorphism (SNP) present on each gene in each individual in a diseased group (cases) and a control group (controls). Hence, it was decided to determine genotypes (genotyping) using invader probes stocked by the RIKEN SNP Research Center (RIKEN SRC). For the 64 cartilage expression GPCR genes selected in Example 1, database search was performed for the presence or absence of invader probes stocked by the RIKEN SRC. As a result, 47 genes were found to have invader probes registered.

For 8 genes wherein the number of probes per gene is 1, it was decided to perform genotyping using the probe without limitation on conditions. For the remaining 39 genes wherein the number of probes per gene is 2 or more, it was decided to select haplotype tagging SNPs (htSNPs) using the genotype data from the RIKEN SRC, and use them as genotyping probes. The procedures for selection of the htSNPs are described below. First, all invader probes present in the 39 genes were checked against the RIKEN SRC's genotyping data, and 544 probes fulfilling Hardy-Weinberg's equilibrium (HWE) were selected ($P \geq 0.01$). These 544 probes included probes wherein the minor allele frequency (MAF) was 10% or more; from genotype data thereon, haplotype blocks were estimated using Gabriel's method. For each haplotype block estimated, SNPs that express all the haplotypes occurring at a frequency of 1% or more in the block were selected as htSNPs. Of the 39 genes for which selection of htSNPs was performed, 37 genes were found to have 1 to 11 htSNPs per gene. The total number of htSNPs for these genes was 162. For the remaining 2 genes (GRM7 and GRM8), the number of htSNPs was 45 and 47, respectively. These 2 genes, because of necessity for typing a larger number of htSNPs than other genes, were excluded from this screening. SNPs on X chromosome were also excluded from the subjects of analysis. After the processes shown above, 167 SNPs for 44 genes were finally identified as subject SNPs for genotyping (Table 2).

Table 2 Candidate genes and primary screening subject SNPs

| Official code | Name of gene | Position | dbsnp |
|---|---|---|---|
| *EDG1* | endothelial differentiation, sphingolipid G-protein-coupled receptor, 1 | 1p21 | rs3765684<br>rs3753194<br>rs3737578<br>rs3737577<br>rs3765683<br>rs2295337 |
| *PTGFR* | prostaglandin F receptor (FP) | 1p31.1 | rs3753380<br>rs3766355<br>rs3766354<br>rs1555541<br>rs3766348<br>rs3766346<br>rs3766345<br>rs3766344<br>rs3766343<br>rs668005<br>rs3766333 |
| *PTGER3* | prostaglandin E receptor 3 (subtype EP3) | 1p31.2 | rs2072947<br>rs541667<br>rs1409164<br>rs646511<br>rs2300166<br>rs2247098<br>rs2300176 |
| *ADORA1* | adenosine A1 receptor | 1q32.1 | rs2275349<br>rs3766568<br>rs3753476<br>rs3766562<br>rs3753474<br>rs3766560<br>rs3766558<br>rs3753473<br>rs3753472 |
| *CXCR4* | chemokine (C-X-C motif) receptor 4 | 2q21 | rs2228014 |
| *CALCRL* | calcitonin receptor-like | 2q32.1 | rs698548<br>rs858745<br>rs1983492<br>rs3771074<br>rs3771073<br>rs3771072<br>rs1355519 |
| *GPR1* | G protein-coupled receptor 1 | 2q33.3 | rs3732083<br>rs3732082<br>rs3755227 |
| *RDC1* | chemokine orphan receptor 1 | 2q37.3 | rs3827553 |
| *CX3CR1* | chemokine (C-X3-C motif) receptor 1 | 3p21.3 | rs3732378<br>rs3732379<br>rs3732380 |
| *CCR9* | chemokine (C-C motif) receptor 9 | 3p21.3 | rs2286486<br>rs3764864<br>rs3774642<br>rs2236938 |
| *PTHR1* | parathyroid hormone receptor 1 | 3p22-p21.1 | rs2242117<br>rs724450<br>rs3792556<br>rs936184 |
| *OXTR* | oxytocin receptor | 3p25 | rs2268491<br>rs2268494<br>rs237897<br>rs237911<br>rs237913<br>rs2301261 |
| *AGTR1* | angiotensin II receptor, type 1 | 3q21-q25 | rs2640539<br>rs3772630<br>rs3772622<br>rs909383<br>rs3772620<br>rs3772616<br>rs388915<br>rs3772615<br>rs5186 |
| *P2RY1* | purinergic receptor P2Y, G-protein coupled, 1 | 3q25.2 | rs3755711 |
| *GNRHR* | gonadotropin-releasing hormone receptor | 4q21.2 | rs3796720<br>rs3756159 |
| *NPY2R* | neuropeptide Y receptor Y2 | 4q31 | rs1047214<br>rs2880415 |
| *EDNRA* | endothelin receptor type A | 4q31.23 | rs1996373<br>rs3756022<br>rs2292766<br>rs2292764<br>rs5335<br>rs5342 |
| *F2R* | coagulation factor II (thrombin) receptor | 5q13 | rs27135<br>rs37243<br>rs2227774 |
| *GRM6* | glutamate receptor, metabotropic 6 | 5q35 | rs2856354<br>rs3733915<br>rs2256966<br>rs2071246 |
| *GRM4* | glutamate receptor, metabotropic 4 | 6p21.3 | rs2229901<br>rs3798528<br>rs3734361<br>rs3734360<br>rs963733<br>rs3756927 |
| *GABBR1* | gamma-aminobutyric acid (GABA) B receptor, 1 | 6p21.31 | rs2267633<br>rs2076482<br>rs29221<br>rs29220 |
| *OPRM1* | opioid receptor, mu 1 | 6q24-q25 | rs1799971<br>rs3798688 |
| *GHRHR* | growth hormone releasing hormone receptor | 7p14 | rs2302019<br>rs2302020<br>rs2302021<br>rs2302022<br>rs3735429<br>rs2270026 |
| *CRHR2* | corticotropin releasing hormone receptor 2 | 7p15.1 | rs2240403 |
| *ADRA1A* | adrenergic, alpha-1A-, receptor | 8p21-p11.2 | rs2291775<br>rs3739216<br>rs1048101<br>rs1383914 |
| *GABBR2 (GPR51)* | gamma-aminobutyric acid (GABA) B receptor, 2 | 9q22.1-q22.3 | rs970388 |
| *EDG2* | endothelial differentiation, lysophosphatidic acid G-protein-coupled receptor, 2 | 9q31.3 | rs3739709<br>rs3780528<br>rs3780525<br>rs3780523<br>rs2277179<br>rs3739708<br>rs3739707<br>rs3739706 |
| *ADRA2A* | adrenergic, alpha-2A-, receptor | 10q24-q26 | rs1800038<br>rs553668<br>rs3750625 |
| *DRD2* | dopamine receptor D2 | 11q23 | rs2242592<br>rs1079594<br>rs1125394 |
| *C3AR1* | complement component 3a receptor 1 | 12p13.31 | rs2072449 |
| *CMKLR1* | chemokine-like receptor 1 | 12q24.1 | rs2280169 |
| *EDNRB* | endothelin receptor type B | 13q22 | rs3818416<br>rs2296281<br>rs2070591<br>rs3759476<br>rs3759475 |
| *PTGER2* | prostaglandin E receptor 2 (subtype EP2), 53kDa | 14q22 | rs1254600<br>rs2075797<br>rs17197 |
| *GPR68* | G protein-coupled receptor 68 | 14q31 | rs2236324 |
| *GPR65* | G protein-coupled receptor 65 | 14q31-q32.1 | rs3742704 |
| *BDKRB1* | bradykinin receptor B1 | 14q32.1-q32.2 | rs2071084 |
| *BDKRB2* | bradykinin receptor B2 | 14q32.1-q32.2 | rs5224<br>rs3809418 |
| *SSTR5* | somatostatin receptor 5 | 16p13.3 | rs2076419<br>rs169068 |
| *EDG4* | endothelial differentiation, lysophosphatidic acid G-protein-coupled receptor, 4 | 19p12 | rs1054685<br>rs873870 |
| *TBXA2R* | thromboxane A2 receptor | 19p13.3 | rs3786990<br>rs3786989<br>rs2238630<br>rs2238631<br>rs2238632<br>rs2238634 |
| *PLAUR* | plasminogen activator, urokinase receptor | 19q13 | rs2302524<br>rs2302525<br>rs2239372<br>rs2239374<br>rs2286960<br>rs3760977 |
| *FPRL2* | formyl peptide receptor-like 2 | 19q13.3-q13.4 | rs3732125 |
| *FPR1* | formyl peptide receptor 1 | 19q13.4 | rs867228<br>rs1042229<br>rs2070746<br>rs2070745 |
| *ADORA2A* | adenosine A2a receptor | 22q11.23 | rs3810617<br>rs2267076<br>rs2236624<br>rs2236625 |

Example 3 Selection of knee OA susceptibility polymorphs using stepwise screening

For the correlation analysis, the 2-stage stepwise screening technique was used. The case population used in the screening was a group of patients who visited a number of medical institutions in Japan, in whom an OA change in the knee joint was observed in X-ray diagnostic imaging (radiographic OA patient population). For primary screening, genotyping was performed on genomic DNAs extracted from a population of 368 Japanese knee OA patients and a population of 323 controls by the invader method according to the method of Ohnishi et al. [J Hum Genet. 2001;46(8): 471-7.]. Of the 167 SNPs subjected to the genotyping, 4 SNPs exhibited no polymorphism. Also, 5 SNPs did not fulfill the HWE ($P \geq 0.01$) in the control population; furthermore, 6 SNPs did not permit genotyping because probes did not work. For 155 SNPs, excluding these SNPs, a correlation analysis was performed by $\chi^2$ test or Fisher's exact test; 13 SNPs exhibited a correlation of $P < 0.05$. For these 13 SNPs, genotyping was performed in a population of 276 Japanese knee OA patients that did not overlap the primary screening, and a correlation analysis was performed using genotype data on a population of 654 controls registered with the RIKEN SRC database; 2 SNPs (rs3739216 and rs3739708) exhibited a correlation of $P < 0.05$. However, in rs3739216, the tendencies of MAF in primary screening and secondary screening reversed between the knee OA population and the control population; an analysis on both screening data in combination yielded $P > 0.05$, so that the data was rejected. Eventually, rs3739708 passed the two stages of screening (Table 3). This screening suggested that knee OA susceptibility polymorphs may be present in a linkage disequilibrium (LD) region where rs3739708 is present.

Table 3

Table 3 Correlation of ra3739708 and Knee OA in two independent screening populations

| screening populations | Genotypes[a] | | | | | | | | | | P values | | | |
|---|---|---|---|---|---|---|---|---|---|---|---|---|---|---|
| | Diseased | | | | | Controls | | | | | | | | |
| | 11 | 12 | 22 | Total | Frequency[b] (%) | 11 | 12 | 22 | Total | Frequency[b] (%) | Genotype | Allele | 11 vs | 22 vs |
| 1 | 25 | 123 | 220 | 368 | 6.8 | 11 | 113 | 196 | 320 | 3.4 | 0.14 | 0.28 | 0.049 | 0.69 |
| 2 | 20 | 102 | 154 | 276 | 7.2 | 23 | 217 | 414 | 654 | 3.5 | 0.015 | 0.007 | 0.013 | 0.032 |

a 11, 12 and 22 indicate a minor allele homozygote and heterozygote and a major allele homozygote, respectively.
b Frequency of genotype 11

Example 4 Determination of rs3739708 linkage disequilibrium region using Japanese knee OA population

By the 2-stage screening shown in Example 3, rs3739708, which exhibited a high correlation with knee OA, was selected. Judging from the common disease-common variant hypothesis, true knee OA susceptibility polymorphs are thought to be present in LD regions as in rs3739708. We performed genotyping of the knee OA patient group used in the primary screening for polymorphs present in an about 800kb region, including rs3739708, using invader probes or TaqMan probes already designed by Applied biosystems Co. (probes denoted as Invader or TaqMan in the "Probe type" column in Table 4). Of them, polymorphs wherein the MAF is 10% or more were used to calculate the linkage disequilibrium constant D' for rs3739708. As a result, polymorphs having a relation of D' > 0.9 to rs3739708 were rs7041855 and rs3739707 (Fig. 1a). Meanwhile, because the D' for rs11794726, which is located about 35 kb upstream of rs3739708, and the D' for rs12353088, which is located about 10 kb downstream, were 0.69 and 0.40, respectively, it was suggested that the LD region wherein rs3739708 was present might be present in the about 45 kb region between these 2 SNPs (Fig. 1a). This region is designated as the rs3739708 LD region.

[Table 4]

Table 4 Polymorphs in the rs3739708 LD region

| Number | dbSNP ID | Synonym used in this application | Distance from KDG2 transcription initiation point | Probe type | MAF [a](%) |
|---|---|---|---|---|---|
| 1 | rs3739705 | | -435,250 | Invader | 18.5 |
| 2 | rs744324 | | -392,680 | Invader | 18.4 |
| 3 | rs2297529 | | -379,447 | Invader | 37.0 |
| 4 | rs3818719 | | -373.324 | Invader | 34.8 |
| 5 | n.2274899 | | -373,199 | Invader | 34.8 |
| 6 | rs2274897 | | -338.056 | Invader | 18.3 |
| 7 | rs1571978 | | -333.473 | Invader | 27.1 |
| 8 | rs3780522 | | -324,999 | Invader | 35.7 |
| 9 | rs2014343 | | -58,946 | TaqMan | 41.2 |
| 10 | rs4475574 | | -31,679 | TaqMan | 31.8 |
| 11 | rs4246886 | | -31,279 | TaqMan | 31.8 |
| 12 | rs4246885 | | -31,254 | TsqMsa | 31.7 |
| 13 | rs4978979 | | -30.954 | TaqMan | 6.7 |
| 14 | rs741224 | | -29.205 | TaqMan | 7.8 |
| 15 | rs741223 | | -28,895 | TaqMsu | 7.8 |
| 16 | rs11794726 | | -27,256 | TaqMan | 17.8 |
| 17 | Novel | i-*EDG2*-01 | -26825-26826 | Discoverd SNP | 2.3 |
| 18 | rs3030215 | i-*EDG2*-02 | -23718--23719 | Discoverd SNP | 6.5 |
| 19 | Novel | i-*EDG2*-03 | -18363--18364 | Discoverd SNP | 28.3 |
| 20 | Novel | i-*EDG2*-04 | -14,946 | Discoverd SNP | 12.9 |
| 21 | rs4576495 | i-*EDG2*-05 | -14,799 | TaqMan | 6.6 |
| 22 | Novel | i-*EDG2*-06 | -13,850 | Discoverd SNP | 1.6 |
| 23 | Novel | i-*EDG2*-07 | -3,289 | Disocverd SNP | 13.0 |
| 24 | rs10980706 | i-*EDG2*-08 | -3,262 | Discoverd SNP | 48.6 |
| 25 | rs10980705 | i-*EDG2*-09 | -2,820 | Discoverd SNP | 29.1 |
| 26 | n9919025 | i-*EDG2*-10 | -2,254 | TaqMan | 3.0 |
| 27 | rs4978978 | i-*EDG2*-11 | 2,068 | Discoverd SNP | 6.6 |
| 28 | rs10980704 | i-*EDG2*-12 | -1,828 | Discoverd SNP | 31.3 |
| 29 | rs10980702 | i-*EDG2*-13 | -1,340 | Discoverd SNP | 6.7 |
| 30 | rs10980701 | i-*EDG2*-14 | -1,076 | Discoverd SNP | 6.6 |
| 31 | is12344515 | i-*EDG2*-15 | -866 | Discoverd SNP | 49.6 |
| 32 | Novel | i-*EDG2*-16 | -425 | Discoverd SNP | 48.5 |
| 33 | rs7858454 | i-*EDG2*-17 | -77 | Discoverd SNP | 6.5 |
| 34 | rsl2236414 | i-*EDG2*-18 | 1,039 | Discoverd SNP | 23.7 |

(continued)

| Number | dbSNP ID | Synonym used in this application | Distance from KDG2 transcription initiation point | Probe type | MAF a(%) |
|---|---|---|---|---|---|
| 35 | rs7041855 | i-*EDG2*-19 | 1,637 | TaqMan | 41.8 |
| 36 | Novel | i-*EDG2*-20 | 1,728 | Discoverd SNP | L1 |
| 37 | a1861157 | i-*EDG2*-21 | 3,389 | Discoverd SNP | 6.6 |
| 38 | Novel | i-*EDG2*-22 | 3,624 | Discoverd SNP | 13.0 |
| 39 | n3739706 | i-*EDG2*-23 | 7,369 | Invader | 5.1 |
| 40 | rs3739707 | i-*EDG2*-24 | 7,660 | Invader | 41.7 |
| 41 | rs3739708 | i-*EDG2*-25 | 7,747 | Invader | 23.6 |
| 42 | Novel | i-*EDG2*-26 | 8,510 | Discoverd SNP | 5.1 |
| 43 | rs2287005 | i-*EDG2*-27 | 9,053 | Invader | 3.3 |
| 44 | rs12353088 | | 17,344 | TaqMan | 43.0 |
| 45 | rs2110548 | | 17,432 | TaqMan | 39.3 |
| 46 | rs1030006 | | 21,355 | TaqMan | 39.3 |
| 47 | rs981286 | | 27,105 | TaqMan | 3.8 |
| 48 | rs2192593 | | 36.037 | TaqMan | 3,8 |
| 49 | rs10980675 | | 58,446 | TaqMan | 19.5 |
| 50 | rs1030004 | | 59,660 | TaqMan | 17.2 |
| 51 | rs3758283 | | 63.187 | Invader | 1.1 |
| 52 | rs2277179 | | 65,756 | Invader | 3.5 |
| 53 | rs3780523 | | 101,461 | Invader | 22.5 |
| 54 | rs3780525 | | 108,940 | Invader | 9.7 |
| 55 | rs3780526 | | 108,961 | Invader | 40.5 |
| 56 | rs3780527 | | 132,941 | Invader | 44.1 |
| 57 | rs3780528 | | 133,089 | Invader | 4.2 |
| 58 | rs3739709 | | 162,512 | Invader | 18.6 |
| 59 | rs955888 | | 162,704 | Invader | 0.4 |
| 60 | rs3780529 | | 239,115 | Invader | 19.2 |
| 61 | rs1170327 | | 292,890 | Invader | 27.6 |
| 62 | rs2274419 | | 262,244 | Invader | 25.0 |
| 63 | rs2298492 | | 287,593 | Invader | 34.8 |
| 64 | rs3824468 | | 290,807 | Invader | 0.3 |
| 65 | rs3808886 | | 369,557 | Invader | 0.4 |

a Minor allele frequency in the diseased population in primary screening

Example 5 Polymorph discovery in the rs3739708 LD region

To identify true knee OA susceptibility polymorphs, polymorph discovery was performed on the rs3739708 LD region according to the method of Iida et al. [J Hum Genet. 2005;50(1):42-5.]. The method is briefly described below. Genomic DNAs were extracted from 48 persons, and 16 kinds of mixed genomes of 3 individuals each were used. On the basis of a genome sequence reported in the Genbank database (AC007157.6), primers that amplify the region between rs11794726 and rs12353088 were prepared. Using these primers, PCR was performed on 20 ng of each mixed genomic DNA. The PCR was performed using the GeneAmp PCR system (Applied biosystems), under the conditions shown below. Specifically, after initial denaturation was performed at 94˚C for 2 minutes, a reaction involving three temperatures, at 94˚C for 30 seconds (thermal denaturation), at 60˚C for 30 seconds (annealing), and at 72˚C for 2 minutes (extension reaction), as one cycle, was carried out in 35 cycles, and finally, a final extension reaction was carried out at 72˚C for 7 minutes. Sequencing was performed by a cycle sequence method using a fluorescent dye terminator, with the PCR reaction product as the template. All gene polymorphs were determined using the PolyPhred computer program. As a result, it was found that in the region for which polymorph discovery was performed, there were 24 SNPs and 3 insertion/deletion polymorphs (Fig. 1b, Table 5). Of them, 9 polymorphs were found to be novel polymorphs that have not been registered with the dbSNP published by NCBI.

[Table 5]

Table 5 Polymorphs in the rs3739708 LD region

| Polymorph ID | dbSNP ID | Distance from transcription initiation point | Allele | |
|---|---|---|---|---|
| | | | Major[a] | Minor[a] |
| i-*EDG2*-01 | Novel | -26825~26825 | - | A/ins |
| i-*EDG2*-02 | rs3030215 | -23718~23719 | - | ACAGGAG/del |
| i-*EDG2*-03 | Novel | -18363~18364 | - | T/ins |
| i-*EDG2*-04 | Novel | -14946 | G | A |
| i-*EDG2*-05 | rs4576485 | -14799 | T | C |
| i-*EDG2*-06 | Novel | -13850 | A | T |
| i-*EDG2*-07 | Novel | -3289 | C | T |
| i-*EDG2*-08 | rs10980706 | -3262 | A | C |
| i-*EDG2*-09 | rs10980705 | -2820 | G | A |
| i-*EDG2*-10 | rs9919025 | -2254 | C | T |
| i-*EDG2*-11 | rs4978978 | -2068 | C | G |
| i-*EDG2*-12 | rs10980704 | -1828 | A | T |
| i-*EDG2*-13 | rs10990702 | -1340 | G | T |
| i-*EDG2*-14 | rs10980701 | -1076 | G | C |
| i-*EDG2*-15 | rs12344515 | -866 | A | G |
| i-*EDG2*-16 | Novel | -425 | T | C |
| i-*EDG2*-17 | rs7958454 | -77 | A | G |
| i-*EDG2*-18 | rs12236414 | 1039 | G | T |
| i-*EDG2*-19 | rs7041855 | 1637 | A | C |
| i-*EDG2* -20 | Novel | 1728 | A | G |
| i-*EDG2*-21 | rs1861157 | 3389 | C | T |
| i-*EDG2*-22 | Novel | 3624 | G | A |
| i-*EDG2*-23 | rs3739706 | 7369 | C | T |
| i-*EDG2*-24 | rs3739707 | 7660 | T | G |
| i-*EDG2*-25 | rs3739708 | 7747 | A | T |
| i-*EDG2*-26 | Novel | 8510 | A | G |
| i-*EDG2*-27 | rs2287005 | 9053 | A | G |

a Based on calculations in disease population in primary screening.

Example 6 Confirmation of the rs3739708 LD region

For the polymorphs whose existence was demonstrated in Example 5, a TaqMan probe for genotyping was prepared by Assays-by-Design service (Applied biosystems). Using this TaqMan probe, with genomic DNAs (368 persons) collected from individuals in the knee OA patient group used in the primary screening as the samples, a genotyping reaction was carried out on a 384-multiwell plate. Detection was performed using ABI Prism 7900 (Applied biosystems), and the data was analyzed using Sequence Detection Software (Applied biosystems). For the polymorphs for which no TaqMan probe could be prepared, a PCR primer that amplifies the polymorph region was prepared, PCR was performed with genomic DNA as the template, and genotyping was performed according to the method described in Example 5. Using genotype data on selected polymorphs wherein the MAF is 10% or more, and polymorphs, including rs3739708, present in the 800 kb region used in Example 4, D' was calculated pairwise, and an LD map was generated (Fig. 1c). As a result, it was found that the polymorphs present in the rs3739708 LD region in Example 5 were mutually in the relationship of D'>0.9 (for i-EDG2-24 only, D' > 0.5).

Example 7 Correlation analysis of polymorphs in the rs3739708 LD region in general Japanese population

For all polymorphs present in the rs3739708 linkage disequilibrium region, genotyping was performed on 644 knee OA patients (the population used in the primary and secondary screening) and a population of 640 controls, according to the methods described in Examples 4 and 6, and a correlation analysis was performed. As a result, three polymorphs exhibiting a stronger correlation than with rs3739708 (i-EDG2-25) were identified (Table 6). All these polymorphs exhibited the strongest correlation in a minor allele recessive model. These polymorphs were mutually in a nearly complete linkage (Δ > 0.85).

[Table 6]

Table 6 Correlation of polymorphs in the rs3739708 LD region and knee QA in general Japanese population

| Polymorh ID (i-*EDG2*-x) | Genotype[a] | | | | | | | | 11 Frequency (%) | | P value | | |
|---|---|---|---|---|---|---|---|---|---|---|---|---|---|
| | Diseased | | | | Controls | | | | Diseased | Controls | | | |
| | 11 | 12 | 22 | | 11 | 12 | 22 | 計 | | | Allege | 11 vs | 22 vs |
| 1 | 0 | 31 | 613 | 644 | 0 | 26 | 613 | 639 | 0.0 | 0.0 | 0.5 | - | 0.5 |
| 2 | 0 | 83 | 561 | 644 | 4 | 74 | 562 | 640 | 0.0 | 0.6 | 1.0 | 0.04 | 0.7 |
| 3 | 57 | 254 | 333 | 644 | 29 | 252 | 359 | 640 | 8.9 | 4.5 | 0.01 | 0.002 | 0.1 |
| 4 | 9 | 154 | 481 | 644 | 12 | 159 | 466 | 637 | 1.4 | 1.9 | 0.5 | 0.5 | 0.5 |
| 5 | 0 | 84 | 560 | 644 | 4 | 74 | 560 | 638 | 0.0 | 0.6 | 0.9 | 0.04 | 0.7 |
| 6 | 0 | 19 | 622 | 641 | 0 | 29 | 608 | 637 | 0.0 | 0.0 | 0.1 | - | 0.1 |
| 7 | 9 | 152 | 482 | 643 | 10 | 150 | 472 | 632 | 1.4 | 1.6 | 0.9 | 0.8 | 0.9 |
| 8 | 155 | 329 | 159 | 643 | 135 | 310 | 193 | 638 | 24.1 | 21.2 | 0.03 | 0.2 | 0.03 |
| 9 | 64 | 255 | 325 | 644 | 33 | 251 | 356 | 640 | 9.9 | 5.2 | 0.005 | 0.001 | 0.1 |
| 10 | 0 | 37 | 606 | 643 | 3 | 30 | 604 | 637 | 0.0 | 0.5 | 0.9 | 0.1 | 0.7 |
| 11 | 0 | 86 | 558 | 644 | 5 | 77 | 556 | 638 | 0.0 | 0.8 | 0.9 | 0.02 | 0.8 |
| 12 | 74 | 266 | 304 | 644 | 38 | 275 | 327 | 640 | 11.5 | 5.9 | 0.009 | 0.0004 | 0.2 |
| 13 | 0 | 86 | 556 | 642 | 6 | 77 | 557 | 640 | 0.0 | 0.9 | 0.8 | 0.01 | 0.8 |
| 14 | 0 | 87 | 557 | 644 | 4 | 78 | 556 | 638 | 0.0 | 0.6 | 1.0 | 0.04 | 0.7 |
| 15 | 151 | 326 | 166 | 643 | 140 | 316 | 182 | 638 | 23.5 | 21.9 | 03 | 0.5 | 03 |
| 16 | 154 | 329 | 160 | 643 | 133 | 311 | 194 | 638 | 24.0 | 20.8 | 0.03 | 0.2 | 0.03 |
| 17 | 0 | 84 | 559 | 643 | 4 | 78 | 556 | 638 | 0.0 | 0.6 | 0.8 | 0.04 | 0.9 |
| 18 | 48 | 225 | 371 | 644 | 30 | 221 | 387 | 638 | 7.5 | 4.7 | 0.08 | 0.04 | 03 |
| 19 | 119 | 315 | 209 | 643 | 106 | 282 | 250 | 638 | 18.5 | 16.6 | 0.03 | 0.4 | 0.01 |
| 20 | 1 | 13 | 629 | 643 | 0 | 16 | 622 | 638 | 0.2 | 0.0 | 0.8 | 0.3 | 0.7 |
| 21 | 0 | 86 | 558 | 644 | 4 | 80 | 556 | 640 | 0.0 | 0.6 | 0.8 | 0.04 | 0.9 |
| 22 | 9 | 152 | 483 | 644 | 10 | 152 | 476 | 638 | 1.4 | 1.6 | 0.8 | 0.8 | 0.9 |
| 23 | 0 | 64 | 580 | 644 | 2 | 69 | 567 | 638 | 0.0 | 03 | 0.4 | 0.2 | 0.5 |
| 24 | 111 | 327 | 206 | 644 | 99 | 302 | 236 | 637 | 17.2 | 15.5 | 0.08 | 0.4 | 0.1 |
| 25 | 45 | 225 | 373 | 643 | 22 | 212 | 404 | 638 | 7.0 | 3.4 | 0.007 | 0.004 | 0.1 |
| 26 | 0 | 66 | 578 | 644 | 2 | 69 | 567 | 638 | 0.0 | 0.3 | 0.5 | 0.2 | 0.6 |
| 27 | 2 | 36 | 604 | 642 | 3 | 55 | 578 | 636 | 03 | 0.5 | 0.03 | 0.6 | 0.03 |

a 11, 12 and 22 indicate a minor allele homozygote and hetezygote and a major allle homozygote, respectively.

Example 8 Haplotype analysis

Using the genotyping data obtained in Example 7, a haplotype analysis in the rs3739708 linkage disequilibrium region was performed. The SNPs used in the haplotype analysis were 13 SNPs wherein the minor allele frequency exceeded 10%. As a result, it was found that in this region, eight haplotypes accounted for 95% of the entire study population (population of knee OA patients and controls) (Table 7). When these haplotypes were subjected to a correlation analysis, no haplotype exhibiting a higher correlation than the three SNPs identified in Example 7 was observed (Table 7). This finding strongly suggests that in the rs3739708 linkage disequilibrium region, true disease-susceptible polymorphs may be present in the three SNPs that exhibited a high correlation in Example 7.

[Table 7]

Table 7 Haplotype correlation analysis

| Haplotype | Haplotype frequency | | *P* value |
|---|---|---|---|
| | Knee QA patients | Controls | |
| I | 0.457 | 0.501 | 0.026 |
| II | 0.194 | 0.167 | 0.083 |
| III | 0.130 | 0.127 | 0.83 |
| IV | 0.047 | 0.053 | 0.50 |
| V | 0.052 | 0.048 | 0.63 |
| VI | 0.045 | 0.027 | 0.018 |
| VII | 0.023 | 0.019 | 0.51 |
| VIII | 0.016 | 0.014 | 0.81 |

Example 9 Reproducibility analysis in cohort population for polymorphs highly correlated with knee OA in general Japanese population

For four polymorphs highly correlated with knee OA present in the rs3739708 LD region (i-EDG2-03, 09, 12 and 25), a correlation analysis in a Mie Prefecture cohort (Miyagawa Village and Nansei Town) was performed. The correlation analysis was performed on a population of subjects judged to have OA on X-ray images (238 subjects, OA population) for a case group, and a population of subjects who had no OA findings on X-ray images and had no pain (279 subjects, non-OA population) for a control group. As a result, i-EDG2-09, 12 and 25 exhibited a correlation of $P < 0.05$ in a minor allele recessive model (Table 8). This result, combined with Example 7, means that the correlations of i-EDG2-09, 12 and 25 with knee OA were reproduced in the two independent case/control populations. The final correlation value for i-EDG2-09 is 0.0000015 as obtained by multiplying the P-values for two independent populations (general Japanese population and cohort population). This remains a significant value even after Bonferroni's correction is performed with the number of SNPs used in the primary screening (155 SNPs).

[Table 8]

Table 8 Correlation test in Mie Prefecture cohort population

| Polymorh ID (i-*EDG2*-x) | Genotype[a] | | | | | | | | 11 Frequency (%) | | *P* value[b] | Odds ratio[b] (95% confidence interval) |
|---|---|---|---|---|---|---|---|---|---|---|---|---|
| | Diseased (raodiographic OA) | | | | Controls (non-OA) | | | | Diseased | Controls | | |
| | 11 | 12 | 22 | | 11 | 12 | 22 | | | | | |
| 3 | 14 | 81 | 143 | 238 | 8 | 103 | 163 | 274 | 5.9 | 2.9 | 0.099 | 2.08 (0.86-5.05) |
| 9 | 18 | 78 | 142 | 238 | 6 | 101 | 172 | 279 | 7.6 | 2.2 | 0.0036 | 3.72 (1.45-9.53) |
| 12 | 17 | 86 | 135 | 238 | 6 | 108 | 165 | 279 | 7.1 | 2.2 | 0.0061 | 3.50 (1-36-9.03) |
| 25[c] | 12 | 72 | 154 | 238 | 5 | 88 | 185 | 278 | 5.0 | 1.8 | 0.040 | 2.9 (1.01-835) |

a 11, 12 and 22 indicate a monor allele homozygote and heterozygote and a major allele homozygote, respectively.
b P values and odds ratios were calculated using a recessive model (11 versus 12+22).
c i-EDG2-5 is an SNP identical to re3739708..

Example 10 Expression analysis of EDG2 gene in human tissues

Results of a correlation analysis suggested that the EDG2 gene might be a knee OA susceptibility gene. EDG2 is a GPCR having lysophosphatidic acid (LPA) as a ligand thereof. For LPA, four receptors, including EDG2, have been reported (EDG2, EDG4, EDG7 and GPR23). To analyze the tissue distributions of these four LPA receptors, an expression quantitation analysis by quantitative real-time PCR in various human tissues was performed. On 500 ng of total RNA of each of various human tissues (Human Total RNA Master Panel, Clontech), reverse transcription was performed using Multiscribe reverse transcriptase (Applied biosystems) and a random hexamer, whereby a cDNA was obtained. Quantitation of the LPA receptor genes was performed using QuantiTect STBR Green PCR (Qiagen) on the ABI PRISM 7700 sequence detection system (Applied biosystems) as directed in the documentation attached. The number of copies of each gene was calculated using a working curve. The amount of each gene was standardized by being divided by the number of copies of the G3PDH gene. The primers used in the gene quantitation are shown in Table 9. A Toyobo product was used as the primer for the G3PDH gene. As a result, the EDG2 gene was found to be strongly expressed in the whole brain and spinal cord. In these organs, the amount of the EDG2 gene expressed was about 20 to 100 times as high as other LPA receptor genes; this gene is a dominant LPA receptor (Fig. 2). The EDG2 gene was also found to be expressed at moderate levels in the adrenal, cerebellum, lung, placenta, prostate, testis, thyroid, trachea, and uterus (Fig. 2). In these tissues, EDG2 was not always a dominant LPA receptor.

[Table 9]

Table 9 Primer sequences used in quantitative real-time PCR

| | Primer sequence (5' → 3') | | | |
|---|---|---|---|---|
| Target gene | Forward primer | SEQ ID NO | Reverse primer | SEQ ID NO |
| human *EDG2* | TGTCTCGGCATAGTTCTGGA | 4 | CATTTCTTTGTCGCGGTAGG | 5 |
| human *EDg4* | TTGTCTTCCTGCTCATGGTG | 6 | TGCAGGACTCACAGCCTAAA | 7 |
| human *EDG7* | TGACTGTCTTAGGGGCGTTT | 8 | TTCTCCTGAGAGAAGCAGCA | 9 |
| human *GPR23* | TTCCTGGCCATTGTCTATCC | 10 | GGAATGATAAACCCAACAACTTC | 11 |
| human *TNFA* | CCTGCCCCAATCCCCTTTATT | 12 | CCCTAAGCCCCCAATTCTCT | 13 |
| human *IL1B* | TCCAGGGACAGGATATGGAG | 14 | TGAAGACAAATCGCTTTTCCA | 15 |
| human *IL6* | AGTGAGGAACAAGCCAGAGC | 16 | GAGGTGCCCATGCTACATTT | 17 |
| rabbit *Edg2* | AATCGGGACACCATGATGAG | 18 | CAGAGGATCTGCCTGAAGGT | 19 |
| rabbit *Tnfa* | ATGGTCACCCTCAGATCAGC | 20 | TTGACCGCTGAAGAGAACCT | 21 |
| rabbit *Il1b* | TGGCACGTATGAGCTGAAAG | 22 | CCAGCTGCAGGGTAGGTTTA | 23 |
| rabbit *Il6* | TGGTGCTGAAGAACATCCAA | 24 | AAATTCCGCAAGCAAGGAC | 25 |
| rabbit *Mmpl* | TCCCAGGAATTGGAAACAAG | 26 | ACATGACTGGGCTCACAAAG | 27 |
| rabbit *M* mp3 | TGGGTTTCCCTTCAACCATA | 28 | TTGGGTAAACTCCGACTGT 29 | |
| rabbit *M* mp13 | ATTTTGAAGACACGGGCAAG | 30 | GGGAATTTGTTGGCATGACT | 31 |
| rabbit *G3pdh* | ATCACTGCCACCCAGAAGAC | 32 | ATGAGGTCCACCACCCTGTT | 33 |

Example 11 Expression analysis of the EDG2 gene in OA synovial membrane and OA articular cartilage

To analyze the expression of the four LPA receptors examined in Example 10 in joint tissue, an expression quantitation analysis by quantitative real-time PCR was performed on articular cartilages and synovial tissues of knee OA patients. The method used was in accordance with Example 10. Total RNA was extracted from each tissue (articular cartilage n=4, synovial membrane n=3) using Isogen (Nippon Gene), and reverse transcription was performed on 500 ng of total RNA using Multiscribe reverse transcriptase (Applied biosystems) and a random hexamer, whereby a cDNA was obtained. Quantitation of the LPA receptor genes was performed using QuantiTect STBR Green PCR (Qiagen) on the ABI PRISM 7700 sequence detection system (Applied biosystems) as directed in the documentation attached. The number of copies of each gene was calculated using a working curve. The amount of each gene was standardized by being divided by the number of copies of the G3PDH gene. The primers used in the gene quantitation are shown in Table 9. A Toyobo product was used as the primer for the G3PDH gene. As a result, the EDG2 gene was found to be expressed in knee OA articular cartilage and OA synovial membrane. The amount of the EDG2 gene expressed was higher in synovial membrane at a level about 10 times as high as in articular cartilage. In synovial tissue, the amount of the EDG2 gene expressed was about 25 times as high as other LPA receptor genes, showing that the EDG2 gene is a dominant LPA receptor in synovial membrane (Fig. 3). Meanwhile, in articular cartilage, in addition to the EDG2 gene, the GPR23 gene was expressed at similar levels, showing that EDG2 is not a dominant LPA receptor (Fig. 3).

Example 12 Investigation of activation of transcriptional factor AP-1 in stimulation of synovial cell line E11 cells with phorbol 12-myristate 13-acetate

Because the EDG2 gene is an LPA receptor that is dominant in OA synovial membrane, it was decided to perform a functional analysis of OA susceptibility polymorphs using synovial cells. E11 cells are a cell line established by immortalizing fibroblast-like cells from a rheumatoid arthritis patient synovial membrane with simian SV40 large T antigen (Abe et al, J Rheumatol. 1997 Mar;24(3):420-9.). In the 5'-flanking region of EDG2, where i-EDG2-09 and 12 are present, an AP-1-binding motif is present. Hence, to determine whether or not a transcription activation mechanism by AP-1 is present in E11 cells, E11 cells were stimulated with phorbol 12-myristate 13-acetate (PMA), and the luciferase activity of a construct wherein the binding motif of AP-1 is inserted in front of the luciferase gene (PathDirectAP-1cis-Reporting system, Stratagene) was measured. The method used is described below. The E11 cells were cultured using Dulbecco's Modified Eagle's Medium (DMEM, Sigma) containing 10% fetal bovine serum (FBS), antibiotics (100 U/ml penicillin G and 100 $\mu$g/ml streptomycin) and L-glutamine (200 mM). On the day before the transfection, the E11 cells were seeded to a 24-multiwell plate to obtain a cell density of 5 x $10^4$ cells/well. The following day, using Fugene-6 (Roche diagnostics), 200 ng of the reporter construct and 4 ng of pRL-TK (Promega) as the internal standard for correction of transfection efficiency, per well were transfected. Twenty-four after the transfection, the cells were cultured on a medium containing 0.1, 1 and 10 ng/ml PMA. Twenty-four hours after the stimulation, the cells were solubilized, and luciferase activity was measured using PicaGene Dual SeaPansy System (Toyo Ink). The number of cases was n=2. As a result, it was found that the luciferase activity rose PMA dose-dependently (Fig. 4a). This result shows that in E11 cells, a mechanism for AP-1 activation by PMA stimulation exists.

Example 13 Investigation of expression control of the EDG2 gene by PMA stimulation of synovial cell line E11 cells

The results in Example 12 revealed that in E11 cells, a mechanism for AP-1 activation by PMA stimulation exists. Hence, an investigation was made to determine whether PMA alters the expression of the EDG2 gene in E11 cells over time. The method used is described below. E11 cells were seeded to a 24-multiwell plate to obtain a cell density of 1 x $10^5$ cells/well. The cells were cultured until they became confluent, after which they were stimulated with PMA (10 ng/ml); 0, 2, 4, 9, 12 and 24 hours later, total RNAs were recovered using ISOGEN. In these total RNAs, the amount expressed of the EDG2 gene relative to G3PDH was measured in accordance with the method described in Example 11. The number of cases was n=4. As a result, from 2 hours to 12 hours after the addition of PMA, the amount expressed of the EDG2 gene rose over time (Fig. 4b). This finding shows that the signal for elevation of the expression of the EDG2 gene is activated by PMA stimulation.

Example 14 Influence on transcription activity of i-EDG2-09 by reporter assay (comparison across alleles)

An AP-1-binding motif described in a report by Duncliffe et al. [Immunity. 1997 Feb;6(2):175-85.] is present 1 to 7 bp upstream of i-EDG2-09 out of the 2 polymorphs that exhibited a high correlation with knee OA, and an NFAT-binding motif described in a report by Guo et al. [J Biol Chem. 2001 Dec 28;276(52):48871-8.] is present 11 to 16 bp upstream. The existence of a transcriptional factor-binding motif where AP-1 and NFAT are located in tandem is known in a plurality of genes [Macian F, Lopez-Rodriguez C and Rao A., Oncogene. 2001 Apr 30;20(19):2476-89. Review.]. Meanwhile, in the surrounding sequence, including i-EDG2-12, no distinct transcriptional factor binding sequence was found. Hence, the influence of i-EDG2-09 on the EDG2 gene transcription activity was analyzed by luciferase reporter assay. Oligonucleotides (sense strand and antisense strand) wherein 4bp at the restriction endonuclease NheI adhesion end is added on the 5' side of a region centering at i-EDG2-09, and comprising the AP-1 and NFAT-binding motifs (a region at -2809 to -2839 from the transcription initiation point of EDG2), and 4 bp at the XbaI adhesion end is added on the 3' side, were prepared for both alleles of i-EDG2-09 (G or A). After these oligonucleotides were converted to double-stranded DNAs for each allele, reporter constructs wherein each allele was integrated in tandem 5 times in the NheI site of the pGL3-promoter vector (Promega), which is a luciferase reporter vector, while retaining the orientation 5'→3', were prepared for G allele and A allele. These constructs were transfected to E11 cells cultured in Dulbecco's Modified Eagle's Medium (DMEM, Sigma) containing 10% fetal bovine serum (FBS) and antibiotics (100 U/ml penicillin G and 100 μg/ml streptomycin). The transfection was performed by the procedures shown below. On the day before the transfection, the E11 cells were seeded to a 24-multiwell plate to obtain a cell density of 5 x $10^4$ cells/well. The following day, using Fugene-6 (Roche Diagnostics), 200 ng of the reporter construct and 4 ng of pRL-TK (Promega) as the internal standard for correcting the transfection efficiency per well were transfected. Twenty-four hours after the transfection, the cells were stimulated with a medium containing 10 ng/ml PMA. Five hours after the stimulation, the cells were solubilized, and luciferase activity was measured using the PicaGene Dual SeaPansy System (Toyo Ink). As a result, for both constructs, with the addition of PMA, the luciferase activity rose significantly. Without addition of PMA, there was no difference in transcription activity between G allele and A allele; however, with addition of PMA, a 2.4 times higher transcription activity was confirmed in A allele than in G allele (Fig. 5). From this result, it was found that A allele is an allele that raises transcription activity. Because the number of individuals that are homozygous for A allele is larger in the knee OA patient group, it was suggested that transcription activation of the EDG2 gene might be involved in the onset or progression of knee OA.

Example 15 Investigation of intranuclear factor binding affinity for i-EDG2-09 by gel shift assay (comparison across alleles)

In Example 14, i-EDG2-09 was found to exhibit a difference in transcription activity across alleles. Hence, to determine whether or not this difference in transcription activity depends on a difference in the binding affinity of intranuclear factor for each allele, a gel shift assay was performed using a nuclear extract from PMA-stimulated E11 cells. The method used is described below. After E11 cells were made to be confluent in a 100 mm dish, they were cultured on a medium containing PMA (10 ng/ml) for 0, 1, 2, 4 and 8 hours. After elapse of each time, nuclear extract protein of E11 cells was prepared according to the method of Andrews et al. [Nucleic Acids Res. 1991 Nov 25;19(22):6263-8.]. Oligonucleotide annealing and DIG labeling were performed using a DIG gel shift kit (Roche Diagnostics). The sequences of the oligonucleotides used are shown in Table 10. The DIG-labeled probe was mixed with a nuclear extract from PMA-stimulated E11 cells, and incubated at room temperature for 20 minutes, to form a DNA-protein complex. The DNA-protein complex formed was separated in 0.5% Tris-borate-EDTA buffer using 6% polyacrylamide gel. In the competitive experiments of the DIG-labeled probe and a non-labeled probe, the non-labeled probe in an amount 125 times the amount of the labeled probe was added prior to mixing the labeled probe and the nuclear extract. After electrophoresis, the DNA-protein complex was transferred to nitrocellulose membrane and crosslinked by baking (120°C, 30 minutes), after which signals from the labeled probe were detected using a chemiluminescence detection system (Roche Diagnostics Co.).

[Table 10]

Table 10 Oligonucleotide sequences used in gel shift assay

| Name | | Oligonucleotide sequence (5'→ 3') | SEQ ID NO |
|---|---|---|---|
| i-*EDG2*-9-G | Sense strand: | CTAGCTTTGGAACATAATGAGCTAGGTTTTATGGAT | 34 |
| | Antisense strand: | CTAGATCCATAAAACCTAGCTCATTATGTTCCAAAG | 35 |
| i-*EDG2*-*9*-A | Sense strand: | CTAGCTTTGGAACATAATGAGCTAGTTTATGGAT | 36 |
| | Antisense strand: | CTAGATCCATAAAACTTAGCTCATTATGTTCCAAAG | 37 |
| AP-1 | Sense strand: | CTAGCGCTTGATGAGTCAGCCGGAA | 38 |
| | Antisense strand: | CTAGTTCCGGCTGACTCATCAAGCG | 39 |

First, a gel shift assay was performed using the oligonucleotide of A allele and a nuclear extract from E11 cells stimulated with PMA over time. As a result, specific bands were confirmed on a lane wherein the nuclear extract of E11 cells and labeled oligo were mixed (Fig. 6a, arrows 1 and 2). The signal intensity of band 1 reached a plateau 2 hours after the PMA stimulation, the signal of band 2 rose over time until 8 hours after the PMA stimulation. This finding suggests that an intranuclear factor bindable to the oligonucleotide of A allele may migrate into the nucleus over time. Next, 8 hours after PMA stimulation, when the signal intensity of band 2 became the strongest, the E11 nuclear extract and the nucleotide of A allele or G allele were mixed, and a gel shift assay was performed. As a result, in the oligonucleotide of G allele, as in the oligonucleotide of A allele, bands resulting from the binding of intranuclear factor were confirmed (Fig. 6b, arrows 1 and 2). Because these bands disappeared in the presence of respective non-labeled oligonucleotides, it is shown that the binding is not specific. When band intensity was compared across the alleles, the intensity of band 1 did not differ across the alleles, but the intensity of band 2 was stronger in A allele. Because this band 2 disappeared in the presence of the oligonucleotide of non-labeled AP-1-binding motif sequence, it was suggested that the band might be AP-1. The results above suggested that A allele might raise the binding affinity of AP-1. From Example 14, it is shown that A allele promotes the rise in luciferase activity with the addition of PMA; this experimental result suggested that the rise in luciferase activity in Example 14 might result from a rise in the binding affinity of AP-1 for the binding motif.

Example 16 Analysis of inflammatory cytokine gene expression induction by LPA in human articular cartilage cells

The inflammatory cytokine expression induction reaction of LPA, which is a ligand of EDG2, in human articular cartilage cells was analyzed. The method used is described below. Normal human knee articular cartilage cells (NHAC-kn, Takara) were embedded in alginate beads, and cultured in the attached differentiation medium (CDM) for 3 weeks. After incubation in sodium citrate solution (55 mM in 0.15M NaCl solution), the alginate beads were dissolved, and the cells were recovered via centrifugal operation. The cells were twice washed with 155 mM NaCl solution, after which they were seeded to a 24-well plate at a ratio of 1 x $16^5$ cells/well. Twelve hours before performing LPA (1-Oleoyl-sn-Glycero-3-Phosphate, Sigma) stimulation, the medium was replaced with a DMEM/F-12 medium containing 0.1% fatty acid free bovine serum albumin (Sigma). Twelve hours after the medium replacement, the cells were stimulated with 10 $\mu$M LPA; 0, 2, 4, 8, 12 and 24 hours later, total RNA was extracted using ISOGEN (Nippon Gene), and the amounts expressed of TNFA and IL1B, which are inflammatory cytokine genes, were quantified by a method in accordance with Example 10. The number of cases was n=4. As a result, the amount expressed of the TNFA gene rose about 2 folds transiently 2 hours after addition of LPA (Fig. 7a). For IL1B, no expression raising reaction was observed (Fig. 7b).

Example 17 Analysis of expression induction of inflammatory cytokine gene and matrix metalloprotease gene by LPA in human OA synovial cells

The inflammatory cytokine expression induction reaction of LPA, which is a ligand of EDG2, in OA patient-derived fibroblast-like synovial cells was analyzed. The method used is described below. OA patient-derived fibroblast-like synovial cells (HFLS-OA) were purchased from Cell application Co. The HFLS-OA was cultured using the attached Synoviocyte growth medium. The number of passages for the cells used was 4 or 5. The HFLS-OA was seeded to a 24-well plate at a ratio of 1 x $10^5$ cells/well, and cultured using the attached Synoviocyte growth medium until they became confluent. Twelve hours before performing LPA stimulation, the medium was replaced with Ham's F-12 (Invitrogen) medium containing 0.1% fatty acid free bovine serum albumin (Sigma). In the time-related change analysis, 12 hours after the medium replacement, the cells were stimulated with 10 $\mu$M LPA; 0, 1, 2, 4, 8, 12 and 24 hours later, total RNA was extracted using ISOGEN (Nippon Gene), and the amounts expressed of the TNFA, IL1B, and IL6 genes, which are inflammatory cytokine genes, and of the MMP1, MMP3, and MMP13 genes, which are Matrix metalloproteinase (MMP) genes, were quantified by a method in accordance with Example 10 (n=4). In the dose dependency test, 12 hours after the medium replacement, the cells were stimulated with 0, 0.1, 0.3, 1, 3 and 10 $\mu$M LPA; 2 hours later, total RNA was extracted using ISOGEN (Nippon Gene), and the amounts expressed of the TNFA, IL1B, and IL6 genes, which are inflammatory cytokine genes, were quantified in the same manner as the above for the time-related change test (n=3). The sequences of the primers used are shown in Table 9. As a result of the time-related change test of inflammatory cytokine genes with LPA stimulation, the amounts of TNFA, IL1B and IL6 expressed rose transiently, with peaks reached 2, 4 and 1 hour later, respectively (Fig. 8a, b and c). The amounts expressed of the MMP1, MMP3, and MMP13 genes, which are MMP genes, rose transiently, with peaks reached 8, 8 and 4 hours later, respectively (Fig. 8d, e and f). It is known that in synovial cells, MMP genes have the expression thereof increased by inflammatory cytokines. In this experiment, the rise in the expression of the inflammatory cytokine genes preceded the rise in the expression of the MMP genes; therefore, the rise in the expression of MMP genes with LPA stimulation may be a secondary reaction via an inflammatory cytokine. As a result of the dose dependency test, it was shown that LPA raises the expression of inflammatory cytokine genes dose-dependently (Fig. 8g, h and i). The amounts expressed of the TNFA and IL1B genes nearly reached a plateau at 1 $\mu$M, whereas the expression of the IL6 gene rose dose-dependently up to 10 $\mu$M. The results above showed that LPA induces in OA synovial cells the expression of inflammatory cytokine genes, and induces the expression of MMP genes paracrinely. The results of the LPA receptor expression analysis in OA synovial tissue shown in Example 11 show that the EDG2 gene is dominant over other LPA receptor genes in synovial tissue. From this finding, it is thought that the inflammatory cytokine gene group induction reaction of LPA is very highly likely to be a reaction mediated mainly by the EDG2 gene.

In Example 14, the A allele of i-EDG2-09, which is a knee OA susceptibility polymorph, raised the transcription activity of the EDG2 gene; therefore, AA homozygous individuals possibly have highly expressed EDG2 in their synovial cells and an increased inflammatory cytokine gene expression raising reaction by LPA stimulation. The induced inflammatory cytokines may induce MMPs in joints to cause cartilage destruction and raise the susceptibility to radiographic OA. At the same time, inflammatory cytokines may raise the susceptibility to symptomatic OA by lowering the threshold value of the primary afferent nerve present in the primary synovial membrane.

Example 18 Analysis of expression induction of inflammatory cytokine gene and MMP gene by LPA in rabbit synovial cell line

The inflammatory cytokine expression induction reaction of LPA in a rabbit synovial cell line was analyzed. The method used is described below. A rabbit synovial cell line (HIG-82) was purchased from ATCC. The HIG-82 was cultured using Ham's F-12 medium (Invitrogen) containing 10% fetal bovine serum (FBS) and antibiotics (100 U/ml penicillin G and 100 μg/ml streptomycin). The HIG-82 was seeded to a 24-well plate at a ratio of 1 x $10^5$ cells/well, and cultured until they became confluent. Twelve hours before performing LPA stimulation, the medium was replaced with Ham's F-12 medium containing 0.1% fatty acid free bovine serum albumin (Sigma). In the time-related change analysis, 12 hours after the medium replacement, the cells were stimulated with 10 μM LPA; 0, 2, 4, 8, 12 and 24 hours later, total RNA was extracted using ISOGEN (Nippon Gene), and the amounts expressed of the TNFA and IL1B genes, which are inflammatory cytokine genes, and of the MMP1, MMP3 and MMP13 genes, which are MMP genes, were quantified by a method in accordance with Example 10 (n=4). In the dose dependency test, 12 hours after the medium replacement, the cells were stimulated with 0, 0.1, 1 and 10 μM LPA; 2 hours later, total RNA was extracted using ISOGEN (Nippon Gene), and the amounts expressed of the TNFA and IL1B genes, which are inflammatory cytokine genes, were quantified by the same method as with the time-related change test (n=4). As a result of the time-related change test of inflammatory cytokine genes with LPA stimulation, the amounts expressed of the TNFA and IL1B genes rose transiently, with peaks reached 2 hours later (Fig. 9a and b). The amounts expressed of the MMP1, MMP3 and MMP13 genes, which are MMP genes, rose transiently, with peaks reached 4, 4 and 8 hours later, respectively. As a result of the dose dependency test, it was shown that LPA raised the expression of inflammatory cytokine genes dose-dependently. The amount expressed of the IL1B gene nearly reached a plateau at 1 μM, whereas the amount expressed of the TNFA gene rose dose-dependently up to 10 μM. The results above are well similar to the results for HFLS-OA shown in Example 17, showing that in HIG-82 cells as well, the expression of inflammatory cytokine genes is induced with LPA stimulation, and the expression of MMPs is induced paracrinely.

Example 19 Correlation between frequency of disease-susceptible genotype in i-EDG2-09 and severity of knee OA in Japanese

For i-EDG2-09, the relationship between the severity of knee OA and the frequency of disease-susceptible genotype (A homozygote) was examined in a general population of Japanese patients with knee OA.
As a result, in the population, as the severity of disease increased, the frequency of the disease-susceptible genotype tended to be higher (Fig. 10). Hence, it was found that the genotype and the phenotype correlate with each other.

Example 20 Involvement of EDG2 in LPA-induced inflammatory cytokine raising reaction in human OA synovial cells

To analyze the involvement of EDG2 in the LPA-induced inflammatory cytokine raising reaction in human OA synovial cells, obtained in Example 17, an investigation using an siRNA was performed. The method used is described below. An siRNA against EDG2 was designed by utilizing an siRNA design support system available from the Takara Bio Web site (http://bio.takara.co.jp). Synthesis of the siRNA was outsourced to Takara Bio. The sequence is shown in Table 11. The control siRNA used was such that the sequence of an siRNA against asporin was scrambled. OA patient-derived fibroblast-like synovial cells (HFLS-OA) were purchased from Cell application Co. The HFLS-OA was cultured using the attached Synoviocyte growth medium. The number of passages for the cells used was 4 or 5. The HFLS-OA was seeded to a 24-well plate at a ratio of 1 x $10^5$ cells/well, and cultured using the attached Synoviocyte growth medium. The following day, the siRNA against EDG2 was introduced into the HFLS-OA to obtain a final concentration of 11 nM, using the Trans-IT-TKO transfection regent (Mirus Co.), according to the attached protocol. On the day after the transfection, the medium was replaced with Ham's F-12 (Invitrogen) medium containing 0.1% fatty acid free bovine serum albumin (Sigma). Twelve hours after the medium replacement, the cells were stimulated with 10 μM LPA; 2 hours later, total RNA was extracted using ISOGEN (Nippon Gene), and the amount of the IL6 gene expressed was quantified by a method in accordance with Example 10 (n=4). In the cells incorporating the siRNA against EDG2, the amount of EDG2 expressed was significantly suppressed (Fig. 11a). The amount expressed of the IL6 gene, which rises in response to LPA simultaneously, was also significantly suppressed (Fig. 11b). This result showed that in HFLS-OA, EDG2 is involved in the rise of the expression of the IL6 gene induced by LPA.
[Table 11]

Table 11 siRNA sequence used in EDG2 knockdown

| Name | siRNA sequence (5' → 3') | | | |
|---|---|---|---|---|
| | Sense strand | SEQ ID NO | Antisense strand | SEQ ID NO |
| EDG2-siRNA | GAAAUGAGOGCCACCUUUATT | 40 | UAAAGGUGGCGCUCAUUUCTT | 41 |

Furthermore, the involvement of EDG2 in the LPA-induced inflammatory cytokine raising reaction in human OA synovial cells was analyzed using an antagonist against EDG2. The method used is described below. As the EDG2 antagonist, Ki16425 (Sigma) was used. OA patient-derived fibroblast-like synovial cells (HFLS-OA) were purchased from Cell application Co. The HFLS-OA was cultured using the attached Synoviocyte growth medium. The number of passages for the cells used was 4 or 5. The HFLS-OA was seeded to a 24-well plate at a ratio of 1 x $10^5$ cells/well, and cultured using the attached Synoviocyte growth medium until they became confluent. Twelve hours before performing LPA stimulation, the medium was replaced with Ham's F-12 (Invitrogen) medium containing 0.1% fatty acid free bovine serum albumin (Sigma). Twelve hours after the medium replacement, the cells were treated with Ki16425 (0, 0.3, 1, 3, 10 and 33 μM) for 30 minutes. Thereafter, the cells were stimulated with 10 μM LPA; 2 hours later, total RNA was extracted using ISOGEN (Nippon Gene), and the amount expressed of the IL6 gene was quantified by a method in accordance with Example 10 (n=4). As a result, Ki16425 suppressed the rise in the expression of the IL6 gene by LPA dose-dependently (Fig. 11c). Ki16425 possesses antagonist activity not only against EDG2, but also against EDG7; however, as shown in Example 11, EDG2 is a dominant LPA receptor in synovial tissue, so that Ki16425 is thought to act mainly on EDG2. This result strongly suggests that the inflammatory cytokine expression induction by LPA in HFLS-OA may be mediated by EDG2.

Example 21 Construction of screening system for EDG2 antagonist

A forcible expression vector comprising an ORF sequence of human EDG2 [hEDG2-pcDNA3.1(+)] was purchased from UMR cDNA Resource Center. This plasmid was introduced into McA-RH7777 (Dainippon Pharmaceutical Co., Ltd.) using Lipofectamine LTX (Invitrogen), and the cells were cultured in a medium containing Geneticin for 14 to 20 days to acquire cells that stably express EDG2.
Next, an evaluation was made using FLIPR tetra (Molecular Devices) to confirm that stimulation with LPA raised intracellular Ca concentrations via the EDG2 expressed. That is, these cells were seeded to a 384-well plate, 0.03 to 10 μM LPA (Sigma) was added to each well, and it was confirmed that rises in intracellular $Ca^{2+}$ concentration occurred LPA dose-dependently.
Furthermore, this elevation of intracellular $Ca^{2+}$ concentration by LPA stimulation (0.3 μM) was suppressed dose-dependently by previously adding Ki16425 (Sigma), which has antagonizing action on EDG2 ($10^{-8}$ to $10^{-5}$ M).

Industrial Applicability

According to the present invention, genetic susceptibility to bone and joint diseases can easily be determined. Anti-EDG2 antibody, antisense EDG2, EDG2 antagonists, DNMs of EDG2, and substances capable of regulating the expression or activity thereof can be used as prophylactic/therapeutic agents for bone/joint diseases.
This application is based on a patent application No. 2007-155792 filed in Japan, the contents of which are incorporated in full herein by this reference.

**Claims**

**1.** A diagnostic reagent for a bone/joint disease, comprising either (a) or (b) below:

(a) an antibody against a protein comprising the same or substantially the same amino acid sequence as the amino acid sequence shown by SEQ ID NO:2; or
(b) a nucleic acid comprising a base sequence that encodes a protein comprising the same or substantially the same amino acid sequence as the amino acid sequence shown by SEQ ID NO:2 or a portion thereof.

**2.** The diagnostic reagent according to claim 1, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma.

**3.** A screening method for a substance that regulates inflammatory cytokine expression in joints and/or joint destruction and/or pain in a bone/joint disease, comprising using a protein comprising the same or substantially the same amino acid sequence as the amino acid sequence shown by SEQ ID NO:2 or a partial peptide thereof.

**4.** The method according to claim 3, wherein lysophosphatidic acid is further used.

**5.** The method according to claim 3, wherein the protein comprising the same or substantially the same amino acid sequence as the amino acid sequence shown by SEQ ID NO:2 or partial peptide thereof is provided in the form of a cell that produces the same.

**6.** The method according to claim 5, further comprising using an antibody against a protein comprising the same or substantially the same amino acid sequence as the amino acid sequence shown by SEQ ID NO:2, or a nucleic acid comprising a base sequence that encodes the protein or a portion thereof.

**7.** The method according to claim 3, wherein the method is to be used for screening for a prophylactic/therapeutic substance for a bone/joint disease.

**8.** The method according to claim 7, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma.

**9.** A nucleic acid being a partial sequence of the base sequence shown by SEQ ID NO:3 comprising the bases shown by base numbers 8463 to 8464 in the base sequence (but thymine is inserted between the two bases), and comprising a continuous base sequence of about 15 bases or more.

**10.** A diagnostic method for genetic susceptibility to a bone/joint disease, comprising detecting a polymorph in one or more bases or base sequences selected from the group consisting of the bases or base sequences shown by base numbers 8463 to 8464, 24007, 24999 and 34573 in the partial base sequence of the human EDG2 gene shown by SEQ ID NO:3.

**11.** The method according to claim 10, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma.

**12.** A screening method for a substance that regulates inflammatory cytokine expression in joints and/or joint destruction and/or pain in a bone/joint disease, wherein the substance is to be administered to a human having an allele wherein the base shown by base number 24007 is thymine in the partial base sequence of the human EDG2 gene shown by SEQ ID NO:3, comprising using a nucleic acid comprising a base sequence consisting of the base shown by base number 24007 in the base sequence shown by SEQ ID NO:3 (but the base is adenine) and a base or base sequence adjacent thereto, and a transcription regulatory factor that binds selectively to the base sequence.

**13.** The method according to claim 12, wherein the method is to be used for screening for a prophylactic/therapeutic substance for a bone/joint disease.

**14.** The method according to claim 13, wherein the bone/joint disease is selected from the group consisting of osteoarthritis, osteoporosis, chronic rheumatoid arthritis, arthritis, synovitis, metabolic arthropathy, sport-related arthropathy, congenital skeletal dysplasia, osteochondroma, osteoma and chondroma.

**15.** A method of evaluating the pharmacological effect of a suppressing agent for inflammatory cytokine expression in joints and/or joint destruction and/or pain, comprising examining the expression of a protein comprising the same or substantially the same amino acid sequence as the amino acid sequence shown by SEQ ID NO:2 or the gene that encodes the same, in a bone/joint disease in a subject receiving the suppressing agent.

# FIG. 1

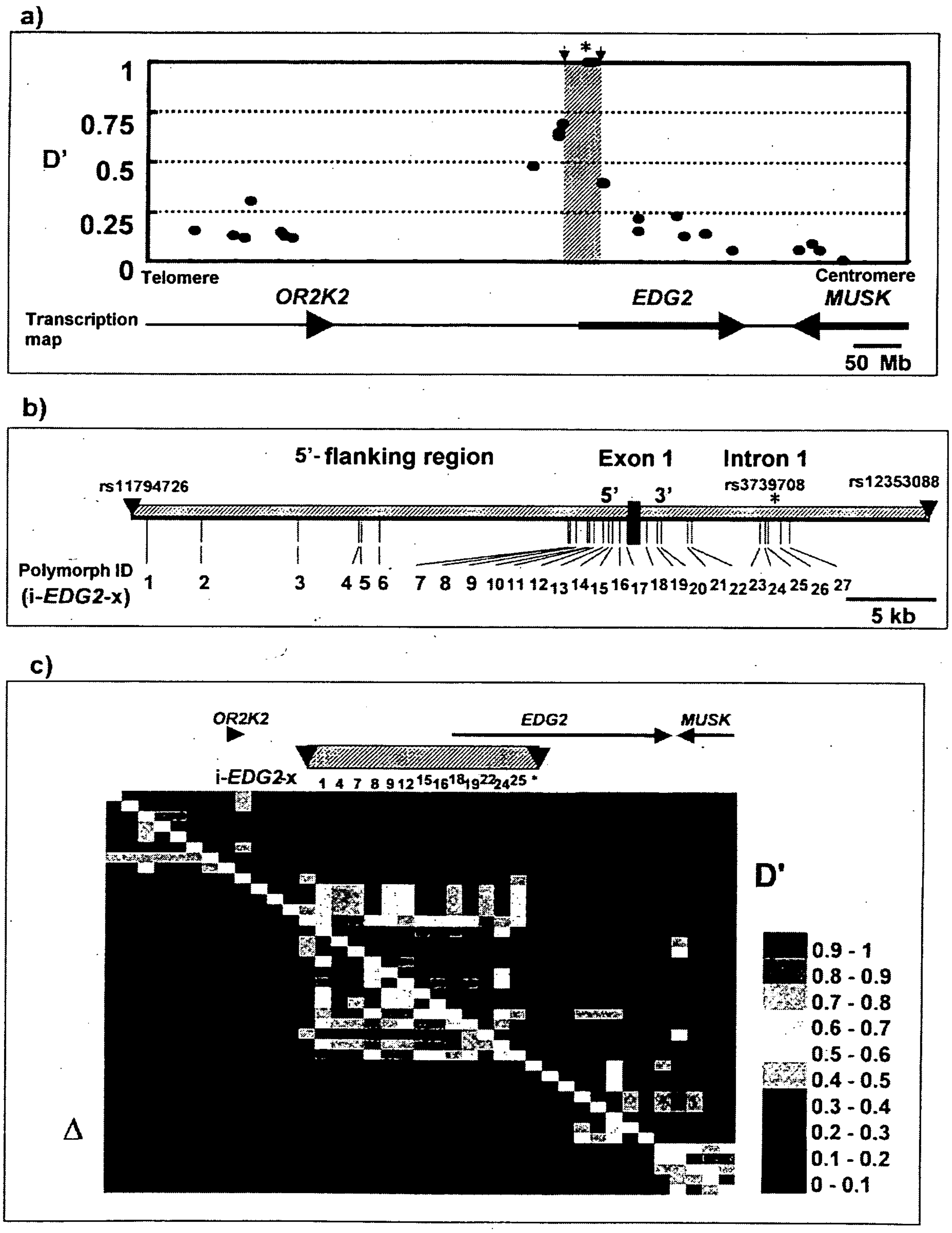
a)
1
0.75
D'
0.5
0.25
0
Telomere
Centromere
OR2K2
EDG2
MUSK
Transcription map
50 Mb
b)
5'-flanking region
Exon 1
Intron 1
rs11794726
5'
3'
rs3739708
rs12353088
Polymorph ID (i-EDG2-x)
1 2 3 4 5 6 7 8 9 10 11 12 13 14 15 16 17 18 19 20 21 22 23 24 25 26 27
5 kb
c)
OR2K2
EDG2
MUSK
i-EDG2-x
1 4 7 8 9 12 15 16 18 19 22 24 25
D'
0.9 - 1
0.8 - 0.9
0.7 - 0.8
0.6 - 0.7
0.5 - 0.6
0.4 - 0.5
0.3 - 0.4
0.2 - 0.3
0.1 - 0.2
0 - 0.1
Δ

FIG. 2

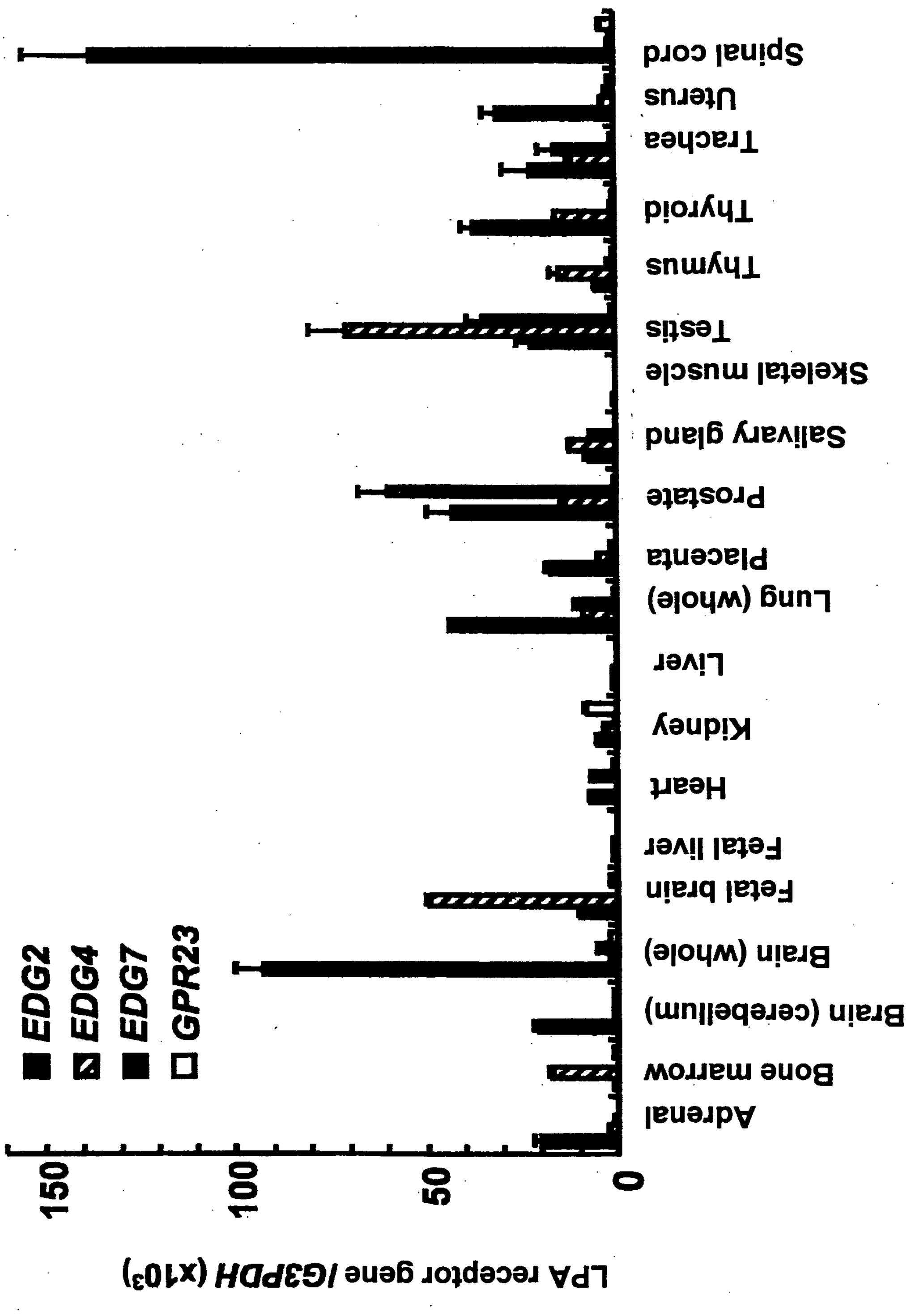
EDG2
EDG4
EDG7
GPR23
LPA receptor gene /G3PDH ($x10^3$)
150
100
50
0
Adrenal
Bone marrow
Brain (cerebellum)
Brain (whole)
Fetal brain
Fetal liver
Heart
Kidney
Liver
Lung (whole)
Placenta
Prostate
Salivary gland
Skeletal muscle
Testis
Thymus
Thyroid
Trachea
Uterus
Spinal cord

FIG. 3

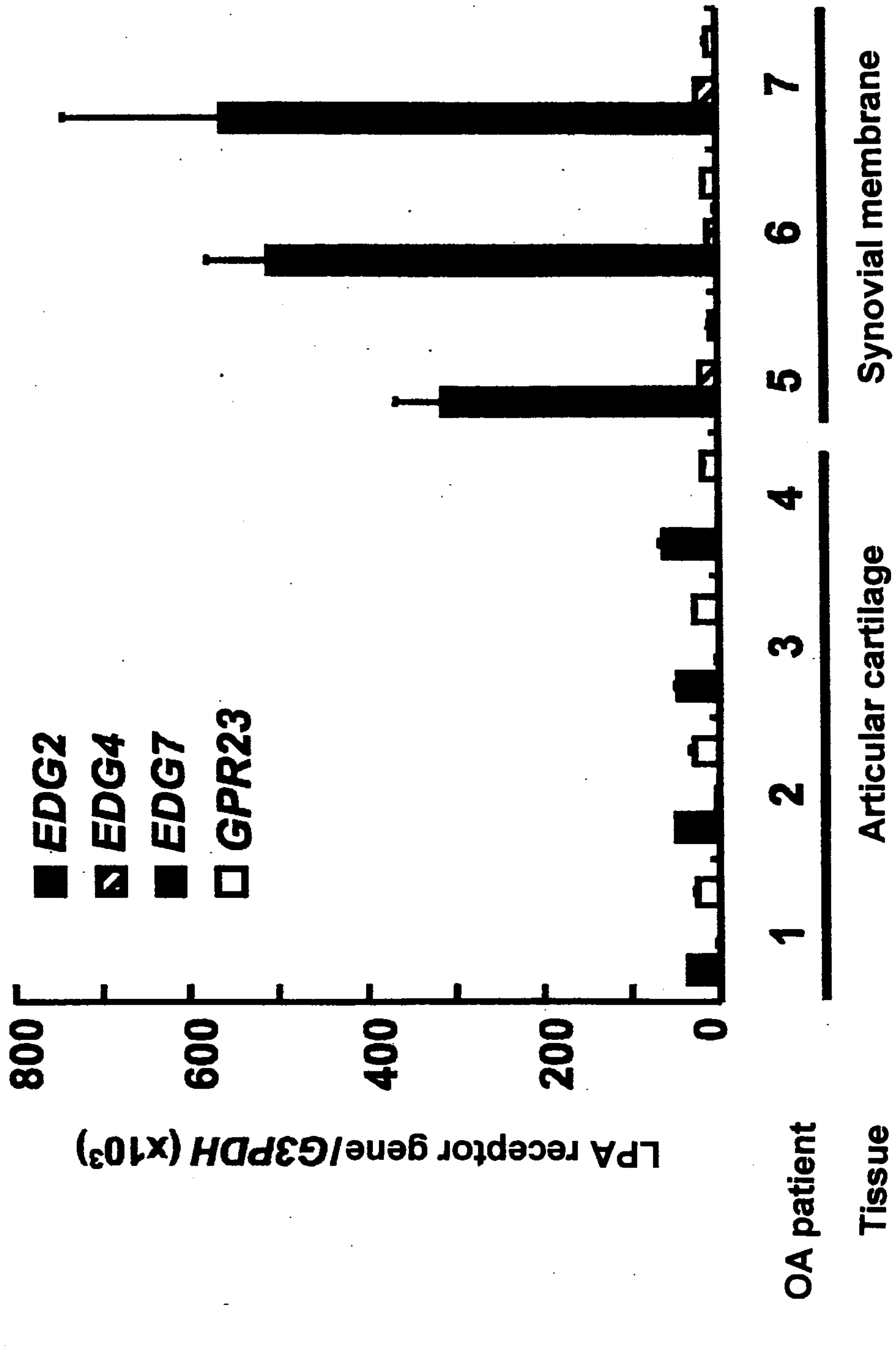
EDG2
EDG4
EDG7
GPR23
LPA receptor gene/G3PDH (x10³)
800
600
400
200
0
OA patient
1
2
3
4
5
6
7
Tissue
Articular cartilage
Synovial membrane

FIG. 4

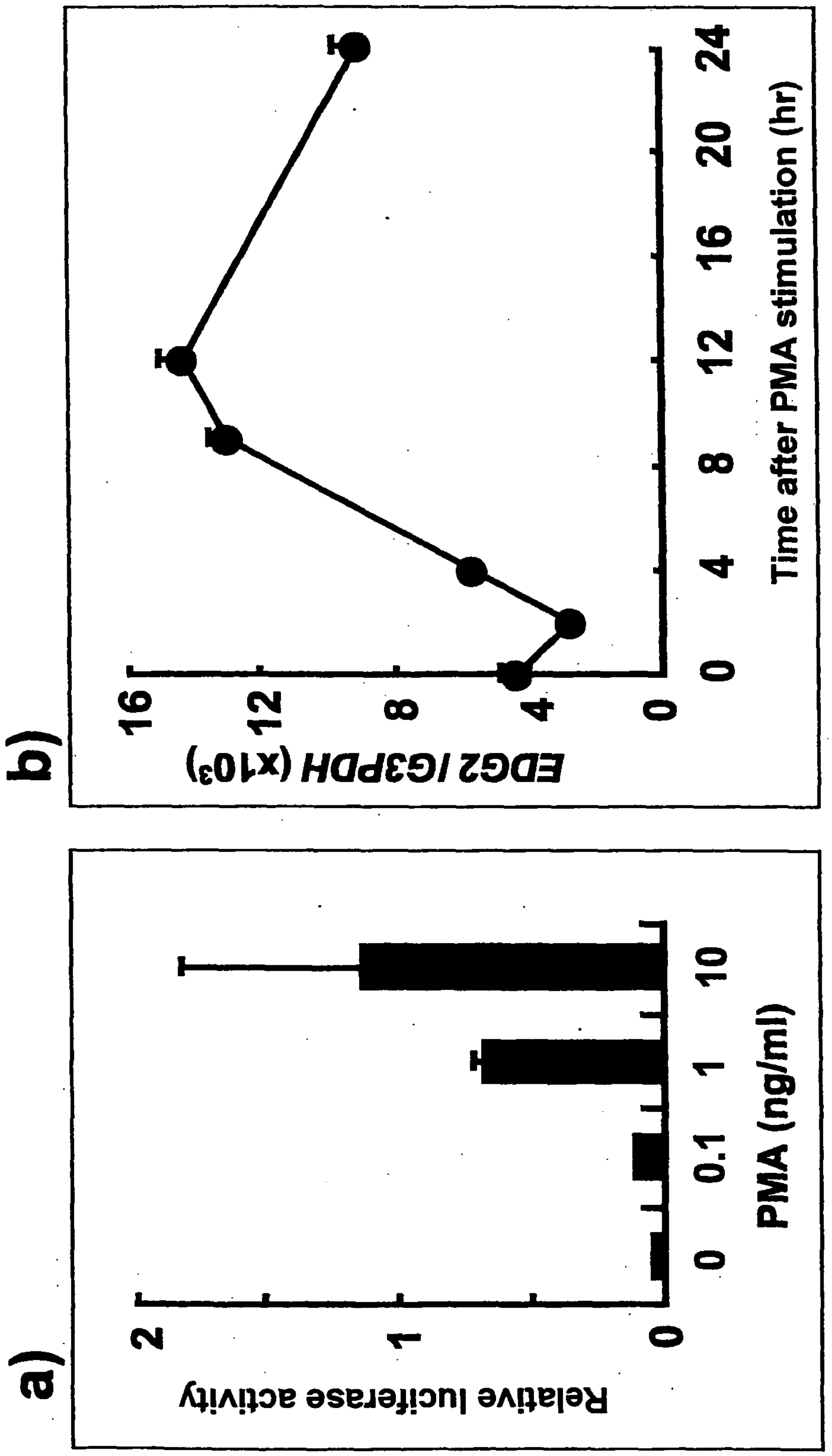
a)
Relative luciferase activity
2
1
0
0
0.1
1
10
PMA (ng/ml)
b)
EDG2 /G3PDH (x10³)
16
12
8
4
0
0
4
8
12
16
20
24
Time after PMA stimulation (hr)

FIG. 5

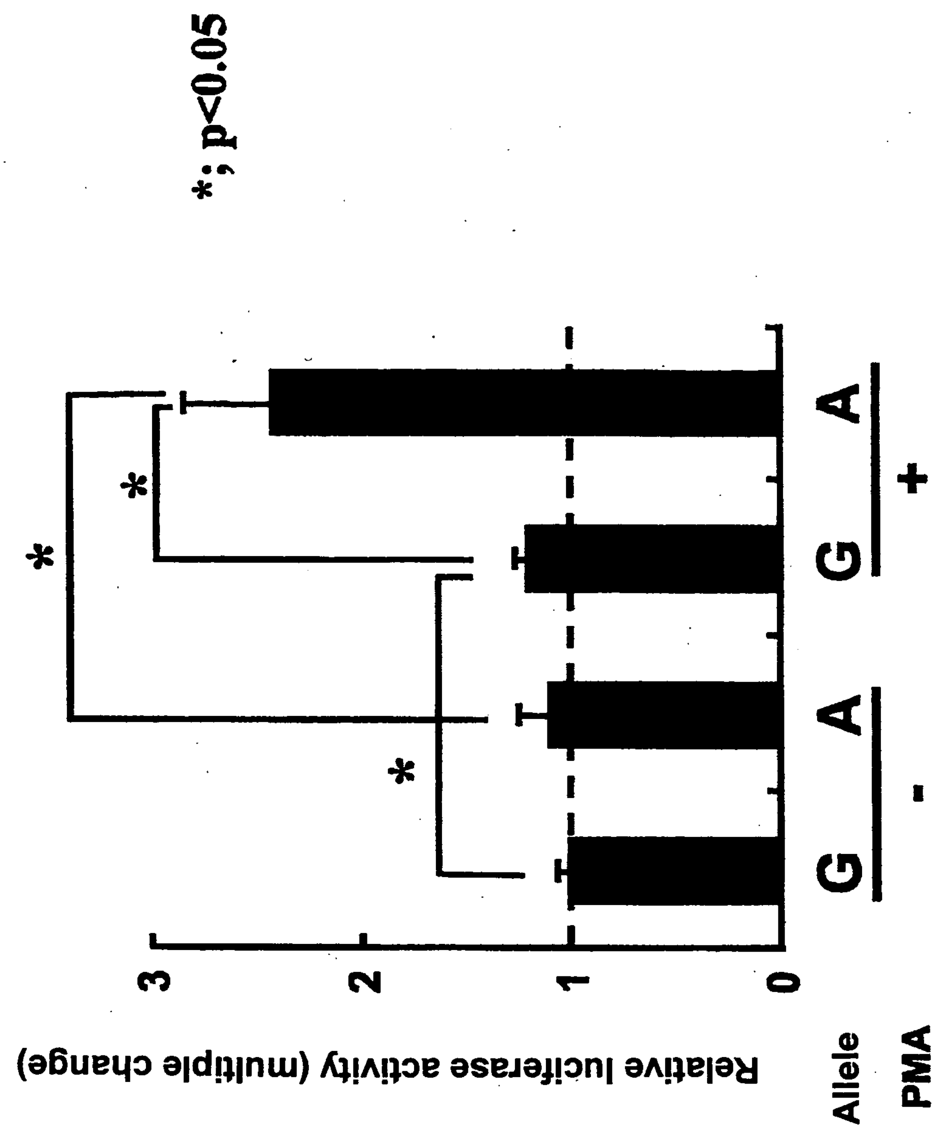
*; p<0.05
Relative luciferase activity (multiple change)
0
1
2
3
Allele
G
A
G
A
PMA
-
+
*
*
*

FIG.6

a)

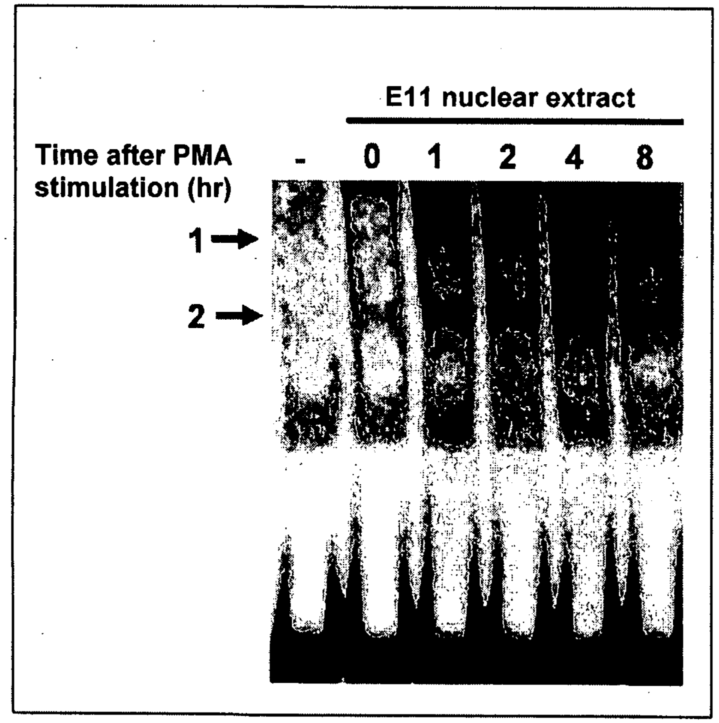
E11 nuclear extract
Time after PMA stimulation (hr)
- 0 1 2 4 8
1
2

b)

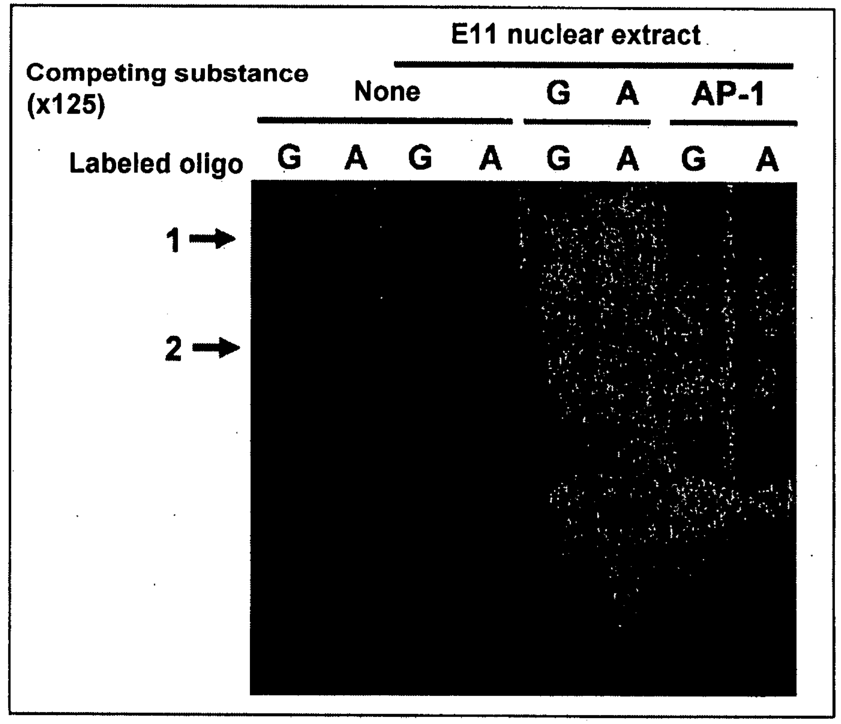
E11 nuclear extract
Competing substance (x125)
None
G A
AP-1
Labeled oligo
G A G A G A G A
1
2

FIG. 7

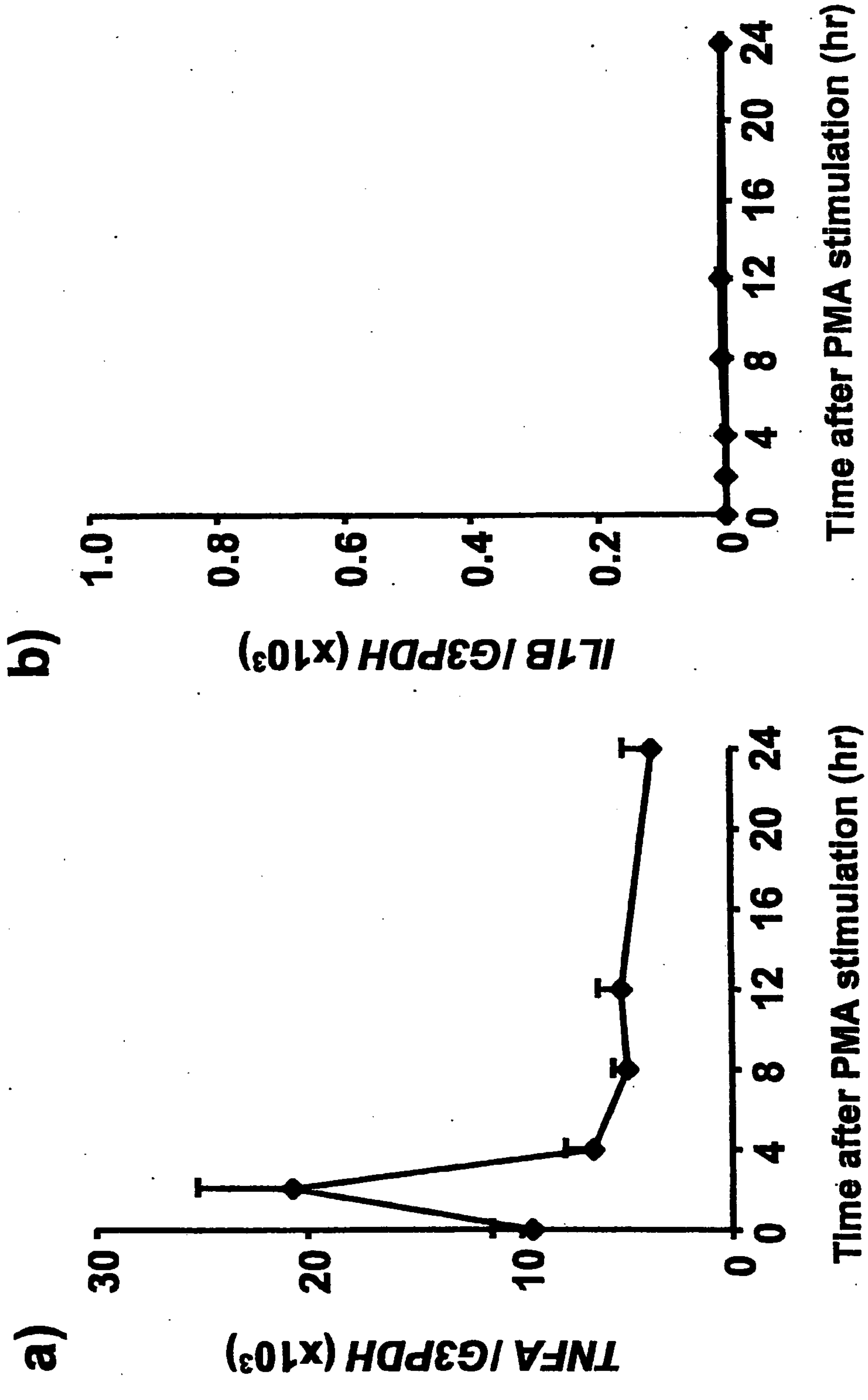
a)
TNFA /G3PDH (x10³)
30
20
10
0
0
4
8
12
16
20
24
Time after PMA stimulation (hr)
b)
IL1B /G3PDH (x10³)
1.0
0.8
0.6
0.4
0.2
0
0
4
8
12
16
20
24
Time after PMA stimulation (hr)

# FIG. 8

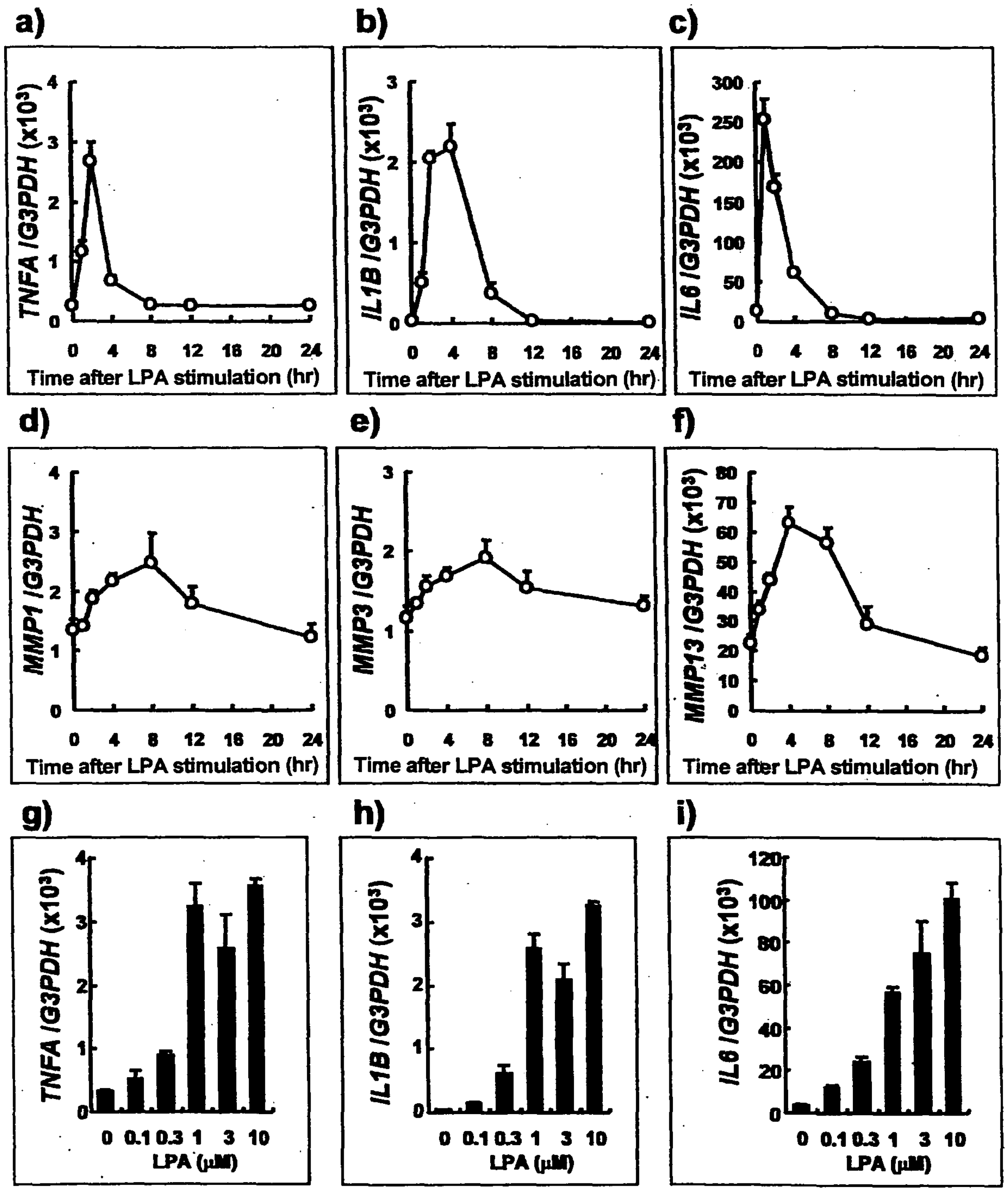
a)
TNFA /G3PDH (x10³)
0 4 8 12 16 20 24
Time after LPA stimulation (hr)
b)
IL1B /G3PDH (x10³)
0 4 8 12 16 20 24
Time after LPA stimulation (hr)
c)
IL6 /G3PDH (x10³)
0 4 8 12 16 20 24
Time after LPA stimulation (hr)
d)
MMP1 /G3PDH
0 4 8 12 16 20 24
Time after LPA stimulation (hr)
e)
MMP3 /G3PDH
0 4 8 12 16 20 24
Time after LPA stimulation (hr)
f)
MMP13 /G3PDH (x10³)
0 4 8 12 16 20 24
Time after LPA stimulation (hr)
g)
TNFA /G3PDH (x10³)
0 0.1 0.3 1 3 10
LPA (μM)
h)
IL1B /G3PDH (x10³)
0 0.1 0.3 1 3 10
LPA (μM)
i)
IL6 /G3PDH (x10³)
0 0.1 0.3 1 3 10
LPA (μM)

FIG. 9

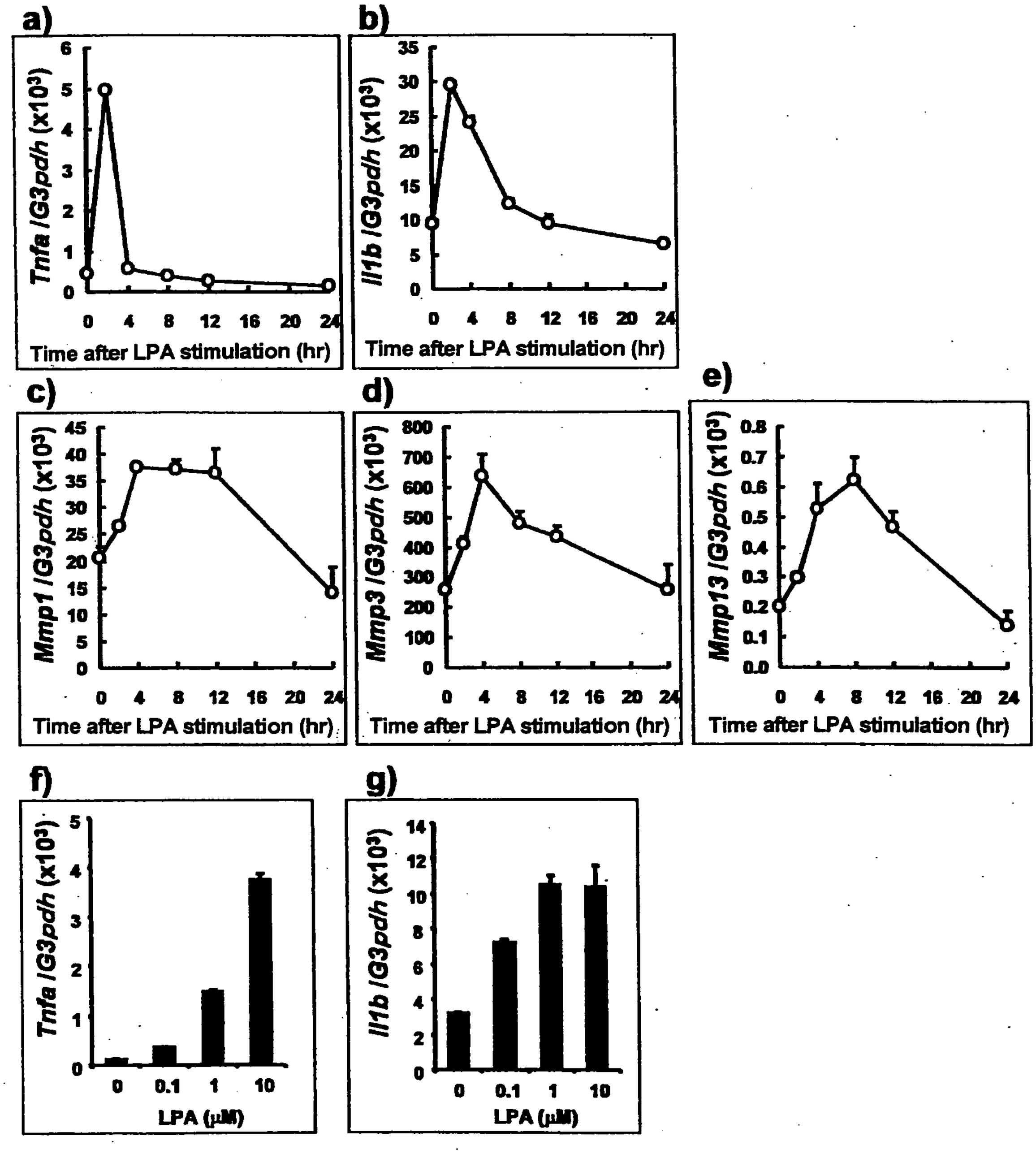

a)
Tnfa /G3pdh (x10³)
Time after LPA stimulation (hr)
b)
Il1b /G3pdh (x10³)
Time after LPA stimulation (hr)
c)
Mmp1 /G3pdh (x10³)
Time after LPA stimulation (hr)
d)
Mmp3 /G3pdh (x10³)
Time after LPA stimulation (hr)
e)
Mmp13 /G3pdh (x10³)
Time after LPA stimulation (hr)
f)
Tnfa /G3pdh (x10³)
LPA (μM)
g)
Il1b /G3pdh (x10³)
LPA (μM)

FIG. 10

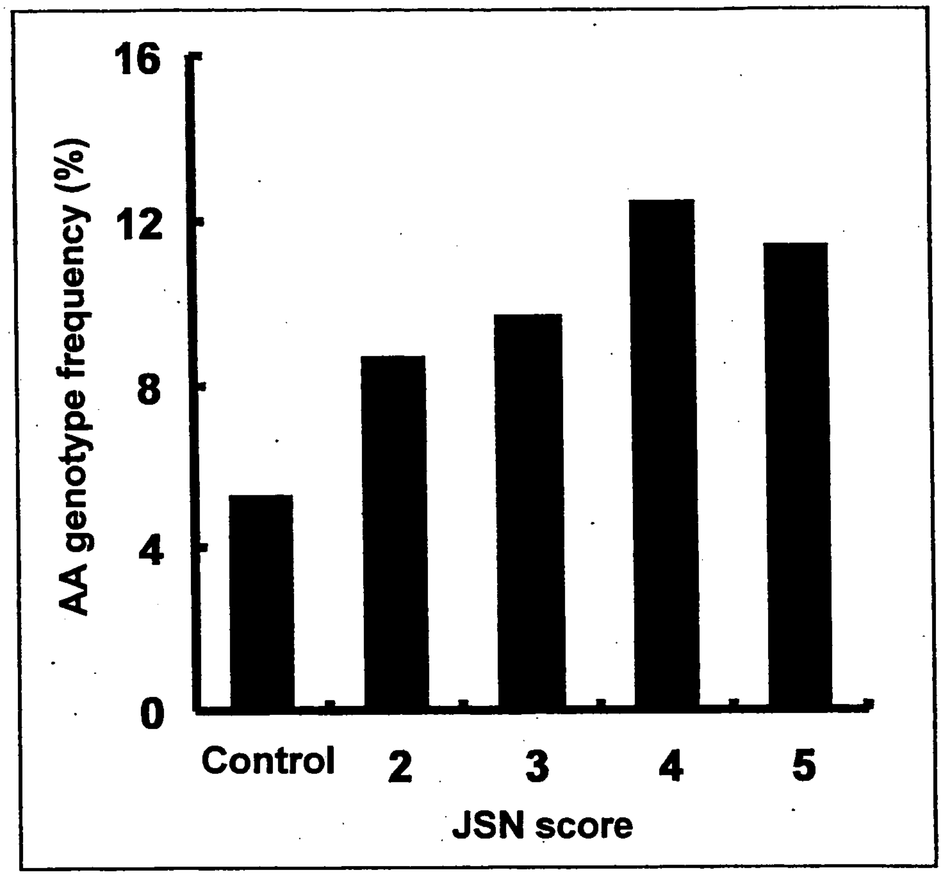

FIG. 11

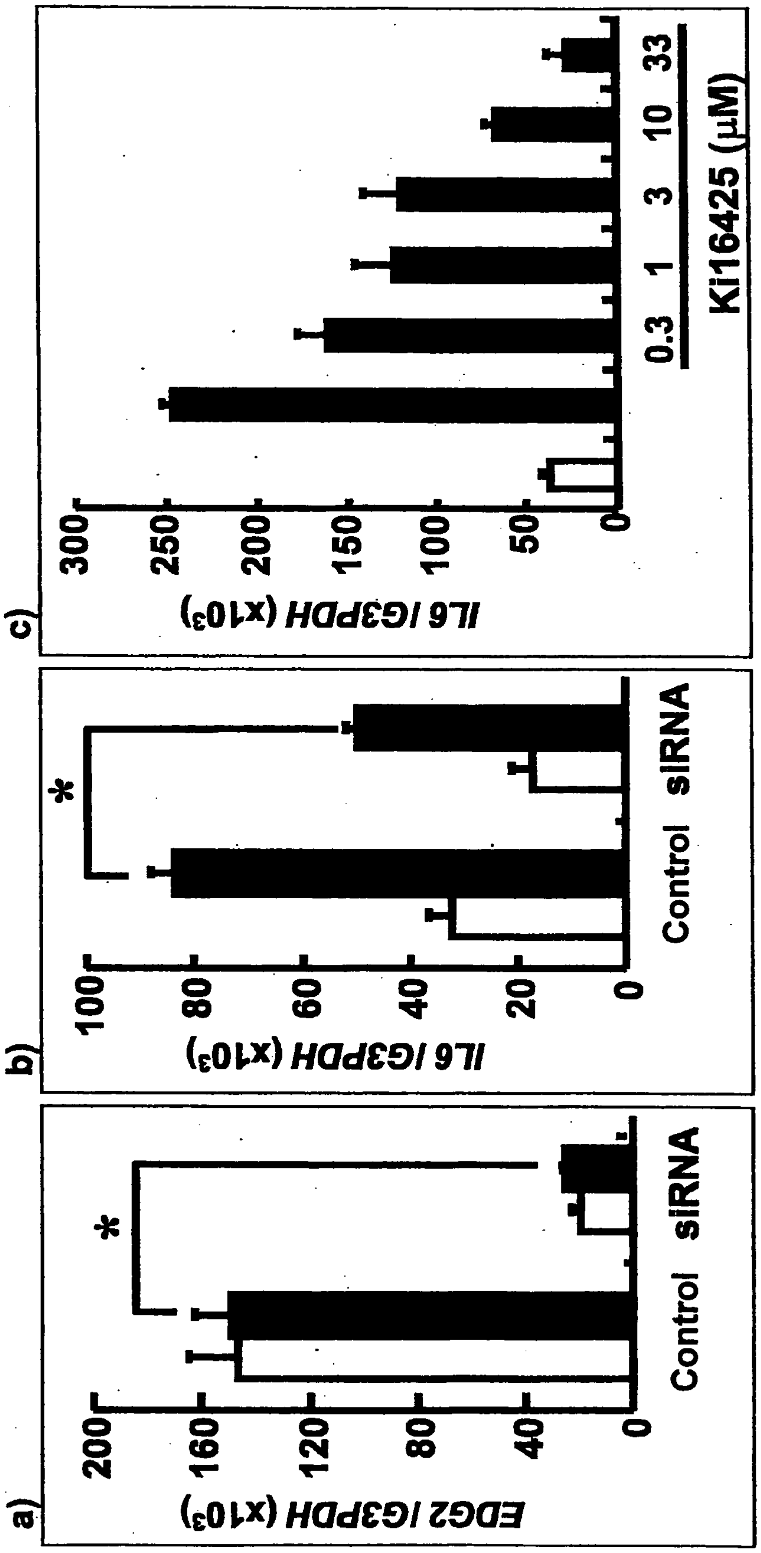


*; P<0.05, student's t-test

**INTERNATIONAL SEARCH REPORT**

International application No.
PCT/JP2008/060700

A. CLASSIFICATION OF SUBJECT MATTER
*A61K39/395*(2006.01)i, *A61K31/7088*(2006.01)i, *A61K38/00*(2006.01)i, *A61K48/00*(2006.01)i, *A61P19/00*(2006.01)i, *A61P19/02*(2006.01)i, *A61P19/10*(2006.01)i, *A61P25/02*(2006.01)i, *A61P29/00*(2006.01)i, *A61P35/00*(2006.01)i,

B. FIELDS SEARCHED

Minimum documentation searched (classification system followed by classification symbols)
A61K39/395, A61K31/7088, A61K38/00, A61K48/00, A61P19/00, A61P19/02, A61P19/10, A61P25/02, A61P29/00, A61P35/00, A61P43/00, C12Q1/68, G01N33/15, G01N33/50, C07K16/28, C12Q1/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2004-000209 A (Sumitomo Pharmaceuticals Co., Ltd.),<br>08 January, 2004 (08.01.04),<br>Full text; particularly, examples 6, 9<br>(Family: none) | 1-8<br>1-8,12-14 |
| X<br>Y | Zhao C. et al., Lysophosphatidic acid and its receptors in the pathogenesis of rheumatoid arthritis, FASEB Journal, 2006.03.06, Vol.20, No.4, Part1, page A113, ISSN: 0892-6638, [retrieved on 23 Jul. 2008] which was retrieved from the Internet:<http://www.fasebj.org/cgi/content/meeting_abstract/20/4/A113-a> | 1-8<br>1-8,12-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 July, 2008 (23.07.08) | 12 August, 2008 (12.08.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No. PCT/JP2008/060700

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Boucharaba A. et al., The type 1 lysophosphatidic acid receptor is a target for therapy in bone metastases, Proc. Natl. Acad. Sci. USA, 2006, Vol.103, No.25, pages 9643 to 9648, ISSN: 0027-8424 | 1-8<br>1-8, 12-14 |
| X<br>Y | JP 11-508135 A (Incyte Pharmacetricals, Inc.), 21 July, 1999 (21.07.99), Full text; particularly, page 10, lines 14 to 18; page 17, lines 16 to 23 & WO 1997/000952 A2 & EP 0840787 A2 | 1, 2<br>1-8, 12-14 |
| X<br>Y | Database DDBJ/EMBL/GenBank [online], Accession No.AC007157 (VERSION: AC007157.6, GI:4646258) [retrieved on 23 Jul. 2008] which was retrieved from the Internet: <http://www.ncbi.nlm.nih.gov/entrez/viewer.fcgi?db=nuccore&id=4646258> 27-APR-1999 uploaded, Birren,B. et al., Definition: Homo sapiens, clone hRPK.78_A_1, complete sequence | 9<br>12-14 |
| Y | Hiromi NOCHI et al., "Kansetsu Katsumaku Saibo no Lysophosphatidic Acid to Sphingosine 1-phosphate no COX-2 no Hatsugen Zokyo Sayo", Seikagaku, 25 August, 2002 (25.08.02), Vol.74, No.8, page 951, 3P-717, ISSN: 0037-1017 | 1-8 |
| Y | Hiromi NOCHI et al., "Lysophosphatidic Acid ya Sphingosine 1-phosphate wa Kansetsu Katsumaku Saibo no COX-2 Hatsugen o Zokyo suru", The Pharmaceutical Society of Japan Nenkai Yoshishu, 05 March, 2002 (05.03.02), Vol.122nd, No.4, page 30, 27[P]I-380, ISSN: 0918-9823 | 1-8 |
| Y | NOCHI H. et al., Effects of lysophosphatidic acid and proinflammatory cytokines on the expression of cyclooxygenase-2 in rheumatoid synovial cells, Seikagaku, 2003.08.25, Vol.75, No.8, page 870, 2P-570, ISSN: 0037-1017 | 1-8 |
| Y | Hiromi NOCHI et al., "Kansetsu Katsumaku Saibo no Lysophospholipid ni yoru Ensho Hanno ni Oyobosu Kobunshiryo Hyaluronic Acid no Eikyo", The Pharmaceutical Society of Japan Nenkai Yoshishu, 05 March, 2004 (05.03.04), Vol.124th, No.4, page 39, 30[P1]II-121, ISSN: 0918-9823 | 1-8 |
| Y | Hiromi NOCHI et al., "Kansetsu Rheumatism no Byotai Keisei ni Okeru Lysophospholipid no Yakuwari", Proceedings of the Japanese Society for Immunology, 05 November, 2004 (05.11.04), Vol.34, page 162, 2-C-W21-22-P, ISSN: 0919-1984 | 1-8 |

Form PCT/ISA/210 (continuation of second sheet) (April 2007)

**INTERNATIONAL SEARCH REPORT**

International application No.
PCT/JP2008/060700

C (Continuation). DOCUMENTS CONSIDERED TO BE RELEVANT

| Category* | Citation of document, with indication, where appropriate, of the relevant passages | Relevant to claim No. |
|---|---|---|
| Y | Duncliffe K.N. et al., A T cell-specific enhancer in the interleukin-3 locus is activated cooperatively by Oct and NFAT elements within a DNase I-hypersensitive site, Immunity, 1997, Vol.6, No.2, pages 175 to 185, ISSN: 1074-7613 | 12-14 |
| Y | Macian F. et al., Partners in transcription: NFAT and AP-1, Oncogene, 2001, Vol.20, No.19, pages 2476 to 2489, ISSN: 0950-9232 | 12-14 |
| Y | McCarthy G.M. et al., Molecular mechanism of basic calcium phosphate crystal-induced activation of human fibroblasts. Role of nuclear factor kappab, activator protein 1, and protein kinase c, Journal of Biological Chemistry, 1998, Vol.273, No.52, pages 35161 to 35169, ISSN: 0021-9258 | 12-14 |
| Y | Sun Y. et al., Basic calcium phosphate crystals induce matrix metalloproteinase-1 through the Ras/mitogen-activated protein kinase/c-Fos/ AP-1/metalloproteinase 1 pathway. Involvement of transcription factor binding sites AP-1 and PEA-3, Journal of Biological Chemistry, 2002, Vol.277, No.2, pages 1544 to 1552, ISSN: 0021-9258 | 12-14 |
| Y | Fahmi H. et al., Peroxisome proliferator-activated receptor gamma activators inhibit MMP-1 production in human synovial fibroblasts likely by reducing the binding of the activator protein 1, Osteoarthritis and Cartilage, 2002, Vol.10, No.2, pages 100 to 108, ISSN: 1063-4584 | 12-14 |
| P,X | Zhao C. et al., Regulation of lysophosphatidic acid receptor expression and function in human synoviocytes: implications for rheumatoid arthritis?, Molecular Pharmacology, 2008.02, Vol.73, No.2, pages 587 to 600, ISSN: 0026-895X | 1-8 |
| P,X | Mototani H. et al., A functional SNP in EDG2 increases susceptibility to knee osteoarthritis in Japanese, Human Molecular Genetics, 2008, Vol.17, No.12, pages 1790 to 1797, ISSN: 0964-6906 | 1-9,12-14 |

Form PCT/ISA/210 (continuation of second sheet) (April 2007)

**INTERNATIONAL SEARCH REPORT**

International application No.
PCT/JP2008/060700

Continuation of A. CLASSIFICATION OF SUBJECT MATTER
(International Patent Classification (IPC))

*A61P43/00*(2006.01)i, *C12Q1/68*(2006.01)i, *G01N33/15*(2006.01)i, *G01N33/50*(2006.01)i, *C07K16/28*(2006.01)n, *C12Q1/02*(2006.01)n

(According to International Patent Classification (IPC) or to both national classification and IPC)

Form PCT/ISA/210 (extra sheet) (April 2007)

**INTERNATIONAL SEARCH REPORT**

International application No.
PCT/JP2008/060700

**Box No. II Observations where certain claims were found unsearchable (Continuation of item 2 of first sheet)**

This international search report has not been established in respect of certain claims under Article 17(2)(a) for the following reasons:

1. [X] Claims Nos.: 10, 11, 15
because they relate to subject matter not required to be searched by this Authority, namely:
Claims 10, 11 and 15 involve a method for treatment of a human body by therapy or a diagnostic method, thus relate to a subject matter which this International Searching Authority is not required, under the provisions of PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
Claim 9 relates to a nucleic acid comprising a nucleotide sequence which is a partial sequence of the nucleotide sequence depicted in SEQ ID NO:3 contains the nucleotide sequence TTA and which is composed of about 15 or more continuous nucleotides. However, the nucleotide sequence is already known, as disclosed in Document 5. Therefore, the nucleotide sequence cannot be regarded as a special technical feature which makes any contribution over the prior art.
Thus, the invention of claim 9 does not share no common special technical feature with the inventions of claims 1-8 and 12-14 which relate to a diagnostic
(continued to extra sheet)

1. [ ] As all required additional search fees were timely paid by the applicant, this international search report covers all searchable claims.
2. [X] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.

Form PCT/ISA/210 (continuation of first sheet (2)) (April 2007)

INTERNATIONAL SEARCH REPORT

International application No.
PCT/JP2008/060700

Continuation of Box No.III of continuation of first sheet(2)

agent for a bone/joint disease or a method for the screening of a substance capable of controlling the expression of an inflammatory cytokine in a joint and/or the destruction of a joint (e.g., the destruction of an articular cartilage) and/or a pain in a bone/joint disease. Consequently, it cannot be considered that these inventions are so linked as to form a single general inventive concept.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006014013 A
- JP 2000333673 A
- US 6331415 B
- US 5225539 A
- US 5585089 A
- US 5693761 A
- US 5693762 A
- JP 5227970 A
- JP HEI5227970 A
- US 5939598 A
- US 5545806 A
- EP 1258484 A1
- WO 03055507 A
- WO 03023063 A
- JP 2007155792 A

### Non-patent literature cited in the description


- **Mototani et al.** *Human Molecular Genetics,* 2005, vol. 14, 1009-1017
- **Kizawa et al.** *Nature Genetics,* 2005, vol. 37 (2), 138-44
- **Hecht, J.H. et al.** *The Journal of Cell Biology,* 1996, vol. 135 (4), 1071-1083
- **An, S. et al.** *Biochemical and Biophysical Research Communications,* 1997, vol. 231, 619-622
- **Renback, K. et al.** *Molecular Brain Research,* 2000, vol. 75, 350-354
- **Inoue, M. et al.** *Nature Medicine,* 2004, vol. 10 (7), 712-718
- **Renback, K. et al.** *Neuroscience Letters,* 1999, vol. 270, 59-61
- **Gardell, S.E. et al.** *Trends in Molecular Medicine,* 2006, vol. 12 (2), 65-75
- **Bowie et al.** *Science,* 1990, vol. 247, 1306-1310
- **Karlin et al.** *Proc. Natl. Acad. Sci. USA,* 1993, vol. 90, 5873-5877
- **Altschul et al.** *Nucleic Acids Res.,* 1997, vol. 25, 3389-3402
- **Needleman et al.** *J. Mol. Biol.,* 1970, vol. 48, 444-453
- **Myers ; Miller.** *CABIOS,* 1988, vol. 4, 11-17
- **Pearson et al.** *Proc. Natl. Acad. Sci. USA,* 1988, vol. 85, 2444-2448
- **M. Bodanszky ; M.A. Ondetti.** Peptide Synthesis. Interscience Publishers, 1966
- **Schroeder ; Luebke.** The Peptide. Academic Press, 1965
- **J. Sambrook et al.** Molecular Cloning. Cold Spring Harbor Lab. Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **Vaughn, J. L. et al.** *In Vivo,* 1977, vol. 13, 213-217
- **Maeda et al.** *Nature,* 1985, vol. 315, 592
- *Proc. Natl. Acad. Sci. U.S.A.,* 1972, vol. 69, 2110
- *Gene,* 1982, vol. 17, 107
- *Molecular and General Genetics,* 1979, vol. 168, 111
- *Methods in Enzymology,* 1991, vol. 194, 182-187
- *Proc. Natl. Acad. Sci. USA,* 1978, vol. 75, 1929
- *Bio/Technology,* 1988, vol. 6, 47-55
- **Saibo Kogaku.** *Cell Engineering*
- New Cell Engineering Experimental Protocol. Shujunsha, 1995, 263-267
- *Virology,* 1973, vol. 52, 456
- **Miller.** Journal of Experiments in Molecular Genetics. Cold Spring Harbor Laboratory, 1972, 431-433
- **Bostian, K.L. et al.** *Proc. Natl. Acad. Sci. USA,* 1980, vol. 77, 4505
- **Bitter, G.A. et al.** *Proc. Natl. Acad. Sci. USA,* 1984, vol. 81, 5330
- **Grace, T.C.C.** *Nature,* 1962, vol. 195, 788
- *Science,* 1952, vol. 122, 501
- *Virology,* 1959, vol. 8, 396
- *The Journal of the American Medical Association,* 1967, vol. 199, 519

- *Proceeding of the Society for the Biological Medicine,* 1950, vol. 73, 1
- **Pratt J.M. et al.** Transcription and Translation. IRL Press, 1984, 179-209
- **Johnston et al.** *Nature,* 1957, vol. 179, 160-161
- **Erickson A.H. et al.** *Meth. Enzymol.,* 1996, vol. 96, 38-50
- **Spirin A.S. et al.** *Science,* 1988, vol. 242, 1162-1164
- **J. Kawakami et al.** *Pharm. Tech. Japan,* 1992, vol. 8, 247
- *PHARM. TECH. JAPAN,* 1992, vol. 8, 395
- Antisense Research and Applications. CRC Press, 1993
- *Proc. Natl. Acad. Sci. USA,* 2001, vol. 98 (10), 5572-5577
- *Nucleic Acids Res.,* 2001, vol. 29 (13), 2780-2788
- *Nature,* 2001, vol. 411 (6836), 494-498
- **Koehler ; Milstein.** *Nature,* 1995, vol. 256, 495
- *Bio/Technology,* 1991, vol. 9, 88-89
- **Kabat et al.** Sequences of Proteins of Immunological Interest. Public Health Service, NIH, 1991
- *Immunol. Today,* 1996, vol. 17, 391-397
- *Nat. Genet.,* 1997, vol. 15, 146-156
- *Nat. Biotechnol.,* 1996, vol. 14, 845-851
- **Tomizuka et al.** *Nat. Genet.,* 1997, vol. 16, 133-143
- *Nat. Biotechnol.,* 2000, vol. 18, 1086-1090
- *Nat. Biotechnol.,* 2002, vol. 20, 889-894
- **Holt et al.** *Curr. Opin. Biotechnol.,* 2000, vol. 11, 445-449
- *J. Biol. Chem.,* 1999, vol. 274, 18218-18230
- *J. Mol. Biol.,* 1991, vol. 222, 581-597
- *Nat. Biotechnol.,* 1996, vol. 14, 309-314
- *Annu. Rev. Immunol.,* 1994, vol. 12, 433-455
- *Proc. Natl. Acad. Sci. USA,* 2000, vol. 14, 7969-7974
- *J.Mol. Biol.,* 2000, vol. 296, 57-86
- *Nat. Biotechnol.,* 2000, vol. 18, 852
- *Proc. Natl. Acad. Sci. USA,* 1995, vol. 92, 3938
- *J. Immunol. Methods,* 2003, vol. 272, 219-233
- *Mol. Pharmacol.,* 2003, vol. 64 (4), 994-1005
- *Mol. Pharmacol.,* 2001, vol. 60 (4), 776-784
- *Yeast,* 2000, vol. 16, 11-22
- Immunochemical Techniques (Part A). Meth. Enzymol. vol. 70
- Immunochemical Techniques (Part B). METH. ENZYMOL. vol. 73
- Immunochemical Techniques (Part C). METH. ENZYMOL. vol. 74
- Immunochemical Techniques (Part D: Selected Immunoassays). METH. ENZYMOL. vol. 84
- Immunochemical Techniques (Part E: Monoclonal Antibodies and General Immunoassay Methods). METH. ENZYMOL. vol. 92
- Immunochemical Techniques (Part I: Hybridoma Technology and Monoclonal Antibodies). METH. ENZYMOL. Academic Press, vol. 121
- **Wallace et al.** *Proc. Natl. Acad. Sci. USA,* 1983, vol. 80, 278-282
- *Biochem. Biophys. Res. Commun.,* 2000, vol. 278, 659-664
- **Ohnishi et al.** *J Hum Genet.,* 2001, vol. 46 (8), 471-7
- **Iida et al.** *J Hum Genet.,* 2005, vol. 50 (1), 42-5
- **Abe et al.** *J Rheumatol.,* March 1997, vol. 24 (3), 420-9
- **Duncliffe et al.** *Immunity,* February 1997, vol. 6 (2), 175-85
- **Guo et al.** *J Biol Chem.,* 28 December 2001, vol. 276 (52), 48871-8
- **Macian F ; Lopez-Rodriguez C ; Rao A.** *Oncogene,* 30 April 2001, vol. 20 (19), 2476-89
- **Andrews et al.** *Nucleic Acids Res.,* 25 November 1991, vol. 19 (22), 6263-8